# EUROPÄISCHE PATENTSCHRIFT

(11) **EP 1 689 446 B1**
(45) Veröffentlichungstag und Bekanntmachung des Hinweises auf die Patenterteilung: **07.01.2015**
(21) Anmeldenummer: 04803146.2
(22) Anmeldetag: 15.11.2004
(51) Int. Cl.: A61K 48/00, C12N 15/861

(54) **NEUE ADENOVIREN, DAFÜR CODIERENDE NUKLEINSÄUREN UND DEREN VERWENDUNG**
NOVEL ADENOVIRUSES, NUCLEIC ACIDS THAT CODE FOR THE SAME AND THE USE OF SAID VIRUSES
ADENOVIRUS, ACIDES NUCLEIQUES LES CODANT ET UTILISATIONS ASSOCIEES

(30) Priorität: 14.11.2003 DE 10353152; 14.04.2004 DE 102004018117
(43) Veröffentlichungstag der Anmeldung: 16.08.2006
(73) Patentinhaber: Holm, Per Sonne, 82256 Fürstenfeldbruck (DE)
(72) Erfinder: Holm, Per Sonne, 82256 Fürstenfeldbruck (DE)
(74) Vertreter: Bohmann, Armin K.
(86) Internationale Anmeldenummer: PCT/EP2004/012931
(87) Internationale Veröffentlichungsnummer: WO 2005/052143

(56) Entgegenhaltungen:
- DE-A1- 10 150 945
- US-A1- 2002 086 411
- HOLM P S ET AL: "YB-1 relocates to the nucleus in adenovirus infected cells and facilitates viral replication E2 gene expression through the E2 late promoter" JOURNAL OF BIOLOGICAL CHEMISTRY, AMERICAN SOCIETY OF BIOLOGICAL CHEMISTS, BALTIMORE, MD, US, Bd. 277, Nr. 12, 22. März 2002 (2002-03-22), Seiten 10427-10434, XP002237181 ISSN: 0021-9258
- FUEYO JUAN ET AL: "A mutant oncolytic adenovirus targeting the Rb pathway produces anti-glioma effect in vivo" ONCOGENE, Bd. 19, Nr. 1, 6. Januar 2000 (2000-01-06), Seiten 2-12, XP002336078 ISSN: 0950-9232
- HOLM PER S ET AL: "Multidrug-resistant cancer cells facilitate E1-independent adenoviral replication: Impact for cancer gene therapy." CANCER RESEARCH, Bd. 64, Nr. 1, 1. Januar 2004 (2004-01-01), Seiten 322-328, XP002336079 ISSN: 0008-5472
- BALAGUÉ C. ET AL: "Human papillomavirus E6E7-mediated adenovirus cell killing: selectivity of mutant adenovirus replication in organotypic cultures of huma keratinocytes.", J. VIROL., vol. 75, no. 16, August 2001 (2001-08), pages 7602-7611, XP002215977,
- SHENK T. ET AL: "Functional analysis of Adenovirus-5 host-range deletion mutants defective for transformation of rat embryo cells.", SYMP. QUANT. BIOL., vol. V, 1980, pages 367-375, XP008103427,
- MYMRUYK J.S.; BAYLEY S.T.: "Multiple pathways for activation of E2A expression in human KB cells by the 243R E1A protein of adenovirus type 5.", VIRUS RESEARCH, vol. 33 , pages 89-97, XP023893185,
- BRYDAK L B ET AL: "Immunogenicity of influenza vaccine in patients with hemato-oncological disorders.", LEUKEMIA & LYMPHOMA JAN 1999, vol. 32, no. 3-4, January 1999 (1999-01), pages 369-374, XP008165890, ISSN: 1042-8194
- KHURI F R ET AL: "a controlled trial of intratumoral ONYX-015, a selectively-replicating adenovirus, in combination with cisplatin and 5-fluorouracil in patients with recurrent head and neck cancer", NATURE MEDICINE, NATURE PUBLISHING GROUP, NEW YORK, NY, US, vol. 6, no. 8, 1 August 2000 (2000-08-01), pages 879-885, XP008126723, ISSN: 1078-8956, DOI: 10.1038/78638
- WILDNER O ET AL: "THE ROLE OF THE E1B 55 KDA GENE PRODUCT IN ONCOLYTIC ADENOVIRAL VECTORS EXPRESSING HERPES SIMPLEX VIRUS-TK: ASSESSMENT OF ANTITUMOR EFFICACY AND TOXICITY", CANCER RESEARCH, AMERICAN ASSOCIATION FOR CANCER RESEARCH, US, vol. 60, no. 15, 1 August 2000 (2000-08-01), pages 4167-4174, XP009061080, ISSN: 0008-5472

## Beschreibung

Die vorliegende Erfindung betrifft einen Adenovirus gemäß den Ansprüchen sowie, eine Nukleinsäure codierend für den Adenovirus, ein Replikationssystem umfassend die Nukleinsäure, einen Vektor umfassend die Nukleinsäure und/oder das Replikationssystem, eine Zelle umfassend den Adenovirus und/oder die Nukleinsäure und/oder das Replikationssystem und/oder den Vektor, einen nicht-menschlichen Organismus umfassend den Adenovirus, die Nukleinsäure, das Replikationssystem, den Vektor oder die Zelle, die Verwendung des Adenovirus, der Nukleinsäure, des Replikationssystems, des Vektors oder der Zelle, eine pharmazeutische Zusammensetzung umfassend den Adenovirus, die Nukleinsäure, das Replikationssystem, den Vektor oder die Zelle, und einen Kit umfassend den Adenovirus.

Bei der Behandlung von Tumoren werden derzeit eine Vielzahl von Therapiekonzepten verfolgt. Neben der Verwendung chirurgischer Techniken stehen dabei die Chemotherapie und die Strahlentherapie im Vordergrund. All diese Techniken sind jedoch für den Patienten mit nicht unerheblichen Nebenwirkungen verbunden. Mit der Verwendung von replikationsselektiven onkolytischen Viren wurde eine neue Plattform für die Behandlung von Tumoren geschaffen. Dabei wird eine selektive intratumorale Replikation eines viralen Agens herbeigeführt, die in der Folge zur Virusreplikation, Lyse der infizierten Tumorzelle und Verbreitung des Virus auf benachbarte Tumorzellen führt. Infolge der Beschränkung der Replikationsfähigkeit des Virus auf Tumorzellen bleibt normales Gewebe von der Replikation und damit der Lyse durch das Virus verschont.

Derzeit finden verschiedene virale Systeme in klinischen Studien mit dem Ziel der Tumorlyse Anwendung. Ein Beispiel für einen derartigen Adenovirus ist dl1520 (Onyx-015), der bereits erfolgreich in den klinischen Phasen I und II eingesetzt wurde (Khuri, F. et al. Nature Medicine 6, 879-885, 2000). Onyx-015 ist ein Adenovirus, bei dem das E1B-55kDA-Gen vollständig deletiert ist. Die vollständige Deletion des E1B55kDa-Proteins des Adenovirus beruht dabei auf der Beobachtung, dass im adenoviralen Vektor die Replikation und somit die Lyse von Zellen mit defektem p53 möglich ist (Kirn, D. et al., Proc. Am. Soc. Clin. Oncol. 17, 391a, 1998), wobei normale Zellen nicht geschädigt werden. Genauer ist das E1B-55kDa-Genprodukt beteiligt an der Inhibierung von p53, dem Transport viraler mRNA und dem Abschalten der Proteinsynthese der Wirtszelle. Die Inhibierung von p53 erfolgt dabei durch Ausbilden eines Komplexes aus p53 und dem adenoviral codierten B1B-55kDa Protein und/oder Komplex bestehend aus E1B-55kDA und E4orf6. Von p53, codiert von TP53, geht ein komplexer regulatorischer Mechanismus aus (Zambetti, G.P. et al., FASEB J. 7, 855-865, 1993), der u.a. auch dazu führt, dass eine effiziente Replikation von Viren wie Adenoviren in der Zelle unterdrückt wird. Das Gen TP 53 ist in etwa 50 % aller menschlichen Tumoren deletiert oder mutiert mit der Folge, dass es zu keiner - erwünschten - Apoptose infolge einer Chemotherapie oder einer Bestrahlungstherapie kommt und damit der Erfolg dieser Tumorbehandlungen im Normalfall unterbleibt.

Ein weiteres Konzept tumorlytischer Viren beruht auf der Beobachtung, dass wenn das E1A-Protein in einer bestimmten Weise deletiert vorliegt bzw. eine oder mehrere Mutationen aufweist, welche die Bindung von Rb/E2F und/oder p107/E2F und/oder p130/E2F nicht beeinflussen, solche Adenoviren den Eintritt der infizierten Zellen in die S-Phase nicht induzieren und zur Replikation in Tumorzellen befähigt sind, die kein funktionelles Rb-Protein aufweisen. Zudem kann das E1A-Protein am N-Terminus deletiert vorliegen bzw. eine oder mehrere Mutationen im Bereich der Aminosäurepositionen 1 bis 76 des E1A-Proteins umfassen, um die Bindung von E1A an p300 zu unterbinden, um somit eine selektivere Replikation in Tumorzellen zu bewerkstelligen. Diese Vorgehensweisen sind beispielhaft beschrieben in dem europäischen Patent EP 0 931 830. Beispiele derartiger Adenoviren sind AdΔ24, d1922 - 947, E1Ad/01/07 und CB016 (Howe, J. A. et al., Molecular Therapy 2, 485-495, 2000; Fueyo, J. et al., Oncogene 19, 2-12, 2000; Heise, C. et al., Nature Medicine 6, 11341139,2001; Balague, C. et al., J. Virol. 75, 7602-7611, 2001). Diese im Stand der Technik bekannten adenoviralen Systeme zur Onkolyse weisen somit bestimmte Deletionen im E1A Protein auf, wobei diese Deletion vorgenommen worden war unter der Annahme, dass intakte Rb-Proteine bzw. Komplex bestehend aus intaktem Rb-Protein und E2F einer effiziente Replikation in vivo entgegenwirken würden und um eine adenovirale Replikation in vivo nur in Rb-negativen/mutierten Zellen sicher zu stellen. Diese adenoviralen Systeme nach dem Stand der Technik gehen auf E1A zurück, um mittels des frühen E2-Promotors (engl. E2 early Promotor) und freiem E2F die in vivo Replikation zu steuern (Dyson, N. Genes & Development, 12, 2245-2262, 1998).

Eine weitere Form von tumorlytischen adenoviralen Systemen beruht auf dem Einsatz selektiver Promotoren, um das virale Onkogen E1A spezifisch zu exprimieren, was eine selektive Replikation in Tumorzellen erlaubt (Rodriguez, R. et al., Cancer Res. 57, 2559-2563, 1997).

Wie vorstehend beschrieben kommt es bei den verschiedenen Konzepten adenoviraler tumorlytischer Viren auf die Auswahl eines für das dem jeweiligen Konzept zugrundeliegenden Wirkmechanismus geeigneten zellulären Hintergrundes an. Mit anderen Worten, die verschiedenen derzeit bekannten adenoviralen Systeme zur Tumorlyse können nur bei Vorliegen bestimmter molekularbiologischer Voraussetzungen angewandt werden. Dies beschränkt die Anwendung derartiger Systeme auf bestimmte Patientenkollektive.

Ein besonderes Problem bei der Behandlung von Tumorerkrankungen stellt sich dann ein, wenn die Patienten eine sogenannte klassische Vielfachresistenz (engl. multidrug resistence (MDR)) entwickeln, die eine besonders gut untersuchte Resistenzform von Tumoren gegenüber Zytostatika darstellt (Gottesman und Pastan, Annu. Rev. Biochem. 62, 385-427, 1993). Sie beruht auf der Überexpression des membranständigen Transportproteins P-Glykoprotein, das zu den sogenannten ABC-Transportern gehört (Stein, U. et al., JBC 276, 28562-69, 2001, J. Wijnholds, Novartis Found Symp., 243, 69-79, 2002). Bargou, R. C. et al. und Oda, Y. et al (Bargou, R. C. et al., Nature Medicine 3, 447-450, 1997; Clin. Cancer Res. 4, 2273-2277, 1998) konnten zeigen, dass die Kernlokalisation des humanen Transkriptionsfaktors YB-1 unmittelbar an der Aktivierung der Expression des P-Glykoproteins beteiligt ist Weitere Untersuchungen belegen, dass YB-1 durch verschiedene Streßbedingungen in den Zellkern gelangt, wie z.B. UV-Bestrahlung, Zytostatika-Applikation (Koike, K. et al., FEBS Lett 17, 390-394, 1997) und Hyperthermie (Stein, U. et al., JBC 276, 28562-69, 2001). Weitere Untersuchungen belegen, dass die nukläre Lokalisation von YB-1 einen Einfluss auf einen weiteren ABC-Transporter ausübt. Dieser ABC-Transporter wird als MRP (engl. multidrug resistance-related protein) bezeichnet und ist mit der Ausbildung des sogenannten atypischen, nicht P-Glykoprotein-abhängigen Vielfachresistenz beteiligt (Stein, U. et al., JBC 276,28562-69,2001).

US-Patentanmeldung 2002/0086411 A1 offenbart Mittel zur Behandlung von malignen Erkrankungen unter Verwendung des Proteins YB-1.

Der vorliegenden Erfindung liegt die Aufgabe zugrunde, eine technische Lehre und insbesondere ein Mittel bereitzustellen, welches erlaubt, einen Organismus, speziell einen menschlichen Organismus bzw. ein Patientenkollektiv spezifisch mit tumorlytisch wirkenden Agenzien zu behandeln. Weiterhin ist es eine der vorliegenden Erfindung zugrundeliegenden Aufgabe, ein Mittel bereitzustellen, welches geeignet ist, bei Patienten mit Tumorerkrankungen eine Tumorlyse herbeizuführen, die gegenüber Zytostatika resistent sind, insbesondere solche, die eine Vielfachresistenz zeigen. Schließlich ist es eine der vorliegenden Erfindung zugrundeliegende Aufgabe, einen Adenovirus bereitzustellen, der für die Zelllyse geeignet ist

Die der vorliegenden Erfindung zu Grunde liegende Aufgabe wird erfindungsgemäßen gelöst durch den Gegenstand der beigefügten unabhängigen Ansprüche. Bevorzugte Ausführungsformen ergeben sich aus den beigefügten abhängigen Ansprüchen.

Die der vorliegenden Erfindung zu Grunde liegende Aufgabe wird erfindungsgemäßen gelöst in einem ersten Aspekt durch eine Adenovirus, der ein E1B55kD-Protein und/oder ein E4orf6-Protein zeitlich vor einem E1A-Protein exprimiert, wobei der Adenovirus in Zellen repliziert, die YB-1 im Kern aufweisen, aber nicht in Zellen, die YB-1 nicht im Kern aufweisen.

In einer Ausführungsform des ersten Aspekts ist vorgesehen, dass der Adenovirus eine Kombination aus E1B55kD-Protein und E4orf6-Protein zeitlich vor einem E1A-Protein exprimiert.

In einer Ausführungsform des ersten Aspekts ist vorgesehen, dass das E1A-Protein ein E1A12S-Protein ist.

In einer Ausführungsform des ersten Aspekts ist vorgesehen, dass der Adenovirus mindestens eine Nukleinsäure umfasst, die für ein Protein codiert, das aus der Gruppe ausgewählt ist, die aus E1B55kD-Protein, E4orf6-Protein und E1A-Proteinen besteht, wobei das mindestens eine Protein unter der Kontrolle eines Promotors steht, der verschieden ist von dem Promotor, der die Expression des Proteins in einem Wildtyp-Adenovirus steuert.

In einer Ausführungsform des ersten Aspekts ist vorgesehen, dass das mindestens eine Protein ein E1B55kD-Protein ist.

In einer Ausführungsform des ersten Aspekts ist vorgesehen, dass das mindestens eine Protein ein E1A-Protein, bevorzugterweise ein E1A12S-Protein ist.

In einer Ausführungsform des ersten Aspekts ist vorgesehen, dass die Expression des E1B55kD-Proteins von einem Promotor gesteuert wird, wobei der Promotor ausgewählt ist aus der Gruppe, die Tumor-spezifische Promotoren, Organ-spezifische Promotoren, Gewebespezifische Promotoren, heterologe Promotoren und adenovirale Promotoren umfasst, wobei der adenovirale Promotor verschieden ist von dem E1B-Promotor.

In einer Ausführungsform des ersten Aspekts ist vorgesehen, dass die Expression des E4orf6-Proteins von einem Promotor gesteuert wird, wobei der Promotor ausgewählt ist aus der Gruppe, die Tumor-spezifische Promotoren, Organ-spezifische Promotoren, Gewebespezifische Promotoren, heterologe Promotoren und adenovirale Promotoren umfasst, wobei der adenovirale Promotor verschieden ist von dem E4-Promotor.

In einer Ausführungsform des ersten Aspekts ist vorgesehen, dass der adenovirale Promotor der E1A-Promotor ist.

In einer Ausführungsform des ersten Aspekts ist vorgesehen, dass die Expression des E1A-Proteins von einem Promotor gesteuert wird, wobei der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei der adenovirale Promotor verschieden ist von dem E1A-Promotor.

In einer Ausführungsform des ersten Aspekts ist vorgesehen, dass der die Expression des E1A-Proteins steuernde Promotor YB-1-gesteuert oder YB-1-regulierbar ist.

In einer Ausführungsform des ersten Aspekts ist vorgesehen, dass der die Expression des E1A-Proteins steuernde Promotor der adenovirale E2-late-Promotor ist.

In einer Ausführungsform des ersten Aspekts ist vorgesehen, dass das E4orf6-Protein, und das E1B55kD-Protein unter der Kontrolle eines gleichen oder eines gemeinsamen Promotors stehen.

In einer Ausführungsform des ersten Aspekts ist vorgesehen, dass der Adenovirus das Protein YB-1 durch mindestens ein adenovirales Protein im Zellkern bereitstellt oder die Bereistellung von YB-1 im Zellkern durch mindestens ein adenovirales Protein vermittelt,
wobei bevorzugterweise das adenovirale Protein verschieden ist von E1A, wobei das adenovirale Protein ein Komplex aus E4orf6 und E1B55kD ist.

In einer Ausführungsform des ersten Aspekts ist vorgesehen, dass der Adenovirus das Protein YB-1 durch mindestens ein adenovirales Protein für die adenovirale Replikation bereitstellt oder die Bereitstellung von YB-1 für die adenovirale Replikation durch mindestens ein adenovirales Protein vermittelt, wobei das adenovirale Protein ein Komplex aus E4orf6 und E1B55kD ist.

In einer Ausführungsform des ersten Aspekts ist vorgesehen, dass die Nukleinsäure des Adenovirus zumindest eine funktional inaktive adenovirale Region aufweist, wobei die Region ausgewählt ist aus der Gruppe, die aus der E1-Region, der E3-Region, der E4-Region und Kombinationen davon besteht.

In einer Ausführungsform des ersten Aspekts ist vorgesehen, dass die Region die E1-Region ist.

In einer Ausführungsform des ersten Aspekts ist vorgesehen, dass die Region die E3-Region ist.

In einer Ausführungsform des ersten Aspekts ist vorgesehen, dass die Region die E4-Region ist.

In einer Ausführungsform des ersten Aspekts ist vorgesehen, dass die Region aus der E1-Region, der E3-Region und der E4-Region besteht.

In einer Ausführungsform des ersten Aspekts ist vorgesehen, dass der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E1B55kD-Protein ist.

In einer Ausführungsform des ersten Aspekts ist vorgesehen, dass der Promotor verschieden ist von dem E1B-Promotor.

In einer Ausführungsform des ersten Aspekts ist vorgesehen, dass der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei der Promotor verschieden ist vom E1B-Promotor.

In einer Ausführungsform des ersten Aspekts ist vorgesehen, dass der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E4orf6-Protein ist.

In einer Ausführungsform des ersten Aspekts ist vorgesehen, dass der Promotor verschieden ist von dem E4-Promotor.

In einer Ausführungsform des ersten Aspekts ist vorgesehen, dass der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei die adenoviralen Promotoren verschieden sind von dem E4-Promotor.

In einer Ausführungsform des ersten Aspekts ist vorgesehen, dass der Promotor der E1A-Promotor ist.

In einer Ausführungsform des ersten Aspekts ist vorgesehen, dass der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E1A-Protein, bevorzugterweise ein E1A12S-Protein ist.

In einer Ausführungsform des ersten Aspekts ist vorgesehen, dass der Promotor verschieden ist von dem E1A-Promotor.

In einer Ausführungsform des ersten Aspekts ist vorgesehen, dass der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht.

In einer Ausführungsform des ersten Aspekts ist vorgesehen, dass der Adenovirus eine Nukleinsäure umfasst, wobei die Nukleinsäure für YB-1 codiert.

In einer Ausführungsform des ersten Aspekts ist vorgesehen, dass die für YB-1 codierende Nukleinsäure unter der Kontrolle eines Promotors steht, wobei der Promotor bevorzugterweise der E2-late-Promotor ist.

In einer Ausführungsform des ersten Aspekts ist vorgesehen, dass die für YB-1 codierende Nukleinsäure unter der Kontrolle eines Promotors steht, wobei der Promotor YB-1 abhängig ist bzw. YB-1- reguliert ist.

In einer Ausführungsform des ersten Aspekts ist vorgesehen, dass die für YB-1 codierende Nukleinsäure ein Teil der Expressionskassette umfassend eine für ein E1A-Protein codierende Nukleinsäure ist, bevorzugterweise eine für ein E1A 12S-Protein codierende Nukleinsäure ist.

In einer Ausführungsform des ersten Aspekts ist vorgesehen, dass die für das E1A-Protein codierende Nukleinsäure von der für das YB-1 codierende Nukleinsäure durch eine IRES-Sequenz getrennt ist.

In einer Ausführungsform des ersten Aspekts ist vorgesehen, dass die für das E4orf6-Protein codierende Nukleinsäure und die für das das E1B55kD-Protein codierende Nukleinsäure in einer Expressionskassette enthalten sind, und die beiden codierenden Sequenzen durch eine IRES-Sequenz voneinander getrennt sind.

In einer Ausführungsform des ersten Aspekts ist vorgesehen, dass der Promotor der Expressionskassette ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei die adenoviralen Promotoren verschieden sind von dem E4-Promotor und verschieden sind von dem E1B-Promotor, bevorzugterweise verschieden sind von dem Wildtyp-E4-Promotor und verschieden sind von dem Wildtyp-E1B-Promotor.

In einer Ausführungsform des ersten Aspekts ist vorgesehen, dass der Adenovirus eine Expressionskassette umfassend einen Promotor und eine Nukleinsäuresequenz umfasst, wobei die Nukleinsäuresequenz ausgewählt ist aus der Gruppe, die aus Aptameren, Ribozymen, Aptazymen, Antisense-Molekülen und siRNAs besteht.

In einer Ausführungsform des ersten Aspekts ist vorgesehen, dass der Adenovirus eine Expressionskassette umfassend einen Promotor und eine Nukleinsäuresequenz umfasst, wobei die Nukleinsäuresequenz eine codierende Nukleinsäure ist, wobei die Nukleinsäure für ein Molekül codiert, das ausgewählt ist aus der Gruppe, die aus Peptiden, Polypeptiden, Proteinen, Antikalinen, Antikörpern und Antikörperfragmenten besteht.

In einer Ausführungsform des ersten Aspekts ist vorgesehen, dass der Adenovirus eine Expressionskassette umfassend einen Promotor und eine Nukleinsäuresequenz umfasst, wobei die Nukleinsäuresequenz ausgewählt ist aus der Gruppe, die aus apoptoseinduzierende Genen, Prodrug-Genen, Protease-Inhibitoren, Tumorsuppressorgenen, Zytokinen und Angiogenese-Inhbitoren besteht.

In einer Ausführungsform des ersten Aspekts ist vorgesehen, dass der Adenovirus replikationsfähig ist in Zellen, die dereguliertes YB-1 aufweisen, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

In einer Ausführungsform des ersten Aspekts ist vorgesehen, dass die Zellen YB-1 Zellzyklus-unabhängig YB-1 im Kern aufweisen.

Die der vorliegenden Erfindung zu Grunde liegende Aufgabe wird erfindungsgemäßen gelöst in einem zweiten Aspekt durch eine Nukleinsäure codierend für einen Adenovirus nach der ersten Aspekt der Erfindung.

Die der vorliegenden Erfindung zu Grunde liegende Aufgabe wird erfindungsgemäßen gelöst in einem dritten Aspekt durch ein Replikationssystem umfassend eine Nukleinsäure nach dem zweiten Aspekt der Erfindung und eine Nukleinsäure eines Helfervirus, wobei die Nukleinsäure des Helfervirus eine oder mehrere der Expressionskassetten des Adenovirus nach dem ersten Aspekt der Erfindung umfasst.

In einer Ausführungsform des dritten Aspekts ist vorgesehen, dass dem Adenovirus oder der diesen codierenden Nukleinsäure die vom Helfervirus umfasste Expressionskassette fehlt.

Die der vorliegenden Erfindung zu Grunde liegende Aufgabe wird erfindungsgemäßen gelöst in einem vierten Aspekt durch einen Vektor umfassend eine Nukleinsäure nach dem zweiten Aspekt der Erfindung und/oder ein Replikationssystem nach dem dritten Aspekt der Erfindung.

In einer Ausführungsform des vierten Aspekts ist vorgesehen, dass der Vektor ein Expressionsvektor ist

Die der vorliegenden Erfindung zu Grunde liegende Aufgabe wird erfindungsgemäßen gelöst in einem fünften Aspekt durch eine. Zelle umfassend einen Adenovirus nach dem ersten Aspekt der Erfindung und/oder eine Nukleinsäure nach dem zweiten Aspekt der Erfindung und/oder ein Replikationssystem nach dem dritten Aspekt der Erfindung und/oder einen Vektor nach dem dritten Aspekt der Erfindung.

In einer Ausführungsform des fünften Aspekts ist vorgesehen, dass die Zelle eine eukaryontische Zelle, bevorzugterweise eine tierische Zelle, bevorzugtererweise eine Säugtierzelle ist.

In einer Ausführungsform des fünften Aspekts ist vorgesehen, dass die Säugetierzelle eine Zelle ist, die ausgewählt ist aus der Gruppe, die aus Zellen von Mäusen, Ratten, Meerschweinchen, Schweinen, Schafen, Ziegen, Rindern, Pferden, Hunden, Katzen und Menschen besteht.

Die der vorliegenden Erfindung zu Grunde liegende Aufgabe wird erfindungsgemäßen gelöst in einem sechsten Aspekt durch einen nicht-menschlicher Organismus, bevorzugterweise ein Säugetierorganismus umfassend einen Adenovirus nach dem ersten Aspekt der Erfindung, eine Nukleinsäure nach dem zweiten Aspekt der Erfindung, ein Replikationssystem nach dem dritten Aspekt der Erfindung, einen Vektor nach dem vierten Aspekt der Erfindung oder eine Zelle nach dem fünften Aspekt der Erfindung, wobei bevorzugterweise der Organismus ausgewählt ist aus der Gruppe, die aus Mäusen, Ratten, Meerschweinchen, Schweinen, Schafen, Ziegen, Rindern, Pferden, Hunden und Katzen besteht.

Die der vorliegenden Erfindung zu Grunde liegende Aufgabe wird erfindungsgemäßen gelöst in einem siebten Aspekt durch die Verwendung eines Adenovirus nach dem ersten Aspekt der Erfindung, einer Nukleinsäure nach dem zweiten Aspekt der Erfindung, eines Replikationssystem nach dem dritten Aspekt der Erfindung, eines Vektor nach dem vierten Aspekt der Erfindung oder einer Zelle nach dem fünften Aspekt der Erfindung zur in vitro Replikation von Adenovirus.

Die der vorliegenden Erfindung zu Grunde liegende Aufgabe wird erfindungsgemäßen gelöst in einem achten Aspekt durch die Verwendung eines Adenovirus nach dem ersten Aspekt der Erfindung, einer Nukleinsäure nach dem zweiten Aspekt der Erfindung, eines Replikationssystem nach dem dritten Aspekt der Erfindung, eines Vektor nach dem vierten Aspekt der Erfindung oder einer Zelle nach dem fünften Aspekt der Erfindung zur in vitro Herstellung von Adenovirus.

Die der vorliegenden Erfindung zu Grunde liegende Aufgabe wird erfindungsgemäßen gelöst in einem neunten Aspekt durch die Verwendung eines Adenovirus nach dem ersten Aspekt der Erfindung, einer Nukleinsäure nach dem zweiten Aspekt der Erfindung, eines Replikationssystem nach dem dritten Aspekt der Erfindung, eines Vektor nach dem vierten Aspekt der Erfindung oder einer Zelle nach dem fünften Aspekt der Erfindung zur Herstellung eines Medikamentes.

In einer Ausführungsform des achten und neunten Aspekts ist vorgesehen, dass die Zelle, in der der Adenovirus repliziert, YB-1 im Kern aufweist, bevorzugterweise YB-1 Zellzyklusunabhängig im Kern aufweist.

In einer Ausführungsform des achten und neunten Aspekts ist vorgesehen, dass die Zelle, in der der Adenovirus repliziert, dereguliertes YB-1 aufweist, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

In einer Ausführungsform des neunten Aspekts ist vorgesehen, dass das Medikament für die Behandlung von Tumorerkrankungen ist.

In einer Ausführungsform des neunten Aspekts ist vorgesehen, dass die Tumorerkrankung ausgewählt ist aus der Gruppe, die aus malignen Erkrankungen, Krebs, Krebserkrankungen und Tumoren besteht.

In einer Ausführungsform des neunten Aspekts ist vorgesehen, dass die Tumoren ausgewählt sind aus der Gruppe, die aus soliden, nichtsoliden, malignen und benignen Tumoren besteht.

In einer Ausführungsform des neunten Aspekts ist vorgesehen, dass zumindest ein Teil der den Tumor ausbildenden Zellen YB-1 im Kern aufweist, bevorzugterweise YB-1 Zellzyklusunabhängig im Kern aufweist.

In einer Ausführungsform des neunten Aspekts ist vorgesehen, dass zumindest ein Teil der den Tumor ausbildenden Zellen dereguliertes YB-1 aufweist, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

In einer Ausführungsform des neunten Aspekts ist vorgesehen, dass dass zumindest ein Teil der den Tumor ausbildenden Zellen Rb-positiv oder Rb-negativ ist.

In einer Ausführungsform des neunten Aspekts ist vorgesehen, dass zumindest ein Teil der den Tumor ausbildenden Zellen eine Resistenz gegen Antitumormittel eine Mehrfachresistenz gegen pharmakologische Wirkstoffe und/oder eine durch Bestrahlung bedingte Resistenz aufweisen.

In einer Ausführungsform des neunten Aspekts ist vorgesehen, dass die Resistenz gegen gegen Zytostatika gerichtet ist.

In einer Ausführungsform des neunten Aspekts ist vorgesehen, dass der Patient für den das Medikament bestimmt ist, eine Mehrzahl von Zellen aufweist, wobei die Zellen solche Zellen sind, wie in einem der vorstehenden Ausführungsformen beschrieben sind.

In einer Ausführungsform des neunten Aspekts ist vorgesehen, dass das Medikament mindestens ein weiteres pharmazeutisch aktiven Mittels umfasst.

In einer Ausführungsform des neunten Aspekts ist vorgesehen, dass das Medikament zusammen mit einem weiteren pharmazeutisch aktiven Mittel verabreicht wird oder werden soll.

In einer Ausführungsform des neunten Aspekts ist vorgesehen, dass das weitere pharmazeutische Mittel ausgewählt ist aus der Gruppe, die aus Zytokinen, Metalloproteinase-Inhibitoren, Angiogenese-Inhibitoren, Zytostatika, Tyrosinkinase-Inhibitoren, Zellzyklus-Inhibitoren, Proteosomen-Inhibitoren, Inhibitoren der Signaltransduktionskaskade, Proteinkinasen und rekombinanten Antikörpern besteht.

In einer Ausführungsform des neunten Aspekts ist vorgesehen, dass das Medikament vor, während oder nach einer Bestrahlung verabreicht wird.

In einer Ausführungsform des neunten Aspekts ist vorgesehen, dass die Bestrahlung zum Zwecke der Behandlung eines Tumors verabreicht wird.

In einer Ausführungsform des neunten Aspekts ist vorgesehen, dass dass die Zelle oder der zu behandelnde Organismus einer Maßnahme unterzogen wird, wobei die Maßnahme aus der Gruppe ausgewählt ist, die aus Bestrahlung, Gabe von Zytostatika und Hyperthermie besteht.

In einer Ausführungsform des neunten Aspekts ist vorgesehen, dass die Maßnahme lokal oder systemisch angewandt wird.

In einer Ausführungsform des neunten Aspekts ist vorgesehen, dass die Bestrahlung mit energiereicher Strahlung erfolgt, bevorzugterweise mit einer Strahlung, wie sie bei der Behandlung von Tumorerkrankungen verwendet wird.

In einer Ausführungsform des neunten Aspekts ist vorgesehen, dass die Tumorerkrankung ausgewählt ist aus der Gruppe, die aus Brusttumoren, Knochentumoren, Magentumoren, Darmtumoren, Gallenblasentumoren, Bauspeicheldrüsentumoren, Lebertumoren, Nierentumoren, Gehirntumoren, Eierstocktumoren, Haut- und Hautanhangsorgantumoren, Kopf-/Nackentumoren, Gebärmuttertumoren, Synovialtumoren, Kehlkopftumoren, Speiseröhrentumoren, Zungentumoren und Prostatatumoren, bevorzugterweise eine der vorstehenden Tumorerkrankungen, die wie in einem der vorangehenden Ausführungsformen beschrieben ausgeprägt ist.

In einer Ausführungsform des neunten Aspekts ist vorgesehen, dass der tumor-spezifische Promotor ein solcher Promotor ist, der spezifisch ist für den Tumor, für den das Medikament verwendet wird.

Die der vorliegenden Erfindung zu Grunde liegende Aufgabe wird erfindungsgemäßen gelöst in einem zehnten Aspekt durch eine pharmazeutische Zusammensetzung umfassend einen Adenovirus nach dem ersten Aspekt der Erfindung, eine Nukleinsäure nach dem zweiten Aspekt der Erfindung, ein Replikationssystem nach dem dritten Aspekt der Erfindung, einen Vektor nach dem vierten Aspekt der Erfindung oder eine Zelle nach dem fünften Aspekt der Erfindung, und optional ein pharmazeutisch geeignetes Trägermittel.

In einer Ausführungsform des neunten Aspekts ist vorgesehen, dass Medikament eine Kombination von mindestens zwei Mittel umfasst, wobei ein jedes Mittel einzeln und unabhängig aus der Gruppe ausgewählt ist, die aus Cytostatika besteht.

In einer Ausführungsform des neunten Aspekts ist vorgesehen, dass mindestens zwei der Mittel an unterschiedlichen Zielmolekülen angreifen.

In einer Ausführungsform des neunten Aspekts ist vorgesehen, dass mindestens zwei der Mittel über einen unterschiedlichen Wirkmechanismus aktiv sind.

In einer Ausführungsform des neunten Aspekts ist vorgesehen, dass mindestens ein Mittel die Infizierbarkeit einer Zelle erhöht, in der das Virus repliziert.

In einer Ausführungsform des neunten Aspekts ist vorgesehen, dass mindestens ein Mittel die Verfügbarkeit einer Komponente der Zelle beeinflusst, wobei die Komponente die Aufnahme des Virus vermittelt.

In einer Ausführungsform des neunten Aspekts ist vorgesehen, dass mindestens ein Mittel den Transport von YB-1 in den Zellkern vermittelt, bevorzugterweise erhöht.

In einer Ausführungsform des neunten Aspekts ist vorgesehen, dass mindestens ein Mittel ein Histon-Deacylase-Inhibitor ist.

In einer Ausführungsform des neunten Aspekts ist vorgesehen, dass der Histon-Deacylase-Inhibitor aus der Gruppe ausgewählt ist, die aus Trichostatin A, FR 901228, MS-27-275, NVP-LAQ824, PXD101 Apicidin und Scriptaid besteht.

In einer Ausführungsform des neunten Aspekts ist vorgesehen, dass mindestens ein Mittel aus der Gruppe ausgewählt ist, die aus Trichostatin A, FR 901228, MS-27-275, NVP-LAQ824, PXD101, Apicidin und Scriptaid besteht.

In einer Ausführungsform des neunten Aspekts ist vorgesehen, dass mindestens ein Mittel ein Topoisomerase-Inhibitor ist.

In einer Ausführungsform des neunten Aspekts ist vorgesehen, dass der Topoisomerase-Inhibitor aus der Gruppe ausgewählt ist, die aus Camptothecin, SN-38, Topotecan, DX-895If, Irinotecan 9-aminocamptothecin, 9-nitrocamptothecin, Etoposid und Daunorubicin besteht.

In einer Ausführungsform des neunten Aspekts ist vorgesehen, dass das Mittel aus Trichostatin A und Irinotecan besteht. on den mindestens zwei Mittel ist.

In einer Ausführungsform des neunten Aspekts ist vorgesehen, dass mindestens eine Einheitsdose des Virus von mindestens einer Einheitsdose von einem oder den mindestens zwei Mitteln getrennt ist.

Die der vorliegenden Erfindung zu Grunde liegende Aufgabe wird erfindungsgemäßen gelöst in einem elften Aspekt durch ein Kit umfassend einen Virus nach dem ersten Aspekt der Erfindung, und mindestens zwei Mittel, wobei ein jedes Mittel einzeln und unabhängig aus der Gruppe ausgewählt ist, die aus Cytostatika besteht.

In einer bevorzugten Ausführungsform des ersten Aspektes ist dabei vorgesehen, dass das E1A 12S-Protein ein solches von Ad5, insbesondere von Wildtyp Ad 5, oder ein E1A 12S-Protein von Adenovirus delta 24.

In einer Ausführungsform der Verwendung gemäß dem neunten Aspekt der vorliegenden Erfindung ist vorgesehen, dass das Medikament zusammen mit einem weiteren pharmazeutisch aktiven Mittel verabreicht wird oder werden soll.

In einer Ausführungsform der Verwendung gemäß dem neuenten Aspekt der vorliegenden Erfindung ist vorgesehen, dass das weitere pharmazeutische Mittel ausgewählt ist aus der Gruppe, die Zytokine, Metalloproteinase-Inhibitoren, Angiogenese-Inhibitoren, Zytostatika beispielsweise beispielsweise Irinotecan und CPT-11 gegen Kolorektalkarzinome und Daunorubicin gegen Leukämien, Zellzyklus-Inhibitoren beispielsweise wie das CYC202 welches CDK2/CyclinE-kinasaktivität inhibiert und gegen Kolorektaltumore eingesetzt werden kann (McClue SJ, Int. J. Cancer 2002, 102, 463-468) und BAY 43-9006 welches Raf-1 inhibiert und beispielsweise gegen Mammacarcinoma wirksam ist (Wilhelm SM et al., Cancer Res. 2004, 64, 7099-7109), Proteosomen-Inhibitoren wie das PS-341 welches die 26S Proteasomenaktivität inhibiert und gegen Kopf- und Halsplattenepithelkarzinom eingesetzt wird (Fribley A et al., Mol Cell Biol 2004 Nov; 24(22): 9695-704), rekombinante Antikörper beispielsweise gegen den EGF-Rezeptor (Herceptin für Mammacarcinom und Prostatatumore; H.G. van der Poel, European Urology 2004, 1-17; Erbitux gegen Hals-Kopftumore; Bauman M et al., Radiother. Oncol., 2004, 72, 257-266) und Inhibitoren der Signaltransduktionskaskade wie das STI 571 welches unteranderem c-kit reprimiert und gegen gastrointensinale Tumore eingesetzt werden kann (H.G. van der Poel, European Urology 2004, 45, 1-17), ABT-627 ein Endothelininhibitor welches unter anderem gegen Prostatatumore eingesetzt werden kann (H.G. van der Poel, European Urology 2004, 45, 1-17), SU5416 welches die Phosphorylierung des VEGF Tyrosinkinaserezeptor inhibiert und unteranderem gegen Glioblastom und Prostatakarzinom eingesetzt werden kann (Bischof M et al Int. J. Radiat. Oncol. Biol. Phys. 2004; 60 (4): 1220-32), ZD1839 welches EGFR- Tyrosinaseaktivität inhibiert und unteranderem gegen Prostatatumore eingesetzt werden kann (H.G. van der Poel, European Urology 2004, 45, 1-17); Rapamycin-Derivate beispielsweise CCI-779 und RAD001 welche mTOR inhibieren und gegen Prostatatumore eingesetzt werden kann (H.G. van der Poel, European Urology 2004, 45, 1-17). Es ist im Rahmen der vorliegenden Erfindung, dass die verschiedenen hierin beschrieben Adenoviren bzw. die hierin erfindungsgemäß zu verwendenen Adenoviren mit den vorstehend genannten Verbindungen grundsätzlich für eine jede Indikation verwendet werden können, die hierin im Zusammenhang damit beschrieben ist. In einer besonders bevorzugten Ausführungsform ist die Indikation eine solche, wie sie für eine jede der vorstehend genannten pharmazeutisch wirksamen Verbindungen beschrieben ist.

In einer weiteren Ausführungsform ist vorgesehen, dass das Zielmolekül ausgewählt ist aus der Gruppe, die Resistenz-relevante Faktoren, Anti-Apoptose-Faktoren, Onkogene, Angiogenese-Faktoren, DNA-Synthese-Enzyme, DNA-Reperaturenzyme, Wachstumsfaktoren und deren Rezeptoren, Transkriptionsfaktoren, Metalloproteinasen, insbesondere Matrix-Metalloproteinasen, und Plasminogenaktivator vom Urokinase-Typ umfasst. In einer Ausführungsform sind die Resistenz-relevanten Faktoren bevorzugt aus der Gruppe ausgewählt, die P-Glykoprotein, MRP und GST umfasst, und umfassen auch die dafür codierenden Nukleinsäuren. In einer Ausführungsform sind die Anti-Apopotose-Faktoren ausgewählt aus der Gruppe, die BCL2 umfasst, und umfassen auch die dafür codierenden Nukleinsäuren. In einer Ausführungsform sind die Onkogene ausgewählt aus der Gruppe, die Ras, insbesondere mutiertes Ras, Rb und Myc umfasst, und umfassen auch die dafür codierenden Nukleinsäuren. In einer Ausführungsform sind die Angiogenese-Faktoren ausgewählt aus der Gruppe, die VEGF und HMG-Proteine umfasst, und umfassen auch die dafür codierenden Nukleinsäuren. In einer Ausführungsform sind die DNA-Synthese-Enzyme ausgewählt aus der Gruppe, die Telomerase umfasst, und umfassen auch die dafür codierenden Nukleinsäuren. In einer Ausführungsform sind die DNA-Reparaturenzyme ausgewählt aus der Gruppe, die Ku-80 umfasst, und umfassen auch die dafür codierenden Nukleinsäuren. In einer Ausführungsform sind die Wachstumsfaktoren ausgewählt aus der Gruppe, die PDGF, EGF und M-CSF umfasst, und umfassen auch die dafür codierenden Nukleinsäuren In einer weiteren Ausführungsform ist vorgesehen, dass die Rezeptoren insbesondere solche der Wachstumsfaktoren sind, wobei bevorzugterweise die Wachstumsfaktoren aus der Gruppe ausgewählt sind, die PDGF, EGF und M-CSF umfasst, und umfassen auch die dafür codierenden Nukleinsäuren. In einer Ausführungsform sind die Transkriptionsfaktor ausgewählt aus der Gruppe, die YB-1 umfasst, und umfassen auch die dafür codierenden Nukleinsäuren. In einer Ausführungsform sind die Metalloproteinasen insbesondere Matrix-Metalloproteinasen. In einer bevorzugten Ausführungsform sind die Matrix-Metalloproteinasen ausgewählt aus der Gruppe, die MMP-1 und MMP-2 umfasst, und umfassen auch die dafür codierenden Nukleinsäuren. In einer Ausführungsform sind die Plasminogenaktivatoren vom Urokinase-Typ ausgewählt aus der Gruppe, die uPa-R umfasst, und umfassen auch die dafür codierenden Nukleinsäuren.

In einer bevorzugten Ausführungsform ist vorgesehen, dass das weitere pharmazeutische Mittel ausgewählt ist aus der Gruppe, die Zytokine, Metalloproteinase-Inhibitoren, Angiogenese-Inhibitoren, Zytostatika beispielsweise beispielsweise Irinotecan und CPT-11 gegen Kolorektalkarzinoma und Daunorubicin gegen Leukämien, Zellzyklus-Inhibitoren beispielsweise wie das CYC202 welches CDK2/CyclinE-kinasaktivität inhibiert und gegen Kolorektaltumore eingesetzt werden kann (McClue SJ, Int. J. Cancer 2002, 102, 463-468) und BAY 43-9006 welches Raf-1 inhibiert und beispielsweise gegen Mammacarcinoma wirksam ist (Wilhelm SM et al., Cancer Res. 2004, 64, 7099-7109), Proteosomen-Inhibitoren wie das PS-341 welches die 26S Proteasomenaktivität inhibiert und gegen Gehirntumore eingesetzt wird (Yin D. et al., Oncogene 2004), rekombinante Antikörper beispielsweise gegen den EGF-Rezeptor (Herceptin für Mammacarcinom und Prostatatumore; H.G. van der Poel, European Urology 2004, 1-17; Erbitux gegen Hals-Kopftumore; Bauman M et al., Radiother. Oncol., 2004, 72, 257-266) und Inhibitoren der Signaltransduktionskaskade wie das STI 571 welches unteranderem c-kit reprimiert und gegen gastrointensinale Tumore eingesetzt werden kann (H.G. van der Poel, European Urology 2004, 45, 1-17), ABT-627 ein Endothelininhibitor welches unter anderem gegen Prostatatumore eingeetzt werden kann ( H.G. van der Poel, European Urology 2004, 45, 1-17), SU5416 welches die Phosphorylierung des VEGF-Tyrosinkinaserezeptor inhibiert und unteranderem gegen Hals/Kopftumore eingesetzt werden kann (Cooney et al., Cancer Chemother. Pharmacol 2004), ZD1839 welches die EGFR-Tyrosinaseaktivität inhibiert und unteranderem gegen Prostatatumore eingesetzt werden kann (H.G. van der Poel, European Urology 2004, 45, 1-17); Rapamycin-Derivate beispielsweise CCI-779 und RAD001 welche mTOR inhibieren und gegen Prostatatumore eingesetzt werden kann (H.G. van der Poel, European Urology 2004, 45, 1-17). Es ist im Rahmen der vorliegenden Erfindung, dass die verschiedenen hierin beschrieben Adenoviren bzw. die hierin erfindungsgemäß zu verwendenen Adenoviren mit den vorstehend genannten Verbindungen grundsätzlich für eine jede Indikation verwendet werden können, die hierin im Zusammenhang damit beschrieben ist In einer besonders bevorzugten Ausführungsform ist die Indikation eine solche, wie sie für eine jede der vorstehend genannten pharmazeutisch wirksamen Verbindungen beschrieben ist.

In einer Ausführungsform insbesondere des neunten Aspekts der Erfindung ist vorgesehen, dass mindestens ein Mittel aus der Gruppe ausgewählt ist, die Trichostatin A, FR 901228 (gegen Pankreastumore, Sato N et al., Int. J. Oncol. 2004, 24, 679-685; MS-27-275 (gegen Prostatatumore; Camphausen K et al., Clinical Canver Research 2004, 10, 6066-6071), NVP-LAQ824 (gegen Leukämien; Nimmanapalli R et al., Cancer Res. 2003, 63, 5126-5135; PXD101 (gegen Ovarialtumore, Plumb JA et al, Mol. Cancer Ther. 2003, 2, 721-728) Scriptaid (gegen Mammakarzinome, Keen JC et al., Breast Cancer Res. Treat. 2003, 81, 177-186), Apicidin (gegen Melanom, Kim SH et al., Biochem. Biophys. Res. Commun. 2004, 315, 964-970) umfasst. Die Wirkungsweise von Histondeacetylaseinhibitoren ist beschrieben unter anderem in Lindemann RK et al., Cell Cycle 2004, 3, 77-86. Es ist im Rahmen der vorliegenden Erfindung, dass die verschiedenen hierin beschrieben Adenoviren bzw. die hierin erfindungsgemäß zu verwendenen Adenoviren mit den vorstehend genannten Verbindungen grundsätzlich für eine jede Indikation verwendet werden können, die hierin im Zusammenhang damit beschrieben ist. In einer besonders bevorzugten Ausführngsform ist die Indikation eine solche, wie sie für eine jede der vorstehend genannten pharmazeutisch wirksamen Verbindungen beschrieben ist.

In einer bevorzugten Ausführungsform insbesondere des neunten Aspekts der Erfindung ist vorgesehen, der Topoisomerase-Inhibitor aus der Gruppe ausgewählt ist, die Camptothecin, Irinotecan, Topotecan, DX-895If, SN-38, 9-aminocamptothecin, 9-nitrocamptothecin, Daunorubicin und Etoposide umfasst. Diese können gegen verschiedene Tumore eingsetzt werden beispielsweise Kolorektaltumore, Pankreastumore, Ovarialkarzinome, Lungentumore und Prostatakarzinome. Die Anwendungsgebiete sind beschrieben unteranderem bei Recchia F et al., British J. Cancer 2004, 91, 1442-1446; Cantore M et al., Oncology 2004, 67, 93-97; Maurel J. et al., Gynecol. Oncol 2004, 95,114-119; Amin A. et al., Urol. Oncol. 2004, 22, 398-403; Kindler HL et al., Invest. New Drugs 2004, 22, 323-327, Ahmad T. et al., Expert Opin. Pharmacother. 2004, 5, 2333-2340; Azzariti A. et al., Biochem Pharmacol. 2004, 68, 135-144; Le QT et al., Clinical Cancer Res. 2004, 10, 5418-5424. Es ist im Rahmen der vorliegenden Erfindung, dass die verschiedenen hierin beschrieben Adenoviren bzw. die hierin erfingdungsgemäß zu verwendenen Adenoviren mit den vorstehend genannten Verbindungen grundsätzlich für eine jede Indikation verwendet werden können, die hierin im Zusammenhang damit beschrieben ist. In einer besonders bevorzugten Ausführungsform ist die Indikation eine solche, wie sie für eine jede der vorstehend genannten pharmazeutisch wirksamen Verbindungen beschrieben ist.

Die vorstehend offenbarten erfindungsgemäßen Adenoviren, insbesondere jene, wie sie im Zusammenhang mit den Aspekte eins bis acht der vorliegenden Erfindung beschrieben werden, werden hierin auch als Adenoviren der Gruppe I bezeichnet, und die Adenoviren, die ein transaktivierendes Onkogenprotein aufweisen, wie beispielsweise E1A, und/oder die hierin und insbesondere vorstehend als erfindungsgemäß verwendet bezeichnet werden, werden hierin auch als Adenoviren der Gruppe II bezeichnet. Adenoviren der Gruppe I und der Gruppe II werden hierin auch insgesamt als Adenoviren oder als erfindungsgemäße Adenoviren oder erfindungsgemäße Viren bezeichnet.

Der vorliegenden Erfindung liegt die überraschende Erkenntnis zugrunde, dass die Umkehrung der Reihenfolge der Expression von adenoviralen Genen zu einer effizienten Replikation und gegebenenfalls zu einer Lyse der von dem Adenovirus infizierten Zelle führt. Dabei ist hinsichtlich der zeitlich geänderten Exprimierung der adenoviralen Gene besonders auf ein E1B-Protein und ein E4-Protein abzustellen, die hierin auch als erstes Protein einzeln bzw. zusammen bezeichnet werden, die vor einem zweiten Protein exprimiert werden. Das zweite Protein ist aus der Gruppe ausgewählt, die E1A-Proteine umfasst. Mit dieser Umkehrung der Expressionsreihenfolge gegenüber Wildtyp-Adenoviren, bei denen zuerst ein E1A-Protein exprimiert wird und erst dann das E1B-Protein und ein B4-Protein, wird gewährleistet, dass Transkriptionsfaktoren aktiviert werden, beispielsweise in den Zellkern der infizierten Zelle transportiert werden und dort das weitere replikative Geschehen von Adenoviren beeinflussen oder steuern. Die Kinetik der adenoviralen Transkripte in Adenoviren vom Wildtyp ist beispielsweise beschrieben in Glenn G. M. und Ricciardi R P. Virus Research 1988, 9, 73-91, die berichten, dass die E1A-Transkripte, d. h. das E1A12S-Transkript und das E1A13S-Transkript im Wildtyp normalerweise vor den Transkripten bzw. Translationsprodukten E4orf6 und E1B55k detektierbar sind. Bei dem einen E1B-Protein handelt es sich im vorliegenden Falle um das E1B-55kD-Protein. Bei einem E4-Protein handelt es sich im vorliegenden Falle um das E4orf6-Protein. Bei dem E1A-Protein handelt es sich im vorliegenden Falle und auch insgesamt hierin sofern nichts Gegenteiliges angegeben wird, bevorzugterweise um ein E1A12S-Protein oder um ein solches E1A-Protein, wie es hierin im Zusammenhang mit dem E1A-modifizierten Adenoviren offenbart ist.

Es ist im Rahmen der vorliegenden Erfindung, dass das E1A-Protein, insbesondere auch das E1A12S-Protein grundsätzlich substituierbar ist Derartige substituierte E1A-Proteine bzw. E1A12S-Proteine werden hierin, sofern nicht anders angegeben, auch als E1A-Protein bzw. E1A12S-Protein bezeichnet bzw. sollen von diesem Begriff mit umfasst sein. Anstelle des E1A12S-Proteins kann auch ein E1A-Protein verwendet werden, welches eine Tumorsuppressorfunktion aufweist, wie beispielweise beschrieben bei Dickopp A, Esche H, Swart G, Seeber S, Kirch HC, Opalka B. Cancer Gene Ther. 2000, Jul;7(7):1043-50. Weitere Derivate von E1A-Proteinen, insbesondere E1A12S-Protein, wie hierin verwendet und/oder als solche bezeichnet, sind auch allgemein solche Proteine, die in der Lage sind, den Faktor E2F aus dem Komplex Rb/E2F freizusetzen. Dazu gehören, unter anderem, Simian virus 40 tumor antigen (SV40 large T-Antigen), Papillomavirus E7 Protein (HPV E7), wie beschrieben von Chellappan S. et al., Proc. Natl. Acad. Sci. USA 1992, 89, 4549-4533.

Es ist auch im Rahmen der vorliegenden Beschreibung, dass Derivate von E4orf6 und E1B55k verwendet werden, wobei die Bezeichnung E4orf6 und E1B55k, wie hierin verwendet, diese Derivate umfasst. Die Derivate sind beispielsweise beschrieben in Shen Y et al., J. of Virology 2001, 75,4297-4307; Querido E. et al., J. of Virology 2001, 75,699-709.

Es wird beschrieben dass ein E1B-Protein vor dem B1A-Protein exprimiert wird, oder dass ein E4-Protein vor einem E1A-Protein exprimiert wird, oder sowohl ein E1B-Protein als auch ein E4-Protein vor dem E1A-Protein, jeweils wie vorstehend beschrieben, exprimiert wird.

Ein solchermaßen ausgestaltetes Adenovirus weist insbesondere bei Infektion einer Zelle, die YB-1 im Kern exprimiert, insbesondere zellzyklusunabhängig im Kern exprimiert, oder ein dereguliertes YB-1 aufweist, insbesondere im Zytoplasma aufweist, ein besonders hohes Maß an Replikationsfähigkeit auf. Ohne im Folgenden darauf festgelegt sein zu wollen, geht der vorliegende Erfinder davon aus, dass unter dem Einfluss des vor einem E1A-Protein exprimierten E1B-Protein und/oder E4-Protein, ein Komplex aus E1B-Protein und E4-Protein bzw. einzelne dieser beiden Proteine, in der Lage sind, dereguliertes YB-1 in den Zellkern zu transportieren und dort die Replikation von Adenovirus zu initiieren. Einmal im Zellkern bzw. dort in aktivierter Form vorliegend, kann YB-1, wie hierin beschrieben, insbesondere über den E2-late-Promotor die Replikation wirksam durchführen. Durch die zeitlich vorgezogene Expression eines E1B-Proteins und/oder eines E4-Proteins wird somit vermieden, dass die beim Wildtyp beobachtete Kaskade mit initialer Expression von E1A-Protein erfolgt. Dabei ist das E1A-Protein ein solches, welches insbesondere auf das E1B-Protein und/oder das E4-Protein nicht mehr oder nur mehr schwach transaktivierend wirkt. Bevorzugterweise ist die Transaktivierung nicht ausreichend, um eine effiziente Replikation zu gewährleisten, auch keine Replikation in Zellen, die YB-1 nicht im Zellkern aufweisen. Dabei ist bevorzugt, dass die Transaktivierung nicht erfolgt in Zellen, die Zellzyklus-unabhängig YB-1 im Zellkern nicht aufweisen bzw. kein dereguliertes YB-1 aufweisen.

Weiterhin liegt der vorliegenden Erfindung die überraschende Erkenntnis zugrunde, dass ein Adenovirus zu einer besonders effizienten Replikation dann befähigt ist, wenn dieser mindestens eine Nukleinsäure umfasst, die für ein Protein codiert, wobei das Protein ausgewählt ist aus der Gruppe, die E1B-Proteine, E4-Proteine und E1A-Proteine umfasst, und dass mindestens ein Protein davon unter der Kontrolle eines Promotors steht, der verschieden ist von dem Promotor, der die Expression des jeweiligen Proteins in einem Adenovirus vom Wildtyp steuert. Eine derartige Replikation erfolgt besonders effizient und führt in der Regel zur Tumorlyse bei solchen Zellen, die YB-1 im Kern aufweisen, insbesondere zellzyklusunabhängig YB-1 im Kern aufweisen, oder dereguliertes YB-1, insbesondere dereguliertes YB-1 im Zytoplasma aufweisen. Hinsichtlich der E1B-Proteine, E4-Proteine und E1A-Proteine gilt das vorstehend Gesagte betreffend deren Ausgestaltung. In Adenoviren vom Wildtyp steht das E1B-Protein unter der Kontrolle des E1B-Promotors, das E4-Protein unter der Kontrolle des E4-Promotors und das E1A-Protein unter der Kontrolle des E1A-Promotors steht. Durch Auswahl von Promotoren, die verschieden sind von denjenigen, die in Adenoviren vom Wildtyp die Expression der vorstehend genannten Proteine steuern, wird die Expression der vorgenannten Proteine und damit das regulatorische Zusammenspiel der einzelnen adenoviralen Nukleinsäuren und Proteine verändert. Durch Auswahl der Promotoren kann somit ein anderes zeitliches Expressionsmuster bedingt werden, welches, ohne im Folgenden darauf festgelegt sein zu wollen, zu der beobachteten Replikation in Zellen führt, wobei der Mechanismus derjenige sein kann, wie er vorstehend hinsichtlich der zeitlich unterschiedlichen Expression der adenoviralen Proteine E1B, E4 und E1A bereits beschrieben ist. Ein Beispiel für die konkrete Ausgestaltung der Kontrolle der besagten Proteine durch andere Promotoren als jene, die die Expression des jeweiligen Proteins in einem Wildtyp-Adenovirus steuern, ergeben sich unter anderem aus den Unteransprüchen und aus dem Beispielsteil, wobei hier insbesondere die darin als XVirPSJL1 und XVirPSJL2 bezeichneten Viren beispielhaft hierfür genannten sein sollen. Bevorzugterweise handelt es sich bei dem E1B-Protein um das E1B55kD-Protein, bei dem E4-Protein um das E4orf6-Protein und bei dem E1A-Protein um das E1A12S-Protein.

Die Promotoren, unter die bevorzugterweise das E1B-Protein ebenso wie das E4-Protein gestellt werden, sind ausgewählt aus der Gruppe, die tumorspezifische Promotoren, organspezifische Promotoren, gewebespezifische Promotoren, heterologe Promotoren und adenovirale Promotoren umfasst unter der Voraussetzung, dass bei Verwendung adenoviraler Promotoren diese verschieden sind von dem E1B-Promotor im Falle der Expressionskontrolle des E1B-Proteins und verschieden sind von dem E4-Promotor im Falle der Expressionskontrolle des E4-Proteins. Besonders bevorzugt ist dabei die Verwendung des E1A-Promotors für die Expressionskontrolle des E1B-Proteins und/oder des E4-Proteins. Der E1A-Promotor wird beispielsweise beschrieben von Boulanger P. A. und Blair, G. E. Biochem. J. 1991, 275, 281-299. Darüber hinaus ist auch die Verwendung eines jeglichen anderen heterologen Promotors, d. h. eines Promotors möglich, der verschieden ist von demjenigen, der die Expression des jeweiligen Proteins in einem Adenovirus vom Wildtyp kontrolliert. Beispielhaft sei hier der CMV-Promotor angeführt, wobei den Fachleuten auf dem Gebiet weitere Promotoren offensichtlich sein werden.

Der Promotor, der zur Steuerung des E1A-Proteins verwendet wird, kann ebenfalls ausgewählt sein aus der Gruppe, die tumorspezifische Promotoren, organspezifische Promotoren, gewebespezifische Promotoren, heterologe und adenovirale Promotoren umfasst, unter der Voraussetzung, dass der adenovirale Promotor verschieden ist von dem E1A-Promotor. Es ist dabei im Rahmen der vorliegenden Erfindung, dass ein oder mehrere der vorstehend genannte Proteine, d. h. des E1B-Proteins, des E4-Proteins oder des E1A-Proteins unter der Kontrolle jeweils des gleichen Promotors sind, wobei jedoch bevorzugt ist, dass insbesondere das E1B-Protein und das E4-Protein unter der Kontrolle des gleichen Promotors steht. Besonders bevorzugt ist es, dass das E1A-Protein in seiner Expression durch einen YB-1-gesteuerten oder durch YB-1 regulierbaren Promotor gesteuert wird. Derartige Promotoren sind hierin im Zusammenhang mit anderen Aspekten der vorliegenden Erfindung offenbart. Besonders bevorzugt ist dabei die Verwendung des adenoviralen E2-late-Promotors für die Expressionssteuerung des E1A-Promotors, da dieser zum einen durch YB-1 regulierbar ist und zum anderen bei Abwesenheit von YB-1 nur eine geringe Transkription zeigt, die praktisch vernachlässigbar ist, so dass eine sehr gute Expressionskontrolle des unter der Kontrolle des E2-late-Promotors stehende Nukleinsäure gewährleistet wird. Dies erhöht, insbesondere bei Anwendung im medizinischen Bereich, die biologische Sicherheit ganz erheblich.

Weiterhin hat der vorliegende Erfinder erkannt, dass Adenoviren in Zellen besonders gut replizieren, die YB-1 im Zellkern aufweisen, insbesondere zellzyklusunabhängig YB-1 im Zellkern aufweisen und/oder dereguliertes YB-1, bevorzugterweise dereguliertes YB-1 im Cytoplasma, aufweisen, wenn YB-1 für die Replikation direkt oder indirekt bereitgestellt wird, insbesondere im Zellkern bereitgestellt wird oder die Bereitstellung von YB-1 durch ein adenovirales Protein direkt oder indirekt vermittelt wird, wobei dieses adenovirale Protein verschieden ist von E1A. Dieser Aspekt unterscheidet sich insoweit von dem ebenfalls hierin beschriebenem Aspekt, dass durch Verwendung von transaktivierenden E1A-modifizierten Adenoviren, bevorzugterweise der Adenoviren der Gruppe II, eine Replikation dieser Viren in YB-1-Kern-positiven Tumorzellen, insbesondere YB-1-Zellzyklus-unabhängig-Kern-positiven Tumorzellen, und solche, die dereguliertes YB-1, insbesondere im Cytoplasma aufweisen, replikationsfähig sind, insoweit, als die transaktivierenden Eigenschaften des E1A-Proteins, insbesondere des E1A13S-Proteins hier, d.h. im Zusammenhang mit den Adenoviren der Gruppe I nicht genutzt werden und vielmehr in einer bevorzugten Ausführungsform das E1A13S-Protein funktional inaktiv ist und somit die Transaktivierung auch von E4orf6 und E1B55kD nicht mehr bewerkstelligen kann, die für den Transport von YB-1 bzw. die Bereitstellung von YB-1 im Zellkern direkt oder indirekt beteiligt ist, mit der Folge, dass eine effektive Replikation des Adenovirus, gemäß diesem Aspekt der vorliegenden Erfindung nicht mehr erfolgt. Insoweit erfolgt die Bereitstellung von YB-1 im Zellkern bzw. Bereitstellung von YB-1 für die adenovirale Replikation nun nicht mehr unter der Kontrolle oder direkten oder indirekten Beteiligung des E1A-Proteins, sondern durch ein E1A-unkontrolliertes Exprimieren des E1B-Proteins, insbesondere E1B55kD-Proteins und/oder des E4-Proteins, insbesondere des E4orf6-Proteins.

Diese Ausgestaltung des Adenovirus kann dabei auch durch eine der vorstehend beschriebenen Maßnahmen bereitgestellt werden, beispielsweise dadurch, dass die zeitliche Reihenfolge der Expression des E1B-Proteins und/oder des E4-Proteins gegenüber der Expression des E1A-Proteins vorgezogen wird, oder indem eines oder mehrere der E1B-Proteine, E4-Proteine und E1A-Proteine unter die Kontrolle eines Promotors gestellt wird, der verschieden ist von dem Promotor, der die Expression des jeweils infragestehenden Proteins in einem Wildtyp-Adenovirus steuert.

Schließlich geht der vorliegende Erfinder weiterhin von der überraschenden Erkenntnis aus, dass eine effektive wirksame adenovirale Replikation auch dann erfolgen kann, insbesondere in Zellen, die YB-1 im Zellkern aufweisen, insbesondere Zellzyklus-unabhängig YB-1 im Zellkern aufweisen, oder dereguliertes YB-1, insbesondere im Cytoplasma, aufweisen, wenn zumindest eines der E1B-Proteine, E4-Proteine und E1A-Proteine, insbesondere deren bevorzugte Formen davon, in einer Expressionskassette unter der Kontrolle eines Promotors exprimiert werden. Dabei ist es im Rahmen einer Ausführungsform der vorliegenden Erfindung, dass grundsätzlich drei Expressionskassetten mit jeweils einem einzelnen der besagten Proteine bereitgestellt werden. In einer dazu alternativen Ausführungsform kann eine Expressionskassette auch zwei oder mehrere der Proteine E1B, E4 und E1A bzw. deren Derivate oder möglichen Substitute, besonders im Falle des E1A12S umfassen. Es ist dabei hinsichtlich der Ausprägung der verschiedenen Proteine und der jeweils verwendeten Promotoren grundsätzlich das gleiche anwendbar, wie vorstehend ausgeführt unter dem Aspekt, dass die Adenoviren Nukleinsäuren betreffend die Proteine E1B, E4 und E1A aufweisen. Bei Verwendung derartiger Expressionskassetten ist dabei bevorzugt, wenn die im Genom des Wildtyp-Adenovirus vorkommenden, zu den jeweiligen Proteinen der Expressionskassetten korrespondierenden Proteine bzw. die dafür codierende Nukleinsäure entsprechend vollständig oder teilweise deletiert sind, um zu gewährleisten, dass der Virus stabil ist und Rekombinationen, zumindest in größerem Umfang, vermieden werden.

Die Expressionskassetten können dabei grundsätzlich in jeden Bereich bzw. an jeder Stelle des Adenovirus einkloniert werden, wobei bevorzugterweise eine oder mehrere der Kassetten einzeln oder in Kombination miteinander in die E1-Region, die E3-Region und/oder die E4-Region des Virus eingefügt werden. Dabei können die Nukleinsäuren der E1-, E3- und E4-Region vollständig deletiert werden, teilweise deletiert werden, oder auch gar nicht deletiert werden, wobei jedoch hinsichtlich der erfindungsgemäßen Adenoviren bevorzugt ist, dass die Nukleinsäure, die für das E1A13S-Gen codiert, inaktiviert oder deletiert ist, so dass kein transaktivierendes E1A-Proteins durch den Adenovirus bereitgestellt wird. Der Umfang der Deletion in einer oder mehreren der Regionen E1, E3 und E4 wird bestimmt durch die verwendeten Expressionskassetten und gegebenenfalls weitere eingefügte Fremdgene oder Transgene bzw. der diese umfassenden weiteren Expressionskassetten, d. h. Gene, die verschieden sind von adenoviralen Genen, zumindest verschieden sind in dem Sinne, dass sie in dem regulatorischen Kontext der adenoviralen Nukleinsäuren im Wildtyp-Adenovirus oder der Abfolge der adenoviralen Nukleinsäuren im Wildtyp-Adenovirus an dieser Stelle nicht vorgesehen sind. Es ist im Rahmen der vorliegenden Erfindung, dass die in einer oder meheren der Expressionskassetten enthaltenen, für ein E1B-Protein, ein E4-Protein und/oder ein E1A-Protein codierenden Nukleinsäure(n) im adenoviralen Genom teilweise oder vollständig deletiert ist/sind. In einer Ausführungsform, beispielsweise dem erfindungsgemäßen Adenovirus XvirPSJL 1 oder 2, ist die adenovirale, für E4orf6 codierende Nukleinsäure teilweise und/oder ganz deletiert, die vollständige dafür codierende Nukleinsäure jedoch in der Expressionskassette enthalten. Gleiches wird bevorzugterweise für das E1B55k- (auch als E1 55Kd)-Protein unbd/oder das E1A 12S-Protein realisiert sein. Das Ausmaß der Deletion ist dabei in bevorzugten Ausführungsformen so zu wählen, dass maximal eine Packungsgröße von etwa 103 % der maximalen Packungsgröße des Adenovirus vom Wildtyp erreicht wird, obwohl diese Grenze lediglich eine bevorzugte Grenze ist. Die vorzunehmenden möglichen Deletionen im adenoviralen Genom sind dabei lediglich einer Beschränkung in bevorzugten Ausführungsformen insoweit unterworfen, als dass gewährleistet sein muss, dass noch infektiöse und verpackte Partikel hergestellt werden können. Das genaue Ausmaß der Deletionen kann dabei durch die Fachleute auf dem Gebiet auf der Grundlage der hierin gegebenen Offenbarung im Rahmen von Standardversuchen bestimmt werden.

Als Ausgangspunkt für die Konstruktion der hierin beschriebenen Adenoviren kann dabei ein jegliches Adenovirus vom Wildtyp, aber auch andere Adenoviren, verwendet werden, sofern sie gemäß der technischen Lehre der vorliegenden Erfindung ausgebildet werden. Besonders bevorzugt wird dabei zurückgegriffen auf die Adenoviren der Subgruppe C und innerhalb dieser Gruppe wiederum auf Adenovirus 2 und Adenovirus 5.

Die Begriffe E1B-Protein und E1B-Proteins, E4-Protein und E4-Proteine sowie E1A-Protein und E1-Proteine werden hierin sysnonym verwendet, sofern nichts Gegenteiliges angegeben ist.

Wie hierin verwendet bezeichnet der Begriff "dereguliertes" YB-1 ein YB-1 Molekül oder YB-1-Protein wie hierin beschrieben, das in einer Form vorliegt, das quantitativ und/oder qualitative verschieden ist von YB-1, wie es normalerweise in Zellen, bevorzugterweise in Nicht-Tumorzellen vorliegt. Ein dereguliertes YB-1 kann dadurch charakterisiert werden und als solches identifiziert werden, dass bestimmte Viren bei Vorhandensein von deregulierten YB-1 in der Lage sind, vor einem dieses deregulierte YB-1 aufweisenden zellulären Hintergrund zu replizieren. Die bestimmten Viren sind dabei solche, bei denen das E1A-Protein mutiert ist und eine transkativierende Funktion erfüllt. Beispiele für diese bestimmten Viren sind AD delta 24, dl 922-947, E1 Ad/01/07 und CB 016 und/oder beschrieben von Howe, J. A et al., Molecular Therapy 2, 485-495, 2000; Fueyo J. et al., Oncogene 19, 2-12, 2000; Heise C. et al., Nature Medicine 6, 1134 - 1139, 2001; Balague, C et al., J. Virol. 75, 7602-7611, 2001; Bautista, D.S: et al., Virology 1991, 182, 578-596; Jelsma T.N. et al., Virology 1988, 163, 494-502 ; Wong, H.K. und Ziff E.B., J. of Virology 1994, 68, 4910-4920.] Eine derartige Zelle bzw. eine Zelle mit einem derartigen Hintergrund, kann für die Replikation mit Adenoviren der Gruppe I und/oder der Gruppe II verwendet werden. Darüber hinaus können Tumoren, die derartige Zellen umfassen, mit den erfindungsgemäßen Adenoviren lysiert werden.

Der vorliegenden weiterhin Erfindung liegt die überraschende Erkenntnis zugrunde, dass die DNA-Replikation von E1A-modifizierten Adenoviren in YB-1 Kern-positiven Tumorzellen auf die Aktivierung des E2-late Promotors zurückzuführen ist. Unter E1A-modifizierten Adenoviren sind dabei solche Adenoviren zu verstehen, die (a) keine Replikation oder eine verglichen mit dem jeweiligen Wildtyp verringerte, bevorzugterweise stark verringerte Replikation, in YB-1-Kern-negativen Zellen zeigen, (b) transaktivierend auf mindestens ein virales Gen wirken, wobei das Gen insbesondere ausgewählt ist aus der Gruppe, die E1B-55kDa, E4orf6, E4orf3 und E3ADP umfasst, und/oder (c) zelluläres YB-1 durch den Adenovirus nicht in den Kern transloziert. Optional weisen die erfindungsgemäß verwendeten Adenoviren die weitere Eigenschaft auf, dass nämlich die Bindung des von dem Adenovirus kodierten E1A-Proteins die Bindung von E2F an RB stört bzw. den entsprechenden Komplex aus E2F und Rb aufzulösen in der Lage ist. Adenoviren, die eines oder mehrere der vorstehend genannten Merkmale a) bis c), bevorzugterweise alle Merkmale a) bis c) aufweisen, sind replikationsdefizient in Zellen, die YB-1 nicht im Kern aufweisen.

Unter einer stark verringerten Replikation hierin insbesondere eine solche Replikation verstanden, die gegenüber dem Wildtyp um den Faktor 2, bevorzugterweise um den Faktor 5, bevorzugtererweise um den Faktor 10 und am bevorzugtesten um den Faktor 100 verringert ist. In einer bevorzugten Ausführungsform ist vorgesehen, dass der Vergleich der Replikation bei Verwendung gleicher oder ähnlicher Zellinien, gleicher oder ähnlicher Virustiter für die Infektion (engl. multiplicity of infection, MOI, oder engl. plaque forming unit, pfu) und/oder gleicher oder ähnlicher allgemeiner Versuchsbedingungen durchgeführt wird. Dabei wird unter Replikation insbesondere die Partikelbildung verstanden. In weiteren Ausführungsformen kann jedoch als Mass für die Replikation der Umfang der Synthese viraler Nukleinsäure verstanden sein. Erfahren zur Bestimmung des Umfanges der Synthese viraler Nukleinsäuren sind ebenso wie Verfahren zur Bestimmung der Partikelbildung den Fachleuten auf dem Gebiet bekannt.

Die hierin beschriebenen Erkenntnisse bzw. die Verfahren, Verwendungen oder Nukleinsäuren, Proteine, Replikationssysteme und dergleichen sind nicht notwendigerweise auf Adenoviren beschränkt. Grundsätzlich gibt es auch bei anderen Viren derartige Systeme, die hiermit ebenfalls umfasst sind.

Bei Verwendung der erfindungsgemäßen Viren öder bei der erfindungsgemäßen Verwendung der hierin beschriebenen Viren kann eine dem Wildtyp nahezu vergleichbare Replikation bereits bei einer Infektionsrate von 1 bis 10 pfu/Zelle erreicht werden gegenüber 10 bis 100 pfu/Zelle gemäß dem Stand der Technik.

Unter zellulärem YB-1 soll hierin ein jedes YB-1 verstanden werden, welches von einer Zelle codiert und bevorzugterweise auch exprimiert wird, wobei dieses YB-1 in der Zelle insbesondere vor der Infektion der betreffenden Zelle mit einem Adenovirus, bevorzugterweise einem Adenovirus, und/oder einem Helfervirus, wie hierin beschrieben, vorhanden ist. Es ist jedoch auch im Rahmen der vorliegenden Erfindung, dass zelluläres YB-1 auch ein solches ist, welches erst durch exogene Maßnahmen, wie beispielsweise durch Infektion mit einem Virus, insbesondere mit einem Adenovirus, in die Zelle eingeführt bzw. von dieser produziert wird.

Ohne im folgenden darauf festgelegt sein zu wollen, geht der vorliegende Erfinder davon aus, dass der E2-early Promotor, d. h. der frühe E2-Promotor, im Rahmen der Replikation der hierin erfindungsgemäß verwendeten Viren und der erfindungsgemäßen Verwendung der erfindungsgemäßen Adenoviren nicht über den humanen zellulären E2F-Transkriptionsfaktor eingeschaltet wird. Das Einschalten der Replikation ist dabei unabhängig vom Rb-Status der Zellen, d.h. dass die Tumorzellen, die unter Anwendung der hierin offenbarten Viren infiziert und in der Folge bevorzugt lysiert werden, sowohl funktionelle wie auch inaktive Rb-Proteine besitzen können. Zudem benötigt die adenovirale Replikation unter Verwendung der hierin offenbarten Adenoviren bzw. den hierin offenbarten Bedingungen kein funktionelles p53 Protein, wird jedoch auch durch dessen Vorhandensein nicht nachteilig beeinflusst. Insoweit wendet sich die technische Lehre von dem mit der Anwendung der onkolytischen oder tumorlytischen Adenoviren vom Typ AdΔ24, d1922-947, E1Ad/01/07, CB016 oder jener Adenoviren, wie sie beispielsweise im europäischen Patent EP 0 931 830 beschrieben sind, verfolgten Prinzip ab, bei denen eine und/oder mehrere Deletion(en) im E1A-Protein vorgenommen worden war(en) unter der Annahme, dass intakte funktionelle Rb-Proteine einer effizienten Replikation in vivo entgegenwirken und somit eine adenovirale Replikation in vivo nur in Rb-negativen bzw. Rb-mutierten Zellen sicher zu stellen. Diese adenoviralen Systeme nach dem Stand der Technik gehen auf E1A zurück, um mittels des frühen E2-Promotors (E2-early Promotor) und "freiem E2F" die in vivo Replikation von Adenoviren zu steuern. Gleichwohl können diese im Stand der Technik bekannten Viren erfindungsgemäß verwendet werden, d. h. zur Replikation in Zellen, die YB-1 unabhängig vom Zellzyklus im Kern enthalten oder in Zellen, die dereguliertes YB-1 aufweisen.

Die in dem besagten europäischen Patent EP 0 931 830 beschriebenen Viren und insbesondere Adenoviren können dabei erfindungsgemäß verwendet werden. Konkret handelt es sich bei den in besagtem Patent beschriebenen Viren um solche, die eine Replikaktionsdefizienz aufweisen und denen ein exprimiertes virales Onkoprotein fehlt, das zur Bindung eines funktionellen Rb-Tumorsuppressor-Genprodukts fähig ist. Der Adenovirus kann dabei insbesondere ein solcher sein, dem exprimiertes virales E1A-Onkoprotein fehlt, das zur Bindung eines funktionellen Tumorsuppressor-Genprodukt, insbesondere Rb, fähig ist. Das virale E1A-Onkoprotein kann dabei eine inaktivierende Mutation aufweisen, beispielsweise in der CR1-Domäne an den Aminosäuren 30 bis 85 in Adenovirus Ad5, hierin auch als Ad5 bezeichnet, Ad 5, den Nukleotidpositionen 697-790, und/oder der CR2-Domäne an den Aminosäuren 120 bis 139 in Ad 5, den Nukleotidpositionen 920 bis 967, welche an der Bindung von p105 Rb Protein, p130 und p107 Protein beteiligt sind. Dabei kann auch vorgesehen sein, dass der Adenovirus vom Typ 2 dl 312 oder Adenovirus vom Typ 5 NT dl 1010 ist.

Bei der erfindungsgemäßen Verwendung von Adenoviren zur Herstellung eines Medikamentes, insbesondere zur Herstellung eines Medikamentes für die Behandlung von Tumorerkrankungen und die sonstigen hierin offenbarten Erkrankungen, und bei der erfindungsgemäßen Verwendung von Adenoviren sowie der erfindungsgemäßen Adenoviren zur Replikation in Zellen, die YB-1 im Kern aufweisen, bevorzugterweise Zellzyklus-unabhängig YB-1 im Kern aufweisen oder dereguliertes YB-1 aufweisen, bevorzugterweise im Cytoplasma, erfolgt die Replikation letzten Endes in solchen Zellen, die YB-1 im Kern, bevorzugterweise unabhängig vom Zellzyklus, aufweisen, mithin YB-1-Kern-positiv sind, oder in Zellen, die dereguliertes YB-1 aufweisen. Dabei ist besonders beachtlich, dass die Adenoviren als solche in Zellen, die YB-1 nicht im Kern, sondern im Wesentlichen nur im Cytoplasma enthalten, oder die kein dereguliertes YB-1 aufweisen, nicht oder stark verringert replizieren. Insoweit ist es erforderlich, dass für eine erfolgreiche Replikation dieser Viren YB-1 im Kern bevorzugterweise Zellzyklus-unabhängig, oder dereguliertes YB-1 vorhanden ist. Dies kann beispielsweise, wie auch im Folgenden noch ausgeführt werden wird, durch Anlegen solcher Bedingungen an die Zellen realisiert werden, dass es zur Expression oder dem Vorhandensein von YB-1 bevorzugterweise Zellzyklus-unabhängig, oder dereguliertes YB-1 im Kern oder der Expression von dereguliertem YB-1 kommt. Eine entsprechende Maßnahmen kann z. B. die Codierung bzw. Exprimierung von YB-1 durch die erfindungsgemäß verwendeten und die erfindungsgemäßen Adenoviren sein, die in Ergänzung zu den adenoviralen Genen auch eine genetische Information in sich tragen, die für YB-1 und insbesondere dessen Expression codiert. Andere Maßnahmen, die zum Transport, zur Induktion oder Expression von YB-1 im Kern der Zelle führen, sind das Anlegen von Stressbedingungen wie beispielsweise die Applikation von Zytostatika, Bestrahlung, Hyperthermie und dergleichen an die Zelle bzw. den eine - solche - Zelle enthaltenden Organismus. In einer bevorzugten Ausführungsform ist die Bestrahlung eine Bestrahlung, wie sie beispielsweise bei der Behandlung von Tumorerkrankungen verwendet wird.

Die im Rahmen der vorliegenden Erfindung, insbesondere zur Tumorlyse, verwendeten Adenoviren sowie der erfindungsgemäßen Adenoviren zeichnen sich in bevorzugten Ausführungsformen weiterhin dadurch aus, dass sie in solchen Zellen, die YB-1 im Kern nicht Zellzyklus-unabhängig aufweisen, mithin YB-1-Kern-negativ sind oder die kein dereguliertes YB-1 aufweisen, nicht replizieren.

Ein weiteres Merkmal eines Teils der erfindungsgemäß zu verwendenden Adenoviren, die verschieden sind von den erfindungsgemäßen Adenoviren, besteht darin, dass sie für ein virales Onkoprotein, das hierin auch also Onkogenprotein bezeichnet wird, codieren, wobei es sich bei dem Onkogenprotein bevorzugter Weise um E1A handelt, wobei das Onkogenprotein in der Lage ist, zumindest ein virales Gen zu aktivieren, welches einen Einfluss auf die Replikation des Virus und/oder die Zellyse der von dem Virus infizierten Zelle haben kann. Dabei ist bevorzugt, dass der Einfluss auf die Replikation dergestalt ist, das der Virus bei Vorhandensein des Onkogenproteins besser repliziert als bei Fehlen des Onkogenproteins des jeweilige Virus. Dieser Vorgang wird hierin auch als transaktivierend bezeichnet und insbesondere als E1A-transaktivierend bezeichnet, wenn die Transaktivierung von E1A vermittelt wird. Die Bezeichnung "transaktivieren" oder "Transaktivierung" beschreibt dabei bevorzugt den Vorgang, dass das in Frage stehende virale Onkoprotein auf die Expression und/oder auf die Transkription eines anderen oder mehrerer anderer Gene als das für das virale Onkoprotein selbst codierende Gen Einfluss nimmt, d. h. bevorzugterweise dessen Expression und/oder Translation steuert, und diese(s) insbesondere aktiviert. Derartige virale Gene sind bevorzugter Weise E1B55kDa, E4orf6, E4orf3 und E3ADP sowie beliebige Kombinationen der vorstehend genannten Gene bzw. Genprodukte.

Ein weiteres, wenngleich bevorzugterweise optionales, Merkmal der erfindungsgemäß zu verwendenden Adenoviren ebenso wie der erfindungsgemäßen Adenoviren ist deren Bindungsverhalten bzw. von bestimmten der von ihnen codierten Proteine zu bzw. mit dem Tumor-Suppressor Rb. Es ist grundsätzlich im Rahmen der vorliegenden Erfindung, dass die erfindungsgemäß verwendeten Adenoviren an Rb binden oder nicht binden können. Die Verwendung der beiden alternativen Ausgestaltungsformen der Adenoviren ist dabei unabhängig vom Rb-Status der behandelten oder zu behandelnden Zelle möglich.

Um E1A die Fähigkeit zu verleihen, nicht an Rb binden zu können, sind beispielsweise folgende Deletionen am E1A-Onkoprotein möglich: Deletion in der CR1-Region (Aminosäurepositionen 30-85 in Ad5) und Deletion der CR2-Region (Aminosäurepositionen 120-139 in Ad5). Dabei bleibt die CR3-Region erhalten und kann ihre transaktivierende Funktion auf die anderen frühen viralen Gene ausüben.

Um E1A die Fähigkeit zu verleihen, an Rb binden zu können, sind dagegen folgende Deletionen am E1A-Onkoprotein grundsätzlich möglich: Deletion der CR3-Region (Aminosäurepositionen 140-185); Deletion des N-Terminus (Aminosäurepositionen 1-29); Deletion der Aminosäurepositionen 85-119; und Deletion des C-Terminus (Aminosäurepositionen 186-289). Die hier aufgeführten Bereiche interferieren nicht mit der Bindung von E2F an Rb. Die transaktivierende Funktion bleibt erhalten, ist jedoch gegenüber vom Wildtyp-Ad5 verringert.

Es ist auch im Rahmen der vorliegenden Erfindung, insbesondere betreffend die erfindungsgemäßen Adenoviren, dass das E1A-Protein, insbesondere das E1A12S-Protein so ausgebildet ist, dass es in einer Ausführungsform an Rb zu binden in der Lage ist und in einer anderen Ausführungsform nicht in der Lage ist an Rb zu binden, wobei ein derartiges E1A12 S-Protein ein E1A-Protein und insbesondere ein E1A12S-Protein im Sinne der vorliegenden Erfindung ist, das gleichwohl im Stand der Technik gelegentlich auch als modifiziertes E1A12S bezeichnet wird. Die entsprechende Ausprägung des E1A12S-Proteins ist dabei im Rahmen des Könnens der Fachleute auf dem Gebiet, insbesondere mit Blick auf die vorstehend aufgezeigten Deletionen des E1A-Proteins, hierin auch einfach als E1A bezeichnet.

Derartige im Stand der Technik grundsätzlich bereits bekannte Viren, die keine Transaktivierung zeigen, gelten allgemein als replikationsdefizient. Es ist jedoch das Verdienst des vorliegenden Erfindes, erkannt zu haben, dass sie dennoch zur Replikation in einem geeigneten Hintergrund, insbesondere einem zellulären Hintergrund geeignet sind. Ein derartiger geeigneter zellulärer Hintergrund wird durch das Vorhandensein von YB-1 im Kern, bevorzugterweise eine Zellzyklus-unabhängige Anwesenheit von YB-1 im Kern, oder durch dereguliertes YB-1 bedingt oder bereitgestellt. Der Begriff der Zellen oder zellulären Systeme, wie hierin im Zusammenhang mit einem jeglichen Aspekt der vorliegenden Erfindung verwendet, umfasst dabei Fragmente oder Fraktionen von Zellaufschlüssen ebenso wie Zellen, die in vitro, in vivo oder in situ vorliegen. Insoweit umfasst der Begriff zelluläre Systeme oder Zellen auch solche Zellen, die in einer Zellkultur, Gewebekultur, Organkultur oder in einem Gewebe oder Organ in vivo bzw. in situ, isoliert, in Gruppen oder als Teil von Geweben, Organen oder Organismen oder aber auch als solches in einem bevorzugterweise lebenden Organismus vorliegen. Bei dem Organismus handelt es sich bevorzugter Weise um einen Wirbeltier-Organismus und bevorzugterer Weise um ein Säugetier. Besonders bevorzugterweise ist der Organismus dabei ein menschlicher Organismus. Weitere bevorzugte Organismen sind dabei jene, die im Zusammenhang mit den verschiedenen Aspekten der vorliegenden Erfindung hierin offenbart sind.

Darüber hinaus ist es im Rahmen der vorliegenden Beschreibung dass auf der Grundlage der hierin gegebenen technischen Lehre neue Viren erzeugt werden, die das Replikationsverhalten der hierin beschriebenen und im Stand der Technik bekannten Adenoviren in solchen Zellen zeigen, die YB-1-Kern-positiv, bevorzugt Zellzyklus-unabhängig YB-1-Kern-positiv sind oder dereguliertes YB-1 aufweisen. Mit anderen Worten, insbesondere bevorzugterweise ausgehend von den bereits bekannten Adenoviren können weitere Viren konzipiert werden, die die hierin definierten, für die erfindungsgemäße Verwendung erforderlichen Merkmale aufweisen.

Im Zusammenhang mit der vorliegenden Erfindung ist das modifizierte E1A-Onkoprotein der verschiedenen, erfindungsgemäß zu verwendenden Adenoviren im Gegensatz zu den hierin beschriebenen erfindungsgemäßen Viren in der Lage transaktivierend auf die frühen virale Gene, wie beispielsweise E1BSSK, E4orf3, E4orf6, E3ADP, in YB-1 kernpositiven Zellen oder Zellen, die dereguliertes YB-1 aufweisen, zu wirken. Dabei liegt sonst bevorzugterweise keine Veränderung im viralen Genom vor und der entsprechende Adenovirus kann insoweit ansonsten einen Adenoviren vom Wildtyp oder Abwandlung davon entsprechen.

Zu den hierin offenbarten Viren, die für ein transaktivierendes Onkogenprotein im Sinne der vorliegenden Erfindung codieren oder ein solches umfassen, gehören beispielsweise die Adenoviren AdeΔ24, dl922-947, E1Ad/01/07, CB106 und/oder die im europäischen Patent EP 0931 830 beschriebenen Adenoviren, die jeweils in der Lage sind, transaktivierend auf die frühen Gene z.B. E1B, E2, E3 und/oder E4 zu wirken, und ist vergleichbar mit Adenovirus vom Wildtyp, insbesondere vom Wildtyp Ad5 aufweisen. Verantwortlich für die Transaktivierung ist in diesen Fällen ein bestimmter Bereich des E1A-Proteins. Innerhalb von verschiedenen Adenovirus-Serotypen kommen 3 hoch konservierte Bereiche im E1A Protein vor. Der Bereich CR1 von 41-80 AS, CR2 von 120-139 und CR3 von 140-188 AS. Die transaktivierende Funktion beruht hauptsächlich auf das Vorhandensein der CR3-Region im E1A Protein. Die AS-Sequenz von CR3 liegt in den oben genannten Adenoviren unverändert vor. Dies führt dazu, dass die Transaktivierung der frühen Gene E1B, E2, E3 und E4 unabhängig davon erfolgt, ob YB-1 im Kern oder im Zytoplasma vorhanden ist.

Im rekombinanten Adenovirus dl520 ist dahingegen der CR3-Bereich deletiert worden. Damit exprimiert dl520 ein sogenanntes E1A12S Protein, welches nicht die AS-Sequenz der CR3-Region aufweist. Dies führt dazu, dass dl520 nur eine ganz schwache transaktivierende Funktion ausüben kann, insbesondere auf die E2-Region, und somit in YB-1 Kern-negativen Zellen nicht repliziert. In YB-1 Kern-positiven Zellen übernimmt YB-1 die Transaktivierung der E2-Region, und ermöglicht somit eine effziente Replikation dl520. Darauf beruht die Verwendung von Systemen wie dl520 bzw. auf der Grundlage von dl520 zu den hierin offenbarten Zwecken. Ein weiterer wichtiger Unterschied zwischen beiden vorstehend beschriebenen Gruppen von Adenoviren, d. h. beispielsweise delta 24 (hierin auch als AdΔ24 bezeichnet) und beispielsweise dl520 besteht darin, dass bei dl520 die frühen Gene E1B, E3 und E4 stärker in Zellzyklusunabhängigen YB-1 Kern-positiven Zellen oder Zellen, die dereguliertes YB-1 aufweisen, als in YB-1 Kern-negativen Zellen oder Zellen, die kein dereguliertes YB-1 aufweisen, transaktiviert werden. Bei delta 24 bestehen hingegen keine bzw. nur geringfügige Unterschiede. Die Transaktivierung von dl520 bzw. genauer des E1A12S Proteins ist allerdings erheblich schwächer im Vergleich zum Wildtyp-Adenovirus. Diese Transaktivierung reicht allerdings aus, um eine effiziente Replikation in YB-1 kernpositiven Zellen durchzufiihren, wie dies auch in Beispiel 10 gezeigt ist. Die hierin und speziell in diesem Zusammenhang beschriebene Ausgestaltung des EMA-Proteins bzw. der dafür codierenden Nukleinsäure in der Form, dass das E1A-Protein gegenüber dem Wildtyp-Onlcogenprotein E1A eine oder mehrere Deletionen und/oder Mutationen aufweist, wobei die Deletion bevorzugt eine solche ist, die ausgewählt ist aus der Gruppe, die Deletionen des Bereiches CR3 und Deletionen des N-Terminus und Deletionen des C-Terminus umfasst, einschließlich und besonders bevorzugt jener Ausgestaltungen des E1A-Proteins wie im Zusammenhang mit dl520 oder AdΔ24, dl922 bis 947, E1Ad/01/07, CB106 und/oder die im europäischen Patent EP 0 931 830 beschriebenen Adenoviren, stellen Ausführungsformen von Viren, insbesondere von Adenoviren dar, deren Replikation durch YB-1 über die Aktivierung des E2-late-Promotors gesteuert wird, bevorzugterweise überwiegend über die Aktivierung des E2-late-Promotors. Weitere Ausgestaltungen des E1A-Proteins, die diese Form der Replikation von Adenoviren erlauben, können auf der Grundlage der hierin gemachten Offenbarung von den Fachleuten auf dem Gebiet hergestellt werden. Die vorstehend beschriebene Ausgestaltung des E1A-Proteins ist eine solche, wie sie auch im Zusammenhang mit den erfindungsgemäßen Adenoviren, die hierin auch als erfindungsgemäße Adenoviren oder Adenoviren der Gruppe I bezeichnet werden, verwendet werden kann.

Bei erfindungsgemäßen Adenoviren, insbesondere den Adenoviren der Gruppe I, die hierin auch als Abwandlungen bezeichnet werden und die erfindungsgemäß verwendet werden können, liegt typischerweise eine E1-Deletion, eine E1/E3-Deletion und/oder eine E4-Deletion vor, d. h. die ensprechenden Adenoviren sind nicht in der Lage, funktional aktive E1- und/oder E3- und/oder E4-Expressionsprodukte bzw. entsprechende Produkte zu erzeugen, oder mit anderen Worten, diese Adenoviren sind lediglich in der Lage, funktional inaktive E1-, E3- und/oder E4-Expressionsprodukt zu erzeugen, wobei ein funktional inaktives E1-, E3- und/oder E4-Expressionsprodukt ein solches ist, welches entweder gar nicht als Expressionsprodukt, sei es auf der Ebene der Transkription und/oder der Translation, oder in einer Form vorliegt, bei der es zumindest eine der ihm im Adenovirus vom Wildtyp zukommende Funktion nicht aufweist. Diese dem Expressionsprodukt im Adenovirus vom Wildtyp zukommenden Funktion(en) ist/sind den Fachleuten auf dem Gebiet bekannt und beispielsweise beschrieben in Russell, W. C., Journal ofVirology, 81,2573-2604,2000. Russell (a.a.O.) beschreibt im übrigen auch Prinzipien der Konstruktion von Adenoviren und adenoviralen Vektoren. Es ist auch im Rahmen der vorliegenden Erfindung, dass das modifizierte E1A-Onkoprotein, d. h. das nicht mehr transaktivierende E1A-Protein wie beispielsweise E1A12S, E1B-5SK, E4orf6 und/oder E3ADP (adenoviral death protein (ADP)) (Tollefson, A. et al., J. Virology, 70, 2296-2306, 1996.) in einem solchen Vektor einzeln oder in beliebiger Kombination zur Expression gebracht wird/werden. Dabei können die einzelnen genannten Gene ebenso wie die hierin offenbarten Transgene unabhängig von einander entweder in der E1 und /oder E3 und/oder E4-Region einkloniert und mit Hilfe eines geeigneten Promoters oder unter Kontrolle eines geeigneten Promotors zur Expression gebracht werden. Grundsätzlich sind die Regionen E1, E3 und E4 als Klonierungsstellen innerhalb der adenoviralen Nukleinsäure gleichermaßen geeignet, wobei die für die Klonierung nicht benutzten Regionen entweder vorhanden, teilweise und/oder ganz deletiert werden können. Für den Fall, dass diese Regionen vorhanden sind, insbesondere vollständig vorhanden sind, ist es im Rahmen der vorliegneden Erfindung, dass diese entweder intakt ist und bevorzugterweise ein Translationsprodukt und/oder ein Transkriptionsprodukt liefert, und/oder nicht intakt ist und bevorzugterweise kein Translationsprodukt und/oder Transkriptionsprodukt liefert. Geeignete Promotoren sind in Ausführungsformen jene, wie sie hierin im Zusammenhang mit der Steuerung bzw. Expression von E1A, insbesondere des modifizierten E1A, offenbart sind.

Schließlich ist vorgesehen, dass die verwendeten Adenoviren der Gruppe II hinsichtlich E1B defizient, insbesondere hinsichtlich E1B 19 kDadefizient sind. Dabei wird hierin allgemein unter dem Begriff defizient ein Zustand verstanden, bei dem E1B die im Wildtyp inhärente Gesamtheit der Eigenschaften nicht aufweist und mindestens eine dieser Eigenschaften fehlen.

Das Adenovirus BCL2-Homolog E1B19k verhindert die E1A induzierte Apoptose durch Interaktion mit den pro-apoptotischen Proteinen Bak und Bax. Dadurch wir eine maximale Replikation und/oder Partikelbildung in infizierten Zellen ermöglicht (Ramya Sundararajan und Eileen White, Journal of Virology 2001, 75, 7506-7516). Das Fehlen von E1B 19k führt zur besseren Freisetzung der Viren, da es falls vorhanden vermutlich die Funktion des adenoviralen Death-Proteins minimiert. Durch eine solche Deletion wird der Virus induzierte cytopathische Effekt erhöht (Ta-Chiang Liu et al., Molecular Therapy, 2004) und führt somit zur stärkeren Lyse von infizierten Tumorzellen. Zudem bewirkt das Fehlen von E1B19k, dass TNF-alpha auf die Replikation von solchen Adenoviren in Tumorzellen keinen Einfluß ausübt, währen in normalen Zellen die Behandlung zu einer geringeren Replikation und Freisetzung infektiöser Viren führt. Somit wird die Selektivität und Spezifität erhöht (Ta-Chiang Liu et al., Molecular Therapy, 2004, 9, 786-803).

Die Adenoviren der Gruppe II, sind grundsätzlich im Stand der Technik zumindest in einigen Ausführungsformen bekannt. Bei diesen erfindungsgemäß verwendeten Adenoviren handelt es sich bevorzugter Weise um rekombinante Adenoviren, insbesondere auch dann, wenn eine Veränderung gegenüber dem Wildtyp vorgenommen wurde im Sinne der hierin gegebenen technischen Lehre. Es ist im Rahmen der Kenntnisse der Fachleute auf diesem Gebiet, für die Erfindung unwesentliche adenoviralen Nukleinsäuresequenzen zu deletieren bzw. zu mutieren. Derartige Deletionen können z. B die für einen Teil der E3 und E4 codierende Nukleinsäure betreffen wie hierin auch beschrieben. Bei einer Deletion von E4 ist dabei besonders bevorzugt, wenn sich diese nicht auf das Protein E4orf6 erstreckt, mithin der erfindungsgemäß zu verwendende Adenovirus E4orf6 codiert. In bevorzugten Ausführungsformen können diese adenoviralen Nukleinsäuren noch in das virale Kapsid verpackt werden und damit infektiöse Partikel ausbilden. Gleiches gilt für die erfindungsgemäße Verwendung der Nukleinsäuren. Generell gilt es auch noch festzuhalten, dass die adenoviralen Systeme hinsichtlich einzelner oder mehrerer Expressionsprodukte defizient sein können. Dabei ist zu berücksichtigen, dass dies sowohl im Zusammenhang mit den Adenoviren der Gruppe I als auch den Adenoviren der Gruppe II zum einen darauf beruhen kann, dass die für das Expressionsprodukt codierende Nukleinsäure vollständig oder in dem Maße mutiert oder deletiert ist, dass im wesentlichen kein Expressionsprodukt mehr gebildet wird, oder darauf, dass regulative bzw. die Expression steuernde Elemente wie Promotoren oder Transkriptionsfaktoren fehlen oder anders als im Wildtyp aktiv sind, sei es auf der Ebene der Nukleinsäure (Fehlen eines Promotors; cis-wirkende Element) oder auf der Ebene des Translations- bzw. Transkriptionssystems (trans-wirkende Elemente). Gerade der letztere Aspekt kann dabei vom jeweiligen zellulären Hintergrund abhängen.

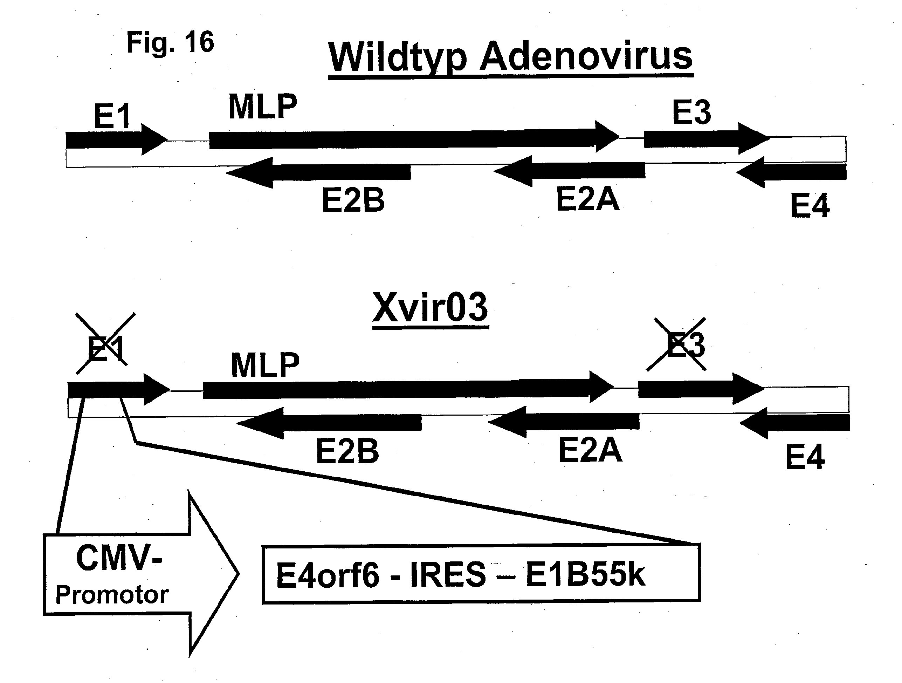

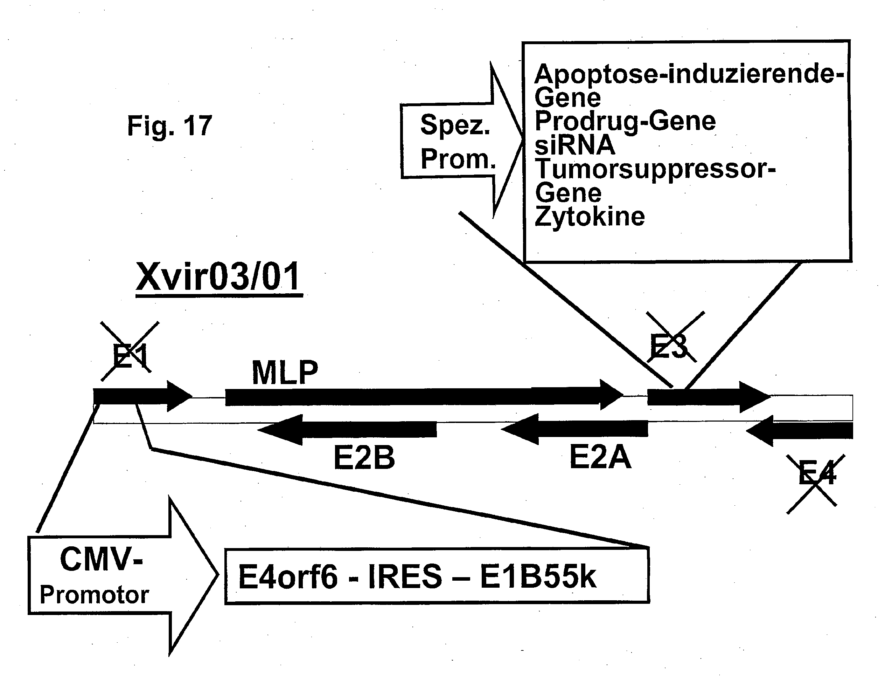

Neben der Verwendung von an und für sich bereits bekannten Adenoviren gemäß der vorliegenden Erfindung können auch neue Adenoviren wie die Adenoviren der Gruppe II in dem Umfang verwendet werden, wie dies für die anderen hierin beschriebenen Adenoviren bereits offenbart ist. Die neuen Adenoviren ergeben sich aus der hierin offenbarten technischen Lehre. Besonders bevorzugte Vertreter sind dabei beispielsweise die hierin in Fig. 16 und Fig. 17 dargestellten Viren Xvir03 und Xvir03/01, deren Konstruktionsprinzip auch in den Beispielen 11 und 12 weiter veranschaulicht ist.

Im Falle des Vektors Xvir03 wurde in der E1-Region ein CMV-Promotor kloniert, der die durch eine IRES-Sequenz getrennten Nukleinsäuren für E1B 55k und E4ORF6 codiert. Dabei kann die E3- und E4-Region deletiert und/oder Intakt vorhanden sein. Infolge Einklonierung dieser beiden Gene bzw. der daraus hergestellten Genprodukte kommt es zu einer hohen Replikationseffizienz, wobei die Selektivität der Replikation in Zellen, insbesondere Tumorzellen, insoweit beibehalten wird, als dass eine Replikation, insbesondere in YB-1-Kern-positiven Zellen und insbesondere in solchen Zellen erfolgt, bei denen YB-1 dereguliert im Sinne der vorliegenden Offenbarung vorliegt. Zellen, in denen YB-1 dereguliert vorliegt, sind dabei in einer Ausführungsform solche, die eine erhöhte Expression von YB-1, bevorzugt Kompartiment-unabhängig, aufweisen verglichen mit normalen oder Nicht-Tumorzellen. Das Einklonieren von E1B 55k und E4orf6 kann aber auch in der E4-Region erfolgen, wobei die E3-Region intakt oder/und deletiert werden kann.

Eine Weiterentwicklung des Virus Xvir03 stellt der Virus Xvir03/01 dar, bei dem unter der Kontrolle eines spezifischen Promotors, insbesondere eines Tumor- oder Gewebe-spezifischen Promotors, therapeutische Gene oder Transgene in einer bevorzugten Ausführungsform einkloniert sind. Dabei kann die E3- und E4-Region deletiert und/oder Intakt vorhanden sein.Weiterhin ist es im Rahmen eines derartigen Virus, dass auch die Region E4 funktional inaktiv ist, bevorzugterweise deletiert ist. Die hierin beschriebenen Transgene können dabei auch in die E4-Region kloniert werden, wobei dies alternativ oder ergänzend zum Klonieren der Transgene in die E3-Region erfolgen kann.

Die hierin und insbesondere im Folgenden beschriebenen Transgene können im übrigen auch im Zusammenhang mit den oder durch die erfindungsgemäßen Adenoviren, d. h. den Adenoviren der Gruppe I, bzw. deren Nukleinsäuren, oder Replikationssystemen zur Expression gebracht werden und sind damit im Zusammenhang mit einer Expressionskassette umfassend einen Promotor und eine Nukleinsäuresequenz, eine derartige Nukleinsäuresequenz, wobei diese für ein oder mehrere der besagten Transgene codiert umfasst. Als Klonierungsort im adenoviralen Genom eignet sich besonders die E1-, E3- und/oder die E4-Region, ist aber nicht darauf beschränkt. Transgene, wie hierein verwendet, können virale Gene, bevorzugterweise adenovirale Gene sein, die bevorzugterweise im Wildtyp-Adenovirus nicht im Genom bzw. an der Stelle im Genom vorkommen, an der sie in dem konkreten Virus nun vorliegen, oder therapeutische Gene.

Therapeutische Gene können dabei Prodrug-Gene, Gene für Zytokine, Apoptose-induzierende Gene, Tumorsuppressorgene, Gene für Metalloproteinasen-Inhibitoren und/oder Angiogenese-Inhibitoren, Tyrisinkinase-Inhibitoren sein. Ferner können siRNA, Aptameren, Antisense und Ribozyme exprimiert werden, die bevorzugterweise gegen Krebs-relevante Zielmoleküle gerichtet sind. Bevorzugterweise ist das einzelne oder die mehreren Zielmoleküle aus der Gruppe ausgewählt, -die Resistenz-relevante Faktoren, Anti-Apoptose-Faktoren, Onkogene, Angiogenese-Faktoren, DNA-Synthese-Enzyme, DNA-Reperaturenzyme, Wachstumsfaktoren und deren Rezeptoren, Transkriptionsfaktoren, Metalloproteinasen, insbesondere Matrix-Metalloproteinasen, und Plasminogenaktivator vom Urokinase-Typ umfasst. Bevorzugte Ausführungsformen davon sind solche, die hierin im Zusammenhang mit anderen Aspekten der Erfindung bereits offenbart sind.

Mögliche Prodrug-Gene, wie sie in bevorzugten Ausführungsformen verwendet werden können, sind beispielsweise *Cytosine deaminase, Thymidin kinase, Carboxypeptidase, Uracil phosphoribosyltransferase*; oder *Purin Nukleosid Phosphorylase (PNP)*; [Kirn et al, Trends in Molecular Medicine, Volume 8, No.4 (Suppl), 2002; Wybranietz W.A. et al., Gene Therapy, 8, 1654-1664, 2001; Niculescu-Duvaz et al., Curr. Opin. Mol. Therapy, 1, 480.486, 1999; Koyama et al., Cancer Gene Therapy, 7, 1015-1022, 2000; Rogers et al., Human Gene Therapy, 7, 2235-2245, 1996; Lockett et al., Clinical Cancer Res., 3, 2075-2080, 1997; Vijayakrishna et al., J. Pharmacol. And Exp. Therapeutics, 304, 1280-1284, 2003].

Mögliche Zytokine, wie sie in bevorzugten Ausführungsformen verwendet werden können, sind beispielsweise *GM-CSF, TNF-alpha, Il-12, Il-2, Il-6, CSF, oder Interferon-Gamma*; [Gene Therapy, Advances in Pharmacology, Volume 40, Editor: J. Thomas August, Academic Press; Zhang und Degroot, Endocrinology, 144, 1393-1398,2003; Descamps et al., J. Mol. Med., 74, 183-189, 1996; Majumdar et al., Cancer Gene Therapy, 7, 1086-1099, 2000].

Mögliche Apoptose-induzierende Gene, wie sie in bevorzugten Ausführungsformen verwendet werden können, sind beispielsweise Decorin [Tralhao et al., FASEB J, 17, 464-466, 2003]; Retinoblastoma 94 [Zhang et al., Cancer Res.,63, 760-765, 2003]; Bax und Bad [Zhang et al, Hum. Gene Ther., 20, 2051-2064,2002]; Apoptin [Noteborn und Pietersen, Adv. Exp. Med. Biol., 465, 153-161, 2000]; ADP [Toth et al., Cancer Gene Therapy, 10, 193-200, 2003]; bcl-xs [Sumantran et al., Cancer Res, 55, 2507-2512, 1995]; E4orf4 [Braithwaite und Russell, Apoptosis, 6, 359-370,2001]; FasL, Apo-1 und Trail [Boehringer Manheim, Guide to Apoptotic Pathways, Arai et al., PNAC, 94, 13862-13867, 1997]; Bims [Yamaguchi et al., Gene Therapy, 10, 375-385,2003; GNR163: Oncology News, 17 Juni, 2000].

Mögliche Tumorsuppressor-Gene, wie sie in bevorzugten Ausführungsformen verwendet werden können, sind beispielsweise *E1A, p53, p16, p21, p27* oder *MDA-7* [Opalka et al., Cell Tissues Organs, 172, 126-132, 2002 , Ji et al., Cancer Res., 59, 3333-3339, 1999, Su et al., Oncogene, 22,1164-1180,2003].

Mögliche Angiogenese-Inhibitoren, wie sie in bevorzugten Ausführungsformen verwendet werden können, sind beispielsweise *Endostatin* oder *angiostatin* [Hajitou et al., FASEB J., 16, 1802-1804,2002], und Antikörper gegen VEGF [Ferrara, N., Semin Oncol 2002 Dec; 29 (6 Suppl 16): 10-4].

Mögliche Metalloproteinase-Inhibitoren, wie sie in bevorzugten Ausführungsformen verwendet werden können, sind beispielsweise *Timp-3* [Ahonen et al., Mol Therapy, 5, 705-715, 2002]; *PAI-1* [Soff et al., J. Clin. Invest., 96, 2593-2600, 1995]; *Timp-1* [Brandt K. Curr. Gene Therapy, 2,255-271,2002].

Weitere Transgene die sowohl von den Adenoviren der Gruppe I als auch der Gruppe II exprimiert werden können, sind auch Tyrosinkinase-Inhibitoren. Beispielhafte Tyrosinkinasen sind dabei der EGFR (epidermal growth factor receptor) [Onkologie, Entstehung und Progression maligner Tumoren; Verfasser: Christoph Wagner, Georg Thieme Verlag, Stuttgart, 1999]. Ein bevorzugter Tyrosinkinase-Inhbitor is Herceptin [Zhang H et al., Cancer Biol Ther. 2003, Jul-Aug; 2 (4 Suppl 1): S122-6].

siRNA (short interfering RNA), wie sie im Rahmen der vorliegenden Erfindunge verwendet werden kann, besteht aus zwei, bevorzugt zwei getrennten RNA-Strängen, die infolge Basenkomplementarität miteinander hybridisieren, d.h. im wesentlichen basengepaart vorliegen, und weist bevozugterweise eine Länge von bis zu 50 Nukleotiden auf, bevorzugterweise zwischen 18 und 30 Nukleotiden, bevorzugtererweise weiniger als 25 Nukleotide und am bevorzugtesten 21, 22 oder 23 Nukleotide, wobei sich diese Zahlenwerte auf einen Einzelstrang der siRNA, insbesondere auf die Länge des Bereiches eines Einzelstranges, der mit einem, genauer dem zweiten Einzelstrang hybridisiert bzw. basenpaart, beziehen. siRNA induziert oder vermittelt spezifisch den Abbau von mRNA. Die dazu erforderliche Spezifität wird durch die Sequenz der siRNA und damit ihren Bindungsort vermittelt. Die abzubauende Zielsequenz ist dabei im wesentlichen komplementär zu dem ersten oder zu dem zweiten die siRNA aufbauenden Strang. Obwohl die genauen Wirkmechanismen noch unklar sind, wird vermutet, dass siRNA für Zellen eine biologische Strategie darstellt, während der Entwicklung bestimmte Allele zu hemmen und sich vor Viren zu schützen. Die durch siRNA vermittelte RNA-Interferenz wird als Verfahren zur spezifischen Unterdrückung oder gar völligen Ausschaltung der Expression eines Proteins durch Einbringen einer genspezifischen doppelsträngigen RNA benutzt. Für höhere Organismen ist eine 19 bis 23 Nukleotid lange siRNA deshalb besonders geeignet, weil sie nicht zur Aktivierung der unspezifischen Abwehrreaktion, zur sogenannten Interleukin-Antwort führt. Die direkte Transfektion von doppelsträngiger RNA aus 21 Nukleotiden mit symmetrischen 2-nt 3' Überhängen konnte eine RNA-Interferenz in Säugetierzellen vermitteln und zeigte im Vergleich zu anderen Technologien wie Ribozymen und Antisense-Molekülen eine hohe Effizienz (Elbashir, S. Harborth J. Lendeckel W. Yalvcin, A. Weber K Tuschl T: Duplexes of 21-nucleotide RNAs mediate RNA interference in cultured mammalian cells. Nature 2001, 411: 494-498). Nur wenige siRNA-Moleküle genügten, um die Expression des Zielgens zu unterdrücke. Um die Limitierungen exogen zugeführter siRNA, die insbesondere in der transienten Natur des Interferenz-Phänomens und der spezifischen Abgabe (engl. delivery) der siRNA-Moleküle liegen, zu umgehen, werden im Stand der Technik auch Vektoren eingesetzt, die eine endogene siRNA-Expression erlauben. Hierfür werden beispielsweise Oligonukleotide mit einer Länge von 64 Nukleotiden, die die 19 Nukleotide lange Zielsequenz einschließen, sowohl in sense- als auch in antisense- Orientierung, getrennt durch eine beispielsweise 9 Nukleotide lange Spacer-Sequenz, in den Vektor eingebaut. Das resultierende Transkript faltet sich zu einer Haarnadelstruktur mit einer Stammstruktur (engl.: stem) von beispielsweise 19 Basenpaaren. In der Zelle wird die Schleife rasch abgespaltet, so daß eine funktionelle siRNA entsteht (Brummelkamp et al., Science, 296, 550-553,2002)

Die für YB-1 codierende Nukleinsäure, die in einer Ausführungsform der erfindungsgemäß zu verwendenden Adenoviren, insbesondere der Gruppe II, aber auch der erfindungsgemäßen Adenoviren, d. h. der Adenoviren der Gruppe I, Bestandteil der Adenoviren sein kann, kann eine einen Kerntransport von YB-1 vermittelnde Nukleinsäuresequenz umfassen. Als Adenoviren bzw. adenovirale Systeme und damit die entsprechenden Nukleinsäuren können im Zusammenhang damit und in Kombination mit diesen erfindungsgemäßen Nukleinsäuren die erfindungsgemäßen Nukleinsäuren, Adenoviren und adenoviralen Systeme sowie die im Stand der Technik bekannten Adenoviren wie beispielsweise Onyx-015, AdΔ24, dl922-947, E1Ad/01/07, CB016, dl 520 und die im Patent EP 0931 830 beschriebenen Adenoviren verwendet werden. Geeignete, den Kerntransport vermittelnde Nukleinsäuresequenzen sind den Fachleuten auf dem Gebiet bekannt und beispielsweise beschrieben in (Whittaker, G.R. et al., Virology, 246, 1-23, 1998; Friedberg, E.C., TIBS 17, 347, 1992; Jans, D.A. et al., Bioessays 2000 Jun; 22(6): 532-44; Yoneda, Y., J. Biocehm. (Tokyo) 1997 May; 121(5): 811-7; Boulikas, T., Crit. Rev. Eukaryot. Gene Expr. 1993; 3(3): 193-227; Lyons RH, Mol. Cell Biol., 7, 2451-2456, 1987). Bei den Kerntransport vermittelnden Nukleinsäuresequenzen können verschiedene Prinzipien verwendet werden. Ein derartiges Prinzip besteht beispielsweise darin, dass das YB-1 als Fusionsprotein mit einem Signalpeptid ausgebildet oder versehen wird und infolge des Signalpeptids YB-1 in den Zellkern geschleust wird und damit die erfindungsgemäße Replikation der Adenoviren erfolgt.

Ein weiteres Prinzip, welches bei der Ausgestaltung der erfindungsgemäß verwendeten Adenoviren, insbesondere der Gruppe II, aber auch der erfindungsgemäßen Adenoviren, d. h. der Adenoviren der Gruppe I, zur Anwendung gelangen kann, besteht darin, dass YB-1 mit einer Transportsequenz versehen wird, die dazu führt, dass YB-1, bevorzugter Weise ausgehend von einer Synthese im Cytoplasma, in den Zellkern geschleust oder transloziert wird und dort die virale Replikation befördert. Ein Beispiel für eine besonders wirksame, den Kerntransport vermittelnde Nukleinsäuresequenz stellt die TAT-Sequenz von HIV dar, die neben weiteren geeigneten derartigen Nukleinsäuresequenzen beispielsweise beschrieben ist in Efthymiadis, A., Briggs, LJ, Jans, DA., JBC 273, 1623-1628, 1998. Dabei ist es Rahmen der vorliegenden Erfindung, dass die erfindungsgemäß verwendeten Adenoviren, insbesondere der Gruppe II, aber auch der erfindungsgemäßen Adenoviren, d. h. der Adenoviren der Gruppe I, die Nukleinsäuresequenzen umfassen, die für die den Kerntransport codierenden Peptide codieren.

Es ist im Rahmen der vorliegenden Erfindung, dass YB-1 in vollständiger Länge vorliegt, insbesondere in einer Form, die dem Wildtyp von YB-1 entspricht. Es ist weiterhin im Rahmen der vorliegenden Erfindung, dass YB-1 als Derivat, zum Beispiel, in verkürzter oder trunkierter Form verwendet wird oder vorliegt. Ein YB-1-Derivat, wie es im Rahmen der vorliegenden Erfindung verwendet werden kann oder vorliegt, ist dabei ein solches, das bevorzugterweise an den E2 late-Promotor zu binden in der Lage ist und dadurch die Genexpression von der adenoviralen, E2-Region aktiviert. Derartige Derivate umfassen insbesondere die hierin offenbarten YB-1-Derivate. Weitere Derivate können durch Deletion einzelner oder mehrerer Aminosäuren am N-Terminus, am C-Termirms oder innerhalb der Aminosäuresequenz erzeugt werden. Es ist im Rahmen der vorliegenden Beschreibung, dass auch YB-1-Fragmente als YB-1-Proteine im Sinne der vorliegenden Erfindung verwendet werden. In der Publikation von Jürchott K et al. [JBC 2003, 278, 27988-27996] werden verschiedene YB-1-Fragmente offenbart, die sich durch Deletionen am C- und am N-Terminus auszeichnen. Die Verteilung der verschiedenen YB-1-Fragemente hat ergeben, dass sowohl die cold-shock-Domäne (CSD) wie auch der C-Terminus für den Zellzyklus-regulierten Transport von YB-1 in den Zellkern von Bedeutung ist. Es ist daher im Rahmen der vorliegenden Erfindung, dass ein verkürztes YB-1 (hierin auch als YB-1-Protein bezeichnet) in Verbindung mit der erfindungsgemäßen Expression von E1B55k und E4orf6 besser in den Kern wandert und somit eine stärkere CPE induziert, ohne zwangsläufig besser an denE2-late-Promotor zu binden im Vergleich zum nativenYB-1, wobei nicht ausgeschlossen werden soll, dass auch ein verkürztes YB-1 besser in den Kern wandert und beides macht, d.h. CPE induziert und an den E2-late-Promotor bindet. Schließlich können derartige verkürzten YB-1-Fragemente auch besser in den Kern wandern und dort effektiver an den E2-late-Promotor binden, ohne dass eine besserer CPE induziert wird. Es ist auch im Rahmen der vorliegenden Erfindung, dass verkürzte YB-1-Proteine oder -Fragmente weitere Sequenzen umfassen, wie hierin im Zusammenhang mit dem Vollängen-YB-1 beschrieben, insbesonder Zelllokalisationssignalsequenzen (NLS) und dergleichen.

Hinsichtlich der vorstehend genannten verschiedenen weiteren, von den Adenoviren codierten bzw. exprimierten Genen und Genprodukten ist es grundsätzlich auch möglich, dass diese in beliebiger Kombination codiert bzw. exprimiert werden.

Im Rahmen der vorliegenden Erfindung sollen hierin die Begriffe Adenovirus und adenovirale Systeme als im wesentlichen die gleiche Bedeutung aufweisend verstanden werden. Unter Adenovirus soll dabei insbesondere das vollständige Viruspartikel verstanden werden umfassend das Kapsid und die Nukleinsäure. Der Begriff adenovirales System stellt insbesondere darauf ab, dass die Nukleinsäure gegenüber dem Wildtyp verändert ist. Bevorzugt umfassen derartige Änderungen solche im Aufbau des Genoms des Adenovirus wie sie durch Deletieren und/oder Hinzufügen und/oder Mutieren von Promotoren, regulativen Sequenzen und/oder codierenden Sequenzen wie beispielsweise Leserahmen entstehen. Der Begriff adenovirale Systeme wird darüber hinaus bevorzugt in dem Zusammenhang verwendet, dass es sich dabei um einen Vektor handelt, der beispielsweise in der Gentherapie verwendet werden kann.

Die vorstehend gemachten Ausführungen, einschließlich jeglicher Verwendungen sowie die Ausbildungen der Adenoviren bzw. adenoviralen Systeme gelten im gleichen Maße für die dafür codierenden Nukleinsäuren und umgekehrt.

Im Zusammenhang mit der vorliegenden Erfindung ist es möglich, dass die erfindungsgemäß verwendeten Adenoviren, insbesondere die Adenoviren der Gruppe II, aber auch jene der Gruppe I bzw. die für sie codierenden Nukleinsäuren eine jede entsprechende adenovirale Nukleinsäure ist, die zu einem Replikationsereignis für sich oder in Verbindung mit weiteren Nukleinsäuresequenzen führt. Dabei ist es möglich, wie hierin ausgeführt, dass mittels Helferviren die für die Replikation erforderlichen Sequenzen und/oder Genprodukte bereitgestellt werden. Sofern hierin auf codierende Nukleinsäuresequenzen Bezug genommen wird und es sich dabei um solche Nukleinsäuresequenzen handelt, die bekannt sind, ist es im Rahmen der Erfindung, dass nicht nur die identische Sequenz verwendet wird, sondern auch hiervon abgeleitete Sequenzen. Unter abgeleiteten Sequenzen sollen hierin insbesondere solche Sequenzen verstanden sein, die noch zu einem Genprodukt, sei es eine Nukleinsäure oder ein Polypeptid, führen, das eine Funktion aufweist, die einer oder der Funktion der nicht abgeleiteten Sequenz entspricht. Dies kann durch einfache, dem Fachmann geläufige Routinetests festgestellt werden. Ein Beispiel für derartige abgeleitete Nukleinsäuresequenzen sind jene Nukleinsäuresequenzen, die für das gleiche Genprodukt, insbesondere für die gleiche Aminosäuresequenz kodieren, jedoch infolge der Degeneriertheit des genetischen Codes eine andere Basenabfolge aufweisen.

Hinsichtlich des erfindungsgemäßen Adenovirus der Gruppe II und/oder des entsprechenden erfindungsgemäßen adenoviralen Replikationssystems, bzw. deren erfindungsgemäße Verwendung ist dabei in einer Ausführungsform vorgesehen, dass, sofern nicht anders angegeben, die adenovirale Nukleinsäure für die Expression des Onkogenproteins, insbesondere des E1A-Proteins defizient ist, d.h. entweder für das 12S E1A-Protein (hierin auch als E1A12S-Protein bezeichnet) nicht codiert oder für das 13S E1A-Protein (hierin auch als E1A13S-Protein bezeichnet) nicht codiert oder für sowohl das 12S E1A-Protein als auch das 13S E1A-Protein nicht codiert, oder modifiziert ist, wie hierin definiert, und das adenovirale Replikationssystem weiter eine Nukleinsäure eines Helfervirus umfasst, wobei die Nukleinsäure des Helfervirus eine Nukleinsäuresequenz umfasst, die für das Onkogenprotein, insbesondere das E1A-Protein codiert, welches die folgenden Eigenschaften aufweist bzw. dem Adenovirus die folgenden Eigenschaften verleiht, nämlich dass dieser bevorzugterweise nicht replizierend in YB-Kern-negativen Zellen aber sehr wohl in vom Zellzyklus unabhängig YB-1-Kern-positiven Zellen oder Zellen, die dereguliertes YB-1 aufweisen, replizierend ist, transaktivierend auf mindestens ein virales Gen, insbesondere E1B55kDa, E4orf6, E4orf3 und/oder E3ADP, in YB-1 kernpositiven Zellen wirkt, und/oder zelluläres YB-1 nicht in den Kern transloziert. Es ist im Rahmen der vorliegenden Erfindung, dass die hierin beschriebenen Transgene einzeln oder gemeinsam von dem Helfervirus codiert und/oder exprimiert werden. Dies gilt sowohl für die Helferviren für die Adenoviren der Gruppe I wie auch der Gruppe II.

Weiterhin ist bei einem derartigen erfindungsgemäßen adenoviralen Replikationssystem in einer Ausführungsform vorgesehen, dass die adenovirale Nukleinsäure und/oder die Nukleinsäure des Helfervirus als replizierbarer Vektor vorliegt.

Dabei ist es weiter im Rahmen der vorliegenden Erfindung, dass die für die Adenoviren der Gruppe I und/oder Gruppe II codierende Nukleinsäure(n) in einem Vektor, bevorzugterweise in einem Expressionsvektor vorliegt/vorliegen und dieser Expressionsvektor erfindungsgemäß verwendet wird.

In einem weiteren Aspekt betrifft die vorliegende Erfindung auch eine Vektorgruppe umfassend mindestens zwei Vektoren, wobei die Vektorgruppe insgesamt ein adenovirales Replikationssystem für die Adenoviren der Gruppe I und/oder Gruppe II umfasst, wie hierin beschrieben, und die Vektorgruppe erfindungsgemäß verwendet wird. Dabei kann vorgesehen sein, dass eine jede Komponente des adenoviralen Replikationssystems auf einem eigenen Vektor, bevorzugter Weise einem Expressionsvektor angeordnet ist.

Schließlich betrifft die vorliegende Erfindung in einem weiteren Aspekt auch die Verwendung einer Zelle, die eine oder mehrere der Nukleinsäuren, wie sie für die erfindungsgemäße Verwendung der hierin beschriebenen Adenoviren der Gruppe I und/oder Gruppe II, die bevorzugterweise erfindungsgemäß verwendet werden sollen, codiert, und/oder ein entsprechendes adenovirales Replikationssystem und/oder einen entsprechenden Vektor und/oder eine erfindungsgemäße Vektorgruppe umfasst, zu denselben Zwecken, wie hierin für die verschiedenen Adenoviren beschrieben

Die vorstehend beschriebenen Konstrukte von Adenoviren und insbesondere deren Nukleinsäuren bzw. die dafür codierenden Nukleinsäuren können auch in eine Zelle, insbesondere eine Tumorzelle, in Teilen eingebracht werden, wobei dann infolge der Anwesenheit der verschiedenen Einzelkomponenten diese so zusammenwirken, als stammten die Einzelkomponenten von einer einzelnen Nukleinsäure bzw. einem einzelnen oder mehreren Adenoviren.

Die erfindungsgemäß verwendeten, für Adenoviren der Gruppe I und/oder Gruppe II, entsprechende adenovirale Systeme oder Teile davon codierenden Nukleinsäuren können als Vektoren vorliegen. Bevorzugter Weise handelt es sich um virale Vektoren. Im Falle der adenovirale Nukleinsäuren umfassenden Nukleinsäuren ist das Viruspartikel dabei bevorzugterweise der Vektor. Es ist jedoch auch im Rahmen der vorliegenden Erfindung, dass die besagten Nukleinsäuren in einem Plasmidvektor vorliegen. In einem jeden Fall weist der Vektor Elemente auf, die für die Vermehrung der inserierten Nukleinsäure, d. h. Replikation und ggf. Expression der inserierten Nukleinsäure sorgen bzw. diese steuern. Geeignete Vektoren, insbesondere auch Expressionsvektoren, und entsprechende Elemente sind den Fachleuten auf dem Gebiet bekannt und beispielsweise beschrieben in Grunhaus, A., Horwitz, M.S., 1994, Adenoviruses as cloning vectors. In Rice, C., Hrsg., Seminars in Virology, London: Saunders Scientific Publications.

Der oben beschriebenen Ausführungsform, dass die verschiedenen Elemente der besagten Nukleinsäure nicht notwendigerweise auf nur einem Vektor enthalten sein müssen, trägt der Aspekt der Erfindung Rechnung, der die Vektorgruppe betrifft. Eine Vektorgruppe umfasst entsprechend mindestens zwei Vektoren. Ansonsten gilt betreffend die Vektoren bzw. die Vektorengruppe das hierin allgemein zu Vektoren Ausgeführte.

Die Adenoviren der Gruppe I und/oder Gruppe II sind durch die verschiedenen hierin offenbarten Nukleinsäuren bzw. Genprodukte charakterisiert und können ansonsten all jene den Fachleuten auf dem Gebiet bekannten Elemente umfassen, wie dies auch bei Adenoviren vom Wildtyp der Fall ist (Shenk, T.: Adenoviridae: The virus and their replication. Fields Virology, 3. Auflage, Hrsg. Fields, B.N., Knipe, D.M., Howley, P.M. et al., Lippincott-Raven Publishers, Philadelphia, 1996, Kapitel 67).

Zur Verdeutlichung, aber nicht der Beschränkung der vorliegenden Erfindung soll im Folgenden kurz allgemein auf die Replikation von Adenoviren eingegangen werden.

Die Replikation von Adenoviren ist ein ausgesprochen komplexer Vorgang und greift im Regelfalle auf den humanen Transkriptionsfaktor E2F zurück. Während einer viralen Infektion werden zunächst die "frühen Gene" E1, E2, E3 und E4 exprimiert. Die Gruppe der "späten Gene" ist für die Synthese der viralen Strukturproteine verantwortlich. Für die Aktivierung sowohl der frühen wie auch der späten Gene spielt die E1-Region bestehend aus zwei Transkriptionseinheiten E1A und E1B, welche für verschiedene E1A- und E1B-Proteine codieren, eine entscheidende Rolle, da sie die Transkription der E2, E3, E4-Gene induzieren (Nevins, J. R., Cell 26,213-220, 1981). Zudem können die E1A-Proteine in ruhenden Zellen die DNA-Synthese induzieren und so deren Eintritt in die S-Phase einleiten (siehe Boulanger and Blair, 1991). Darüber hinaus interagieren sie mit den Tumorsuppressoren der Rb-Klasse (Whyte, P. et al., Nature 334, 124-127, 1988). Dabei wird der zelluläre Transkriptionsfaktor E2F freigesetzt. Die E2F-Faktoren können dann an die entsprechenden Promotorbereiche sowohl zellulärer wie auch viraler Gene binden (insbesonder an den adenoviralen E2 early Promotor) und die Transkription und somit die Replikation einleiten (Nevins, J. R., Science 258, 424-429, 1992). Die Aktivität von pRb bzw. E2F wird durch Phosphorylierung reguliert. Die hypophosphorylierte Form von pRb tritt hauptsächlich in der G1- und M-Phase auf. Dagegen tritt die hyperphosphorylierte Form von pRb in der S- und G2-Phase auf. Durch die Phosphorylierung von pRb wird E2F aus dem Komplex aus E2F und hypophosphoryliertem pRb freigesetzt. Die Freisetzung von E2F aus dem Komplex aus E2F und hypophosphoryliertem pRb führt zur Transkription von E2F-abhängigen Genen. Das E1A-Protein bindet nur an die hypophosphorylierte Form des pRb, wobei die Bindung von E1A an pRb hauptsächlich über die CR2-Region des E1A-Proteins erfolgt. Zudem bindet es auch an die CR1-Region, allerdings mit einer schwächeren Affinität (Ben-Israel and Kleiberger, Frontiers in Bioscience, 7, 1369-1395,2002; Helt und Galloway, Carcinogenesis, 24, 159-169, 2003).

Für das Einleiten bzw. die Durchführung der Replikation werden insbesondere die Genprodukte der E2-Region benötigt, da sie für drei essentielle Proteine kodieren. Die Transkription der E2-Proteine wird durch zwei Promotoren gesteuert, den "E2-Early E2F-abhängigen", hierin auch als E2-early Promotor oder früher E2-Promotor bezeichnet, und den "E2-late" Promoter (Swaminathan und Thimmapaya, The Molecular Repertoire of Adenoviruses III: Current Topics in Microbiology and Immunology,Vol 199, 177-194, Springer Verlag 1995). Zudem spielen die Produkte der E4-Region zusammen mit dem E1A- und E1B-55kDa-Protein eine wichtige Rolle für die Aktivität von E2F bzw. die Stabilität von p53. Zum Beispiel wird durch eine direkte Interaktion des von der E4-Region codierten E4orf6/7-Proteins mit dem Heterodimer bestehend aus E2F und DP1 der E2-Promoter noch stärker transaktiviert (Swaminathan und Thimmapaya, JBC 258, 736-746, 1996). Weiterhin wird p53 durch den Komplex bestehend aus E1B-55kDa und E4orf6 inaktiviert (Steegenga, W. T. et al., Oncogene 16, 349-357, 1998), um einen erfolgreichen lytischen Infektionszyklus durchlaufen zu können. Ferner besitzt das E1B-55kDa Protein eine weitere wichtige Funktion insoweit, als dass es in Wechselwirkung mit dem E4orf6 Protein den Export der viralen RNA aus dem Zellkern fördert, wohingegen die zelleigenen RNAs im Kern zurückgehalten werden (Bridge und Ketner, Virology 174, 345-353, 1990). Eine weitere wichtige Beobachtung ist die, dass der Proteinkomplex bestehend aus E1B-55kDa/E4orf6 in den sogenannten "viral inclusion bodies" lokalisiert ist. Es wird angenommen, dass diese Strukturen Orte der Replikation und Transkription darstellen (Ornelles und Shenk, J. Virology 65, 424-429, 1991).

Eine weitere für die Replikation und insbesondere für die Freisetzung von Adenoviren besonders wichtige Region ist die E3-Region. Die E3-Region enthält genauer die genetische Information für eine Vielzahl von relativ kleinen Proteinen, die für den adenoviralen Infektionszyklus in vitro, d.h. in der Zellkultur nicht essentiell sind. Sie spielen jedoch für das Überleben des Virus während einer akuten und/oder latenten Infektion in vivo eine bedeutende Rolle, da sie unter anderem immunregulatorische und apoptotische Funktion(en) besitzen (Marshall S. Horwitz, Virololgie, 279, 1-8, 2001; Russell, a.a.O.). Es konnte gezeigt werden, dass ein Protein mit einer Größe von ca. 11,6 kDa den Zelltod induziert. Das Protein wurde infolge seiner Funktion als ADP - für den englischen Begriff adenovirus death protein - bezeichnet (Tollefson, J. Virology, 70, 2296-2306, 1996). Das Protein wird vornehmlich in der späten Phase des Infektionszyklus gebildet. Ferner führt die Überexpression des Proteins zu einer besseren Lyse der infizierten Zellen (Doronin et al., J. Virology, 74, 6147-6155,2000).

Weiterhin ist dem vorliegenden Erfinder bekannt, dass E1A-deletierte Viren, d.h. insbesondere solche Viren, die kein 12S E1A-Protein und auch kein 13S E1A-Protein exprimieren, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Ferner war bekannt, dass von den von E1A insgesamt 5 codierten Proteinen zwei Proteine, nämlich das 12S- und das 13S-Protein, die Expression der anderen adenoviralen Gene steuern bzw. induzieren (Nevins, J. R., Cell 26, 213-220, 1981; Boulanger, P. und Blair, E.; Biochem. J. 275, 281-299, 1991). Dabei hat sich gezeigt, dass hauptsächlich die CR3-Region des 13S-Proteins die transaktivierende Funktion ausübt (Wong HK und Ziff EB., J Virol., 68, 4910-20, 1994). Adenoviren, die bestimmte Deletionen in der CR1- und/oder CR2-Region und/oder CR3-Region des 13S-Proteins aufweisen, sind weitestgehend replikationsdefekt, wirken aber noch bei einzelnen Zelllinien transaktivierend auf die viralen Gene bzw. Promotoren, insbesondere auf die E2 Region (Wong HK, Ziff EB., J Virol. 68, 4910-20, 1994; Mymryk, J. S. und Bayley, S. T., Virus Research 33, 89-97,1994).

Nach Infektion einer Zelle, typischerweise einer Tumorzelle, mit einem Wildtyp-Adenovirus wird YB-1 vermittelt durch E1A, E1B-55K und E4orf6 in den Kern induziert und co-lokalisiert mit E1B-55K im Kern in den viral inclusion bodies, was eine effektive Replikation des Virus im Zellkern sowohl in vitro als auch in vivo erlaubt. Dabei war bereits früher festgestellt worden, dass auch E4orf6 an E1B-55 K bindet (Weigel, S. und Dobbelstein , M. J. Virology, 74, 764-772, 2000; Keith N. Leppard, Seminars in Virology, 8, 301-307, 1998.) und somit den Transport bzw. die Verteilung von E1B-55K in den Kern vermittelt, was eine optimale Virusproduktion bzw adenovirale Replikation gewährleistet. Durch das Zusammenwirken von E1A, E1B-55K und YB-1 bzw. durch den Komplex aus E1B-55K/E4orf6 mit YB-1 und der Kolokalisation von YB-1 und E1B-55K im Kern in den sogenannten viral inclusion bodies ist eine erfindungsgemäße effiziente Replikation des Virus, und damit die Verwendung der hierin beschriebenen Viren zur Replikation in Zellen, die YB-1-Kern-positiv sind, bevorzugterweise solche Zellen, die Zellzyklus-unabhängig YB-1 im Zellkern enthalten und/oder Zellen, die dereguliertes YB-1 enthalten oder aufweisen, bzw. zur Herstellung eines Medikamentes zur Behandlung von Erkrankungen, bei denen YB-1-Kern-positive Zellen, bevorzugterweise solche Zellen, die Zellzyklus-unabhängig YB-1 im Zellkern enthalten und/oder Zellen, die dereguliertes YB-1 enthalten oder aufweisen, beteiligt sind, möglich. Die dadurch vor diesem zellulären Hintergrund mögliche Replikation führt zu einer Lyse der Zelle, Freisetzung des Virus und Infektion und Lyse benachbarter Zellen, so dass im Falle der Infektion einer Tumorzelle bzw. eines Tumors letztlich eine Lyse des Tumors, d.h. eine Onkolyse, eintritt.

YB-1 gehört zu einer Gruppe hoch konservierter Faktoren, die an der invertierten CAAT-Sequenz, der sogenannten Y-Box, binden. Sie können sowohl auf der Ebene der Transkription als auch der Translation regulatorisch wirken (Wolffe, A. P. Trends in Cell Biology 8, 318-323, 1998). Es werden immer mehr Y-Box-abhängige Regulationswege bei der Aktivierung aber auch bei der Hemmung Wachstums- und Apoptose-assoziierter Gene aufgefunden (Swamynathan, S. K. et al.,FASEB J. 12, 515-522, 1998). So interagiert YB-1 direkt mit p53 (Okamoto, T. et al., Oncogene 19, 6194-6202, 2000), spielt eine wichtige Rolle bei der Fas-Genexpression (Lasham, A. et al., Gene 252, 1-13, 2000), MDR und MRP-Genexpression (Stein, U. et al., JBC 276, 28562-69, 2001; Bargou, R. C. et al., Nature Medicine 3, 447-450, 1997) und bei der Aktivierung von Topoisomerasen und Metalloproteinasen (Mertens. P. R. et al., JBC 272, 22905-22912, 1997; Shibao, K. et al., Int. J. Cancer 83, 732-737, 1999). Zudem ist YB-1 an der Regulation der mRNA-Stabilität (Chen, C-Y. et al., Genes & Development 14, 1236-1248, 2000) und an Reparaturvorgängen beteiligt (Ohga, T. et al.,Cancer Res. 56, 4224-4228, 1996; Izumi H. et al., Nucleic Acid research 2001, 29, 1200 - 1207; Ise T. et al., Cancer Res. 1999, 59, 342 - 346).

Die nukleäre Lokalisation von YB-1 in Tumorzellen, sei es dass YB-1 Zellzyklus-unabhängig im Zellkern vorliegt, sei es dass im Cytoplasma vorhandenes dereguliertes YB-1 durch die Adenoviren der Gruppe I und/oder II in den Zellkern transloziert wird, führt zu einer E1A-unabhängigen viralen Replikation, bei der insbesondere weder ein 12S E1A-Protein noch ein 13S E1A-Protein exprimiert vorliegt bzw. verwendet wird, (Holm, P. S. et al. JBC 277, 10427-10434, 2002) und im Falle der Überexpression des Proteins YB-1 zu einer multidrug resistance (Vielfachresistenz). Zudem ist bekannt, dass die adenoviralen Proteine wie z. B. E4orf6 und E1B-55K einen positiven Effekt auf die virale Replikation ausüben (Goodrum, F. D. und Ornelles, D. A, J. Virology 73, 7474-7488, 1999), wobei ein funktionelles E1A Protein für das Einschalten der anderen viralen Genprodukte (z. B. E4orf6, E3ADP und E1B-55K) verantwortlich ist (Nevins J. R., Cell 26, 213-220, 1981). Dies unterbleibt jedoch bei den im Stand der Technik bekannten E1A-minus Adenoviren, bei denen das 13S E1A-Protein nicht vorhanden ist. Die Kernlokalisation von YB-1 in multidrug resistenten Zellen, die YB-1 im Kern aufweisen, erlaubt die Replikation bzw. Partikelbildung derartiger E1A-minus Viren. Hierbei ist jedoch die Effizienz der viralen Replikation bzw. Partikelbildung im Vergleich zum Wildtyp Ad5 um ein Vielfaches geringer. Eine Kombination von YB-1, welches entweder bereits im Zellkern der Tumorzelle, sei es dass YB-1 Zellzyklus-unabhängig im Zellkern vorliegt, sei es dass im Cytoplasma vorhandenes dereguliertes YB-1 durch die Adenoviren der Gruppe I und/oder II in den Zellkern transloziert wird, enthalten ist, oder durch äußere Faktoren (z.B. Applikation von Zytostatika oder Bestrahlung oder Hyperthermie) in den Zellkern induziert, d.h. veranlasst wird, im Zellkern vorhanden zu sein, insbesondere unabhängig vom Zellzyklus vorhanden zu sein, oder als Transgen durch einen Vektor eingeführt wird, mit einem System, bevorzugterweise mit einem adenoviralen System, welches die adenoviralen Gene einschaltet, aber nicht zur viralen Replikation fähig ist, stellt demgegenüber überraschenderweise ein System dar, welches eine sehr effektive virale Replikation bzw. Partikelbildung durch YB-1 vermittelt und damit eine Onkolyse erlaubt. Gleiches gilt auch für die erfindungsgemäßen Adenoviren, d.h. die Adenoviren der Gruppe I, die durch ihre spezielle Ausgestaltung und Ausnutzung des Effektes, dass ein E1B-Protein, bevorzugterweise das E1B55K-Protein, und/oder ein E4-Protein, bevorzugterweise das E4orf6-Protein, für eine effektive Bereitstellung von YB-1, bevorzugterweise im Kern für eine wirksame Replikation geeignet sind. Geeignete Zytostatika, die zusammen mit den hierein offenbarten Adenoviren im Rahmen der verschiedenen Aspekte der vorliegenden Erfindungen verwendet werden können, sind beispielsweise solche, die zu den folgenden Gruppen gehören: Anthracycline, wie beispieleweise Daunomycin und Adriamycin; Alkylanzien, wie beispeislweise Cyclophosphamid; Alkaloide, wie beispielsweise Etoposid; Vin-Alkaloide, wie beispielsweise Vincristin und Vinblastin; Antimetabolite wie beispielsweise 5-Fluorouracil und Methrothrexat; Platin-Derivate, wie beispielsweise cis-Platin; Topoisomerase-Inhibitoren, wie beispielsweise Camphothecin, CPT-11; Taxane, wie beispielsweise Taxol, Paclitaxel, Histon-Deacetylasenhibitoren wie beispielsweise FR901228, MS-27-275, Trichostatin A, MDR-Modulatoren, wie beispielsweise MS-209, VX-710 und Geldanamycin-Derivate wie beispielsweise 17-AAG. Die hierin offenbarten Adenoviren, insbesondere rekombinanten Adenoviren, welche nur in YB-1-Kern-positiven Zellen zur Replikation befähigt sind bzw. solchen Zellen, die dereguliertes YB-1, bevorzugterweise im Cytoplasma aufweisen, sind in ihrer Fähigkeit, transaktivierend auf die viralen Gene E1B-55K, E4orf6, E4orf3 und E3ADP zu wirken, beschränkt, verglichen mit den diesbezüglichen transaktivierenden Fähigkeiten von Adenoviren vom Wildtyp, insbesondere vom Wildtyp Ad5. Der vorliegende Erfinder hat nun überraschenderweise festgestellt, dass diese beschränkte transaktivierende Fähigkeit dadurch aufgehoben werden kann, dass die entsprechenden Gene und insbesondere EIB-55K und E4orf6 in Verbindung mit der Kernlokalisation von YB-1 zur Expression gebracht werden. Wie in den Beispielen hierin gezeigt wird, erhöht sich die virale Replikation bzw. Partikelbildung unter diesen Umständen auf ein Niveau, welches vergleichbar ist mit dem Replikationsverhalten bzw. Partikelbildungsverhalten von Adenoviren vom Wildtyp.

Bei dem Medikament, im Rahmen dessen oder bei dessen Herstellung die hierin beschriebenen Adenoviren erfindungsgemäß verwendet werden, ist vorgesehen, dass dieses in der Regel systemisch appliziert wird, gleichwohl es auch im Rahmen der vorliegenden Erfindung ist, wenn dieses lokal appliziert oder abgegeben wird. Die Applikation erfolgt mit der Absicht, dass insbesondere jene Zellen mit dem Adenovirus infiziert werden und insbesondere darin eine Replikation der Adenoviren erfolgt, bei denen eine Beteiligung, bevorzugter Weise kausal, an der Ausbildung eines Zustandes, typischerweise einer Erkrankung, vorliegt, zu deren Diagnose und/oder Prävention und/oder Behandlung das erfindungsgemäße Medikament verwendet wird.

Ein derartiges Medikament ist bevorzugter Weise für die Behandlung von malignen Erkrankungen, Tumorerkrankungen, Krebserkrankungen, Krebs und Tumoren, wobei diese Begriffe hierin im wesentlichen synonym verwendet werden, sofern nichts Gegenteiliges angegeben ist, vorgesehen. Dabei sind jene Tumorerkrankungen besonders bevorzugt, bei denen entweder YB-1 bereits im Zellkern, bevorzugterweise unabhängig vom Zellzyklus infolge des der Tumorerkrankung zugrundeliegenden Mechanismus, insbesondere des zugrundeliegenden pathologischen Mechanismus, vorliegt, oder aber durch äußere Maßnahmen die Anwesenheit von YB-1 im Zellkern bedingt wird, wobei die Maßnahmen geeignet sind, YB-1 in den Zellkern zu transferieren, dort zu induzieren oder dort zu exprimieren. Der Begriff Tumor oder Tumorerkrankung soll hierbei sowohl maligne wie benigne Tumoren, jeweils auch solide oder diffuse Tumoren umfassen, und entsprechende Erkrankungen bezeichnen. Dabei kann vorgesehen sein, dass das Medikament mindestens eine weitere pharmazeutisch wirksame Verbindung enthält. Die Art und der Umfang dieser weiteren pharmazeutisch aktiven Verbindungen wird dabei von der Art der Indikation abhängen, für die das Medikament eingesetzt wird. Im Falle der Verwendung des Medikamentes für die Behandlung und/oder die Prophylaxe von Tumorerkrankungen werden typischerweise Zytostatika, wie beispielsweise cis-Platin und Taxol, Daunorubicin, Adriamycin und/oder Mitoxantron oder andere der hierin beschriebenen Zytostatika oder Gruppen von Zytostatika verwendet, insbesondere jene, wie sie im Zusammenhang mit der durch Zytostatika verursachten Kernlokalisation von YB-1 beschrieben sind..

Das erfindungsgemäße Medikament kann dabei in verschiedenen Formulierungen vorliegen, bevorzugter Weise in einer flüssigen Form. Weiterhin wird das Medikament Hilfsstoffe wie Stabilisatoren, Puffer, Konservierungsstoffe und dergleichen enthalten, die dem Fachmann auf dem Gebiet der Galenik bekannt sind.

Der vorliegende Erfinder hat weiterhin überraschend gefunden, dass die Wirksamkeit der hierin beschriebenen und insbesondere der erfindungsgemäß verwendeten Viren dadurch erhöht werden kann, dass er in Kombination mit mindestens zwei Mitteln verwendet wird, wobei ein jedes der mindestens zwei Mittel einzeln und unabhängig aus der Gruppe ausgewählt ist, die Cytostatika umfasst.

Wie hierin in einer bevorzugten Ausführungsform verwendet sind Cytostatika insbesondere chemische oder biologische Verbindungen, die während oder nach der Verabreichung an eine Zelle oder einen Organismus, der eine oder diese Zelle enthält, dazu führen, dass die Zelle nicht mehr wächst und/oder sich nicht mehr teilt bzw. die Zellteilung und/oder das Zellwachstum verlangsamen. Cytostatika sind dabei auch solche Verbindungen, die erst in der Zelle oder in dem diese enthaltenden Organismus zu einem Cytostatikum im vorstehend beschriebenen Sinne werden. Insoweit umfasst der Begriff der Cytostatika auch Prä-Cytostatika.

Cytostatika werden bevorzugt nach ihrem Wirkmechanismus eingeteilt. Dabei werden die folgenden Gruppen verschieden, die grundsätzlich alle im Rahmen der vorliegenden Erfindung verwendet werden können:
- Alkylanzien, d.h. chemische Verbdinungen, die ihre zytotoxische Wirkung durch Alkylierung von Phosphat-, Amio-, Sulfhydryl-, Carboxyl- und Hydroxylgruppen der Nukleinsäure sowie Protein bedingen. Derartige Verbindungen wirken selbst oft kanzerogen. Typische Beispiele dieser Gruppe von Cytostatika sind Cisplatin bzw. Platin-Derivate, Cyclophosphamid, Dacarbazin, Mitomycin, Procarbazin.
- Antimetabolite, d.h. Verbindungen, die aufgrund struktureller Ähnlichkeit oder Fähigkeit zur Bindung einen Stoffwechselprozess blockieren oder beeinträchtigen. Innerhalb der Gruppe der Antimetabolite unterscheidet man wiederum zwischen strukturähnlichen Antimetaboliten, strukturverändernden Antimetaboliten und den indirekt wirkenden Antimetaboliten. Bei den strukturähnlichen Antimetaboliten konkurrieren diese aufgrund chemischer Ähnlichkeit mit dem Metaboliten, ohne Übernahme dessen Funktion. Strukturverändernde Antimetaboliten binden den Metaboliten, was dessen Funktion oder Resorption verhindert oder den Metaboliten chemisch modifiziert. Indirekt wirkende Antimetaboliten beeinträchtigen die Funktion des Metaboliten, beispielsweise über Bindung von Ionen. Typische Beispiele dieser Gruppe sind Folsäureantagonisten wie z.B. Methotrexat, Pyrimidinanaloga wie z.B. Fluoruracil, Purinanaloga wie z.B. Azathioprin und Mercaptopurin.
- Mitosehemmstoffe, d.h. die Zellteilung hemmen. Innerhalb der Gruppe der Mitosehemmstoffe erfolgt eine Unterscheidung in Zellteilungsgifte, Spindelgifte und Chromosomengifte. Typische Beispiels dieser Gruppe sind Taxane und Vinca-Alkaloide. Die Taxana können dabei wiederum in die beiden Hauptgruppen Taxole und Taxotere eingeteilt werden, wobei ein besonders bevorzugtes Taxol Paclitaxel ist und ein besonders bevorzugtes Taxoter Docetaxel ist.
- Antibiotika mit hemmender Wirkung auf die DNA-abhängige RNA-Polymerase. Typische Beispiels dafür sind die Anthrazykline, so zum Beispiel Bleomycin, Daunorubicin, Doxorubicin und Mitomycin.
- Topoisomerase-Hemmer, insbesondere Topoisomerase-I-Hemmer. Topoisomerase-Hemmer sind chemische Verbindungen, die die Tertiärstruktur der DNA bestimmen, indem sie in einem dreistufigen Prozess die Veränderung der DNA-Verweindungszahl katalysieren. Dabei werden im wesentlichen zwei Formen von Topoisomerasen unterschieden. Topoisomerasen vom Typ I spalten nur einen DNA-Strang und sind ATP-unabhängig, wohingegen Topoisomerasen vom Typ II beide Stränge einer DNA spalten, wobei diese ATP-abhängig sind. Typische Beispiele hierfür sind Irinotecan und Topotecan für Topoisomerase-I- -Inhibitoren und Etoposid und Daunorubicin für Topoismerase II-Inhibitoren.

Im Rahmen der vorliegenden Erfindung werden mindestens zwei Mittel aus der vorstehenden Gruppe ausgewählt, Es ist jedoch auch im Rahmen der vorliegenden Erfindung, dass insbesondere auch drei, vier oder fünf verschiedene Mittel ausgewählt werden. Die folgenden Ausführungen werden für die Ausführungsform der vorliegenden Erfindung gemacht, bei der lediglich zwei Mittel zusammen mit dem Virus verwendet werden. Die Ausführungen gelten sinngemäß auch für die Ausführungsform, bei der mehr als zwei Mittel verwendet werden.

Bevorzugterweise unterscheiden sich die Mittel darin, dass sie an unterschiedlichen Zielmolkülen angreifen, oder in der Literatur an verschiedene Moleküle angreifend beschrieben werden. Es ist im Rahmen, dass das Mittel auch zwei oder mehr verschiedene Verbindungen umfasst, die an das gleiche Zielmolekül binden. Es ist jedoch auch im Rahmen der vorliegenden Erfindung, dass das eine Mittel an einem ersten Ort des Zielmoleküls bindet, während das zweite Mittel an einem zweiten Ort des Zielmoleküls bindet.

Es ist ebenfalls im Rahmen der vorliegenden Erfindung, dass mindestens zwei der Mittel über einen unterschiedlichen Wirkmechanismus aktiv sind. Aktiv bedeutend in einer bevorzugten Ausführungsform, dass die das Zellwachstum und/oder Zellteilung inhibierende oder verzögernde Wirkung der chemischen Verbindung jeweils über einen unterschiedlichen Wirkmechanismus aktiv ist. In einer besonders bevorzugten Ausführungsform bedeutet der Begriff aktiv, dass dadurch die Effizienz der Replikation eines Virus, insbesondere des erfindungsgemäßen Virus, der hierin beschriebenen Viren und/oder der hierin erfindungsgemäß zu verwendenden Viren , erhöht wird verglichen mit einer Situation, bei der eines und/oder beide der Mittel nicht verwendet werden. Als Maß für die Effizienz der Replikation des Virus wird bevorzugterweise die Anzahl der für die Zelllyse erforderliche Anzahl von Viren, bevorzugtererweise ausgedrückt als pfu/Zelle.

In einer besonders bevorzugten Ausführungsform ist eines der mindestens zwei Mittel ein solches, das die Infizierbarkeit der Zelle, in der die Replikation des Virus erfolgen soll, bevorzugt selektiv erfolgen soll, mit dem Virus, bevorzugterweise dem hierin beschriebenen Virus und/oder dem hierin erfindungsgemäß zu verwendenden Virus, erhöht. Dies kann beispielweise dadurch erfolgen, dass die Aufnahme des Virus durch die Zelle erhöht wird. Die Aufnahme des Virus, insbesondere von Adenovirus, wird beispielsweise durch den Coxsackievirus-Adenovirus Rezeptor vermittelt (CAR) (Mizuguchi und Hayakawa, GENE 285,69-77,2002) vermittelt. Eine erhöhte Expression von CAR wird beispielsweise durch Trichostatin A bedingt (Vigushin et al., Clinical Cancer Research, 7, 971-976, 2001).

In einer weiteren Ausführungsform ist eines der mindestens zwei Mittel ein solches, welches die Verfügbarkeit einer Komponente innerhalb der Zelle erhöht, wobei die Komponente eines solche ist, die die Replikation des Virus, bevorzugterweise dem hierin beschriebenen Virus und/oder dem hierin erfindungsgemäß zu verwendenden Virus, erhöht.

In einer weiteren Ausführungsform ist eines der mindestens zwei Mittel ein solches, welches den Transport von YB-1 in den Zellkern vermittelt. Ein derartiges Mittel kann aus der Gruppe ausgewählt sein, die Topoisomerase-Hemmer, Alkylanzien, Antimetabolite und Mitose-Hemmstoffe umfasst. Bevorzugte Topoisomerase-Hemmer sind Camptothecin, Irinotecan, Etoposide und deren jeweilige Analoga. Bevorzugte Mitose-Hemmstoffe sind Daunorubicin, Doxorubicin Paclitaxel und Docetaxel. Bevorzugte Alkylanzien sind Cisplatin und deren Analoga- Bevorzugte Antimetabolite sind Fluoruracil und Methotrexat.

In einer besonders bevorzugten Ausführungsform ist das eine der mindestens zwei Mittel ein solches, das die Infizierbarkeit der Zelle erhöht, insbesondere die Expression von CAR erhöht, und das zweite der mindestens zwei Mittel ein solches, das den Transport von YB-1 in den Zellkern erhöht, wobei bevorzugtererweise als chemische Verbindung eine solche verwendet wird, die die entsprechende erforderliche Eigenschaft aufweist, wie bevorzugt vorstehend erwähnt.

In einer weiteren Ausführungsform ist vorgesehen, dass das eine der mindestens zwei Mittel ein Histon-Deacylase-Inhibitor ist. Ein bevorzugter Histon-Deacylase-Inhibitor ist ein solcher, der aus der Gruppe ausgewählt ist, die Trichostatin A, FR901228, MS-27-275, NVP-LAQ824 und PXD101 umfasst. Trichostatin A ist beispielsweise beschrieben in Vigushin et al., Clinical Cancer Research, 7, 971-976, 2001; FR901228 ist beispielsweise beschrieben in Kitazono et al., Cancer Res., 61, 6328-6330, 2001; MS-27-275 beschrieben in Jaboin et al., Cancer Res., 62, 6108-6115, 2002; PXD101 beschrieben in Plumb et al., Mol. Cancer Ther., 8, 721-728, 2003; NVP-LAQ824 beschrieben in Atadja et al., Cancer Res., 64, 689-695, 2004.

In einer noch weiteren Ausführungsform ist vorgesehen, dass das eine der mindestens zwei Mittel ein Topoisomerase-Inhibitor, bevorzugterweise in Topoisomerase I-Inhibitor ist. Ein bevorzugter Topoismerase-Inhibitor ist ein solcher, der aus der Gruppe ausgewählt ist, die Camptothecine, Irinotecan, Topotecan, SN-38, 9-aminocamptothecin, 9-nitrocamptothecin und DX-895If umfasst. Irinotecan und SN-38 ist beispielsweise beschrieben in Gilbert et al., Clinical Cancer Res., 9,2940-2949,2003; DX-895IF beschrieben in van Hattum et al., British Journal of Cancer, 87, 665-672, 2002; Camptothecin beschrieben in Avemann et al., Mol. Cell. Biol., 8,3026-3034, 1988; 9-aminocamptothecin, 9-nitrocamptothecin beschrieben in Rajendra et al., Cancer Res., 63, 3228-3233, 2003. Ein weitere Topoisomerase-Inhibitor ist Daunorubicin, welches verwendet werden kann. Daunorubicin beschrieben in M. Binaschi et al., Mol. Pharmacol., 51, 1053-1059.

In einer besonders bevorzugten Ausführungsform ist das eine der mindestens zwei Mittel ein Histon-Deacylase-Inhibitor und das andere der mindestens zwei Mittel ein Topoismerase-Inhibitor.

In einer Ausführungsform ist vorgesehen, dass das erfindungsmäße Mittel und/oder das erfindungsgemäß hergestellte Mittel den Virus getrennt von einem oder mehreren der mindestens zwei Mittel, die mit dem Virus erfindungsgemäß kombiniert sind, enthält. Dabei ist bevorzugt, dass der Virus von einem jeglichen Mittel, das mit dem Virus kombiniert wird, getrennt. Bevorzugterweise ist die Trennung eine räumliche Trennung. Die räumliche Trennung kann dabei so erfolgen, dass der Virus in einer anderen Verpackung vorliegt als die Mittel. Bevorzugterweise handelt es sich bei der Verpackung um eine Einzeldose, d.h. der Virus und die Mittel sind in Einzeldosen verpackt. Die Einzeldosen können wiederum zu einer Packung zusammengefasst sein. Es ist jedoch auch im Rahmen der vorliegenden Erfindung, dass die Einzeldosen des Virus mit einer oder mehreren Einzeldosen eines oder mehrerer der Mittel kombiniert oder verpackt ist.

Die Art der Verpackung hängt dabei in der den Fachleuten auf dem Gebiet bekannten Art von der Art der Verabreichung ab. Bevorzugterweise wird der Virus als Lyophilisat oder in einer geeigneten flüssigen Phase vorliegen. Bevoruigterweise werden die Mittel in fester Form vorliegen, z.B. als Tabletten oder Kapseln, sind jedoch nicht darauf beschränkt. Alternativ können die Mittel auch in flüssiger Form vorliegen.

Es ist im Rahmen der vorliegenden Erfindung, dass der Virus systemisch oder lokak verabreicht wird. Es ist auch im Rahmen der vorliegenden Erfindung, dass die mit dem Virus kombinierten Mittel einzeln unabhängig voneinander oder gemeinsam systemisch oder lokal verabreicht werden. Den Fachleuten auf dem Gebiet sind weitere Verabreichungsformen bekannt.

Es ist weiterhin um Rahmen der vorliegenden Erfindung, dass der Virus und die mit ihm kombinierten Mittel zeitlich getrennt oder gleichzeitig verabreicht werden. Bei zeitlich getrennter Verabreichung ist es bevorzugt, dass die Mittel vor der Verabreichung des Virus verabreicht werden. Die Zeitspanne, wie lange vorher die Mittel vorher verabreicht werden, hängt von der Art der verwendeten Mittel ab und ergibt sich für den Fachmann aus der Wirkungsweise der verwendeten Mittel. Dabei kann auch die Verabreichung der mindestens zwei Mittel wiederum gleichzeitig oder zu unterschiedlichen Zeitpunkten erfolgen. Bei zeitlich unterschiedlicher Verabreichung ergeben sich die Zeitpunkte wiederum aus den den Mitteln zugrundeliegenden Wirkmechanismen und können von den Fachleuten auf der Grundlage davon festgelegt werden.

Die vorstehenden Ausführungen, die im Zusammenhang mit den Medikamenten gemäß der vorliegenden Erfindung, die hierin auch als pharmazeutische Zusammensetzungen offenbart und/oder bezeichnet werden, gemacht wurden, gelten sinngemäß auch für eine jegliche Zusammensetzung, so auch für Zusammensetzungen, wie sie erfindungsgemäß für die Replikation von Viren verwendet werden, bevorzugterweise für die *in vitro* Replikation von Viren. Die vorstehenden Ausführungen gelten auch für den erfindungsgemäßen Kit. bzw. den erfindungsgemäß verwendeten Kit, der neben der hierin beschrieben Viren bzw. den erfindungsgemäß verwendeten Viren, auch ein oder eine Kombination von Mitteln, wie hierin beschrieben, umfassen kann. Derartige Kits umfassen den Virus und/oder das oder die Mittel in einer für die Anwendung fertigen Form und bevorzugterweise noch eine Gebrauchsanweisung. Weiterhin gelten die vorstehenden Ausführungsformen auch für die hierin offenbarten Nukleinsäuren und die erfindungsgemäß verwendeten Nukleinsäuren, und die erfindungsgemäßen Replikationssysteme und die dafür codierenden Nukleinsäuren bzw. die erfindungsgemäß verwendeten Replikationssysteme und die erfindungsgemäß verwendeten dafür codierenden Nukleinsäuren.

Der vorliegende Erfinder hat desweiteren überraschenderweise festgestellt, dass die erfindungsgemäße Verwendung der hierin beschriebenen Viren, bevorzugterweise der Adenoviren der Gruppe I und/oder der Gruppe II mit besonders großer Erfolgsrate bei solchen Tumoren bzw. für die Herstellung von Medikamenten für die Behandlung solcher Tumoren verwendet werden kann, bei denen YB-1 unabhängig vom Zellzyklus im Zellkern vorkommt. Normalerweise ist YB-1 im Cytoplasma, insbesondere auch im perinukleären Plasma, vorhanden. In der G1/S-Phase des Zellzyklus findet sich YB-1 im Zellkern von sowohl normalen wie auch Tumorzellen, wobei jedoch ein Teil des YB-1 im Cytoplasma verbleibt [Jürchott K et a., JBC 2003, 278, 27988-27996.]. Dies ist jedoch nicht ausreichend, um eine virale Onkolyse unter Verwendung derartiger modifizierten Adenoviren zu bewerkstelligen. Die im Stand der Technik beschriebene vergleichsweise geringe Wirksamkeit von derartigen attenuierten Adenoviren beruht letztlich auf deren fehlerhaften Anwendung. Mit anderen Worten, es können derartige adenovirale Systeme, insbesondere auch mit einer größeren Wirksamkeit dort eingesetzt werden, wo die molekularbiologischen Voraussetzungen für eine virale Onkolyse unter Verwendung dieser, hierin beschriebenen attenuierten oder modifizierten Adenoviren, bevorzugterweise der Adenoviren der Gruppe I und/oder der Gruppe II, gegeben sind. Im Falle der hierin erfindungsgemäß zu verwendend beschriebenen Adenoviren, wie beispielsweise AdΔ24, dl922-947, E1Ad/01/07, CB016, dl520 und die im europäischen Patent EP 0 931 830 beschriebenen rekombinanten Adenoviren, liegen diese Voraussetzungen bei solchen Tumorerkrankungen vor, deren Zellen eine vom Zellzyklus unabhängige Kernlokalisation von YB-1 aufweisen. Diese Form der Kernlokalisation kann dabei durch die Art des Tumors selbst bedingt sein, oder aber durch die hierin beschriebenen erfindungsgemäßen Agenzien oder Maßnahmen bewirkt werden. Die vorliegende Erfindung definiert somit eine neue Gruppe von Tumoren bzw. Tumorerkrankungen und damit auch von Patienten, die mit den erfindungsgemäßen Viren, bevorzugterweise der Gruppe I und/oder der Gruppe II, aber auch mit den im Stand der Technik bereits beschriebenen attenuierten oder modifizierten Adenoviren noch wirksam behandelt werden können.

Eine weitere Gruppe von Patienten, die erfindungsgemäß unter Verwendung der Adenoviren der Gruppe I und/oder der Gruppe II, und auch der hierin als erfindungsgemäß zu verwenden beschriebenen, im Stand der Technik als solches wenigstens zum Teil bekannten Adenoviren, oder unter Verwendung der hierin erstmalig beschriebenen Adenoviren behandelt werden können, insbesondere unter Verwendung solcher Adenoviren, die Mutationen bzw. Deletionen im E1A-Protein aufweisen, welche die Bindungen von Rb/E2f nicht stören oder aber in YB-1-Kernnegativen Zellen nicht replizieren oder eine stark verringerte Replikation, wie hierin definiert und/oder ein deletiertes Onkoprotein, insbesondere E1A, aufweisen bzw. zeigen, wie beispielsweise im Falle der Viren AdΔ24, dl922-947, E1Ad/01/07, CB106 und der im europäischen Patent EP 0931 830 beschriebenen Adenoviren, sind jene Patienten, bei denen durch Anlegen oder Realisieren bestimmter Bedingungen gewährleistet wird, dass YB-1 in den Kern wandert oder dort induziert oder dorthin transportiert wird oder YB-1 dereguliert vorliegt. Die Verwendung Adenoviren der Gruppe I und/oder der Gruppe II bei dieser Patientengruppe beruht insoweit auf der Erkenntnis, dass die Induktion der viralen Replikation auf der Kernlokalisation von YB-1 mit anschließender Bindung von YB-1 an den E2-late-Promotor beruht. Infolge der hierin offenbarten Erkenntnisse können Adenoviren wie beispielsweise AdΔ24, dl922-947, E1Ad/01/07, CB106 und/oder die im europäischen Patent EP 0931 830 beschriebenen, auch in solchen Zellen replizieren, die YB-1 Kern-positiv sind und/oder in Zellen, in den YB-1 dereguliert im Sinne der vorliegenden Erfindung vorliegt. Insoweit können diese Adenoviren erfindungsgemäß für die Behandlung von Erkrankungen bzw. Patientengruppen verwendet werden, die Zellen mit diesen Eigenschaften aufweisen, insbesondere dann, wenn diese Zellen an der Ausbildung der jeweiligen zu behandelnden Erkrankung beteiligt sind. Dies begründet den Erfolg von AdΔ24, d1922-947, E1Ad/01/07, CB016 und der im Patent EP 0931 830 beschriebenen Adenoviren bzw. der Adenoviren der Gruppe I und/oder II bei der Behandlung solcher Tumoren, die YB-1 Zellzyklus-unabhängig im Kern oder YB-1 dereguliert im Sinne der vorliegenden Offenbarung aufweisen. Eine weitere Gruppe von Patienten, die unter Verwendung der hierin als zu verwenden beschriebenen Viren bzw. unter Verwendung der hierin erstmalig beschriebenen Viren, insbesondere Adenoviren, bzw. der Adenoviren der Gruppe I und/oder Gruppe II behandelbar sind, sind somit solche, die YB-1 Kern-positiv sind und/oder YB-1 Kern-positiv sind als Ergebnis der nachfolgend beschriebenen Behandlungen und/oder solche Patienten, die eine der nachfolgenden Maßnahmen, bevorzugt im Sinne einer Behandlung, vor der Verabreichung der Adenoviren erfahren haben, gleichzeitig mit der Gabe der entsprechenden Viren erfahren oder nach der Verabreichung der Adenoviren erfahren werden. Dabei ist es im Rahmen der vorliegenden Lehre, dass YB-1 Kern-positive Patienten solche Patienten sind, die insbesondere in einer Anzahl der einen Tumor ausbildenden Zellen YB-1 unabhängig vom Zellzyklus im Kern aufweisen und/oder dereguliertes YB-1 aufweisen. Zu diesen Maßnahmen gehört die Verabreichung von Zytostatika, wie sie hierin insgesamt beschrieben sind und/oder im Rahmen einer Tumortherapie verwendet werden. Weiterhin gehört zu dieser Gruppe von Maßnahmen Bestrahlung, insbesondere eine Bestrahlung, wie sie im Rahmen einer Tumortherapie angewandt wird. Bestrahlung bedeutet dabei insbesondere die Bestrahlung mit energiereicher Strahlung, bevorzugterweise mit radioaktiver Strahlung, bevorzugtererweise wie sie im Rahmen einer Tumortherapie verwendet wird. Eine weitere Maßnahme stellt Hyperthermie bzw. das Anlegen von Hyperthermie dar, bevorzugterweise Hyperthermie, wie sie im Rahmen einer Tumortherapie verwendet wird. In einer ganz besonders bevorzugten Ausführungsform ist dabei vorgesehen, dass die Hyperthermie lokal angewandt wird. Schließlich ist eine weitere Maßnahme die Hormonbehandlung, insbesondere die Hormonbehandlung, wie sie bei der Tumorbehandlung zur Anwendung gelangt. Im Rahmen einer derartigen Hormonbehandlung werden Anti-Östrogene und/oder Anti-Androgene verwendet. Dabei werden Anti-Östrogene, wie beispielsweise Tamoxifen, insbesondere bei der Therapie von Brustkrebs verwendet und Anti-Androgene, wie beispielsweise Flutamid oder Cyproteronacetat, bei der Therapie von Prostatakrebs verwendet.

Die hierin offenbarten Adenoviren können auch für die Behandlung von Tumoren, verwendet werden, wobei die Tumoren ausgewählt sind aus der Gruppe, die Primärtumoren, Sekundärtumoren, Tertiärtumoren und metastasierende Tumoren umfassen. Dabei ist bevorzugt, wenn die Tumoren zumindest eines der folgenden Merkmale aufweisen, nämlich dass sie YB-1 im Kern Zellzyklus-unabhängig aufweisen, unabhängig von der Ursache dafür, und/oder, dass sie dereguliertes YB-1 aufweisen.

Es ist im Rahmen der vorliegenden Erfindung, dass die Zellen, in denen die erfindungsgemäßen Adenoviren replizieren oder replizierbar sind bzw. die Tumoren, die derartige Zellen umfassen solche sind, die mit einem oder mehreren der hierin beschriebenen Merkmale ausgestaltet, insbesondere dem Merkmal, dass sie YB-1 im Kern Zellzyklus-unabhängig aufweisen, unabhängig von der Ursache dafür, und/oder dem Merkmal, dass sie dereguliertes YB-1 aufweisen, und diese Zellen bzw. Tumoren mit einem erfindungsgemäßen Adenovirus der Gruppe I und/oder Gruppe II behandelt werden können., bzw. die Adenoviren für die Herstellung eines Medikamentes zur Behandlung derselben verwendet werden können, wobei die Adenoviren eine für YB-1 codierende Nukleinsäure exprimieren. Es existieren somit bevorzugt drei Kategorien von Zellen und damit auch Tumoren, in denen die erfindungsgemäßen Adenoviren der Gruppe I und II replizieren können bzw. die unter Verwendung dieser Adenoviren behandelt, bevorzugterweise lysiert werden können:
- Gruppe A:: Zellen, die Zellzyklus-unabhängig YB-1 im Kern aufweisen;
- Gruppe B:: Zellen, die YB-1 nicht im Kern aufweisen, insbesondere nicht Zellzyklus-unabhängig im Zellkern aufweisen, aber dereguliertes YB-1 aufweisen; und
- Gruppe C:: Zellen, Zellen, die YB-1 nicht im Kern aufweisen, insbesondere nicht Zellzyklus-unabhängig im Zellkern aufweisen, und kein dereguliertes YB-1 aufweisen.

Für die Zellen der Gruppe A können die erfindungsgemäßen Adenoviren, insbesondere die Adenoviren der Gruppe I, zur Replikation oder Lyse verwendet werden, die kein zusätzliches YB-1 exprimieren. Es ist jedoch auch möglich, dass solche erfindungsgemäßen Adenoviren, insbesondere die Adenoviren der Gruppe I, zur Replikation oder Lyse verwendet werden, die zusätzliches YB-1 exprimieren. Gleiches gilt für die Gruppe B. Der Grund scheint, ohne im folgenden darauf festgelegt sein zu wollen, darin zu liegen, dass infolge der Wirkung des E1B-Proteins, insbesondere des E1B55K-Proteins, und/oder des E4-Proteins, insbesondere des E4orf6-Proteins, eine wirksame Replikation durch Lokalisation von YB-1 im Kern bzw. Transfer desselben in den Kern gewährleistet wird. Das YB-1, das von den Adenoviren zusätzlich exprimiert wird, unterstützt diese Prozess.

Im Falle der Gruppe C werden bevorzugterweise die erfindungsgemäßen Adenoviren, insbesondere die Adenoviren der Gruppe I, zur Replikation oder Lyse verwendet werden, die zusätzliches YB-1 exprimieren. Der Grund hierfür scheint, ohne wiederum darauf festgelegt sein zu wollen, darin zu bestehen, dass die obigen Prozesse der viralen Replikation vor dem konkreten zellulären Hintergrund nicht in dem Umfang ablaufen, dass eine wirksame Replikation ablaufen könnte. Erst mit der Zurverfügungstellung des von YB-1 codierten bzw. exprimierten YB-1 kommt es zu einer wirksamen Replikation, wobei der zugrundeliegende Mechanismus darin zu bestehen scheint, dass Überexpression von YB-1 zur Kernlokalisation von YB-1 führt, wie auch von Bargou [Bargout R.C. et al., Nature Medicine 1997,3,447-450) und Jürchott [Jürchott K. et al., JBC 2003, 278, 27988-27996] beschrieben.

Es ist im Rahmen der vorliegenden Erfindung, wenn einige der den Tumor aufbauenden Zellen YB-1 entweder inhärent oder nach Induktion bzw. aktivem Einführen in den Kern dort aufweisen oder aber hinsichtlich YB-1 dereguliert im Sinne der vorliegenden Offenbarung vorliegt so daß etwa 5 % oder ein jeglicher Prozentsatz darüber, d. h. 6 %, 7 %, 8 % etc. der den Tumor aufbauenden Zellen derartige YB-1 Kern-positive Zellen oder Zellen, in denen YB-1 dereguliert vorliegt. Für eine Reihe von Tumoren, wie beispielsweise Brusttumor, Osteosarcom, Ovar, Synovialkarzinom oder Lungenkarzinom kann der Anteil der Zellen des Tumors, die YB-1 dereguliert aufweisen oder eine vom Zellzyklus unahängige Kernlokalisation von YB-1 aufweisen, etwa 30 bis 50 % betragen [Kohno K. et al., BioEssays 2003, 25, 691-698]. Derartige Tumoren können in besonders bevorzugterweise mit den erfindungsgemäβen Adenoviren behandelt werden. Die Kernlokalisation von YB-1 kann durch Stress von außen bzw. lokal appliziertem Stress induziert werden. Diese Induzierung kann beispielsweise durch Bestrahlung, insbesondere UV-Bestrahlung, Anwendung von Zytostatika, wie sie unter anderem hierin ebenfalls offenbart sind, und Hyperthermie erfolgen. Im Zusammenhang mit Hyperthermie ist beachtlich, dass diese zwischenzeitlich sehr spezifisch, insbesondere örtlich spezifisch, realisiert werden kann und somit ebenfalls spezifisch einen Kerntransport von YB-1 in den Zellkern bedingen kann und infolgedessen die Voraussetzungen für eine Replikation des Adenovirus und damit einer Zell- und Tumorlyse, die bevorzugt lokal beschränkt erfolgt, gegeben sind (Stein U, Jurchott K, Walther W, Bergmann S, Schlag PM, Royer HD. J Biol Chem. 2001,276(30):28562-9; Hu Z, Jin S, Scotto KW. J Biol Chem. 2000 Jan 28; 275(4):2979-85; Ohga T, Uchiumi T, Makino Y, Koike K, Wada M, Kuwano M, Kohno K. J Biol Chem. 1998, 273(11):5997-6000).

Das erfindungsgemäße Medikament würde somit auch an solche Patienten und Patientengruppen verabreicht werden bzw. wäre für solche bestimmt, bei denen durch geeignete Vor- oder Nachbehandlungen oder gleichzeitige Behandlung ein Transport von YB-1, insbesondere in die entsprechenden Tumorzellen, bedingt werden würde bzw. eine dereguliertes YB-1 in der Zelle generiert werden würde.

Auf der Grundlage der hierein gegebenen technischen Lehre ist es für den Fachmann im Rahmen seiner Fähigkeiten, geeignete Modifikationen insbesondere von E1A vorzunehmen, die beispielsweise Deletionen oder Punktmutationen umfassen können, um so verschiedene Ausführungsformen der einsetzbaren Adenoviren zu erzeugen.

Wie bereits vorstehend ausgerührt, sind die Adenoviren der Gruppe I und/oder der Gruppe II in der Lage, in solchen Zellen bzw. zellulären Systemen zu replizieren, die YB-1 im Kern aufweisen. Für die Frage, inwieweit diese auch erfindungsgemäß verwendeten Adenoviren zur Replikation und damit zur Tumorlyse in der Lage sind, ist der Status der Zellen hinsichtlich des Vorhandenseins oder Nichtvorhandenseins von Rb, d. h. des Retinoblastom-Tumorsupressor-Produktes, unabhängig. Weiterhin ist es im Rahmen der erfindungsgemäßen Verwendung der besagten Adenoviren nicht erforderlich, auf den p53-Status der infizierten, zu infizierenden oder zu behandelnden Zellen abzustellen, da bei Verwendung der hierin offenbarten adenoviralen Systeme im Zusammenhang mit YB-1-Kern-positiven Zellen, d.h. Zellen, die unabhängig vom Zellzyklus YB-1 im Kern aufweisen, dieser ebenso wie der Rb-Status auf das Replikationsgeschehen des Adenovirus keinen Einfluss für die Ausführung der hierin offenbarten technischen Lehre hat.

Das transaktivierernde Onkogen bzw. Onkogenprotein, insbesondere E1A, bevorzugterweise der Adenoviren der Gruppe II kann dabei entweder unter der Kontrolle der eigenen natürlichen adenoviralen Promotoren stehen und/oder aber über einen Tumor- oder Gewebe-spezifischen Pro-motor gesteuert werden. Geeignete nicht-adenovirale Promotoren können ausgewählt sein aus der Gruppe, die Cytomegalovirus-Promotor, RSV-(Rous sarcoma. Virus) - Promotor, Adenovirus-basierender Promotor Va I und den nicht-viralen YB-1-Promotor (Makino Y. et al., Nucleic Acids Res. 1996, 15, 1873-1878) umfasst. Weitere Promotoren, die im Zusammenhang mit einem jeden Aspekt der hierin offenbarten Erfindung verwendet werden können, stellen der Telomerase-Promotor, der Alpha-Fetoprotein (AFP)-Promotor, der Caecinoembryonic Antigen Promotor (CEA) (Cao, G., Kuriyama, S., Gao, J., Mitoro, A., Cui, L., Nakatani, T., Zhang, X., Kikukawa, M., Pan, X., Fukui, H., Qi, Z. Int. J. Cancer, 78, 242-247, 1998), der L-Plastin-Promotor (Chung, I., Schwartz, PE., Crystal, RC., Pizzorno, G, Leavitt, J., Deisseroth, AB. Cancer Gene Therapy, 6, 99-106, 1999), Argenin-Vasopressin-Promotor (Coulson, JM, Staley, J., Woll, PJ. British J. Cancer, 80, 1935-1944, 1999), E2f-Promotor (Tsukada et al. Cancer Res., 62, 3428 - 3477, 2002), Uroplakin II Promotor (Zhang et al., Cancer Res., 62, 3743-3750, 2002) und der PSA-Promotor (Hallenbeck PL, Chang, YN, Hay, C, Golightly, D., Stewart, D., Lin, J., Phipps, S., Chiang, YL. Human Gene Therapy, 10, 1721-1733, 1999), Tyrosinase-Promotor (Nettelbeck, DM. Anti-Cancer-Drugs, 14, 577-584, 2003), Cyclooxygenase-2 Promotor (Nettelbeck, DM., Rivera, AA, Davydova, J., Dieckmann, D., Yamamoto, M., Curiel, DT. Melanoma Res., 13,287-292, 2003) und induzierende Systeme wie beispielssweise Tetracyclin (Xu, XL., Mizuguchi, H., Mayumi, T., Hayakawa, T. Gene, 309, 145-151, 2003) dar. Ferner stellt der in der deutschen Patentanmeldung DE 101 50 984.7 beschriebenen YB-1 abhängigen E2-late-Promotor von Adenoviren einen Promotor dar, wie er im Rahmen der vorliegenden Erfindung verwendet werden kann.

Hinsichtlich des Telomerase-Promotors ist bekannt, dass dieser in humanen Zellen von zentraler Bedeutung ist. So wird die Telomeraseaktivität durch die transkriptionelle Kontrolle des Telomerase reverse Transkriptase-Gens (hTERT), welches die katalytische Untereinheit des Enzyms darstellt, reguliert. Die Expression der Telomerase ist in 85% der humanen Tumorzellen aktiv. Im Unterschied dazu ist sie in den meisten normalen Zellen inaktiv. Ausgenommen davon sind Keimzellen und embryonales Gewebe (Braunstein, I. et al., Cancer Research, 61, 5529-5536, 2001; Majumdar, A. S. et al., Gene Therapy 8, 568-578, 2001). Genaue Untersuchungen am hTERT-Promotor haben gezeigt, dass Fragmente des Promotors von 283 bp bzw. 82 bp vom Initiationscodon entfernt für eine spezifische Expression in Tumorzellen ausreichend sind (Braunstein I. et al.; Majumdar AS et al., aaO). Daher eignet sich dieser Promotor bzw. die spezifischen Fragmente, um eine spezifische Expression eines Gens und insbesondere eines Transgens, bevorzugterweise eines hierin offenbarten Transgens, nur in Tumorzellen zu erzielen. Der Promotor soll die Expression des modifizierten Onkogens, bevorzugt des E1A-Onkogenproteins, nur in Tumorzellen ermöglichen. Auch ist die Expression eines Transgens, insbesondere eines solchen, das ausgewählt ist aus der Gruppe, die E4orf6, E1B55kD, ADP und YB-1 umfasst, in einem adenoviralen Vektor unter einem dieser Promotoren in einer Ausführungsform vorgesehen. Es ist auch im Rahmen der vorliegenden Erfindung, dass der Leserahmen des transaktivierenden Onkogenproteins, insbesondere des E1A-Proteins im Leserahmen (engl. "in frame") mit einem oder mehreren der Genprodukte des adenoviralen Systems ist. Der Leserahmen des transaktivierenden E1A-Proteins kann jedoch auch unabhängig davon sein.

Die verschiedenen Transgene, so auch E1B55kD, E4orf6, ADP und dergleichen, insbesondere wenn es sich dabei um virale Gene handelt, können grundsätzlich aus einem jeden entsprechenden Virus, insbesondere Adenovirus, cloniert werden. Im Stand der Technik sind darüber hinaus eine Vielzahl von Plasmiden beschrieben, die die entsprechenden Gene enthalten, und aus denen diese sodann entnommen und in die erfindungsgemäßen Adenoviren und die erfindungsgemäße verwendeten Adenoviren eingefügt werden können. Ein Beispiel für ein Plasmid, das E1B55kD exprimiert, ist beispielsweise beschrieben von Dobbelstein, M. et al., EMBO Journal, 16, 4276-4284, 1997. Die codierende Region des E1B55K-Gens kann zusammen mit der 3'-nicht-codierenden Region beispielsweise aus diesem Gen mittels Bam HI aus dem Plasmid pDCRE1B herausgeschnitten werden. Das entsprechende Fragment umfassend das E1B55kD-Gen sowie die 3'-nicht-codierende Region entspricht den Nukleotiden 2019 bis 4107 des Adenovirus Typ 5. Es ist jedoch auch beispielsweise im Rahmen der vorliegenden Erfindung, dass das E1B55kD-Gen mittels der Restriktionsenzyme Bam HI und BfrI aus diesem Plasmid geschnitten wird und anschließend in den Adenovirus eincloniert wird.

Es ist im Rahmen der vorliegenden Erfindung, dass, für den Fall, dass von den erfindungsgemäßen Viren gesprochen wird, dies sowohl die dafür codierende Nukleinsäuren, wie auch die adenoviralen Partikel, bevorzugterweise einschließlich der entsprechenden Nukleinsäure bezeichnet. Die entsprechende Nukleinsäure kann im Übrigen auch in einem anderen Vektor integriert vorliegen.

Es ist im Rahmen der vorliegenden Erfindung, dass die verschiedenen vorstehend beschriebenen Promotoren auch im Zusammenhang mit den verschiedenen Ausführungsformen der erfindungsgemäßen Adenoviren, bevorzugterweise der Adenoviren der Gruppe I, verwendet werden, insbesondere dann, wenn ein anderer Promotor als derjenige verwendet werden soll, der die Expression des jeweiligen Proteins oder Expressionsproduktes im Wildtyp-Adenovirus steuert. Die vorstehend aufgeführten Promotoren sind somit geeignete heterologe Promotoren im Sinne der vorliegenden Erfindung. In bevorzugten Ausführungsformen der erfindungsgemäßen Adenoviren, insbesondere den Adenoviren der Gruppe I, ist vorgesehen, dass bei Anwendung der Adenoviren bei Zellen der Gruppen A und B, wie vorstehend definiert, so erfolgt, dass die Promotoren für die Expression des E1B-Proteins und/oder des E4-Proteins von einem derartigen heterologen Promotor erfolgt, wohingegen bevorzugt, aber nicht ausschließlich, die Expression des E1A-Proteins von YB-1 gesteuert wird. Die Expression des E1A-Proteins steht in diesen und anderen Ausführungsformen bevorzugt unter der Kontrolle eines von YB-1 steuerbaren Promotors wie beispielsweise dem adenoviralen E2-late-Promotor. Dies gilt auch für den Fall, dass das E1B-Protein und/oder das E4-Protein in einer Expressionskassette exprimiert wird/werden.

In bevorzugten Ausführungsformen der erfindungsgemäßen Adenoviren, insbesondere den Adenoviren der Gruppe I, ist vorgesehen, dass bei Anwendung der Adenoviren bei Zellen der Gruppe C jeweils unabhängig voneinander ein Tumor- Organ- oder Gewebe-spezifischer Promotor ist. Dabei ist es ausreichend, wenn zumindest einer der Promotoren, die die Expression des E1B-Proteins, des E4-Proteins und/oder des E1A-Proteins steuern ein derartiger spezifischer Promotor ist. Durch diese Tumor-, Organ- oder Gewebespezifität wird gewährleistet, dass eine Replikation der erfindungsgemäßen Adenoviren nur in den Zellen des jeweiligen Tumors, Organs oder Gewebes erfolgt und darüber hinaus kein weiteres Gewebe durch Replikation der Adenoviren geschädigt, beispielsweise lysiert wird. Bevorzugtererweise wird noch ein zweites und bevorzugtererweise werden alle drei Proteine von einem derartigen Tumor- Organ- oder Gewebespezifischen Promotor gesteuert. Mit derartigen Adenoviren ist es möglich, auch jene Zellen zu lysieren, die keinen Tumor bilden oder zu einem solchen werden könnten, sondern aus anderen Gründen, bevorzugterweise medizinischen Gründen aus einem Organismus, bevorzugterweise einem Säugetierorganismus und bevorzugtererweise einem menschlichen Organismus, entfernt oder zerstört werden sollen, beispielsweise weil sie einen unerwünschten Faktor produzieren oder ein im zu großen Maße produzieren.

Hinsichtlich der Eigenschaft der Zellen, zu deren Lyse die hierin beschriebenen erfindungsgemäßen Adenoviren erfindungsgemäß verwendet werden, ist vorgesehen, dass diese in einer Ausführungsform eine Resistenz, bevorzugterweise eine Mehrfach- oder Vielfach-Resistenz zeigen.

Resistenzen, wie sie hierin bezeichnet werden und für die zu behandelnden Tumoren bzw. Patienten charakteristisch sind, sind solche die durch die folgenden Gene vermittelt werden, aber nicht auf diese beschränkt: MDR, MRP, Topoisomerase, BCL2, Glutathion-S-Transferase (GST), Proteinkinase C (PKC). Da die Wirkung von Zytostatika unter anderem auf der Induktion der Apoptose beruht, spielt die Expression von Apoptose-relevanten Genen bei der Ausbildung der Resistenz eine bedeutende Rolle, so dass auch die folgenden Faktoren diesbezüglich relevant sind, nämlich Fas, die BCL2-Familie, HSP 70 und EGFR [Kim et al., Cancer Chemther. Pharmacol. 2002, 50, 343-352].

Dass YB-1 bei resistenten Tumorzellen im Vergleich zu nicht-resistenten Tumorzellen in seiner Expression stark erhöht ist, wird von Levenson et al. [Levenson, V.V. et al., Cancer Res. , 2000, 60, 5027-5030] beschrieben.

Resistenz, wie hierin verwendet, bezeichnet dabei bevorzugterweise eine Resistenz gegen die hierin beschriebenen Zytostatika. Diese Mehrfach-Resistenz geht bevorzugterweise mit der Expression, bevorzugter Weise einer Überexpression, des membranständigen Transporlproteins P-Glycoprotein einher, welches als Marker für die Bestimmung entsprechender Zellen und damit auch von diesem aufweisenden Tumoren bzw. entsprechende Patientengruppen herangezogen werden kann. Der Begriff Resistenz, wie er hierin verwendet wird, umfasst dabei sowohl die auch als klassische Resistenz bezeichnete, durch das P-Glycoprotein vermittelte Resistenz, wie auch die auch als atypische Resistenz bezeichnete Resistenz, die durch MRP vermittelte oder andere, nicht-P-Glycoprotein vermittelte Resistenzen umfasst. Ein weiterer Marker, der mit der Expression von YB-1 korreliert, ist die Topoisomerase II alpha. Insoweit kann in einem Screenen-Verfahren, um zu bestimmen, ob ein Patient mit den Adenoviren erfindungsgemäß mit Aussicht auf Erfolg behandelt werden kann, an Stelle von der bzw. in Ergänzung zur Bestimmung von YB-1 im Kern die Expression von Topoisomerase II alpha herangezogen werden. Ein Marker, der grundsätzlich ähnlich wie das P-Glycoprotein verwendet werden kann, ist MRP. Ein weiterer Marker, zumindest in dem Umfang, als dass colorectrale Karzinomzellen oder Patienten mit einem Colorectalcarcinom betroffen sind, ist PCNA (engl. proliferating cell nuclear antigen (Hasan S. et al., Nature, 15,387-391,2001.), wie beispielsweise beschrieben von Shibao K. et al. (Shibao K et al., Int. Cancer, 83, 732-737, 1999). Schließlich ist, zumindest für den Bereich der Brustkrebs- und Osteosarcoma-Zellen die Expression von MDR (engl. multiple drug resistance) ein Marker im vorstehend beschriebenen Sinne (Oda Y et al., Clin. Cancer Res., 4, 2273-2277, 1998). Ein weiterer möglicher Marker, der erfindungsgemäß verwendet werden kann, ist p73 (Kamiya, M., Nakazatp, Y., J Neurooncology 59, 143-149 (2002); Stiewe et al., J. Biol. Chem., 278, 14230-14236, 2003).

Schließlich sei noch auf YB-1 selbst als prognostischen Marker bei Brustkrebs hingewiesen, der im Rahmen der vorliegenden Erfindung verwendet werden kann. Nur bei Patientinnen mit erhöhter Expression von YB-1 im Primärtumor tritt ein Rezidiv nach Operation und Chemotherapie auf [Janz M. et al. Int. J. Cancer 2002, 97, 278-282].

Es ist somit ein besonderer Vorteil der vorliegenden Erfindung, dass auch jene Patienten unter Anwendung der erfindungsgemäßen Verwendung der Adenoviren, wie hierin beschrieben, therapiert werden können, die ansonsten im medizinisch-klinischen Sinne als "austherapiert" gelten und somit eine weitgehende Behandlung der Tumorerkrankung nach den Methoden des Standes der Technik mit Aussicht auf Erfolg nicht mehr möglich ist, insbesondere die Verwendung von Zytostatika oder Bestrahlung sinnvoller Weise nicht mehr möglich ist bzw. nicht mehr erfolgreich durchgeführt werden kann im Sinne einer Beeinflussung bzw. Verringerung des Tumors. Der Begriff des Tumors bezeichnet hierin allgemein auch eine jegliche Tumor- oder Krebserkrankung, die entweder inhärent YB-1 im Zellkern enthält oder aber durch Realisieren exogener Maßnahmen, wie hierin offenbart, YB-1 im Zellkern, bevorzugterweise unabhängig vom Zellzyklus, und/oder dereguliertes YB-1 aufweist.

Weiterhin können die hierin beschriebenen Viren zur Behandlung grundsätzlich von Tumoren verwendet werden.

Die Tumoren, die durch die hierin beschriebenen Viren insbesondere behandelt werden können, sind bevorzugterweise jene Tumoren, die aus der Gruppe ausgewählt sind, die Tumore des Nervensystems, okulare Tumoren, Tumore der Haut, Tumore der Weichteile, gastrointestinale Tumore, Tumore des respiratorischen Systems, Tumore des Skeletts, Tumore des endokrinen Systems, Tumore des weiblichen Geschlechtssystem, Tumore der Mammadrüse, Tumore des männlichen Geschlechtssystems, Tumore des harnableitenden Systems, Tumore des haematopoetischen Systems sowie vermischte und embryonale Tumore umfasst. Dabei ist es im Rahmen der vorliegenden Erfindung, dass es sich bei diesen Tumoren insbesondere um resistente Tumoren handelt, wie insbesondere hierin definiert.

Die Gruppe der Tumore des Nervensystems umfasst dabei bevorzugterweise:
1. Tumore der Schädelhöhle sowie des Gehirns (Intracranium), bevorzugtererweise Astrozytom, Oligodendrogliom, Menigiom, Neuroblastom, Ganglioneurom, Ependymom, Schwannom, Neurofibrom, Hemangioblastom,Lipom, Craniopharyngiom, Teratom und Chordom;
2. Tumore des Rückenmarkes und des Wirbelkanals, bevorzugtererweise Glioblastom, Meningiom, Neuroblastom, Neurofibrom, Osteosarkom, Chondrosarkom, Hemangiosarkom, Fibrosarkom und Multiple Myelome; und
3. Tumore der peripheren Nerven, bevorzugtererweise Schwannom, Neurofibrom, Neurofibrosarkom und Perineurale Fibroblastome.

Die Gruppe der okularen Tumore umfasst dabei bevorzugterweise:
1. Tumore der Augenlider und der Liddrüsen, bevorzugtererweise Adenom, Adenokarzinom, Papillom, Histiozytom, Mastzelltumor, Basalzelltumor, Melanom, Plattenepithelkarzinom, Fibrom und Fibrosarkom;
2. Tumore der Konjunktiven und der Nickhaut, bevorzugtererweise Plattenepithelkarzinom, Hemangiom, Hemangiosarkom, Adenom, Adenokarzinom, Fibrosarkom, Melanom und Papillom; und
3. Tumore der Orbita, des Nervus opticus und des Augapfels, bevorzugtererweise Retinoblastom, Osteosarkom, Mastzelltumor, Meningiom, Retikulozelltumor, Gliom, Schwannom, Chondrom, Adenokarzinom, Plattenepithelkarzinom, Plasmazelltumor, Lymphom, Rhabdomyosarkom und Melanom.

Die Gruppe der Tumore der Haut umfasst dabei bevorzugterweise:
Tumore des Histiocytom, Lipom, Fiborsarkom, Fibrom, Mastzell Tumor, Malignes Melanom, Papillom, Basalzelltumor, Keratoakanthom, Hemangioperizytom, Tumore der Haarfollikel, Tumore der Schweißdrüsen, Tumore der Talgdrüsen, Haemangiom, Haemangiosarkom, Lipom, Liposarkom, Malignes fibröses Histiozytom, Plasmazytom und Lymphangiom.

Die Gruppe der Tumore der Weichteile umfasst dabei bevorzugterweise:
Tumore des alveoläres Weichteilsarkom, epitheloidzelliges Sarkom, Chondrosarkom der Weichteile, Osteosarkom der Weichteile, Ewing-Sarkom der Weichteile, primitive neuroektodermale Tumore (PNET), Fibrosarkom, Fibrom, Leiomyosarkom, Leiomyom, Liposarkom, malignes fibröses Histiozytom, malignes Hemangioperizytom, Hemangiom, Hemangiosarkom, malignes Mesenchymom, maligner peripherer Nervenscheidentumor (MPNST, malignes Schannom), malignes melanozytisches Schwannom, Rhabdomyosarkom, Synovialsarkom, Lymphangiom und Lymphangiosarkom.

Die Gruppe des Gastrointestinale Tumors umfasst dabei bevorzugterweise:
1. Tumore der Mundhöhle und Zunge, bevorzugtererweise Plattenepithelkarzinom, Fibrosarkom, Merkelzelltumor, Induktives Fibroameloblastom, Fibrom, Fibrosarkom, Virale Papillomatose, Idiopathische Papillomatose, Nasopharyngeale Polypen, Leiomyosarkom, Myoblastom und Mastzelltumor;
2. Tumore der Speicheldrüsen, bevorzugtererweise Adenokarzinom;
3. Tumore des Oesophagus, bevorzugtererweise Plattenepithelkarzinom, Leiomyosarkom, Fibrosarkom, Osteosarkom, Barrettkarzinom und paraoesophageale Tumore;
4. Tumore der exocrines Pankreas, bevorzugtererweise Adenokarzinom; und
5. Tumore des Magens, bevorzugtererweise Adenokarzinom, Leiomyom, Leiomyosarkom und Fibrosarkom.

Die Gruppe der Tumore des respiratorischen Systems umfasst dabei bevorzugterweise:
1. Tumore der Nase und Nasenhöhle, des Kehlkopfes und der Trachea, bevorzugtererweise Plattenepithelkarzinom, Fibrosarkom, Fibrom, Lymphosarkom, Lymphom, Hemangiom, Hemangiosarkom, Melanom, Mastzelltumor, Osteosarkom, Chondrosarkom, Oncozytom (Rhabdomyom), Adenokarzinome und Myoblastom; und
2. Tumore der Lunge, bevorzugtererweise Plattenepithelkarzinom, Fibrosarkom, Fibrom, Lymphosarkom, Lymphom, Hemangiom, Hemangiosarkom, Melanom, Mastzelltumor, Osteosarkom, Chondrosarkom, Oncozytom (Rhabdomyom), Adenokarzinome, Myoblastom, kleinzelliges Karzinom, nicht-kleinzelliges Karzinom, bronchiales Adenokarznom, bronchoalveoläres Adenokarzinom und alveoläres Adenokarzinom.

Die Gruppe der Tumore des Skeletts umfasst dabei bevorzugterweise:
Osteosarkom, Chondrosarkom, Parosteales Osteosarkom, Hemangiosarkom, Synovialzellsarkom, Hemangiosarkom, Fibrosarkom, Malignes Mesenchymom, Riesenzelltumor, Osteom und multilobulares Osteom.

Die Gruppe der Tumore des endokrinen Systems umfasst dabei bevorzugterweise:
1. Tumore der Schilddrüse/Nebenschilddrüse, bevorzugtererweise Adenome und Adenokarzinome;
2. Tumore der Nebenniere, bevorzugtererweise Adenom, Adenokarzinom und Pheochromozytom (Nebennierenmarktumor);
3. Tumore der Hypothalamus/Hypophyse, bevorzugtererweise Adenom und Adenokarzinom;
4. Tumore des endokrines Pankreas, bevorzugtererweise Insulinom (Betazelltumor, A-PUDom) und Zollinger-Ellison Syndrom (Gastrin sezernierender Tumor der Deltazellen des Pankreas); und
5. sowie multiple endokrine Neoplasien (MEN) und Chemodectom.

Die Gruppe der Tumore des weiblichen Geschlechtssystems umfasst dabei bevorzugterweise:
1. Tumore der Ovarien, bevorzugterweise Adenom, Adenokarzinom, Zystadenom, und undifferenzierte Karzinome;
2. Tumore der Uterus, bevorzugtererweise Leiomyom, Leiomyosarkom, Adenom, Adenokarzinom, Fibrom, Fibrosarkom und Lipom;
3. Tumore der Zervix, bevorzugtererweise Adenokarzinom, Adenom, Leiomyosarkom und Leiomyom;
4. Tumore der Vagina und Vulva, bevorzugtererweise Leiomyom, Leiomyosarkom, Fibroleiomyom, Fibrom, Fibrosarkom, Polypen und Plattenepithelkarzinom.

Die Gruppe der Tumore der Mammadrüse umfasst dabei bevorzugterweise:
Fibroadenom, Adenom, Adenokarzinom, mesenchymale Tumore, Karzinome, Karzinosarkome.

Die Gruppe der Tumore des männlichen Geschlechtssystems umfasst dabei bevorzugterweise:
1. Tumore der Hoden, bevorzugtererweise Seminom, Interstitialzelltumor und Sertolizelltumor;
2. Tumore der Prostata, bevorzugtererweise Adenokarzinome, undifferenzierte Karzinome, Plattenepithelkarzinome, Leiomyosarkome und Transitionalzellkarzinom; und
3. Tumore des Penis und der externen Genitalien, bevorzugtererweise Mastzelltumor und Plattenepithelkarzinom.

Die Gruppe der Tumore des hamableitenden Systems umfasst dabei bevorzugterweise:
1. Tumore der Niere, bevorzugtererweise Adenokarzinom, Transitionalzellkarzinom (epitheliale Tumore), Fibrosarkom, Chondrosarkom (mesenchymale Tumore), Wilm's Tumor, Nephroblastom und embryonales Nephrom (embryonal pluripotente Blastome);
2. Tumore der Urether, bevorzugtererweise Leiomyom, Leiomyosarkom, Fibropapillom, Transitionalzellkarzinom;
3. Tumore der Harnblase, bevorzugtererweise Transitionalzellkarzinom, Plattenepithelkarzinom, Adenokarzinom, Botryoid (Embryonales Rhabdomyosarkom), Fibrom, Fibrosarkom, Leiomyom, Leiomyosarkom, Papillom und Hemangiosarko; und
4. Tumore der Urethra, bevorzugtererweise Transitionalzellkarzinom, Plattenepithelkarzinom und Leiomyosarkom.

Die Gruppe der Tumore des hämatopoetischen Systems umfasst dabei bevorzugterweise:
1. Lymphome, Lymphatische Leukämie, Nichtlymphatische Leukämie, Myeloproliferative Leukämie, Hodgkin's Lymphom, Non-Hodgkin's Lymphom.

Die Gruppe der vermischten und embryonalen Tumore umfasst dabei bevorzugterweise:
Hemangiosarkome, Thymom und Mesotheliom.

Ganz besonders bevorzugterweise sind diese Tumoren ausgewählt aus der Gruppe, die Brustkrebs, Ovarialkarzinom, Prostatakarzinom, Osteosarkom, Glioblastom, Melanom, kleinzelliges Lungenkarzinom und Kolorektalkarzinom umfasst. Weitere Tumoren sind jene, die resistent, wie hierin beschrieben, sind, bevorzugt Gene, die mehrfach resistent sind, insbesondere auch solche Tumoren der vorstehend beschriebenen Gruppe. Besonders bevorzugte Tumoren sind dabei auch jene, die aus der Gruppe ausgewählt sind, die Brusttumore, Knochentumore, Magentumore, Darmtumore, Gallenblasentumore, Bauspeicheldrüsentumore, Lebertumore, Nierentumore, Gehimtumore, Eierstocktumore, Haut- und Hautanhangsorgantumore, Kopf-/Nackentumore, Gebärmuttertumore, Synovialtumore, Kehlkopftumore, Speiseröhrentumore, Zungentumore und Prostatatumore umfasst. Dabei ist bevorzugt, dass diese Tumoren hinsichtlich ihrer Ausprägung solche sind, wie sie hierin insgesamt offenbart sind.

Die erfindungsgemäßen Adenoviren, insbesondere der Adenoviren der Gruppe I, und die erfindungsgemäß verwendeten Adenoviren, insbesondere die Adenoviren der Gruppe II

Die Verwendung der hierin offenbarten Adenoviren, insbesondere der Gruppe I und/oder Gruppe II, als Medikamente und besondere bei systemischer Anwendung kann durch ein geeignetes Targeting der Adenoviren verbessert werden. Die Infektion von Tumorzellen durch Adenoviren hängt bis zu einem bestimmten Umfang unter anderem vom Vorhandensein des Coxackievirus-Adenovirus Rezeptor CAR und bestimmten Integrinen ab. Sobald diese stark in Zellen, insbesondere Tumorzellen exprimiert werden, ist eine Infektion bereits bei sehr geringen Titern (pfu/Zelle) möglich. Verschiedene Strategien sind bisher durchgeführt worden, um ein sogenanntes Re-targeting der rekombinanten Adenoviren zu erzielen, z. B. durch Insertion von heterologen Sequenzen in der fiber knob region, Verwendung von bi -spezifischen Antikörpern, beschichten der Adenoviren mit Polymeren, Einfügen von Liganden im Ad fiber, die Substitution des Serotyps 5 knop bzw. 5 fiberb shaft und knop mit dem Serotyp 3 knop bzw. Ad 35 fiber shaft and knob und Modifikationen des penton base (Nicklin S. A. et al., Molecular Therapy 2001,4, 534-542; Magnusson, M. K. et. al., J. of Virology 2001,75, 7280-7289; Barnett B. G. et al., Biochimica et Biophysica Acta 2002, 1575, 1-14). Die Realisierung derartiger weiterer Ausgestaltungen bzw. Merkmale bei den erfindungsgemäßen Adenoviren und den erfindungsgemäß verwendeten Adenoviren, insbesondere bei den Adenoviren der Gruppe I und/oder der Gruppe II, in ihren verschiedenen Aspekten der vorliegenden Erfindung, ist im Rahmen der vorliegenden Erfindung.

Ebenfalls offenbart ist ein Verfahren zum Screenen von Patienten, die mit einem modifizierten Adenovirus, d.h. einem Adenovirus, wie er erfindungsgemäß verwendet wird, wie beispielsweise AdA24, dl922-947, E1Ad/01/07, CB016 oder die im europäischen Patent EP 0931 830 beschriebenen Viren, und/oder einem Adenovirus der Gruppe I und/oder der Gruppe II behandelbar sind, wobei das Verfahren die folgenden Schritte umfasst:
- Untersuchen einer Probe des Tumorgewebes und
- Festellen, ob YB-1 im Kern Zellzyklus-unabhängig lokalisiert ist oder die Zellen dereguliertes YB-1 aufweisen.

Anstelle von oder in Ergänzung zu YB-1 kann auch das Vorhandensein der vorstehend beschriebenen Marker festgestellt werden.

In dem Falle, dass das Tumorgewebe oder ein Teil davon YB-1 im Zellkern, insbesondere Zellzyklus-unabhängig, lokalisiert aufweist oder dereguliertes YB-1 aufweist, können die hierin offenbarten Adenoviren, insbesondere die Adenoviren der Gruppe I und/oder der Gruppe II erfindungsgemäß verwendet werden.

In einer Ausführungsform des Verfahrens ist vorgesehen, dass die Untersuchung des Tumorgewebes unter Verwendung eines Mittels erfolgt, das ausgewählt ist aus der Gruppe, die Antikörper gegen YB-1, Aptamere gegen YB-1, Spiegelmere gegen YB-1 sowie Anticaline gegen YB-1 umfasst. Grundsätzlich die gleichen Mittel können für die entsprechenden Marker hergestellt bzw. verwendet werden. Die Herstellung von Antikörpern, insbesondere monoklonalen Antikörpern, ist den Fachleuten auf dem Gebiet bekannt. Ein weiteres Mittel zum spezifischen Nachweis von YB-1 oder den Markern stellen Peptide dar, die mit hoher Affinität an ihre Zielstrukturen, im vorliegenden Falle YB-1 oder die besagten Marker, binden. Im Stand der Technik sind Verfahren bekannt, wie beispielsweise phage-display, um derartige Peptide zu erzeugen. Dabei wird typischerweise von einer Peptid-Bibliothek ausgegangen, wobei die einzelnen Peptide eine Länge von etwa 8 bis 20 Aminosäuren aufweisen und die Größe der Bibliothek etwa 10² bis 10¹⁸, bevorzugterweise 10⁸ bis 10¹⁵ verschiedene Peptide beträgt. Eine spezielle Form von an Zielmolekülen bindenden Polypeptiden stellen die sogenannten Anticaline dar, wie sie beispielsweise in der deutschen Patentamneldung DE 197 42 706 beschrieben sind.

Ein weiteres Mittel zum spezifischen Binden an YB-1 oder die entsprechenden, hierin offenbarten Marker und damit zum Nachweis einer Zellzyklus-unabhängigen Lokalisation von YB-1 im Zellkern sind die sogenannten Aptamere, d. h. D-Nukleinsäure, die auf RNA- oder DNA-Basis entweder als Einzelstrang oder als Doppelstrang vorliegen und spezifisch an ein Zielmolekül binden. Die Herstellung von Aptameren ist beispielsweise beschrieben im europäischen Patent EP 0 533 838. Eine Sonderform der Aptamere stellen die sogenannten Aptazyme dar, die beispielsweise beschrieben sind von Piganeau, N. et al. (2000), Angew. Chem. Int. Ed., 39, Nr. 29, Seiten 4369 - 4373. Dabei handelt es sich um eine spezielle Ausführungsform von Aptameren insoweit, als dass sie neben dem Aptameranteil noch einen Ribozymanteil aufweisen und nach Bindung oder Freisetzung des an den Aptamerteil bindenden Zielmoleküls der Ribozymanteil katalytisch aktiv wird und ein Nukleinsäuresubstrat spaltet, was mit der Erzeugung eines Signals einhergeht.

Eine weitere Form der Aptamere stellen sogenannte Spiegelmere dar, d. h. zielmolekülbindende Nukleinsäuren, die aus L-Nukleinsäuren hergestellt sind. Das Verfahren zur Herstellung dieser Spiegelmere ist beispielsweise beschrieben in WO 98/08856.

Die Probe des Tumorgewebes kann dabei durch Punktion oder durch einen chirurgischen Eingriff erhalten werden. Die Feststellung, ob im Kern YB-1 Zellzyklus-unabhängig lokalisiert ist, erfolgt dabei häufig unter Verwendung mikroskopischer Techniken und/oder mittels Immunhistoanalyse, typischerweise unter Verwendung von Antikörpern oder einem der weiteren vorstehenden Mitteln. Weitere Verfahren zum Nachweis, dass YB-1 im Kern und insbesondere dort Zellzyklus-unabhängig lokalisiert ist, sind dem Fachmann bekannt. Beispielsweise kann bei dem Durchmustern von gegen YB-1 gefärbten Gewebeschnitten die Lokalisation von YB-1 leicht erkannt werden. Dabei ergibt sich bereits infolge der Häufigkeit des Auftretens von YB-1 im Kern, dass es sich um eine Zellzyklus-unabhängige Lokalisation im Kern handelt. Eine weitere Möglichkeit zum Zellzyklus-unabhängigen Nachweis von YB-1 im Kern besteht in der Durchführung einer Färbung gegen YB-1 und Feststellen, ob YB-1 im Kern lokalisiert ist, und Durchführung der Bestimmung des Zellstadiums der Zellen. Dies bzw. die Detektion von YB-1 kann aber auch unter Verwendung der vorstehend genannten, gegen YB-1 gerichteten Mittel erfolgen. Der Nachweis der Mittel erfolgt dabei durch Verfahrensweisen, die den Fachleuten auf dem Gebiet bekannt sind. Dadurch, dass die besagten Mittel spezifisch gegen YB-1 gerichtet sind und insoweit nicht an andere Strukturen innerhalb der zu untersuchenden Probe, insbesondere der Zellen, binden, kann durch eine geeignete Markierung der Mittel deren Lokalisierung und infolge der spezifischen Bindung an YB-1 auch die Lokalisierung von YB-1 entsprechend nachgewiesen und festgestellt werden. Verfahren zum Markieren der Mittel sind den Fachleuten auf dem Gebiet bekannt. Die gleichen Techniken können auch verwendet werden, um zu bestimmen, ob und wenn ja, wieviele der Zellen der Probe dereguliertes YB-1 enthalten. Da dereguliertes YB-1 gegenüber nicht-deregulierten YB-1 auch eine Überexpression zeigt kann die relative Expression von YB-1 gegenüber einer Referenzprobe verwendet werden, um zu bestimmen, ob YB-1 in der untersuchten Zelle dereguliert vorliegt.

Es ist im Rahmen der vorliegenden Erfindung, dass die erfindungsgemäßen Viren, auch bei Erkrankungen, bevorzugterweise Tumorerkrankungen und bevorzugtererweise Tumorerkrankungen, bei denen zumindet ein Teil der Tumorzellen eine Mehrfach-Resistenz, insbesondere eine multidrug resistance aufweist, verwendet werden kann, bei denen YB-1 dereguliert vorliegt. Dies gilt auch für einen jeden anderen Aspekt, wie er hierin im Zusammenhang mit Zellen und Tumoren beschrieben ist, soweit sie sich auf Zellen und Erkrankungen bezieht, bei denen sichYB-1 im Zellkern befindet, bevorzugterweise unabhängig vom Zellzyklus im Kern befindet.

Die vorliegende Erfindung soll im folgenden anhand der Figuren und Beispiele weiter veranschaulicht werden, wobei sich daraus neue Merkmale, Ausführungsformen und Vorteile der Erfindung ergeben. Dabei zeigt
- Fig. 1: den strukturellen Aufbau der darin als AdE1/E3-minus Adenovirus bezeichneten adenoviralen Vektoren, die E1/E3-deletierte Adenoviren sind, Wildtyp-Adenovirus und Adenovirus dl520;
- Fig. 2: die Bindungsdomänen des E1A-Proteins hinsichtlich der Bindung von p300, p107 und p105;
- Fig. 3: U2OS-Zellen, welche nicht YB-1 im Kern aufweisen, nach Infektion mit dem darin als E1/E3-minus Ad5 bezeichneten E1/E3-deletierten Adenoviren Ad5 und dl520;
- Fig. 4: 257RDB-Zellen, welche YB-1 im Kern aufweisen, nach Infektion mit dem darin als E1/E3-minus Ad5 bezeichneten E1/E3-deletierten Adenoviren Ad5 und Adenovirus dl520;
- Fig. 5: 257RDB-Zellen und U2OS-Zellen, nach Infektion mit dem Adenovirus dl1119/1131;
- Fig. 6: das Ergebnis einer EMSA-Analyse, womit belegt wird, dass YB-1 in den vielfachresistenten Zellen bzw. Zelllinien 257RDB, 181 RDB, MCF-7Ad- im Zellkern vorhanden ist, wohingegen YB-1 in U2OS und HeLa-Zellen nicht im Kern vorhanden ist;
- Fig. 7: den strukturellen Aufbau des E1A-Proteins vom Wildtyp-Adenovirus, von Adenovirus dl520 und Adenovirus dl1119/1131;
- Fig. 8: ein Balkendiagramm, welches die Replikationseffizienz von Adenoviren bei Anwesenheit zusätzlich exprimierter viraler Proteine in Absolutzahlen zeigt; und
- Fig. 9: ein Balkendiagramm, welches die Steigerung der Replikationseffizienz von Adenoviren bei Anwesenheit zusätzlich exprimierter viraler Proteine zeigt;
- Fig. 10: mit U2OS-Zellen bewachsene Näpfen nach Kristallviolett-Färbung und Infektion mit dl520 mit 10 bzw. 30 pfu/Zelle bzw. Kontrolle (K) ohne Gabe von Daunorubicin bzw. mit Gabe von 40 ng Daunorubicin pro ml;
- Fig. 11: mit HeLa-Zellen bewachsene Näpfen nach Kristallviolett-Färbung und Infektion mit dl520 mit 10 bzw. 30 pfu/Zelle bzw. Kontrolle (K) ohne Gabe von Daunorubicin bzw. mit Gabe von 40 ng pro ml Daunorubicin;
- Fig. 12: ein Diagramm des Tumorvolumens über die Zeit von Tumoren verschiedenen Ursprungs (RDB257 und HeLa) nach Behandlung mit PBS bzw. dl520;
- Fig. 13: Aufnahmen von euthanasierten Mäusen, die einen Tumor auf der Grundlage von RDB257-Zellen entwickelten, nach Behandlung mit PBS bzw. mit 5 x 10⁸ pfu dl520;
- Fig. 14: das Ergebnis einer Southern Blot-Analyse eines Zellextraktes (von den sucutan gewachsenen Tumoren) von RDB257-Zellen und HeLa-Zellen nach Infektion mit dl520;
- Fig. 15: ein Balkendiagramm, welches die Replikationseffizienz bzw. die Partikelbildung von dl520 und Wildtyp-Adenovirus in YB-1 Kern-positiven Tumorzellen (257RDB und 181RDB) und YB-1 Kern-negativen Tumorzellen (HeLa, U2OS) zeigt;
- Fig. 16: den strukturellen Aufbau des Adenovirus vom Wildtyp und des adenoviralen Vektors AdXvir03;
- Fig. 17: den strukturellen Aufbau des adenoviralen Vektors AdXVir03/01; und
- Fig. 18A/B: mit 181RDB-Zellen (Fig. 18A) und 257RDB-Zellen (Fig. 18B) bewachsene Näpfe nach Kristallviolett-Färbung und Infektion mit Ad312 (20 pfu/Zelle), Xvir03 (5 pfu/Zelle) und Kontrolle (nicht infiziert), wobei die Krisstallviolettfärbung fünf Tage nach Infektion erfolgte.
- Fig. 19: das Ergebnis einer Northern Blot-Analyse der Expression des E2-Gens in A549-Zellen und U2OS-Zellen nach Infektion mit Adenovirus vom Wildtyp Ad5 und Adenovirus Ad312;
- Fig. 20: das Ergebnis einer Northern Blot-Analyse der Expression des E2-Gens in U2OS-Zellen nach Infektion mit Adenovirus vom Wildtyp und Adenovirus delta24 nach 12 und 24 Stunden;
- Fig. 21: den strukturellen Aufbau des adenoviralen Vektors XvirPSJL1;
- Fig. 22: den strukturellen Aufbau des adenoviralen Vektors XvirPSJL2;
- Fig. 23: mit HeLa-Zellen bewachsene Näpfe nach Kristallviolett-Färbung und Infektion mit Adenovirus dl520 mit unterschiedlichen pfu/Zellen;
- Fig. 24: ein Balkendiagramm, welches die Aktivität von Luciferase in U2OS-Zellen, He-La-Zellen und 257RDB-Zellen bei Verwendung verschiedener Promotorfragmenten des adenoviralen E2-late-Promotors zeigt;
- Fig. 25: ein Balkendiagramm, welches die Anzahl der viralen Partikeln zeigt nach Infektion von U2OS-Zellen mit einem YB-1 exprimierenden Adenovirus und dem Virus Ad312 zeigt, nach zwei und fünf Tagen, wobei zwischen intrazellulär verbliebenen viralen Partikeln (schwarz dargestellt) und freigesetzten, extrazellulären viralen Partikeln (quergestreift dargestellt) unterschieden wird;
- Fig. 26: das Ergebnis einer Southern Blot-Analyse des Replikationsverhaltens von Adenovirus dl 520 in U373 Zellen mit und ohne Behandlung der Zellen mit Irinotecan;
- Fig. 27: das Ergebnis einer Southern Blot-Analyse des Replikationsverhaltens von Adenovirus dl 520 in U373 Zellen mit und ohne Behandlung der Zellen mit Trichostatin A;
- Fig. 28: das Ergebnis einer FACS-Analyse von mit Trichostatin behandelten U 373 Zellen betreffend die Expression des Coxsackievirus-Adenovirus-Rezeptors (CAR), ausgedrückt als prozentualer Anteil der CAR-positiven Zellen;
- Fig. 29: vier verschiedene Panele von Zellrasen zur Darstellung der Wirkung von replizierendem Adenovirus dl520 und Irinotecan und Trichostatin in verschiedenen Kombinationen;
- Fig. 30: eine schematische Darstelluing des ORF von E1B 55K mit dem 3'UTR-Fragment und der Restriktinosschnittstelle Bfr I an Position 3532;
- Fig. 31: die Sequenz der E1B55k-3'UTR Region entsprechend Sequenzposition 3507 bis 4107 von Wildtyp Ad 5; und
- Fig. 32: eine schematische Darstellung eines universellen Schuttle-Plasmids zur Herstellung von E3/E4-modifiziertenden rekombinanten Adenoviren mit RGD-Motiv.

### Beispiel 1: Strukturen von E1A-Modifikationen, wie sie von den erfindungsgemäß verwendeten Adenoviren aufgewiesen werden können

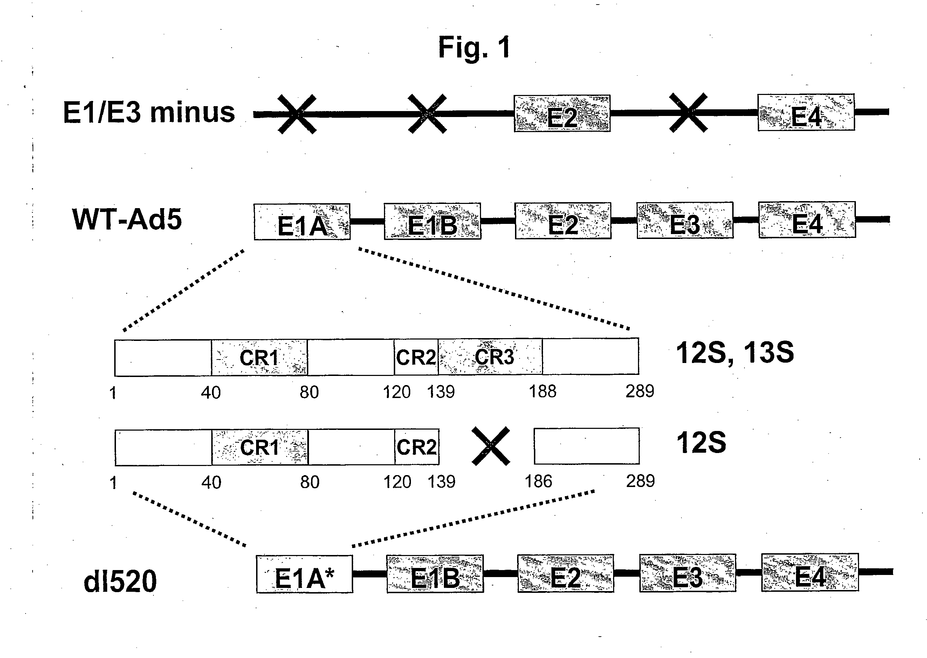

Fig. 1 zeigt den strukturellen Aufbau der adenoviralen Vektoren AdE1/E3-minus, d.h. des E1/E3-deletieren Adenovirus, Wildtyp-Adenovirus und Adenovirus d1520.

Das Adenovirus AdE1/E3-minus weist keine funktionalen für E1A und keine für E1B und keine für E3 codierenden Bereiche auf und dient im Rahmen der durchgeführten Experimente als Toxizitätskontrolle

Das Wildtyp-E1A-Gen kodiert für insgesamt 5 Proteine, die durch alternatives splicing der RNA von E1A hervorgehen. Dabei werden unter anderem zwei unterschiedliche Proteine, nämlich ein 289 Aminosäuren großes Protein und ein 243 Aminosäuren großes Protein hergestellt. D1520 kodiert nicht für das 289 Aminosäure große Protein, da es eine Deletion im CR3-Bereich des E1A-Gens aufweist, das zu einem Fehlen des 13S-Genproduktes führt. Der erfindungsgemäß verwendbare Adenovirus dl520 wird unter Fachleuten als 12S-E1A Virus bezeichnet. Der im Stand der Technik bekannte Adenovirus dl347 (Wong und Ziff, J. Virol., 68, 4910-4920, 1994) ist ebenfalls ein 12S-E1A Virus, der erfindungsgemäß verwendet werden kann.

Innerhalb des 289 Aminosäuren großen Proteins, welches von 13S-E1A mRNA kodiert wird, gibt es 3 Bereiche, die bei den unterschiedlichen adenoviralen Subtypen konserviert vorliegen. Diese werden als CR1, CR2 und CR3 bezeichnet. Während CR1 und CR2 bei beiden E1A-Proteinen (E1A 12S und E1A 13S) vorkommt, d. h. sowohl bei dem 289 Aminosäure langen wie auch bei dem 243 Aminosäure langen Protein, ist der CR3-Bereich nur bei dem größeren der beiden vorstehend genannten Proteine zu finden.

Der CR3-Bereich wird für die Aktivierung der viralen Gene, insbesondere von E1B, E2, E3 und E4 benötigt. Viren, die nur das kleinere, 243 Aminosäuren lange Protein aufweisen, transaktivieren nur sehr schwach die viralen Gene und führen keine adenovirale Replikation in solchen Zellen durch, die YB-1 nicht im Kern aufweisen. Da YB-1 nur in Tumorzellen im Kern vorliegt bzw. nachweisbar ist, eignet sich dieser Vektor, um eine tumorspezifische Replikation zu induzieren.

Durch die Deletion von CR3 in dl520 ist dieser Adenovirus nicht in der Lage, zelluläres YB-1 in den Zellkern zu translokalisieren, hierin auch als Translozieren bezeichnet, und somit auch nicht in der Lage, in YB-1-Kern-negativen Zellen zu replizieren und stellt damit einen der erfindungsgemäß zu verwendenden Viren dar, wobei dieser Virus die erfindungsgemäß erforderliche Transaktivierung aufweist.

### Beispiel 2: Wirkmechanismus von Adenoviren in Abhängigkeit des Rb-Status von Zellen

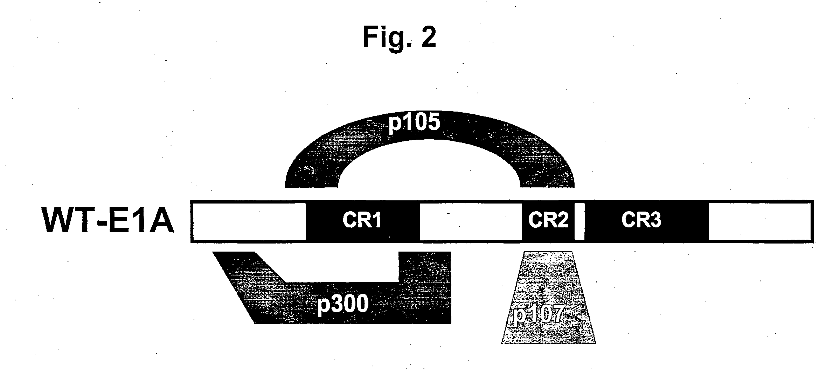

In Fig. 2 sind die Bindungsdomänen des E1A-Proteins hinsichtlich der Bindung von p300, p107 und p105 dargestellt. P300 ist dabei ebenso wie p107 ein zelluläres Bindungsprotein. Die Bindung des Retinoblastoma-Proteins (pRb), ein Tumorsuppressor-Protein, erfolgt über CR1 und CR2. Studien haben gezeigt, dass pRb und p107/p300 in Verbindung mit dem zellulären Transkriptionsfaktor E2F eine transkriptionelle Regulation ausüben. Das Wildtyp E1A-Protein unterbricht die Bindung von E2F an Rb. Das solchermaßen freigesetzte E2F bindet an den E2 early Promotor und induziert dadurch die adenovirale Replikation.

Es ist im Stand der Technik bekannt, dass bestimmte Deletionen im E1A-Onkoprotein dazu führen, dass rekombinante adenovirale Vektoren wie die nachstehend genannten vornehmlich in Rbnegativen Zellen zur Replikation befähigt ist und erfindungsgemäß verwendet werden können. Zum Beispiel weist der adenovirale Vektor dl922-947 eine Deletion in der CR2-Region auf (Aminoäurepositionen 122-129) und der Vektor CB016 Deletionen in den Bereichen CR1 (Aminoäurepositionen 27-80) und CR2 (Aminoäurepositionen 122-129). Der Vektor E1A*dl*01/07 weist eine Deletion im CR2-Bereich auf (Aminoäurepositionen 111-123). Durch eine zusätzliche Deletion am N-Terminus (Aminoäurepositionen 4-25), erfolgt zudem keine Bindung an das Protein p300. Der adenovirale Vektor AdΔ24 weist eine Deletion in der CR2-Region auf (Aminoäurepositionen 120-127). Der im Patent EP 0 931 830 beschriebene adenovirale Vektor weist Deletionen in dem CR1 und CR2-Bereich auf.

Der Bindungsmechanismus von E2F/RB und die durch E1A vermittelte Freisetzung von E2F ist grundlegend verschieden von dem der vorliegenden Erfindung zugrunde liegenden Mechanismus. Nicht die Freisetzung von E2F vom Rb-Protein ist, wie im Stand der Technik angenommen, ein wichtiger, um nicht zu sagen der entscheidende Vorgang der adenoviralen Replikation, sondern die Kernlokalisation des humanen Transkriptionsfaktors YB-1. Dieser Transkriptionsfaktor kommt in normalen Zellen über den größten Teil des Zellzyklus lediglich im Zytoplasma vor. Nach Infektion mit einem Adenovirus wird dieser unter bestimmten Bedingungen in den Kern induziert oder liegt bei bestimmten zellulären Systemen wie bestimmten Tumorerkrankungen, wie z.B. aber nicht darauf beschränkt, Brustkrebs, Ovarialkarzinom, Prostatakarzinom, Osteosarkom, Glioblastom, Melanom, kleinzelliges Lungenkarzinom und Kolorektalkarzinom, bereits im Kern vor.

### Beispiel 3: Infektion von U2OS-Zelle

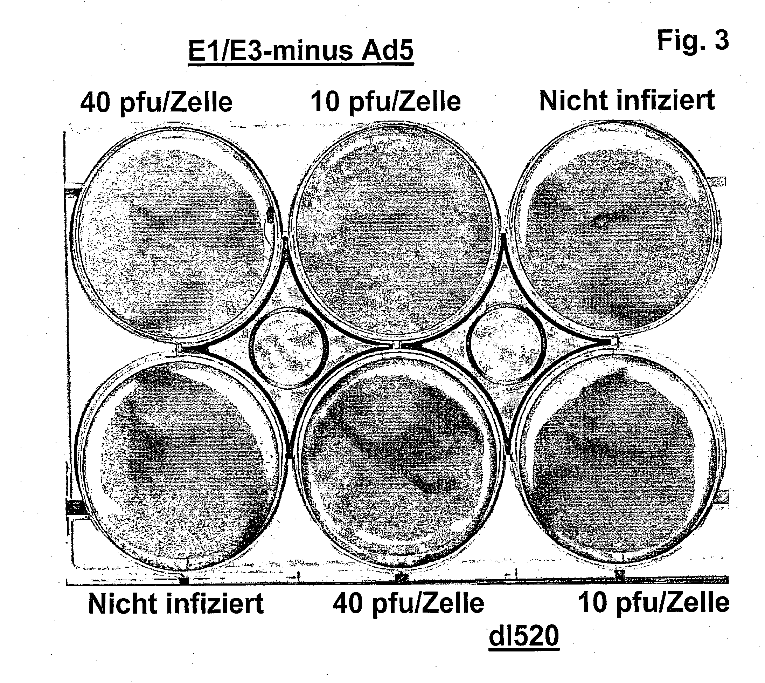

Pro Schale wurden 100.000 U2OS-Zellen ausplattiert. Am nächsten Tag wurden die Zellen wie in Fig. 3 dargestellt mit den verschiedene Adenoviren infiziert. Die Infektion erfolgte in 500 µL serumfreie DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Wie aus Fig. 3 ersichtlich zeigen die U2OS Zellen, welche YB-1 nicht im Kern aufweisen, nach Infektion mit zwei verschiedenen Adenoviren, nämlich dem als E1/E3-minus bezeichneten E1/E3-deletierten Adenovirus und Adenovirus dl520, der erfindungsgemäß verwendet werden kann, keine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert, Danach werden die Platten mit 6 Näpfen mit Wasser gründlich gewaschen und bei Raumtemperatur getrocknet.

Dies bestätigt die der vorliegenden Erfindung zugrundeliegende Erkenntnis, dass das Vorhandensein von YB-1 erforderlich ist, um die erfindungsgemäß zu verwendenden Viren zu einer Lyse von infizierten Zellen zu veranlassen.

### Beispiel 4: Infektion von 257RDB-Zellen

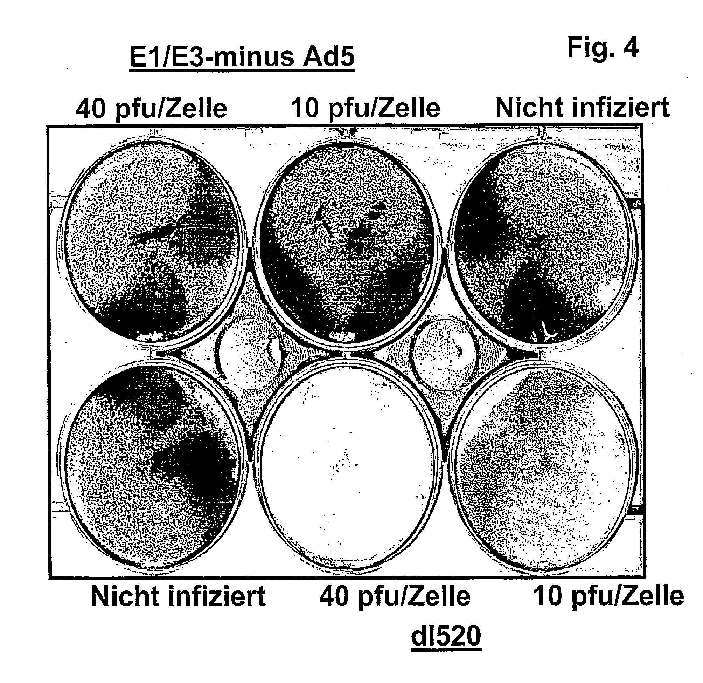

Pro Schale wurden 100.000 257RDB-Zellen ausplattiert. Am nächsten Tag wurden die Zellen wie in Fig. 4 dargestellt mit den verschiedene Adenoviren infiziert. Die Infektion erfolgte in 500 µL serumfreiem DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Das Ergebnis dieses Versuchs ist in Fig. 4 dargestellt. Das Adenovirus als E1/E3-minus Ad5 bezeichnete E1/E3-deletierte Adenovirus zeigt keine Lyse bei niedrigen MOIs (pfu/Zelle) bei

Infektion von 257RDB-Zellen, die YB-1 im Kern aufweisen. Im Gegensatz dazu zeigt dl520, welcher wie in Beispiel 3 belegt, in YB-1-Kern-negativen Zellen nicht repliziert und gleichzeitig mit E1A für ein im Sinne der vorliegenden Erfindung transaktivierendes Onkogenprotein, codiert, eine praktisch vollständige Lyse bei einer MOI (engl. multiplicity of infection) von 40 pfu pro Zelle und eine noch überwiegende Lyse bei einer MOI von 10 pfu pro Zelle. Daraus ergibt sich, dass dl 520 und vergleichbare Viren, wie hierin beispielsweise mit dl1119/1131 oder AdXvir 03 beschrieben, eine gegenüber einem E1-deletierten oder einem E1/E3-deletierten Adenoviurs um etwa eine Größenordnung (etwa zehnfach) verringerte MOI erforderlich machen, was deren Eignung zur klinischen Anwendung begründet.

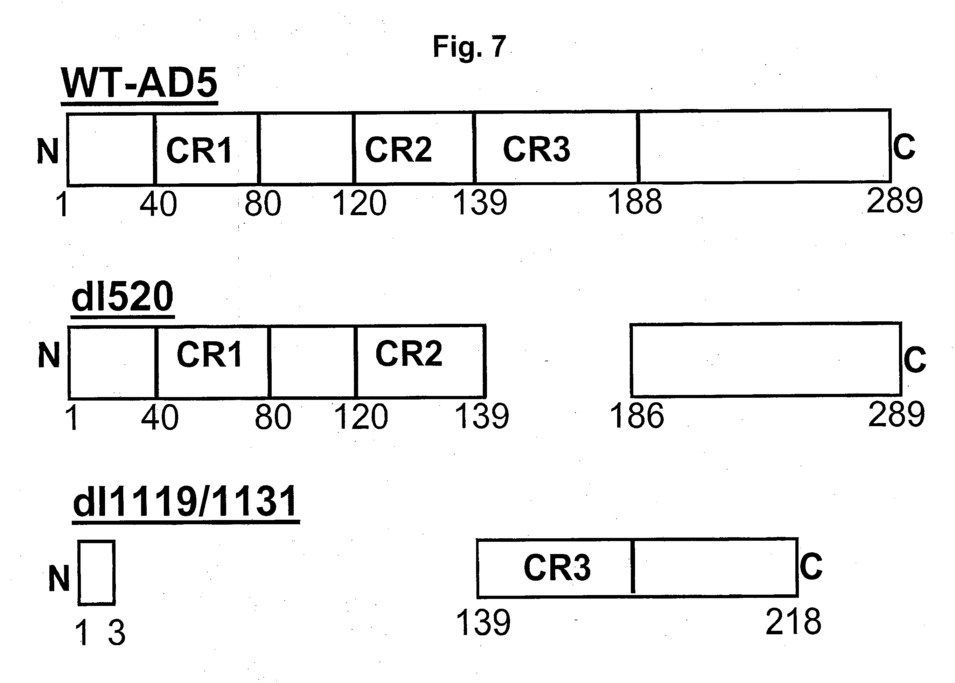

Wie in Fig. 7 dargestellt zeichnet sich das E1A-Protein von dl520 dadurch aus, dass der Bereich CR3 davon deletiert ist, was zu der für die erfindungsgemäße Verwendung des Adenovirus erforderlichen Transaktivierung und Replikation in YB-1-Kern-positiven Zellen führt.

### Beispiel 5: Infektion von 257RDB und U2OS-Zellen mit dl1119/1131

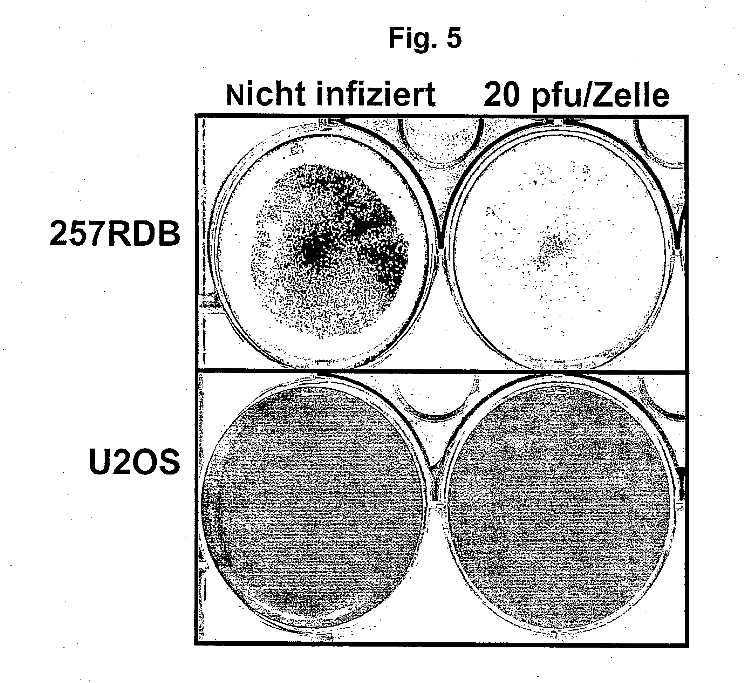

Wie in Fig. 5 dargestellt kommt es bei Infektion von YB-1-Kern-negativen U2OS-Zellen mit dem Adenovirus dl1119/1131, das eine Deletion der Aminosäuren 4-138 des E1A-Proteins bzw. der dafür codierenden Nukleinsäure und ein Stop-Codon nach Aminosäure 218 aufweist, wodurch das exprimierte verkürzte E1A-Protein die CR3-Region des vollständigen E1A-Proteins enthält, bei einer MOI von 20 pfu pro Zelle zu keiner Lyse. Als Negativkontrolle wurde ein nicht-infizierter Zellrasen herangezogen.

Im Gegensatz dazu zeigt sich unter dem Einfluss von Adenovirus dl1119/1131 in einem zellulären System wie 257RDB, welches YB-1 im Kern aufweist, d. h. YB-1-Kern-positiv ist, bereits bei einer MOI von 20 pfu pro Zelle eine praktisch vollständige Lyse des Zellrasens. Insoweit findet sich auch mit diesem Beispiel ein Beleg für die Aussage, dass ein modifiziertes E1A-Onkogenprotein, welches, wie in Fig. 7 dargestellt, beispielsweise lediglich den CR3-Bereich umfasst und dem der Bereich CR1 sowie CR2 fehlt, die für die erfindungsgemäße Verwendung von Adenoviren erforderliche Transaktivierung in YB-1-Kern-positiven Zellen zeigt, mit der Folge einer viralen Replikation. Der Adenovirus dl1119/1131 stellt somit einen weiteren, erfindungsgemäß verwendbaren Adenovirus dar. Dabei ist es im Rahmen der vorliegenden Erfindung, dass auch solche Viren verwendet werden können, die hinsichtlich des CR3-Bereiches wie dl1119/1131 ausgebildet sind, jedoch im Unterschied dazu den Bereich CR1 und/oder CR2 aufweisen.

### Beispiel 6: Nachweis von nuklärem YB-1 bei vielfachresistenten Zellen

Dem Experiment liegt die Überlegung zugrunde, das nukläres YB-1 als Transkriptionsfaktor an die Y-Box (CAAT-Sequenz) innerhalb des mdr1-Promoters (engl. multiple drug resistance promoter) binden sollte. Um dies nachzuweisen, wurde eine sogenannte EMSA-Analyse (e-lectrophoretic mobility shift assay) durchgeführt. Dabei wird Kernprotein isoliert und anschließend werden 1-10 µg Protein mit einem kurzen DNA-Fragment (Oligo) zusammen bei 37° C inkubiert. Um nukläres YB-1 zu bestimmen, wurde folgendes Oligonukleotid benutzt: *mdr1* promoter im Unterschied zu U20S (Position -86 bis -67): TGAGGCTGATTGGCTGGGCA (SEQ. ID. No 1) (die Y-box ist unterstrichen).

Dieses DNA-Fragment wird zuvor mit einer Kinase am 5'-Ende mit ³²P radiaktiv markiert. Anschließend erfolgt die Auftrennung in einem nativen Polyacrylamidgel. Falls das Protein YB-1 an einer Sequenz am Oligonucleotid bindet, ist dies zu erkennen, da ungebundenes Oligonukleotid im Gel schneller wandert als das gebundene Oligonucleotid (Holm, P. S. et al., JBC 277, 10427-10434, 2002; Bargou, R. C. et al., Nature Medicine 3, 447-450, 1997).

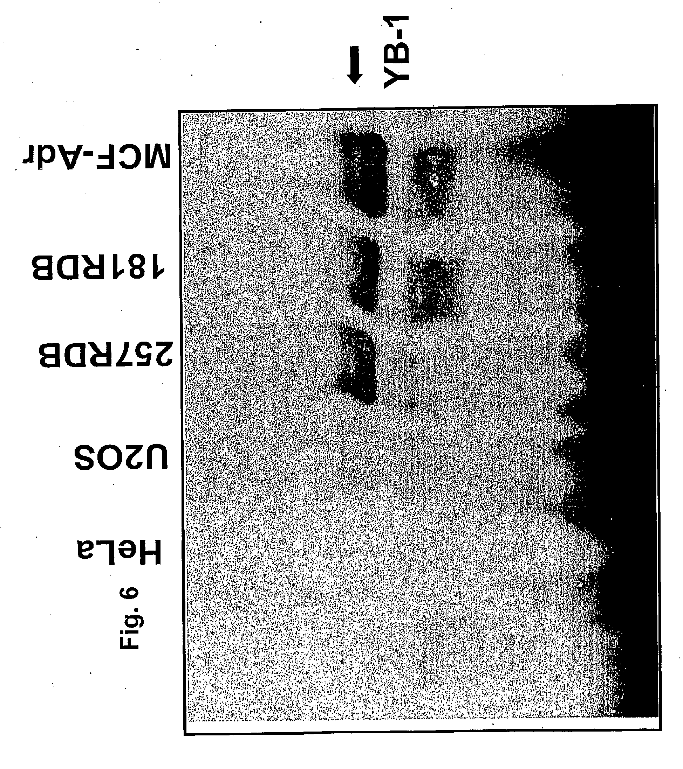

Wie in Fig. 6 dargestellt, konnte im Rahmen einer EMSA-Analyse gezeigt werden, dass YB-1 in den vielfachresistenten Zellen 257RDB, 181RDB und MCF-7Ad-Zellen im Kern im Gegensatz zu den Zelllinien U2OS und HeLa-Zellen vorhanden ist.

Die in Beispiel 4 und 5 gezeigten Ergebnisse belegen, dass die Adenoviren dl520 und dl1119/1131 in YB-1-Kern-positiven Zellen wie z. B. 257RDB im Unterschied zu U205 replizieren und eine Zellyse induzieren. Dies belegt somit die Aussage der erfindungsgemäßen Verwendung der Adenoviren. Weiterhin belegen die Ergebnisse, dass bereits eine schwache Transaktivierung der viralen Gene in YB-1- Kern-positiven Zellen im Vergleich zum Wildtyp-Adenovirus durch die modifizierten oder deletierten E1A-Genprodukte bei Anwesenheit von YB-1 im Zellkern erfolgreich mit einer Replikation und Lyse von derartigen Zellen einhergeht, einschließlich beispielsweise von vielfachresistenten Zellen, und somit die hierin beschriebenen Adenoviren bei der Lyse derartiger Tumoren verwendet werden können.

### Beispiel 7: Steigerung der Replikationseffizienz von E1-minus Adenoviren

In diesem Beispiel wird die Substitution der frühen viralen Gene E1B-55K und E4orf6 durch Transfektion mit dem Plasmid pE4orf6 und Infektion mit dem E1/E3-deletierten Adenovirus Ad-55K gezeigt. Ad-55k ist ein E1/E3 deletiertes Virus, wobei E1B-55k in die E1 kloniert wurde und unter CMV-Kontrolle steht. Diese Substitution wird mit Blick darauf erforderlich, dass A-dYB-1, d. h. ein Adenovirus, der YB-1 exprimiert, diese frühen Gene nicht exprimiert und der vorliegende Erfinder erkannt hat, dass eine Substitution dieser frühen Gene in einem Replikationssystem, bei dem YB-1 im Kern vorhanden ist, in der Lage ist, die Replikationseffizienz bzw. die Partikelbildungseffizienz in einem Umfang vergleichbar derjenigen von Wildtyp-Adenoviren vom Typ Ad5 zu erhöhen.

Dabei wurde wie folgt vorgegangen:
Transfektion von je 10⁵ U2OS-Zelle mit dem Plasmid pE4orf6 mit Hilfe von Lipofectamin. Das Plasmid pE4orf6 trägt die für das frühe virale Gen E4orf6 codierende DNA-Sequenz unter CMV-Kontrolle.

24 h nach der Transfektion mit dem Plasmid pE4orf6 wurden die Zellen mit dem YB-1 exprimierenden E1/E3-deletierten Adenovirus AdYB-1 (50 pfu/Zelle) und dem E1/E3-deletierten E1B-55K Adenovirus Ad-55K (50 pfu/Zelle) infiziert. Ad-55K ist ein E1/E3-deletiertes Virus, welches als Transgen das virale Gen E1B-55K unter CMV-Kontrolle trägt.

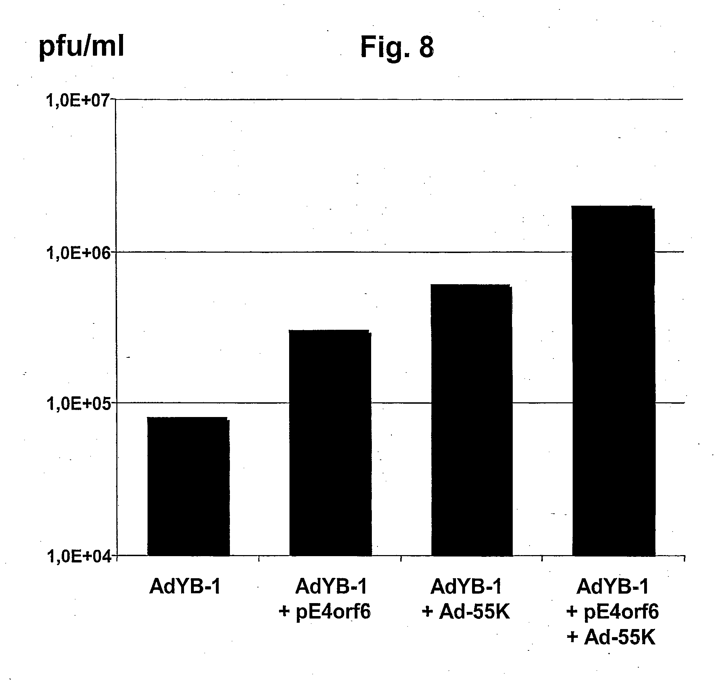

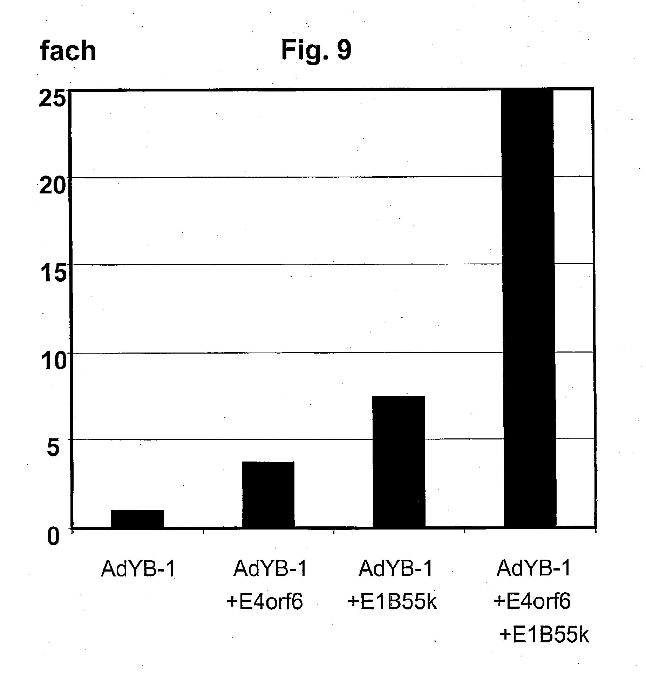

Anschließend wurden die Zellen vom Medium (2ml) 5 Tage nach der Infektion (= post infectionem) entfernt. Die Freisetzung der viralen Partikel aus den isolierten Zellen erfolgte durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in den Figs. 8 und 9 dargestellt. Dabei zeigt Figur 8 das Ergebnis des Plaque Assays, dargestellt in absoluten Zahlen. Die deutlichste Differenz zur Infektion mit AdYB-1 alleine zeigt hierbei die Kombination aus Transfektion mit dem Plasmid pE4orf6 und Co-Infektion mit den beiden Viren AdYB-1 und Ad-55K. Fig. 9 zeigt das Ergebnis von Fig. 8, wobei hier die Steigerung der Replikationseffizienz als Vielfaches der für AdYB-1 ermittelten Replikation dargestellt ist. Die mit Plasmid pE4orf6 transfizierten und anschließend mit AdYB-1 und E1B-55K (Ad-55K) infizierten Zellen produzierten bis zu 25 mal mehr pfu/ml.

Aufgrund dieser Ergebnisse kann gefolgert werden, dass die Substitution von E1B-55K und E4orf6 die Anzahl der gebildeten Viren (pfu/ml) nach Infektion mit dem E1/E3-deletierten Adenovirus AdYB-1 um einen Faktor von bis zu 25 erhöht. Dabei sind die additiven Effekte von E1B-55K und E4orf6 auf die Produktion von plaque forming units (pfu) signifikant größer als die Effekte eines der beiden Genprodukte alleine.

Kontrollversuche mit einem Plasmid, welches EGFP exprimierte, zeigten deutlich, dass in dem gewählten experimentellen Ansatz nur etwas 10 % der Zellen erfolgreich mit dem Plasmid pE4orf6 transfiziert werden konnten. Die Anzahl der in den Zellen gebildeten Partikel, die sowohl E1B-55K als auch E4orf6 exprimierten, ist mit der des humanen Adenovirustyp 5 (Wildtyp) vergleichbar. Dies bestätigt die der vorliegenden Erfindung zugrundeliegende Erkenntnis, dass die Expression von E4orf6 und E1B-55K in Verbindung mit der Kernlokalisation von YB-1 in der Lage ist, eine adenovirale Replikation bzw. Partikelbildung, insbesondere von E1A-deletiertes Adenoviren zu bewerkstelligen, die vergleichbar derjenigen von Wildtyp Ad5 ist.

### Beispiel 8: Verstärkung der Replikation von in YB1-Kern-negativen Zellen nicht replizierenden Adenoviren in YB-1-Kern-positiven Zellen nach Gabe von Zytostatika

Im Stand der Technik ist bekannt, dass durch die Zugabe von verschiedenen Zytostatika die Kernlokalisation des humane Transkriptionsfaktor YB-1 induziert wird. Wie vom vorliegenden Erfinder gefunden, steuert kernlokalisiertes YB-1 die adenovirale Replikation mittels Aktivierung des adenoviralen E2-late Promoters. Die Kombination beider Effekte kann dazu genutzt werden, um eine spezifische Tumorlyse herbeizuführen.

Bei der Durchführung der onkolytischen Assays wurde wie folgt vorgegangen. 200.000 Zellen (HeLa bzw. U2OS) wurden je Napf in 6-Napf-Platten ausplattiert. Am nächsten Tag wurden 40 ng/ml (Endkonzentration) Daunorubicin zugegeben. Nach 3 Stunden Inkubationszeit wurden die Zellen mit 10 bzw. 30 pfu dl520/Zelle infiziert. Anschließend wurden die Zellen in Zytostatikafreiem Medium incubiert. Nach 3-5 Tagen wurden die Zellen mit Kristallviolett angefärbt.

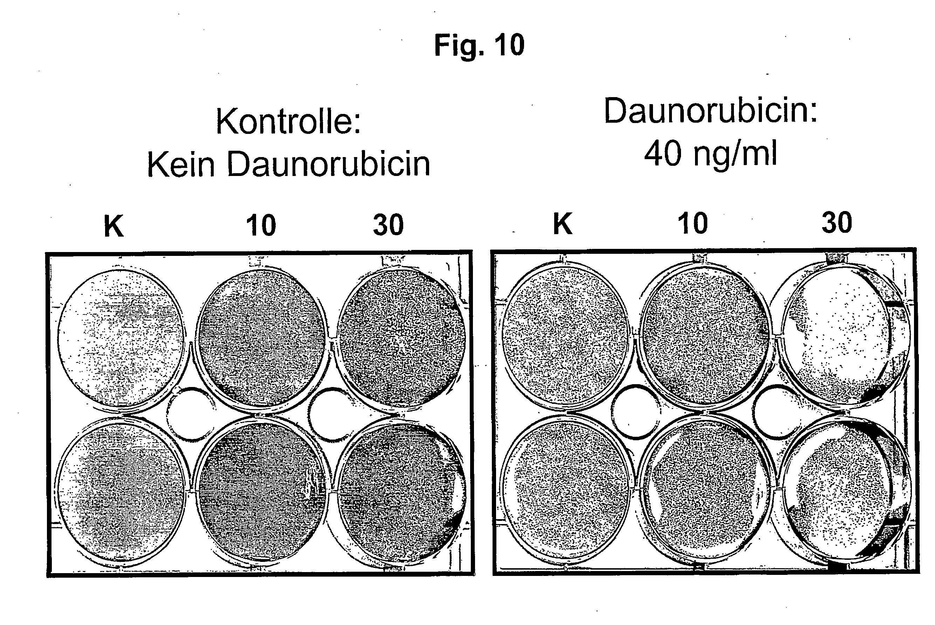

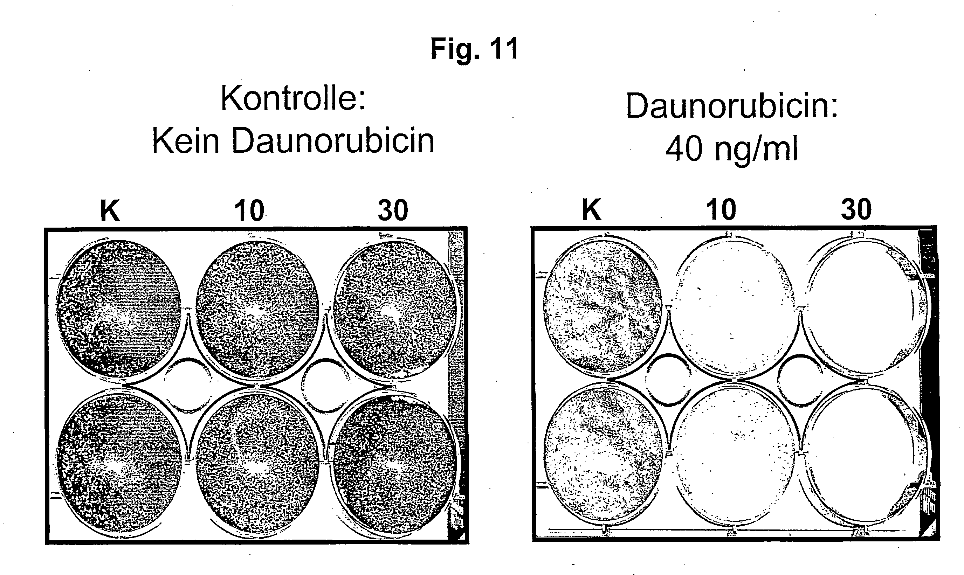

Wie aus Fig. 10 und 11 ersichtlich, induziert die Zugabe von Daunorubicin die Replikation von dl520 durch die Kernlokalisation von YB-1. Somit erzielt dl520 in Verbindung mit dem Zytostatikum Daunorubicin einen größeren tumorlytischen Effekt als Daunorubicin alleine.

### Beispiel 9: In vivo Tumorlyse durch dl520

Die in dieser in vivo Studie verwendeten Zellen HeLa (YB-1 Kern-negativ) und 257RDB (YB-1 Kern-positiv) werden unter sterilen Zellkulturbedingungen expandiert. Kurz vor der Injektion der Zellen in die Mäuse (Stamm CD1NuNu), um einen Tumor subcutan zu setzen, werden diese durch Trypsinierung geerntet, in DMEM-Medium (10 % FKS) aufgenommen, gezählt und einmal mit PBS gespült. Anschließend werden die Zellen zentrifugiert, das PBS abgesaugt und in frischem PBS, der gewünschten Zellzahl entsprechen, portioniert. Die subcutan injizierte Zellzahl betrug in dieser Studie je 5x10⁶ Zellen von beiden Zelllinien. Die Injektion erfolgt subcutan in eine Flanke der Tiere, wobei die HELA-Zellen in die rechte Seite und die 257RDB- Zellen in die linke Flankenseite zur besseren Unterscheidung injiziert wurden. Das Wachstum der Tumoren wurde zweimal wöchentlich kontrolliert und dabei die Länge und die Breite der Tumoren mit einer Schiebleere gemessen. Daraus wurde das Tumorvolumen über folgende mathematische Formel berechnet:
3/4π* a/2* (b/2)² a = Länge, b = Breite

Wenn der Tumor ein Volumen von 200 bis 520 mm³ erreicht hat, wird das Virus bzw. PBS als Negativkontrolle intratumoral appliziert. Die zu injizierenden Volumina waren gleich und betrugen jeweils 50 µl. Dies wird an 3 aufeinanderfolgenden Tagen wiederholt. Die Gesamtdosis an appliziertem Virus betrug 5x10⁸ pfu. Danach wird das Tumorwachstum weiterhin zweimal pro Woche dokumentiert und das Volumen berechnet. Am Ende der Studie werden die Mäuse euthanasiert und die Tumoren für weitere Analysen entnommen.

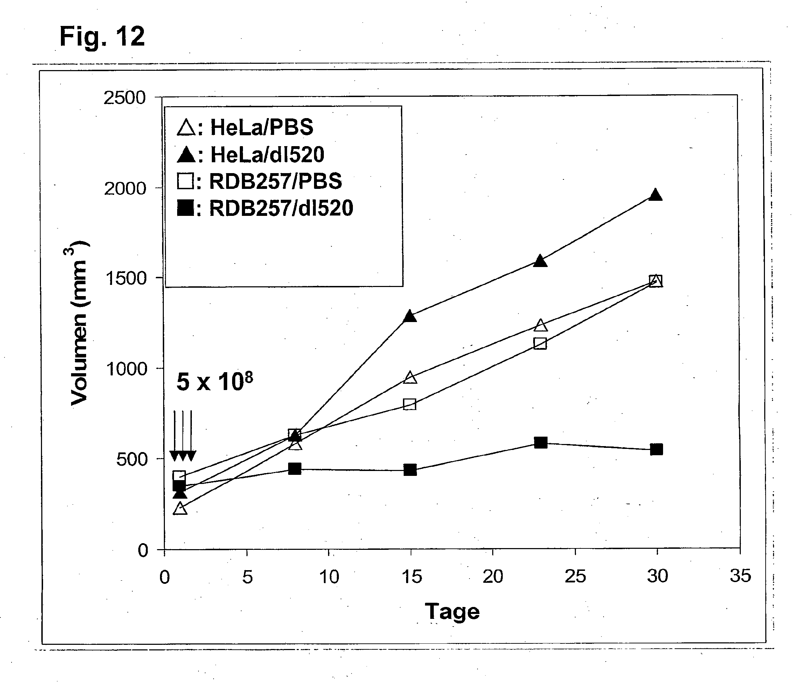

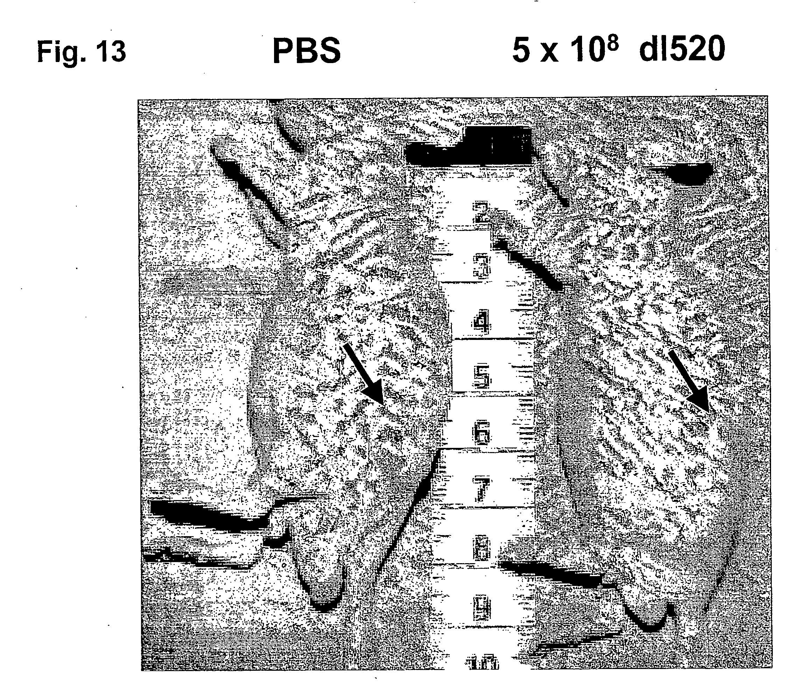

Die Ergebnisse sind in den Figuren 12 und 13 dargestellt.

Fig. 12 zeigt ein Diagramm, welches das Volumen des Tumors in Abhängigkeit von der Zeit und den verschiedenen Behandlungsschemata darstellt. Im Falle der durch RDB257 ausgebildeten Tumoren erfolgt bei Injektion von PBS ein signifikantes Wachstum des Tumors von ca. 438 mm³ bis 1466 mm³. Unter dem Einfluss des erfindungsgemäß verwendeten Vektors dl520 konnte das Tumorwachstum signifikant verringert werden. Ausgehend von einer durchschnittlichen Tumorgröße von 344 mm³ erhöhte sich die Tumorgröße lediglich um 21 % auf insgesamt 543 mm³.

Als Kontrolle wurde im vorliegenden Beispiel der Tumor aus HeLa-Zellen verwendet, der sich bei Gabe von PBS hinsichtlich seines Wachstums ähnlich verhielt, wie der auf RDB257 zurückgehende Tumor bei Gabe von PBS. Mit dl520 behandelte HeLa-Zellen basierte Tumoren zeigten einen noch wesentlicheren Anstieg des Tumorwachstums ausgehend 311 mm³ bis 1954 mm³.

Fig. 13 zeigt Aufnahmen von euthanasierten Nacktmäusen, denen unter Anwendung von RDB257 ein Tumor gesetzt wurde. Es ist deutlich zu erkennen, dass nach der erfindungsgemäßen Applikation des Adenovirus dl520 eine signifikante Rückbildung des Tumors eintrat. Im vorliegenden Falle wurde sogar eine Verringerung des Tumorvolumens erzielt (Tag 1 nach Applikation des Virus dl520: 515 mm³; Tag 30 nach Applikation des Virus dl520: 350 mm³.

### Beispiel 10: Southern Blot von Tumor-DNA

Die DNA wird aus einem Tumorstück isoliert, welches aus der Mitte des in Beispiel 9 gesetzten Tumors entnommen worden ist. Für die Isolierung wird das Dneasy Tissue Kit von der Firma Qiagen benutzt. Die Durchführung der DNA-Isolierung erfolgte nach Herstellerangaben. Dabei wird die DNA aus den Zellen durch eine alkalische Lyse freigesetzt. Anschließend wird isolierte DNA über eine Säule gereinigt. Anschließend wird die Konzentration der isolierten DNA photometrisch bei 260 nm gemessen. Die Analyse erfolgte anhand von 2 µg der DNA-Proben, die mit dem Restriktionsenzym Kpn I 10 Units verdaut wurden. Dann erfolgt eine elektrophoretisch Auftrennung der Proben in einem 0,8%igen Agarosegel. Anschließend wurde die DNA auf eine Nylonmembran geblottet (Durchführung nach dem System von Schleicher & Schuell). Die auf der Membran geblottete DNA wird gegen eine spezifische 1501 bp große DNA-Sonde hybridisiert Die 1501 bp große DNA-Sonde bindet spezifisch an das 3369 bp-großes Kpn I-Fragment innerhalb der E2A-codierenden Ad5-Sequenz. Die Sonde wurde zuvor per PCR (Primer:5'- GTC GGA GAT CAG ATC CGC GT (SEQ.ID. No 2), 5'- GAT CCT CGT CGT CTT CGC TT (SEQ. ID.No.3)) hergestellt und mit ³²P radioaktiv markiert. Anschließend wird die Membran gewaschen und auf eine Filmfolie exponiert.

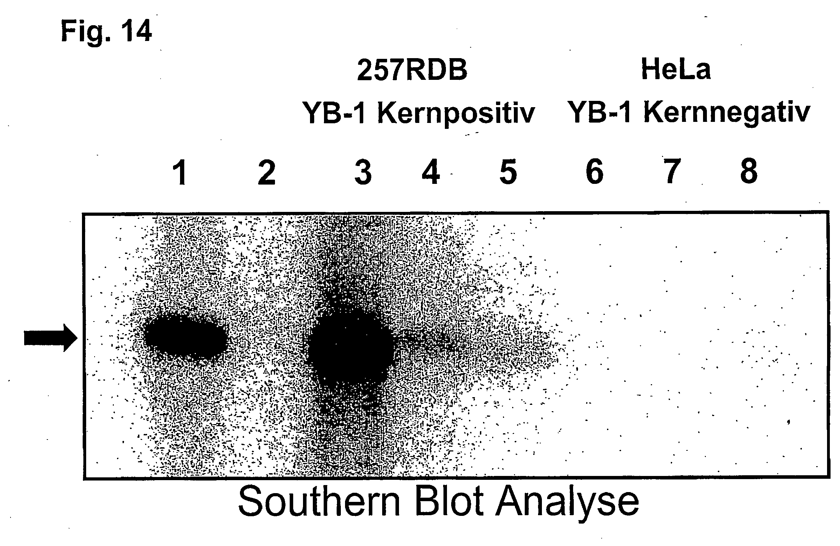

Das Ergebnis des Southern-Blots der Tumor-DNA ist in Fig. 14 dargestellt. Die Analyse belegt, dass nur dl520 in den resistenten Zellen RDB257 in vitro repliziert, wie in den Spuren 3, 4 und 5 dargestellt. Spur 1 zeigt als Positivkontrolle Ad-5d, Spur 6, 7 und 8 DNA von HeLa-Zellen, die mit dl520 infiziert wurden. Nachdem HeLa-Zellen keine YB-1-Kernpositivität zeigen, erfolgte darin keine Replikation des Virus dl520, so dass entsprechend die E2A-Sequenz nicht nachgewiesen werden konnte.

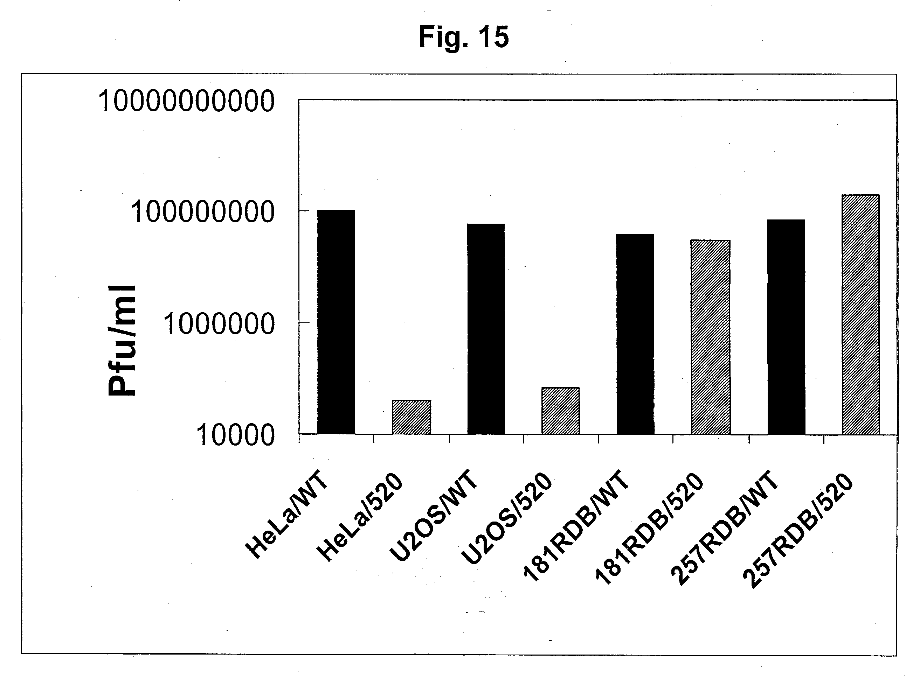

Ein weiteres Ergebnis mit dl520 ist in der Fig. 15 dargestellt. Anhand eines Plaque-assays wurde die Partikelbildung (pfu/ml) nach Infektion mit dl520 und Wildtyp-Adenovirus untersucht. Dabei werden verschiedene YB-1 Kern-positive (257RDB und 181RDB) Tumorzellen und YB-1 kernnegative Tumorzellen mit dl520 und Wildtyp-Adenovirus infiziert.

Dabei wurde wie folgt vorgegangen:
Jeweils 100.000-200.000 Zellen werden in sog. Platten mit 6 Näpfen (engl. 6 well plates) in L 15-Medium (resistente Zellen) bzw. DMEM (nicht-resistente Zellen) jeweils mit 10 % FKS ausplattiert. Nach 24 h erfolgt die Infektion mit dl520 und WT-Adenovirus (10 pfu/Zelle) 3 Tage nach der Infektion (post infectionem) erfolgt die Freisetzung der viralen Partikel aus der Zellsuspension (3 ml) durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in der Fig. 15 dargestellt. Dabei zeigt das Ergebnis des Plaque Assays, dass dl520 in den YB-1 Kern-positiven Zellen (257RDB und 181RDB) vergleichbar dem Wildtyp-Adenovirus repliziert. Insoweit wird bei der erfindungsgemäßen Verwendung der hierin beschriebenen Adenoviren eine Replikationseffizienz vergleichbar derjenigen von Wildtyp-Adenoviren erreicht.

### Beispiel 11: Struktureller Aufbau des adenoviralen Vektors Xvir03

Fig.16 zeigt den strukturellen Aufbau des adenoviralen Vektors Xvir03. Das Adenovirus Xvir03 ist ein sogenanntes E1/E3-deletiertes Adenovirus. Das heisst, dass keine für die adenovirale Replikation funktionellen E1A, E1B (E1B55k und E1B19K Proteine) und E3 Proteine hergestellt werden. Die Deletion der E1 Region erstreckt sich von 342 - 3528; die Deletion der E3 Region von Basenposition 27865 - 30995. Wie hierin verwendet, bezeichnet der Begriff "E1-deletiertes Virus ein solches Virus, bei dem E1 funktional nicht mehr aktiv ist. Dies kann durch eine Inaktivierung bei ansonsten weitestgehend intakter Nukleinsäure- bzw. Aminosäuresequenz erfolgen, kann jedoch auch eine unterschiedlich große Deletion des für E1-Region kodierenden Proteine bedeuten. Durch das Fehlen des E1A und E1B Proteins bzw. der dafür codierenden Nukleinsäuren wird die E4-Region, z.B. E4orf6, nicht oder nur sehr schwach (ca. 1-5% im Vergleich zum Wildtyp-Adenovirus) exprimiert. In der E1-Region werden die viralen Gene E1B55k und E4orf6 vermittelt durch den in den Xvir 03 eingeführten, heterologen CMV-Promoter (Firma Clontech: Plasmid pShuttle) exprimiert. Anstelle des CMV-Promotors können auch jene hierien beschriebenen Promotoren verwendet werden, wie im Zusammenhang mit der Expression von E1A offenbart. Der offene Leserahmen beider Gene ist über eine sogenante IRES-Sequenz (engl.: internal ribosomal entry site) miteinander verbunden (Pelletier, J. and Sonenberg, N. Nature, 1988, 334, 320-325). Dieses Element (Firma Novagen: pCITE) erlaubt die Expression von 2 Proteinen aus einer mRNA.

### Bei der Herstellung des Vektors wurde wie folgt vorgegangen: System Adeno-X der Firma Clontech

Das Plasmid E1B55k-pShuttle ging durch Umklonierung des offenen Leserahmen von E1B55k aus pCGNE1B von M. Dobelstein (Uni Marburg) mit XbaI und BfrI in den pShuttle-Vektor von Clontech hervor. Alternativ kann das BamHI Fragment aus dem pCGNE1B Vektor nach Glätten der Enden in den entsprechend vorbereiteten pShuttle Vektor von Clontech kloniert werden. Anschließend wurde E1B55k in pShuttle mit ApaI linearisiert, die Enden geglättet und mit NheI geschnitten.

In einem zweiten Vektor, dem pcDNA3.1 (+) (Invitrogen), wurden nacheinander das IRES-Element als PCR-Produkt mit dem pCITE-4a(+) der Firma Novagen als Template über TA-Klonierung in die EcoRV-Schnittstelle und das E4orf6 aus dem Plasmid pCMV-E4orf6 (M. Dobelstein, Uni Marburg) über BamHI hineinkloniert = IRES-E4orf6-pcDNA3.1(+). IRES-E4orf6 in pcDNA3.1 (+) wurde linearisert mit NotI, die Enden geglättet und anschließend wurde das Fragment IRES-E4ORF6 mit NheI herausgeschnitten. Das Fragment IRES-E4orfF6 wurde mit dem geöffneten Vektor E1B55k-pShuttle (blunt, NheI) verknüpft. Die Kassette wurde anschließend aus dem E1B55k-IRES-E4orf6-pShuttle zusammen mit dem CMV-Promotor und dem *bovine growth hormone* (BGH)-PolyA in das ΔE1, ΔE3 Adeno-X-Plasmid (Clontech) mit I-Ceu I und PI-SceI kloniert und als AdcmvE1B/IRES/E4orf6 bezeichnet. Danach erfolgte die Adenovirusherstellung nach Herstellerangaben (Clontech). Das mit PacI linearisierte Adenoplasmid mit dem Expressionselement CMV-E1B55k-IRES-E4orf6-BGH polyA wurde mit in HEK293 Zellen transfiziert und nach 11 Tagen *post transfectionem* die sich ablösenden Zellen zusammen mit dem Medium abgenommen, um durch wiederholte Einfrier-Auftau-Zyklen die entstandenen Adenoviren freizusetzten.

Es ist im Rahmen der vorliegenden Erfindung und für den Fachmann im Lichte der vorliegenden Offenbarung durchführbar, dass für die Herstellung der erfindungsgemäßen, bevorzugterweise rekombinanten Adenoviren, insbesondere solche, welche die Kassetten E4orf6-IRES-EIB55k bzw. YB-1-IRES-E1A12S einzeln und/oder zusammen enthalten, auch andere Systeme verwendet werden können, z. B. das System AdEasy der Firma QBIOGENE und Microbix. Zudem können die einzelnen Transgene innerhalb der Kassetten untereinander ausgetauscht werden. Es ist somit im Rahmen der vorliegenden Erfindung, dass auch solche Adenoviren hergestellt und erfindungsgemäß verwendet werden können, bei denen die Kassetten den folgenden Aufbau aufweisen: E1B55k-IRES-E4orf6 bzw. E1A12S-IRES-YB1.

Im Rahmen der vorliegenden Erfindung wurde ein sogenanntes E1/E3-deletiertes rekombinantes Adenovirus, welche die Kassetten E4orf6-IRES-E1B55k enthält verwendet. Es ist jedoch ohne weiteres möglich, dass das Virus nur eine E1-Deletion aufweist, d. h. dass die E3-Region intakt bleibt. Dabei kann wahlweise die E4-Region teilweise und/oder vollständig deletiert und/oder intakt vorliegen.

Bei der Herstellung des Vektors mit Hilfe anderer Systeme wurde wie folgt vorgegangen.

Herstellung des Adenovirus **Ad-Xvir 3'UTR** mit intakter E3-Region mit dem Vektorsystem nach Graham (Firma Microbix).

### Klonierung des Vektors CMV-E40RF6-IRES-E1B55k 3'UTR-polyA in pDelta ElsplA

Für das Plasmid E1B55k 3'UTR -pShuttle (Clontech) wurde der offene Leserahmen mit der 3'-UTR durch Amplifikation aus der DNA des Adenovirus Typ 5 gewonnen (E1B55k Vorwärtsprimer = 5'-ATGGAGCGAAGAAACCC-3' und E1B55k 3'UTR Rückwärtsprimer = 5'-CACGTCCTGGAAAAAATACAC-3') und in die geglättete NheI-Schnittstelle, die mit T-Enden versehen wurde (TA-Klonierung), in das pShuttle-Plasmid der Firma Clontech kloniert. Dadurch wurde das Transgen am 5' Ende mit einem hCMV-Promotor versehen und am 3' Ende mit dem Bovine Growth Hormone Polyadenylierungssignal.

### Klonierung des Vektors E4ORF6-IRES-pcDNA3.1(+)

Die Amplifikate E4ORF6 mit dem Adenovirus Typ 5 DNA als Template (E4orf6 Vorwärtsprimer 5'-CTTCAGGATCCATGACTACGTCCGGCG-3' und E4orf6 Rückwärtsprimer 5'-GAAGTGAATTCCTACATGGGGGTAGAGTCATAATCGT-3') bzw. aus dem Plasmid pCMVE4-34kD mit Bam HI herausgeschnitten (Dobbelstein et al., EMBO, 16,4276-4284,1997) und das IRES-Element mit dem pCITE-4a(+) der Firma Novagen als Template (IRES Vorwärtsprimer = 5'-TCCGGTTATTTTCCACCATATTGC-3' und IRES Rückwärtsprimer = 5'-TTATCATCGTGTTTTTCAAAGG-3') wurden nacheinander in die multiple Klonierungsstelle des pcDNA3.1(+)-Vektor hineinkloniert. Hierzu wurden für das E4Orf6 Transgen Primer verwendet, die eine *Bam*HI Schnittstelle am 5'-Ende und eine EcoRI-Schnittstelle am 3'-Ende des offenen Leserahmens generierten. Das Amplifikat wurde mit den entsprechenden Restriktionsenzymen verdaut und damit die Enden kompatibel für die gerichtete Klonierung in den mit entprechend *Bam*HI und *Eco*RI geöffneten Vektor. Anschließend wurde das Plasmid E4Orf6 in pcDNA3.1(+) mit *Eco*RV linearisiert, T-Enden angehängt und das Amplifikat für das IRES-Element hineinkloniert. Nach Überprüfung der korrekten Orientierung des IRES-Elements wurde der Vektor für die weitere Klonierung benutzt.

Die Verknüpfung der beiden Transgene mit dem IRES-Element entstand durch Umklonierung der Kassette E4ORF6-IRES in das vorweg generierte Plasmid CMV-E1B55k 3'UTR-polyA - pShuttle (Clontech), das mit *Not*I linearisiert, geglättet und anschließend mit *Xba*I geschnitten wurde. E4ORF6-IRES in pcDNA3.1(+) wurde mit *Not*I linearisiert, die Enden geglättet und weiter mit *Nhe*I verdaut. Durch die Ligation des E4ORF6-IRES-Inserts mit dem CMV-E1B55k 3'UTR-polyA -pShuttle (Clontech) entstand XVIR-3'UTR in pShuttle (Clontech).

### Herstellung des verwendeten adenoviralen Shuttlevektor

Da der für das nun benutzte adenovirale Herstellungssystem der Firma Microbix der Shuttlevektor pΔE1sp1A weder einen CMV-Promotor noch ein Bovine Growth Hormone Polyadenylierungssignal enthält, wurden diese Elemente in pΔE1sp1A hineinkloniert. Hierzu wurde pΔE1sp1A mit ClaI linearisiert, geglättet und mit *EcoRI* geschnitten. Die Elemente CMV-MCS (multiple cloning site)-Poly-A wurden aus pShuttle (Clontech) mit MfeI geschnitten, die Enden geglättet, und dann mit *Eco*RI geschnitten. Anschließend wurde die Kassette (Xvir-3'UTR pShuttle von Clontech) mit PmeI in den ebenfalls mit PmeI geschnittenen und dann dephosphorylierten CMV-MCS-Poly-A pΔE1sp1A Vektor umkloniert. Das Klonierungsprodukt Xvir-3'UTR-pΔE1sp1A wurde für die Virusherstellung verwendet.

### Virusherstellung

Xvir-3'UTR-pΔE1sp1A und pBHGE3 (von Microbix, enthält die E3-Region, die dem wildtyp des Adenovirus Typ 5 entspricht) wurden in HEK 293 Zellen kotransfiziert, wo durch Rekombination homologer Sequenzbereiche beider Vektoren das Virus Ad-Xvir-3'UTR E3 entstand.

### Herstellung des Adenovirus Ad-Xvir 3'UTR-AdEASY E3 mit dem AdEASY-System (Firma Qbiogene)

### Herstellung des verwendeten adenoviralen Shuttlevektors

Da der für das hier verwendete System vorliegende Vektor pShuttle-AdEASY weder einen CMV-Promotor noch das Bovine Growth Hormone Polyadenylierungssignal enthielt, wurden diese Elemente in pShuttle-AdEASY hineinkloniert. Hierzu wurde das Plasmid mit *Eco*RI verdaut, die Enden durch Auffüllen mit T4-Polymerase und dNTPs geglättet, das Backbone dephosphoryliert und die beiden entstandenen Schnittprodukte religiert. Durch diese Vorgehensweise wurde die Restriktionserkennungssequenzen für *Eco*RI vernichtet. Das daraus hervorgegangene Plasmid wurde pShuttle(-*Eco*RI)-AdEASY genannt.

Anschließend wurde die Kassette CMV-MCS-polyA aus dem pShuttle von Clontech mit *Mfe*I und *Eco*RI geschnitten, die Enden geglättet und in den Vektor pShuttle (-EcoRI)-AdEASY kloniert, der dafür mit *Xba*I linearisiert, geglättet und dephosphoryliert wurde. Daraus entstand das Plasmid CMV-MCS-PolyA-pShuttle-AdEASY.

In dieses Plasmid wurde die Kassette E4Orf6-IRES-EIB55k-3'UTR mit *Mlu*I und *Eco*RI hineinkloniert.
Dadurch entstand das Plasmid Xvir-3'UTR in pShuttle AdEASY. Dieses wurde mit Bst1107I und *Mro*I linearisiert und zusammen mit dem Rescue-Plasmid pAdEASY in BJ5183 (EC) Bakterien mittels Elektroporation eingebracht. Durch homologe Rekombination entstand das adenovirale Plasmid Ad-Xvir-3'UTR-pAdEASY, das nach Transfektion in HEK293 Zellen zur Virusproduktion führte.

### Einbringung der wt E3 Region in das pAdEASY

Da die E3 Region in dem Plasmid pAdEASY weitgehend deletiert ist, wurde für die Rekonstruktion die E3E4-Region aus dem Plasmid pAdEASY mit *Spe*I und *Pac*I in das Plasmid CMV-MCS-PolyA pShuttle (AdEASY) kloniert und damit das Plasmid E3E4-pShuttle-AdEASY generiert.

Durch Restriktion mit *Nde*I und Religation wurde von zwei *NdeI* Schnittstellen eine deletiert und damit auch die Multiple Klonierungsstelle aus dem Plasmid. Durch diese Prozedur entstand das Plasmid E3E4-pShuttle (-*Nde*I)-AdEASY.

Anschließend wurde das 4007 bp große wtE3-Region Fragment aus wildtyp Adenovirus Typ 5 mit *SpeI* und *NdeI* ausgeschnitten und in das *SpeI* und *NdeI* aufgeschnittene E3E4-pShuttle (-*Nde*I)-AdEASY kloniert. Der so entstandene Vektor wurde wtE3E4-pShuttle (-*Nde*I)-AdEASY benannt.

Anschließend wurde die wtE3 E4-Region aus dem E3E4-pShuttle (-*Nde*I)-AdEASY mit *Spe*I und *Pac*I geschnitten und in das mit *Spe*I und *Pac*I geschnittene pAdEASY reinkloniert, wodurch in dem Plasmid pAdEASY die E3-Region wieder hergestellt wurde (pAdEASY-E3).

Durch die Transformation von BJ5183 (EC) Bakterien mit den Plasmiden Xvir-3'UTR in pShuttle AdEASY und pAdEASY-E3 entstand durch homologe Rekombination Xvir-3'UTR-pAdEASY-E3.

### E4-Manipulation für die genannten Systeme

Um für mögliche therapeutische Gene oder Transgene Platz zu schaffen und um eine unerwünschte homologe Rekombination zu unterbinden kann die E4-Region im Plasmid E3E4-pShuttle (-NdeI)-AdEASY spezifisch deletiert werden. Dabei wird die E4orf6-Region durch Herausschneiden mit PstI und Religation um ca. 0.6 kb, bevorzugterweise 634 bp verkürzt. Dies kann wie beschrieben in Figur 17 Xvir03/01 der Fall sein. Entsprechende Deletionen sind in anderen Systemen zur Herstellung rekombinanter Adenoviren durch den Fachmann durchführbar.

### Klonierung des RGD-Motivs in Ad-Xvir 3'UTR-AdEASY E3 im speziellen (gilt aber auch für alle anderen Systemen)

Für die verbesserte Infektiösität wurde in Anlehnung an Dmitriev et al. 1998 (An Adenovirus Vector with Genetically Modified Fibers Demonstrates Expanded Tropism via Utilization of a Coxsackievirus and Adenovirus Receptor-Independent Cell Entry Mechanism) der HI Loop des Fiber-knob Domäne modifiziert: Die entsprechende Region wurde mit Primern RGD-Hpa fw (5'-GAGgttaacCTAAGCACTGCCAAG-3'), RGD-EcoRV rev (5'-CATAGAGTATGCAGATATCGTTAGTGTTACAGGTTTAGTTTTG-3') sowie RGD-EcoRV fw (5'-GTAACACTAACGATATCTGCATACTCTATGTCATTTTCATGG-3') und RGD-Bfr rev (5'-CAGCGACATGAActtaagTGAGCTGC-3') amplifiziert und dabei eine EcoRV-Schnittstelle generiert. In diese Schnittstelle wurden gepaarte Oligonukleotide kloniert, die für ein Arg-Gly-Asp (RGD)- Peptid kodieren: RGD-Oligo 1 (5'-CACACTAAACGGTACACAGGAAACAGGAGACACAACTTGTGACTGCCGCGGAGACT GTTTCTGCCC-3') und RGD-Oligo 2 (5'-GGGCAGAAACAG TCTCCGCGGCAGTCA CAAGTTGTGTCTCCTGTTTCCTGTGTACCGTTTAGTGTG-3'). Somit befindet sich das RGD-Motiv im HI Loop der Fiberknob-Domäne.

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, in YB-1-Kern-positiven Zellen sowie Zellen, in denen YB-1 dereguliert, d.h. im Vergleich zu Normal-Zellen bzw. Nicht-Tumorzellen überexprimiert vorliegt, zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

### Beispiel 12: Struktureller Aufbau des adenoviralen Vektors Xvir03/01

Wie aus Fig. 17 ersichtlich, ist XVIR03/01 eine Weiterentwicklung von XVIR03. Dabei können in der E3 Region therapeutische Gene wie beispielsweise die hierin beschriebenen Gene und Transgene kloniert werden. Zudem wurde eine Deletion in der E4-Region eingeführt, um eine homologe Rekombination mit dem E4orf6 aus der Expressionskassette von Xvir03 zu vermeiden. Dies führt auch dazu, dass bei diesem Konstrukt größere Transgene kloniert werden können. Die deletierte E3-Region enthält für das Einsetzen einer Kassette nutzbare SacI, NdeI und NheI- Schnittstellen, in die beispielsweise die therapeutischen Transgene einkloniert werden können. Es kann jedoch auch die E3-Region intakt verbleiben und die therapeutischen Gene in die E4-Region kloniert werden. Somit wird unter anderem die Expression des adenoviral death protein ADP sicher gestellt.

### Vorbereitung eines Plasmids zur Klonierung therapeutischer Gene in die E3-Region sowie zur Deletion in der E4-Region: System Adeno-X der Firma Clontech

Das pAdenoX-Plasmid von Clontech verfügt über eine SfuI -Schnittstelle hinter der 3' ITR-Region, die im wildtyp Adenovirus fehlt. Die E3-E4-Region wurde mit SpeI (Position 23644) und SfuI aus pAdenoX (Clontech) in pcDNA3.1(+) (Invitrogen) übernommen = pcDNA3.1-E3Δ27865-30995-E4. Der Großteil des E4ORF6, nämlich 33241-33875 wurde mittels PstI entfernt = pcDNA3.1-E3A27865-30995,E4A33241-33875. Für die Weiterentwicklung von XVIR03 wurde der deletierte E3/E4-Bereich aus pcDNA3.1-E3Δ27865-30995,E4Δ33241-33875 mittels SfuI und SpeI in das Plasmid pAdenoX kloniert = pAdenoX E3Δ27865-30995,E4Δ33241-33875.

Die Expressionskassette wurde anschließend wie für Xvir03 beschrieben aus dem E1B55k-IRES-E4orf6-pShuttle zusammen mit dem CMV-Promotor und dem *bovine growth hormone* (BGH)-PolyA in das pAdenoX E3Δ27865-30995,E4Δ33241-33875 mit I-Ceu I und PI-SceI kloniert und als AdcmvE1B/IRES/E4orf6-ΔE4 bezeichnet. Danach erfolgte die Adenovirusherstellung nach Herstellerangaben (Clontech).

Es ist im Rahmen der vorliegenden Erfindung und für den Fachmann im Lichte der vorliegenden Offenbarung offensichtlich und praktikabel, dass für die Herstellung der erfindungsgemäßen, bevorzugterweise rekombinanten Adenoviren auch andere Systeme verwendet werden können, z. B. das System der Firma QBIOGENE und der Firma Microbix.

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, bei YB-1-Kern-positiven Zellen sowie Zellen, in denen YB-1 dereguliert, d.h. im Vergleich zu Norinal-Zellen bzw. Nicht-Tumorzellen überexprimiert vorliegt zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

### Beispiel 13: Onkolytische Wirkung von Xvir 03 in 257 RDB- und 181 RDB-Zellen

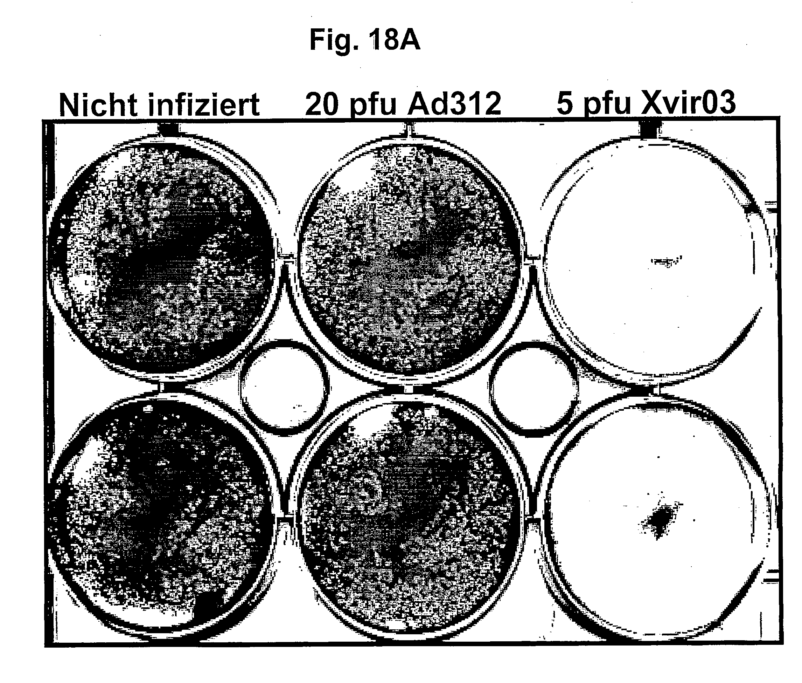

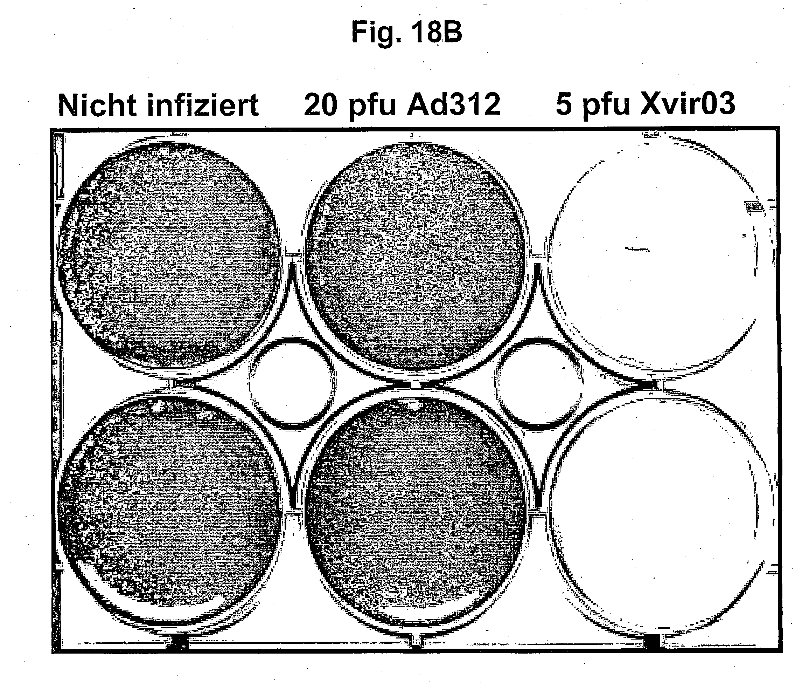

Pro Napf einer Platte mit sechs Näpfen (engl. 6 well plate) wurden jeweils 100.000 Zellen (257RDB und 181RDB) ausplattiert. Am nächsten Tag wurden die Zellen wie in Fig. 18 dargestellt mit Ad312 (20pfu/Zelle) und Xvir03 (5pfu/Zelle) infiziert. Die Infektion erfolgte in 500 µL serumfreie DMEM-Medium für 1 h bei 37° C. Anschließend wurde das Infektionsmedium entfernt und durch 2 ml Vollmedium (10 % FKS/DMEM) ersetzt. Nach 5 Tagen erfolgte die Auswertung mit Hilfe einer Kristallviolettfärbung. Das Ergebnis ist in Figs. 18A und 18B dargestellt.

Wie aus Fig. 18A und 18B ersichtlich, zeigen die vielfachresistenten Zellen, welche YB-1 im Kern aufweisen, nach Infektion mit Ad312 und Xvir03 nur bei Xvir03 eine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert. Danach werden die Platten mit sechs Näpfen mit Wasser gründlich gewaschen und bei Raumtemperatur getrocknet.

Es ist dem vorliegenden Erfinder bekannt, dass E1A-deletierte Viren (z. B. Ad312), die jedoch keine transaktivierenden Adenoviren im Sinne der vorliegenden Erfindung sind, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Der hier eingesetzte Adenovirus Ad312 ist ein E1A-deletiertes Virus. Bei der eingesetzten Menge (20 pfu/zelle), die noch oberhalb des klinisch wünschenswerten Titers liegt, werden die frühen adenoviralen Gene wie z. B. E1B55k und E4orf6 nicht oder nur sehr gering exprimiert (Nevins J. R., Cell 26, 213-220, 1981). Wie bereits beschrieben spielen diese Gene bzw. die Proteine eine bedeutende Rolle bei der viralen Replikation. Im Gegesatz dazu werden diese Gene bzw. Proteine beim Adenovirus Xvir03 exprimiert (Fig 16). Anhand der Fig. 18A und 18B wird ersichtlich, dass die Expression der Gene E1B55k und E4orf6 zur einer effizienten viralen Replikation und Zellyse führen bei gleichzeitig geringem erforderlichen, Infektionstiter (augedrückt als pfu/Zelle). Dies bestätigt die der vorliegenden Erfindung zugrundeliegende Erkenntnis, dass die Expression von E4orf6 und E1B-55K (und das Nicht-Vorhandensen von E1A) in Verbindung mit der Kernlokalisation von YB-1 in der Lage ist, eine sehr effiziente adenovirale Replikation zu induzieren. Die dazu erforderliche Menge von nur 1 bis 5 pfu/Zelle lässt jetzt eine klinischen Anwendung zu.

Dies bestätigt die der vorliegenden Erfindung zugrundeliegende Erkenntnis, dass das Vorhandensein von YB-1 im Kern, insbesondere Zellzyklus-unabhängig, erforderlich ist, um die erfindungsgemäß zu verwendenden Viren zu einer Lyse von infizierten Zellen zu veranlassen.

### Beispiel 14: Northern Blot Analyse der E2-Genexpression von Adenovirus Ad312

In 10 cm Petrischalen wurden jeweils 1 Million A549 und U2OS Zellen ausplattiert. Am nächsten Tag wurden die Zellen mit Ad312 (50pfu/Zelle) und Adwt (diente zur Kontrolle, 5pfu/Zelle) infiziert. Die eingesetzte hohe Virusmenge von Ad312 führt dabei zu einer E1-unabhängigen Replikation in Tumorzellen. Die Infektion erfolgte in 1-2 ml serumfreiem DMEM-Medium für 1 h bei 37° C. Anschließend wurde das Infektionsmedium entfernt und durch 10 ml Vollmedium (10 % FKS/DMEM) ersetzt. Nach 3 Tagen wurde die RNA isoliert. Anschließend wurde die Konzentration der isolierten RNA photometrisch bei 260 nm gemessen. Dann erfolgte eine elektrophoretisch Auftrennung der RNA-Proben in einem 0,8%igen Formaldehyd-Agarosegel. Anschließend wurde die RNA auf eine Nylonmembran geblottet (Durchführung nach dem System von Schleicher & Schuell). Die auf der Membran geblottete RNA wird gegen eine "frühe Sonde" E2 und gegen eine "späte Sonde" E2 hybridisiert. Die 1501 bp große "spät-Sonde" bindet spezifisch hinter dem E2-late Promotor. Die Sonde wurde zuvor per PCR (Primer:5'- GTC GGA GAT CAG ATC CGC GT (SEQ. ID. NO. 4), 5'- GAT CCT CGT CGT CTT CGC TT (SEQ. ID. NO. 5)) hergestellt und mit ³²P radioaktiv markiert. Die frühe Sonde bindet dagegen zwischen dem E2-early und den E2-late Promotor (Position: 226791-227002) und wurde ebenfalls mittels PCR hergestellt (Primer:5'- AGCTGATCTTCGCTTTTG (SEQ. ID. NO. 6), 5'-GGATAGCAAGACTCTGAC AAAG (SEQ. ID. NO. 7). Anschließend wird die Membran gewaschen und auf eine Filmfolie exponiert.

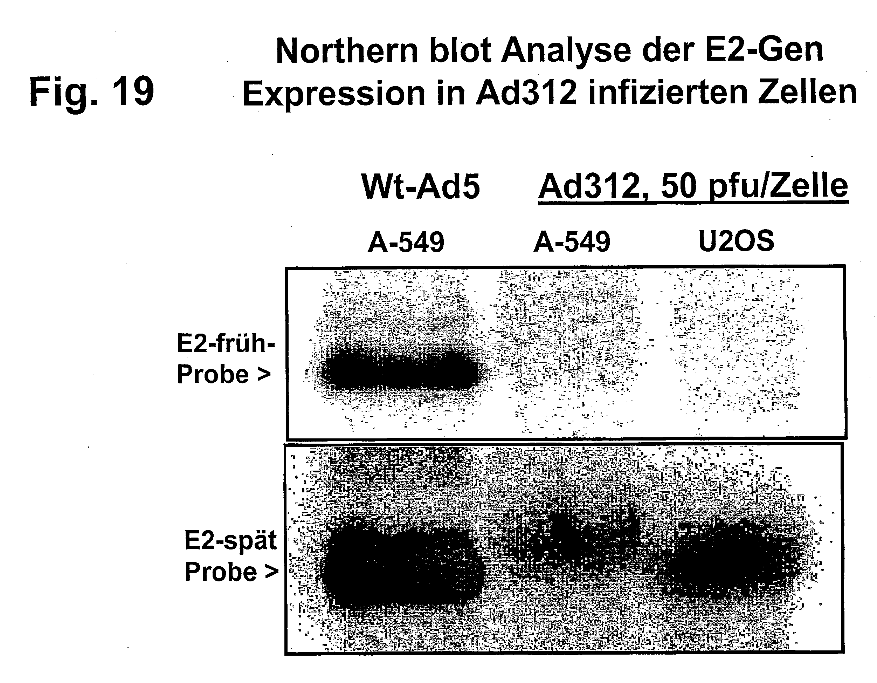

Das Ergebnis ist in Fig. 19 dargestellt. Während in der Kontrollinfektion mit Wildtyp-Adenovirus sowohl die frühe wie auch die späte Probe ein spezifisches Signal liefert, zeigen die mit Ad312 infizierten Tumorzellen nur ein spezifisches Signal bei der Verwendung der späten Probe. Dies bestätigt die der vorliegenden Erfindung zugrundeliegende Erkenntnis, dass die Expression von E4orf6 und E1B-55K und das Nicht-Vorhandensein von E1A überexprimiertes bzw. dereguliertes YB-1 in den Kern transportiert und somit die E2-Genexpression als Voraussetzung für eine effiziente adenovirale Replikation induziert.

### Beispiel 15: Northern Blot Analyse der E2-Genexpression von Adenovirus Addelta24

In 10 cm Petrischalen wurden jeweils 1 Million U2OS Zellen ausplattiert. Am nächsten Tag wurden die Zellen mit Adenovirus delta 24 (Addelta24) (10pfu/Zelle) und Adenovirus vom Wildtyp (Adwt) (diente zur Kontrolle, 10pfu/Zelle) infiziert. Das verwendete Addelta24 rekombinante Adenovirus (Fueyo, J. et al., Oncogene 19,2-12,2000) weist eine spezifische Deletion in der CR2-Region des E1A-Proteins auf und ist somit nur in der Lage, in Rb-negativen Tumoren zu replizieren. Ferner exprimiert das Virus vergleichbar wie Wildtyp-Adenovirus die Gene E1B55k und E4orf6. Die Infektion erfolgte in 1-2 ml serumfreie DMEM-Medium für 1 h bei 37° C. Anschließend wurde das Infektionsmedium entfernt und durch 10 ml Vollmedium (10 % FKS/DMEM) ersetzt. Nach 12 h und 24 h wurde die RNA isoliert. Anschließend wurde die Konzentration der isolierten RNA photometrisch bei 260 nm gemessen. Dann erfolgte eine elektrophoretisch Auftrennung der RNA-Proben in einem 0,8%igen Formaldehyd-Agarosegel. Anschließend wurde die RNA auf eine Nylonmembran geblottet (Durchführung nach dem System von Schleicher & Schuell). Die auf der Membran geblottete RNA wird gegen eine "frühe Sonde" und gegen eine "spät Sonde" hybridisiert. Die 1501 bp große "spät-Sonde" bindet spezifisch hinter dem E2-late Promotor. Die Sonde wurde zuvor per PCR (Primer:5'- GTC GGA GAT CAG ATC CGC GT (SEQ. ID. NO. 4), 5'- GAT CCT CGT CGT CTT CGC TT (SEQ. ID. NO. 5)) hergestellt und mit ³²P radioaktiv markiert. Die frühe Sonde bindet dagegen zwischen dem E2-early und den E2-late Promotor und wurde ebenfalls mittels PCR hergestellt (Primer:5'-AGCTGATCTTCGCTTTTG (SEQ. ID. NO. 6), 5'-GGATAGCAAGACTCTGACAAAG (SEQ. ID. NO. 7)). Anschließend wird die Membran gewaschen und auf eine Filmfolie exponiert.

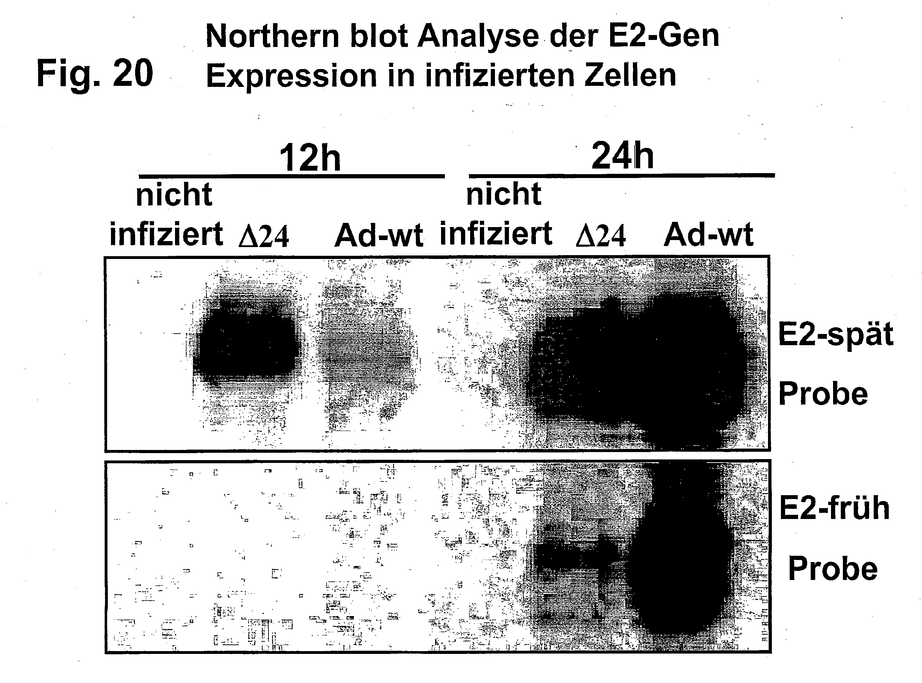

Das Ergebnis ist in Fig. 20 dargestellt.

Nach 12 h zeigt nur die späte Probe ein spezifisches Signal. Erst nach 24 h zeigt auch die frühe Probe ein Signal in den mit Addelta24 infizierten Zellen. Im Vergleich zum Wildtyp-Adenovirus ist das Signal jedoch deutlich schwächer. Auch dieses Ergebnis bestätigt die der vorliegenden Erfindung zugrundeliegende Erkenntnis, dass die Expression von E4orf6 und E1B-55K überexprimiertes bzw. dereguliertes YB-1 in den Kern transportiert, das dann an den E2-late Promotor bindet und somit die E2-Genexpression induziert.

### Beispiel 16: Struktureller Aufbau des adenoviralen Vektors XvirPSJL1 und XvirPSJL2

Beschreibung der Vektoren: Die Vektoren der XvirPSJL-Gruppe, die Ausführungsformen der hierin als Adenoviren der Gruppe I bezeichneten Viren darstellen und beispielhaft durch die Vektoren bzw. Adenoviren XvirPSJL1 und XvirPSJL2 veranschaulicht werden, sind nicht nur wie auch der Adenovirus dl520 in der Lage, in YB-1 Kern-positiven Zellen, insbesondere Tumorzellen zu replizieren, sondern auch in Tumorzellen, in denen YB-1 überexprimiert bzw. dereguliert vorliegt. Während die viralen Gene E1B55k und E4orf6 in dl520 infizierten Zellen nur in YB-1 Kern-positiven Zellen unter dem Einfluss des E1B-Promotors bzw. des E4-Promotors exprimiert werden, erfolgt die Expression von E1B55k und E4orf6 in XvirPSJL über den Cytomegalievirus (CMV)-Promotor. Statt des cmw-Promotors können allerdings auch andere Promotoren, insbesondere Tumor-, Gewebe- oder Organ-spezifische Promotoren und den natürlichen E1A-Promotor, d.h. insbesondere der E1A-Promotor, wie er in Adenoviren vom Wildtyp, insbesondere Ad5 vorkommt, verwendet werden. Durch die Expression von E1B55k und E4orf6 wird das überexprimierte bzw. deregulierte YB-1 in den Kern transportiert und die adenovirale Replikation eingeleitet. Die hierin offenbarten adenoviralen Vektoren der XvirPSJL-Gruppe vereinen somit verschiedenen Elemente und damit Funktionen der adenoviralen Vektoren dl520, Xvir03 und AdYB-1 in einem Vektor. Ähnlich dem Vektor dl520 weisen die XvirPSJL-Viren das E1A12S Gen auf Dieses Gen bzw. das entsprechende Genprodukt ist für die Induktion der S-Phase der infizierten Zelle verantwortlich und fördert die virale Replikation und die Wirkung von Chemotherapeutika und von Bestrahlung. Wie Xvir03 enthalten die XvirPSJL-Viren die Expressionskassette CMV- E4orf6/IRES/ E1B55k, die für eine effiziente Replikation benötigt wird und dereguliertes YB-1, welches bevorzugterweise in Tumorzellen vorhanden ist, indirekt oder direkt in den Kern transportiert. Damit ist eine Replikation nur in solchen Zellen, insbesondere Tumorzellen, möglich, in denen YB-1 überexprimiert bzw. dereguliert vorliegt. Zudem wird P53 durch den Komplex E1B55k/E4orf6 der Degradation zugeführt. Aus dem Virus AdYB-1 stammt die für den humanen Transkriptionsfaktor YB-1 codierende Sequenz. Das endogene, d. h. das in der Zelle bereits vorhandene Protein YB-1 verstärkt die virale Replikation. Sowohl E1A12S wie auch YB-1 werden durch den YB-1-abhängigen adenoviralen Promoter E2-Late in ihrer Expression gesteuert. Auch hier können in einer Ausführungsform spezifische Promotoren verwendet werden, insbesondere Tumor-, Gewebe- oder Organ-spezifische Promotoren verwendet werden.. Als ein weiteres Merkmal dieser Viren liegt eine Deletion in der E4-Region vor. Der Vektor besitzt dort Schnittstellen, in die bei den adenoviralen Vektoren XvirPSJL1 und XvirPSJL2 verschiedene Transgene, wie in der Beschreibung offenbart, beispielsweise Ribozyme, Antisense-Moleküle, siRNA, Apoptoseinduzierende-Gene, Zytokine und Prodrug-Gene, zur Expression gebracht werden können. Deren Expression kann ebenfalls durch Tumor-, Gewebe-oder Organ-spezifische Promotoren gesteuert werden, wie in der Beschreibung offenbart. Die einzelnen Expressionskassetten sind in ihrer Lokalisation, insbesondere hinsichtlich der und innerhalb der E1-, E3- und E4-Region nicht festgelegt, sondern können beliebig angeordnet sein. Dabei können die nicht benötigten Regionen deletiert aber auch intakt bleiben. Die Vektoren replizieren unabhängig vom p53 oder Rb-Status der Tumorzellen.

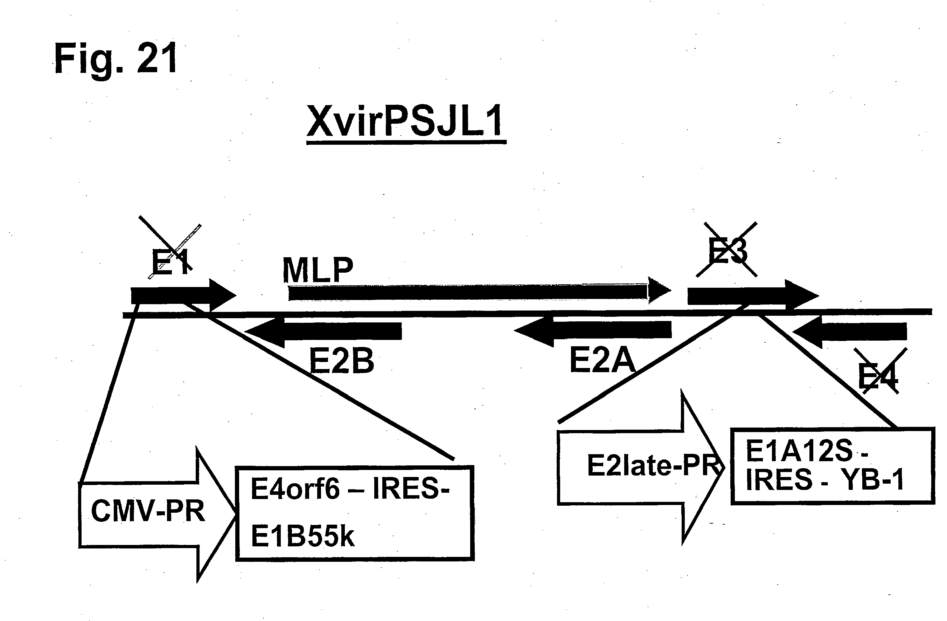

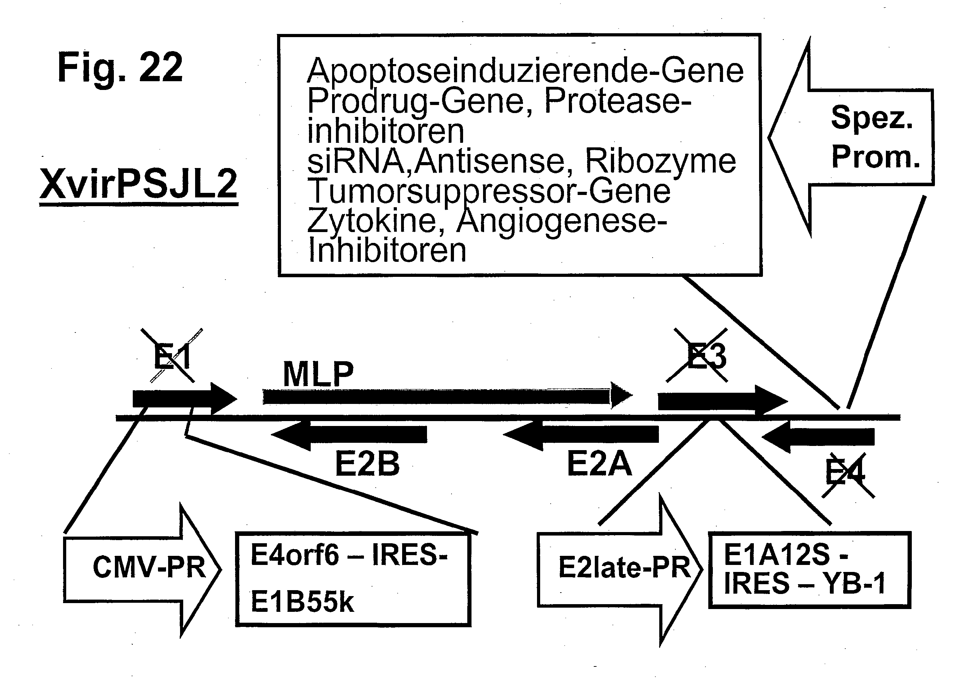

Der struktuelle Aufbau der rekombinanten Adenoviren XvirPSJL1 und Xvir PSJL2 sind in den Abbildungen 21 und 22 dargelegt:

### Herstellung des Vektors XvirPSJL nach dem System Aden-X der Firma Clontech

### Herstellung der Kassette E2-late- YB1IRES/12S:

Das hierin als Ausgangsmaterial verwendete pAdenoX-Plasmid von der Firma Clontech/ BD Biosciences umfasst die genomische Nukleinsäure von Adenovirus Ad5 und verfügt über eine SfuI -Schnittstelle hinter der 3' ITR-Region, die im Wildtyp-Adenovirus fehlt. Die E3-E4-Region wurde mit SpeI (Position 23644) und SfuI aus pAdenoX (Clontech) in pcDNA3.1(+) (Invitrogen) übernommen und als pcDNA3.1-E3Δ27865-30995-E4 bezeichnet. Der Großteil des E4ORF6, nämlich die Basen 33241-33875 wurde mittels PstI entfernt. Das solchermaßen erhaltene Fragment wurde als pcDNA3.1-E3Δ27865-30995, E4Δ33241-33875 bezeichnet.

Der E2late-Promoter wurde aus pGL3-EGFP (Holm et al., JBC 2002, 277, 10427-10434) mit Sacl und NheI ausgeschnitten und in pcDNA3.1-E3Δ27865-30995,E4Δ33241-33875 kloniert. Dabei wird die E3-Region weiter deletiert im Bereich der Basen Δ27593-31509. Das solchermaßen erhaltene Fragment wurde als E2late-pcDNA3.1 E3Δ27593-31509, E4Δ33241-33875 bezeichent.

Die cDNA für das E1A-243AS-Produkt wurde mittels einer RT-PCR hergestellt, isoliert und die Sequenz überprüft und wurde über BamHI und EcoRI in den pcDNA3.1(+) Vektor (Invitrogen) kloniert. E1A-12S-pcDNA3.1+ wurde mit NheI und BamHI linearisiert, die Enden mit T4-Polymerase geglättet und durch Taq-Polymerase und dTTPs mit T-Überhängen versehen. Das IRES-Element wurde als PCR-Produkt (Template = pCITE, Novagen) in den E1A-12S-pcDNA 3.1(+) Vektor kloniert (TA-Cloning-Strategie).

Das YB-1 -EcoRI-Fragment wurde aus dem Vektor pHVad2c (Holm et al., JBC 2002, 277, 10427-10434) isoliert und die Enden geglättet. Der Vektor pShuttle (kommerziell erhältlich von der Firma BD Biosciences) wurde mit XbaI linearisiert, die Enden geglättet und dephosphoryliert und mit der zuvor hergestellten für YB-1 codierenden Nukleinsäure verküpft. Der solchermaßen erhaltene Vektor wurde als YB-1-pShuttle bezeichnet. Die Umklonierung in den pShuttle-Vektor versah die für das YB-1-Fragment codierende Nukleinsäure mit einem STOP-Codon im Leserahmen. Aus YB-1-pShuttle wurde die für das YB-1 codierende Nukleinsäure mittels *Nhe*I und *Bfr*I in den Vektor IRES-E1A-12S in pcDNA3-1 (+) kloniert. Das solchermaßen erhaltene Fragment wurde als YB-1 (*Eco*RI-*Eco*RI mit STOP-Codon)-IRES-E1A-12S- pcDNA3.1(+) bezeichnet.

Anschließend wurde daraus die Kassette YB-1-IRES-E1A12S mit *Pme*I ausgeschnitten und in den mit *Nhe*I linearisierten, geblunteten und dephosphorylierten Vektor E2late-pcDNA3.1 E3Δ27593-31509,E4Δ33241-33875 kloniert. Damit befindet sich die zweite Kassette im deletierten Bereich der E3-Region.

Die transgene Kassette umfassend das Nukleinsäurekonstrukt E2late-YB-1-IRES-E1A12S wurde zusammen mit den restlichen adenoviralen Sequenzen E3Δ27593-31509,E4Δ33241-33875 mittels *Sfu*I und *Spe*I in den Vektor pAdenoX der Firma Clontech kloniert (=AdenoX/E2late-YB-1-IRES-E1A12S/E3Δ27593-31509,E4Δ33241-33875).

Die Kassette CMV-E1B55k/IRES/E4orf6 wurde aus dem oben unter Xvir03 beschriebenen pShuttle mittels I-CeuI und PI-SceI herausgeschnitten und in den Vektor AdenoX/E2late-YB-1-IRES-E1A12S/E3Δ27593-31509,E4Δ33241-33875 eingefügt.

Anschließend wurde der Vektor mit Pac I linearisiert, in 293-Zellen tranfiziert und das rekombinante Adenovirus XvirPSJL 1 bzw. 2, ohne die in der Fig. angegebenen Transgene, nach Herstellerangaben isoliert.

Es ist im Rahmen der vorliegenden Erfindung und für den Fachmann im Lichte der vorliegenden Offenbarung durchführbar, dass für die Herstellung der erfindungsgemäßen, bevorzugterweise rekombinanten Adenoviren, insbesondere solche, welche die Kassetten E4orf6-IRES-E1B55k bzw. E1A12S-IRES-YB-1 einzeln und/oder zusammen enthalten, auch andere Systeme verwendet werden können, z. B. das System der Firma QBIOGENE und MICROBIX. Zudem können die einzelnen Transgene innerhalb der Kassetten, insbesondere der jeweiligen Kassetten untereinander ausgetauscht werden. Ferner kann die Kassette E1A12S-IRES-YB-1 nur aus E1A12S bestehen und/oder aus E1A12S über IRES mit anderen relevanten Genen verknüpft werden.

### Herstellung des Adenovirus AdPSJL-E2late Promotor 12S-AdEASY mit E1A12S in der deletierten E3-Region mit dem AdEASY-System (Firma Microbix).

### Klonierung von PSJL 12S

Zunächst wurde der E2Late-Promotor als gepaarte Oligonukleotide (Upper Primer 5'-TCGAGCTCCGCATTTGGCGGGCGGGATTGGTCTTCGTAGAACCTAATCTCGTGGGCG TGGTAGTCCTCAGGTACAAAT-3' und Lower Primer 5'-AGCTTATTTGTACCTGAGGACTACCACGCCCACGAGATTAGGTTCTACGAAGACCAA TCCCGCCCGCCAAATGCGGAGC-3' in die *Hind*III und *Bgl*II Schnittstelle des pGL3-Enhancer Plasmids kloniert (pGL3-E2Late).

Anschließend wurde das Luziferasegen mit *Nco*I und *Xba*I ausgeschnitten, die Enden geglättet und T-Enden angehängt. An der somit geöffneten Stelle wurde das Transgen E1A12S, das durch RT-PCR mit den Primern E1A 12S Vorwärtsprimer 5'-ATGGCCGCCAGTCTTTTG-3' und E1A 12S Rückwärtsprimer 5'-TTATGGCCTGGGGCGTTTAC-3' amplifiziert wurde, durch TA-Klonierung eingebracht.

Diese Kassette wurde mit *Pvu*I und *Cla*I rausgeschnitten, die Enden geglättet und in die geglättete und dephosphorylierte *Nhe*I-Schnittstelle in die E3-Region von E3E4-pShuttle (-*Nde*I)-AdEASY hineinkloniert. Die Kassette enthält damit den E2Late-Promotor, den offenen Leserahmen E1a-12S und das SV-40 Late Polyadenylierungssignal. Das entstandene Konstrukt ist E2Late-E1a-12S-E3E4-pShuttle (-*Nde*I)-AdEASY.

Anschließend wurde die E2Late-Ela 12S-E3E4 aus dem E2Late-E1a 12S-E3E4-pShuttle (-*Nde*I)-AdEASY mit *Spe*I und *Pac*I geschnitten und in das mit *Spe*I und *Pac*I geschnittene pA-dEASY reinkloniert. Das daraus entstandene Konstrukt wurde mit E2Late-E1a 12S-E3E4-pAdEASY bezeichnet.

Durch die Transformation von BJ5183 (EC) Bakterien mit den Plasmiden Xvir-3'UTR in pShuttle AdEASY und E2Late-E1a 12S-E3E4-pAdEASY entstand durch homologe Rekombination AdPSJL-12S-AdEASY.

### Herstellung des Adenovirus AdPSJL-E2late Promotor-12S-YB-1-AdEASY mit E1A12S und YB-1 in der deletierten E3-Region mit dem AdEASY-System (Firma Microbix)

### Klonierung des Vektors E4ORF6-IRES-pcDNA3.1(+)

Die Amplifikate E1a 12S (Siehe oben) und das IRES-Element (Siehe oben) wurden nacheinander in die multiple Klonierungsstelle des pcDNA3.1(+)-Vektor hineinkloniert. Hierzu wurden das E1a-12S Amplifikat in die geglättete *Bam*HI-Schnittstelle durch TA-Klonierung eingebracht. Anschließend wurde das Plasmid E1a-12S in pcDNA3.1(+) mit *Eco*RV linearisiert, T-Enden angehängt und das Amplifikat für das IRES-Element hineinkloniert. Das daraus entstandene Plasmid wurde anschließend mit *Xho*I linearisiert, die Enden geglättet und das *Eco*RI-*Eco*RI-Schnittprodukt von YB-1, das ohne Stopcodon ist.

Das so entstandene konstrukt E1A-12S-IRES-pcDNA3.1(+) wurde mit *Not*I linearisiert und die Enden geglättet, ebenso wurde das YB-1-EcoRI-Schnittprodukt geglättet und in den dephosphorylierten Vektor E1A-12S-IRES-pcDNA3.1(+) eingebracht. Die Kassette E1A-12S-IRES-YB-1 wurde mit *Pme*I rausgelöst und in das oben beschriebene Plasmid pGL3-E2Late nach Entfernung des Luziferasegens mit *Nco*I und *Xba*I und Glätten und Dephosphorylierung hineinkloniert.

Die Kassette E21ate-E1A-12S-IRES-YB-1 wurde mit *Pvu*I und *Cla*I rausgeschnitten, die Enden geglättet und in die geglättete und dephosphorylierte *Nhe*I-Schnittstelle in die E3-Region von E3E4-pShuttle (-*Nde*I)-AdEASY hineinkloniert. Das entstandene Konstrukt ist E2late Promotor-E1A-12S-IRES-YB-1-E3E4-pShuttle (-*Nde*I)-AdEASY.

Anschließend wurde die E2late Promotor-E1A-12S-IRES-YB-1-E3E4-Kassette aus dem E2late Promotor-E1A-12S-IRES-YB-1-E3E4-pShuttle (-*Nde*I)-AdEASY mit *Spe*I und *Pac*I geschnitten und in das mit *Spe*I und *Pac*I geschnittene pAdEASY reinkloniert. Das resultierende Konstrukt wurde mit E1a-12S-IRES-YB-1-E3E4-pAdEASY bezeichnet.

Durch die Transformation von BJ5183 (EC) Bakterien mit den Plasmiden Xvir-3'UTR in pShuttle AdEASY und E1a-12S-IRES-YB-1-E3E4-pAdEASY entstand durch homologe Rekombination AdPSJL-12S-Yb-1-AdEASY.

### Klonierung der Kassette E2late Promotor-E1A-12S und/oder E2late Promotor-E1A-12S-IRES-YB-1 in die E4-Region

Nach Manipulation bzw. Deletion der E4-Region mit PstI wird 634 bp entfernt. Hierin können die Kassetten E2late Promotor-E1A-12S und/oder E2late Promotor-E1A-12S-IRES-YB-1 in die E4-Region eingebracht werden. Dabei kann die E3 Region alternativ intakt verbleiben.

### Klonierung des RGD-Motivs

Für die verbesserte Infektiösität wurde in Anlehnung an Dmitriev et al. 1998 (An Adenovirus Vector with Genetically Modified Fibers Demonstrates Expanded Tropism via Utilization of a Coxsackievirus and Adenovirus Receptor-Independent Cell Entry Mechanism) der HI Loop des Fiber knoten-Domäne modifiziert: Die entsprechende Region wurde mit Primern RGD-Hpa fw (5'-GAGgttaacCTAAGCACTGCCAAG-3'), RGD-EcoRV rev (5'-CATAGAGTATGCAGATATCGTTAGTGTTACAGGTTTAGTTTTG-3'), sowie RGD-EcoRV fw (5'-GTAACACTAACGATATCTGCATACTCTATGTCATTTTCATGG-3') und RGD-Bfr rev (5'-CAGCGACATGAActtaagTGAGCTGC-3') amplifiziert und dabei eine EcoRV-Schnittstelle generiert. In diese Schnittstelle wurden gepaarte Oligonukleotide kloniert, die für ein Arg-Gly-Asp (RGD)- Peptid kodieren RGD-Oligo 1 (5'-CACACTAAACGGTACACAGGAAACAGGAGACACAACTTGTGAC TGCCGCGGAG ACTGTTTCTGCCC-3') und RGD-Oligo 2 (5'-GGGCAGAAACAG TCTCCGCGGCAGTCACAAGTTGTGTCTCCTGTTTCCTGTGTACCGTTTAGTGTG-3') Somit befindet sich im HI Loop der Fiberknob-Domäne das RGD-Motiv.

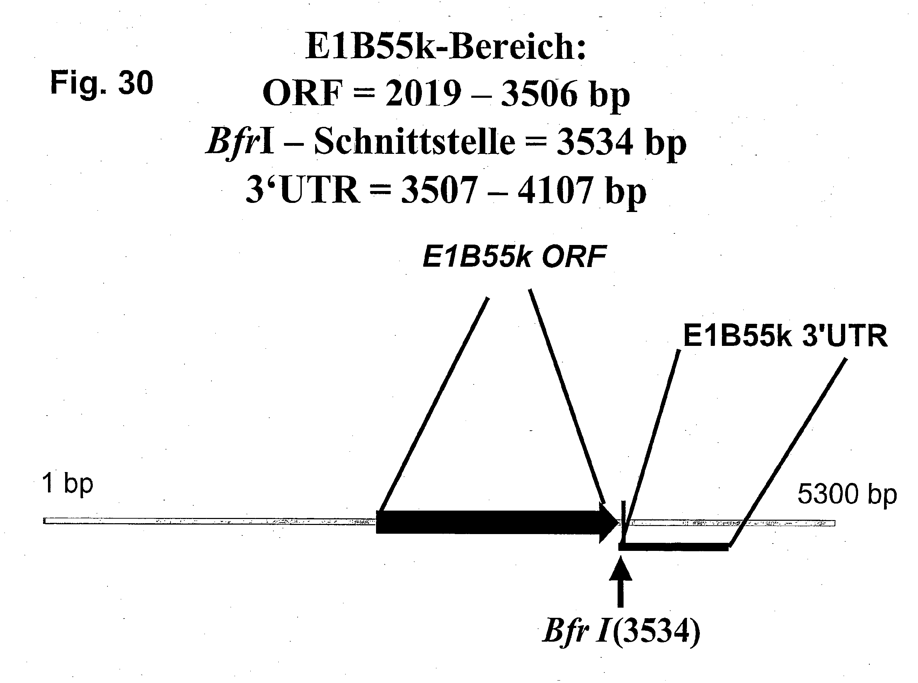

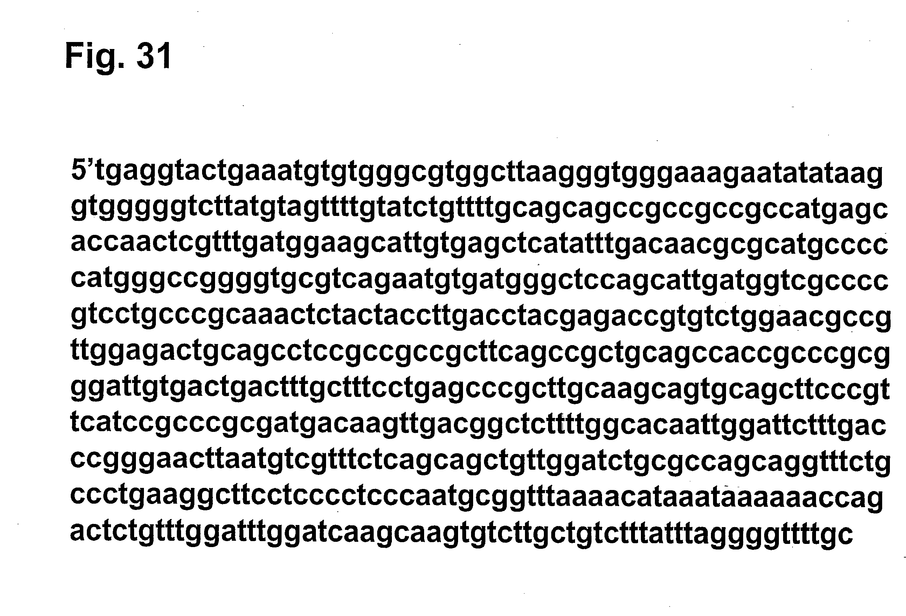

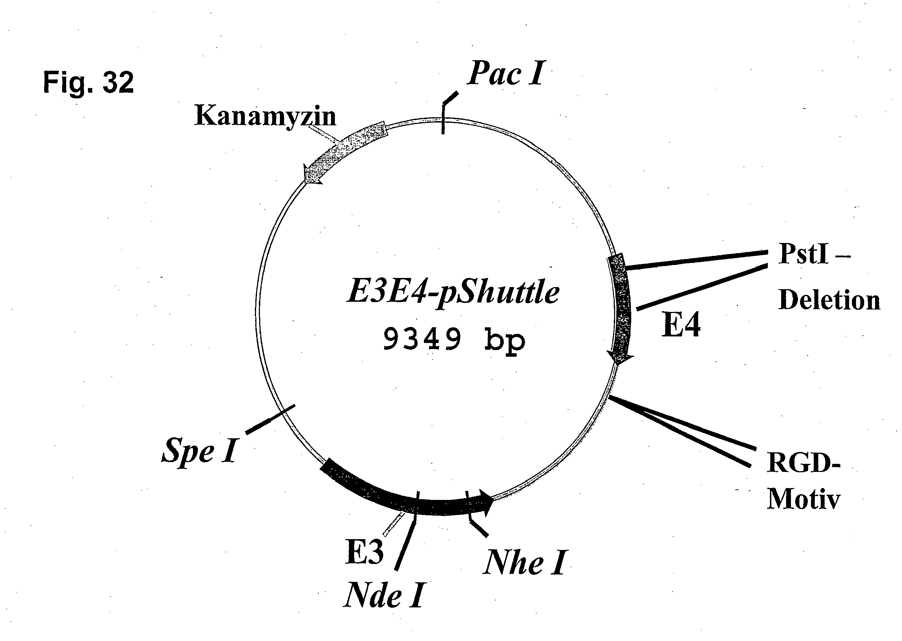

In den Figs. 30 und 31 ist unter anderem das einklonierte E1B55k-3'UTR näher beschrieben und anhand der Fig. 32 ist das pE3/E4-Shuttle Plasmid im Adeasy-System dargestelllt. Das Plasmid zeichnet sich dadurch aus, dass in den Bereichen E3 und E4 Manipulationen bzw. Deletionen vorgenommen sind, die es erlauben, verschiedenartige Kassetten mit Hilfe von spezifischen Restriktions-Schnittstellen beispielsweise NheI für die E3-Region und PstI für die E4-Region zu klonieren, ohne dabei andere offene Leserahmen als das E4orf6 oder das L5 zu beeinträchtigen. Ferner erfolgt das Einfügen einer Sequenz für das RGD-Motiv im Bereich des HI-Loops.

Positionsangaben der Deletionen entsprechen der WT-Adenovirus Sequenz.
E3-Deletion: 28138-30818
E4-Deletion: 33246-33875
RGD-Motiv: 32678 (eingefügt 9 Aminosäuren, zentral 3 Aminosäuren RGD; CDCRGDCFC

### Beispiel 17: Infektion von HeLa-Zellen mit Adenovirus dl520

Pro Schale wurden 100.000 HeLa-Zellen ausplattiert. Am nächsten Tag wurden die Zellen mit verschiedenen Konzentrationen (pfu/ml) von Adenovirus dl520 infiziert. Die Infektion erfolgte in 500 µL serumfreiem DMEM-Medium für 1 h bei 37° C. Anschließend wurde das Infektions-medium entfernt und durch 2 ml Vollmedium (10 % FKS/DMEM) ersetzt. Nach 3-5 Tagen erfolgte die Auswertung mit Hilfe einer Kristallviolettfärbung.

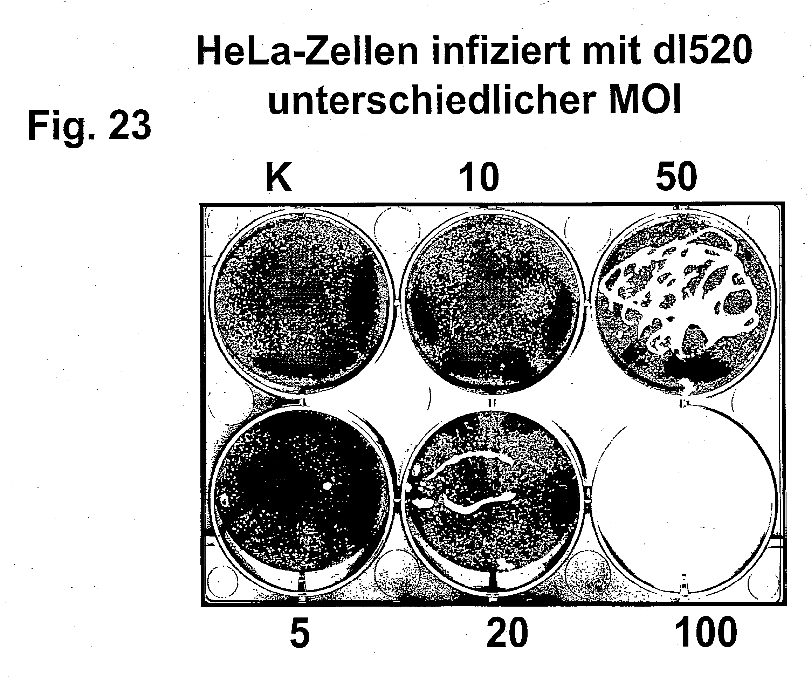

Das Ergebnis dieses Versuchs ist in der Abbildung 23 dargestellt. Das Adenovirus dl520 zeigt keine Lyse bei niedrigen MOIs (5-10 pfu/Zelle) bei Infektion von HeLa-Zellen, welche YB-1 nicht im Kern aufweisen. Im Gegensatz dazu zeigt dl520 eine praktisch vollständige Lyse bei einer MOI (engl. multiplicity of infection) von 100-200 pfu pro Zelle und eine noch überwiegende Lyse bei einer MOI von 50 pfu pro Zelle. Daraus ergibt sich, dass dl520 und vergleichbare Viren, die bei höheren MOI's die adenoviralen Gene E1B55k und E4orf6 einzuschalten in der Lage sind, überexprimiertes bzw. dereguliertes YB-1 direkt oder indirekt in den Kern zu transportieren und somit eine Zelllyse zu induzieren.

### Beispiel 18: Luziferase-Assay zur Bestimmung der E2-late Promoteraktivität

Es ist bekannt, dass YB-1 im Kern an den adenoviralen E2-late-Promotor bindet (Holm et al., JBC 2002, 277, 10427-10434) und sich dieser Promotor auch sehr gut für die Expression von Nukleinsäuren eignet. Die Verwendung des adenoviralen E2-late-Promotors wird insbesondere durch seine Regulierbarkeit durch YB-1 bestimmt, wobei YB-1 als positiver Effektor wirksam ist, d.h. der Promotor erst bei Anwesenheit von YB-1 im Kern aktiv ist. Insoweit ist der besagte adenovirale E2-late-Promotor hochselektiv regulierbar und somit in Systemen verwendbar, in denen YB-1 im Kern vorhanden ist und praktisch eine jegliche Expression der unter der Kontrolle des adenoviralen E2-late-Promotors stehenden Nukleinsäure verhindert für den Fall, dass YB-1 als Effektor bzw. Regulator im Kern nicht vorhanden ist. Der E2-Late Promotor weist 3 Y-Boxen (CCAAT) auf, welche eine Rolle bei der Aktivierung des E2-Gens spielen. Verschiedene E2-late-Promotorkonstrukte wurden hergestellt und auf ihre Spezifität und Aktivität hin unter-sucht. Die Untersuchungen wurden wie folgt durchgeführt.

Die Zellinien EPG-257 RDB (epitheliales Magenkarzinom), bei der YB-1 im Kern vorliegt, He-La (epitheliales Karzinom des Gebärmutterhalses) und U2OS (Osteosarkom) wurden in drei verschiedenen Zellkonzentrationen in 6 Kavitätenplatten ausgesät. Die Kavitäten, die am nächsten Tag eine 70%ige Konfluenz aufwiesen, wurden für die Transfektion verwendet: Für jede Kavität wurden 500 ng über SpinMiniprep (Qiagen) gereinigte Plasmid-DNA von den verschiedenen E2-late-Promotorkonstrukten in Luziferasevektoren (kommerziell erhältlich von der Firma Promega, Ausgangsplasmid: pGL3-enhancer) in 500 µl OptiMEM in ein 1,5 ml Schnappdeckelreaktionsgefäß gegeben und 5 µl DOTAP in 500 µl in ein weiteres Schnappdeckelreaktionsgefäß. Beide Lösungen wurden vereint und gemischt. Für die Komplexbildung wurde das Gemisch für 30 Minuten bei Raumtemperatur inkubiert. Die Zellen wurden dreimal mit PBS gespült und mit dem Transfektionsgemisch überschichtet. Die Platten wurden für 5 Stunden bei 37 °C inkubiert, anschließend wiederum dreimal mit PBS gespült und mit Vollmedium versehen.

Die Zellen wurden 48 h nach der Transfektion mit dem Luzifase Assay System -Kit von Promega (Cat. No. E1500) aufbereitet: Jedes Well wurde mit 500 µl Lysis-Puffer überschichtet, die Zellen nach 10 Minuten bei RT mit einer 1 ml Pipette vom Plattenboden abgespült und in 1,5 ml Schnappdeckelreaktionsgefäße überführt. Die Zellysate wurden anschließend bei 4 °C für 15 Minuten bei 14 000 U/min zentrifugiert. Jeweils 50 µl des Überstandes wurde mit 100 µl Luziferase-Substrat versetzt und mit Topcount (Firma Canberra-Packard GmbH 63303 Dreieich) Microplate Scintillation & Luminescence counter in schwarzen Platten mit 96 Wells bei einer Wellenlänge von 945 nm gemessen.

Die Proteinmessung erfolgte mit dem BCA Protein Assay Reagent Kit, Katalognummer 23227 (PIERCE, Rockford, Illinois, USA) bei 570 nm in einem Bioluminometer (biolumin™ 960) kinetic fluorescece/absorbance-Plattenlesegerät der Firma Molecular Dynamics. Die relativen Lichtsignale der Proben wurden auf die Proteinmenge berechnet (RLU/ µg Protein).

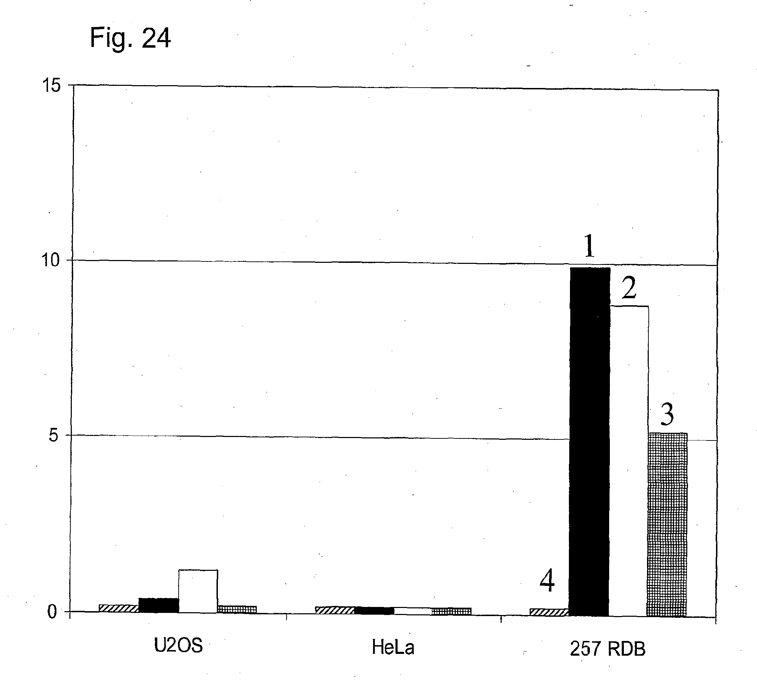

Es wurden die folgenden Plasmide verwendet: pGL3-Enhancer (Firma: Promega), aus dem der Enhancer mittels BamHI (2250 bp) und BsaBI (2003 bp) entfernt wurde diente als Leerkontrolle. In den Enhancer-losen pGL3-Vektor wurden die verschiedenen E2-Promoter Konstrukte über die Schnittstellen Apa I und Sac I in die MCS einkloniert. In den pGL3-Enhancer wurde der hCMV-Promoter mit Bgl II und Hind III einkloniert und diente als Positivkontrolle. Die Positivkontrolle erlaubte eine Abschätzung der Transfektionseffizienz und diente zudem als Referenzwert für die Luziferaseaktivität. Für jede Zellinie wurde die CMV-Kontrolle gleich 100 % gesetzt und die durch die E2-Promotor-Konstrukte erzielte Enzymaktivität darauf bezogen und als Säulendiagram in Fig. 24 dargestellt.

Die verschiedenen Konstrukte wurden wie folgt bezeichnet:
1. umfassend die Y-Box I, II und III entsprechend den Basen 25932 - 26179 bp (bezogen auf die Wildtyp-Adenovirus-Sequenz, siehe Auschnitt der nachfolgend angegebenen adenoviralen E2-Region)
2. umfassend die Y-Box II und III entsprechend den Basen 25932 - 26127 bp (bezogen auf die Wildtyp-Adenovirus-Sequenz, siehe Auschnitt der nachfolgend angegebenen adenoviralen E2-Region)
3. umfassend die Y-Box III entsprechend den Basen 25932 - 26004 bp (bezogen auf die Wildtyp-Adenovirus-Sequenz, siehe Auschnitt der nachfolgend angegebenen adenoviralen E2-Region)
4. umfassend keine Y-box, da Leerkontrolle

### Auschnitt der adenoviralen E2-Region (entnommen aus Virology 1992,186,280-285) (Die YB-1 Bindungsstellen sind in Fettdruck gehalten):

25561 aggaactttatcctagagcgctcaggaatcttgcccgccacctgctgtgcacttcctagc
25621 gactttgtgcccattaagtaccgcgaatgccctccgccgctttggggccactgctacctt
25681 ctgcagctagccaactaccttgcctaccactctgacataatggaagacgtgagcggtgac
25741 ggtctactggagtgtcactgtcgctgcaacctatgcaccccgcaccgctccctggtttgc
25801 aattcgcagctgcttaacgaaagtcaaattatcggtacctttgagctgcagggtccctcg
25861 cctgacgaaaagtccgcggctccggggttgaaactcactccggggctgtggacgtcggct
25921 taccttcgcaaatttgtacctgaggactaccacgcccacgagattaggttctacgaagac
25981 caatcccgcccgccaaatgcggagcttaccgcctgcgtcattacccagggccacattctt
26041 ggccaattgcaagccatcaacaaagcccgccaagagtttctgctacgaaaggacgggg
26101 gtttacttggacccccagtccggcgaggagctcaacccaatcccccgccgccgcagccc
26161 tatcagcagcagccgcgggcccttgcttcccaggatggcacccaaaaagaagctgcagct
26221 gccgccgccacccacggacgaggaggaatactgggacagtcaggcagaggaggttttgga
26281 cgaggaggaggaggacatgatggaagactgggagagcctagacgaggaagcttccgaggt
26341 cgaagaggtgtcagacgaaacaccgtcaccctcggtcgcattcccctcgccggcgcccca

26401 gaaatcggcaaccggttccagcatggctacaacctccgctcctcaggcgccgccggcact
26461 gcccgttcgccgacccaaccgtagatgggacaccactggaaccagggccggtaagtccaa
26521 gcagccgccgccgttagcccaagagcaacaacagcgccaaggctaccgctcatggcgcgg
26581 gcacaagaacgccatagttgcttgcttgcaagactgtgggggcaacatctccttcgcccg
26641 ccgctttcttctctaccatcacggcgtggccttccccgtaacatcctgcattactaccg
26701 tcatctctacagcccatactgcaccggcggcagcggcagcggcagcaacagcagcggcca
26761 cacagaagcaaaggcgaccggatagcaagactctgacaaagcccaagaaatccacagcgg

(SEQ. ID. No. 8)

Die in der Figur 24 dargestellten Ergebnisse belegen eindrucksvoll, dass die einzelnen Promotor-Fragmente, welche unterschiedliche E2-late/Y-boxen enthalten, für die Expression von therapeutischen Transgenen in YB-1 kernpositiven Tumorzellen geeignet sind und somit als Promotoren im Sinne der vorliegenden Erfindung verwendet werden können.

### Beispiel 19: Wirkung von adenoviral exprimiertem YB-1 auf Partikalfreisetzung

Humane Osteosarkomzellen (U2OS) wurden mit dem E1/E3-deletierten adenoviralen Vektor AdYB-1 und dem lediglich E1A-deletierten Ad312 mit einer MOI von 50 pfu/cell infiziert. A-dYB-1 enthält in seinem Genom die für den zellulären Transkriptionsfaktor YB-1 codierende Sequenz und exprimiert so das Y-Box bindende Protein 1 (YB-1). Um die Freisetzung viraler Partikel in Form von 'plaque forming units' (pfu) nach der Infektion zu evaluieren, wurde zum einen nur der Nährmedium-Überstand und zum anderen der verbliebene Zellrasen 2 bzw. 5 Tage post infectionem isoliert. Die intrazellulären Partikel wurden durch 3-maliges 'thaw/freeze' freigesetzt. Die Partikelzahl wurde mit Hilfe eines Plaque Assay auf 293-Zellen ermittelt.

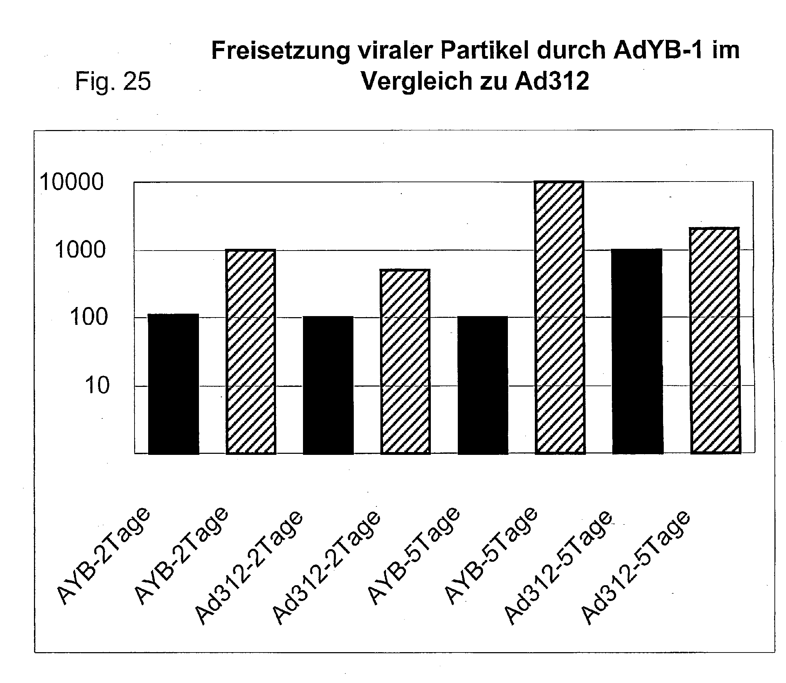

Das Ergebnis ist in Fig. 25 dargestellt, wobei die soliden Balken die intrazellulär verbliebenen viralen Partikel und die quergestreiften Balken die freigesetzten, extrazellulären viralen Partikel anzeigen.

Das in Fig. 25 dargestellte Ergebnis belegt, dass AdYB-1 insgesamt mehr pfu bildet als Ad312 und erheblich mehr Partikel freisetzt. Nach 5 Tagen zeigen die AdYB-1-infizierten Zellen im Gegensatz zu den Ad312-infizierten Zellen deutlich einen cytopathischen Effekt (CPE) an.

### Beispiel 20: Replikation von Adenovirus in Zellen nach Gabe von Irinotecan

Um die Wirkung von Irinotectan auf die adenovirale Replikation zu bestimmen, wurden 10⁶-Tumorzellen U373 in 10 cm² Petrischalen ausplattiert. In einem ersten Ansatz erfolgte nach 24 h die Zugabe von 5µM Irinotecan. Nach wiederum 24 h wurden die Zellen mit 10 pfu/Zelle dl520 infiziert. Nach 3 Tagen Incubation ohne Irinotecan wurde die DNA gemäß der in Beispiel 10 beschriebenen Vorgehensweise isoliert.

In einem parallelen Ansatz wurden die solchermaßen angesetzten U373-Zellen nicht mit Irinotecan präinkubiert. Nach 48-stündiger Kultivierung der Zellen ohne Irinotecan wurden diese mit 10 pfu/Zelle dl520 infiziert und anschließend ohne Irinotecan für weitere 3 Tage inkubiert, Die DNA wurde wie oben beschreiben isoliert.

Anschließend wurden 2µg DNA mit dem Restriktionsenzym Kpn I verdaut und eine Southern Blot Analyse durchgeführt. Als Sonde diente ein mittels PCR hergestellter Abschnitt des adeno-viralen Genoms (Position:22734-24235).

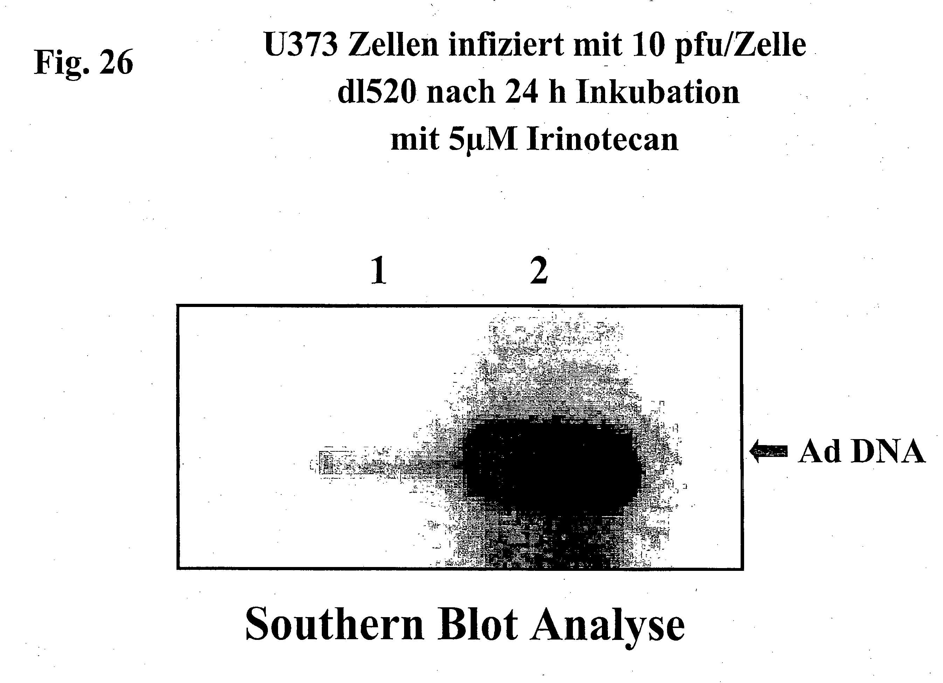

Das Ergebnis ist in Fig. 26 dargestellt. Fig. 26 zeigt, dass nach Inkubation mit Irinotecan die adenovirale Replikation in U 373 Zellen nach Behandlung mit Irinotecan (Bahn 2) gegenüber der unbehandelten Kontrolle, bei der keine Inkubation mit Irinotecan erfolgte (Bahn 1), stark erhöht ist. Die bedeutet, dass unter dem Einfluss von Irinotecan die adenovirale Replikation erhöht ist.

### Beispiel 21: Replikation von Adenovirus in Zellen nach Gabe von Trichostatin A

Um die Wirkung von Trichostatin A auf die adenovirale Replikation zu bestimmen, wurden 10⁶-Tumorzellen U373 in 10 cm² Petrischalen ausplattiert. Nach 24 h erfolgte die Zugabe von 0, 0,25, 0,5 und 0,75µM Trichostatin. Nach wiederum 24 h wurden die Zellen mit 10 pfu/Zelle dl520 infiziert.

Nach 3 Tagen Inkubation im Medium ohne Trichostatin wurde die DNA isoliert. Anschließend wurden 2µg DNA mit dem Restricktionsenzym Kpn I verdaut und eine Southern Blot -Analyse durchgeführt. Als Sonde diente ein mittels PCR hergestellter Abschnitt des adenoviralen Genoms (Position:22734-24235).

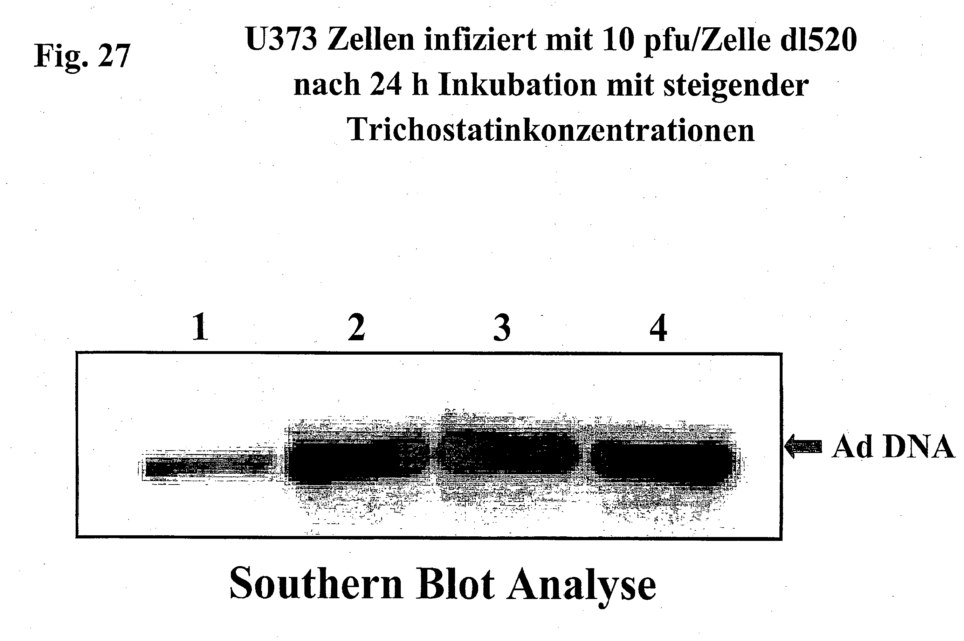

Das Ergebnis ist in Fig. 27 dargestellt. Fig. 27 zeigt, dass nach Inkubation mit steigenden Konzentrationen an Trichostatin A die adenovirale Replikation in U 373 Zellen (Bahnen 2,3 und 4) gegenüber der unbehandelten Kontrolle, bei der keine Inkubation mit Trichostatin A erfolgte (Bahn 1), stark erhöht ist. Die bedeutet, dass unter dem Einfluss von Trichostatin A die adenovirale Replikation erhöht ist.

### Beispiel 22: Beeinflussung der Expression des Coxsackievirus-Adenovirus-Rezeptors (CAR) auf U373 Zellen in Reaktion auf die Gabe von Trichostatin A

200.000 Zellen U373 wurden in 6-Loch-Platten ausplattiert. Nach 24 h wurden die Zellen mit 1 µM Trichostatin A für 24 h kultiviert. Nach wiederum 24 h wurden die Zellen isoliert. Anschließend erfolgte die Analyse der CAR-Expression nach Standardprotokoll mittels Facs-Analyse und dem primären Antikörper anti-CAR Klon RmcB der Firma Upstate und dem sekundären Antikörper Kaninchen-anti-Maus FITC (Firma DAKO).

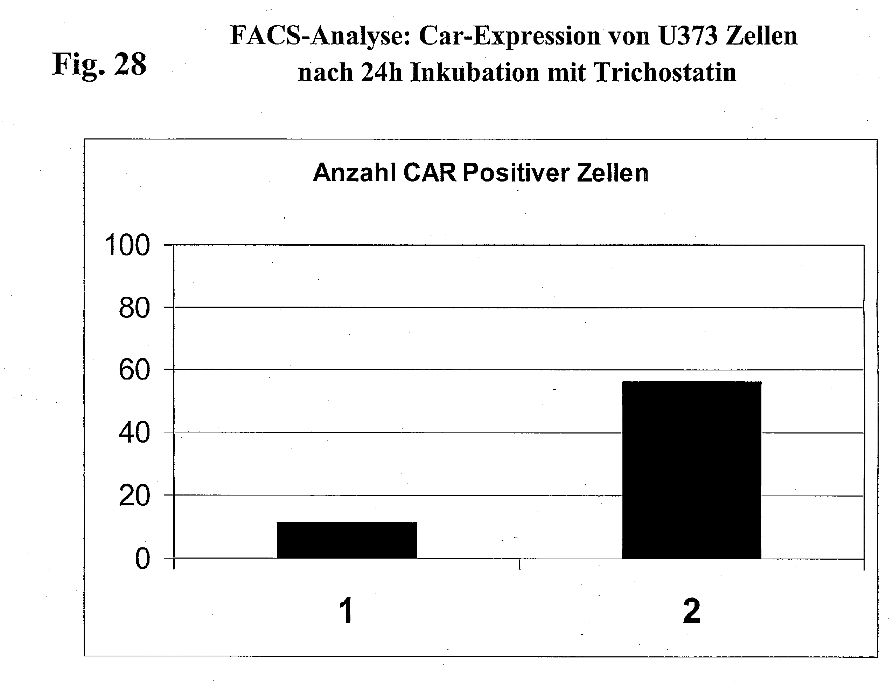

Das Ergebnis ist in Fig. 28 dargestellt. Ohne Trichostatin-Behandlung waren 11,3 % der Zellen CAR-positiv, wohingegen nach Inkubation der Zellen mit 1µM Trichostatin 56, 2 %der Zellen CAR-positiv. Die Zahlen sind % der insgesamt im Test verwendeten Zellen.

Aus Fig. 28 kann somit entnommen werden, dass unter dem Einfluss des Histon-Deacylase-Inhibitors Trichostatin A der für das Binden von Adenoviren wichtige Faktor CAR unter dem Einfluss von Trichostatin A im größeren Maße exprimiert wird bzw. verfügbar ist, was die Effizienz der Transfektion der solchermaßen behandelten Zellen erhöht.

### Beispiel 23: Onkolyse von U373 Zellen durch Adenovirus nach Kombinationsbehandlung der Zellen mit Irinotecan und Trichostatin A.

Es wurden 200.000 U373-Zellen in 6-Loch Platten ausplattiert. Nach 24 h wurden entweder nur 2µM Irinotecan oder nur 1 µM Trichostatin A oder 1 µM Irinotecan + 0.5 µM Trichostatin zum Medium zugegeben. Nach 24 h Inkubation wurden die Zellen mit 10, 20 und 30 pfu/Zelle dl520 infiziert. Nach 3-5 Tagen erfolgte die Auswertung mit Hilfe der Kristallviolettfärbung. Die Versuche wurden in doppelter Ausführung durchgeführt.

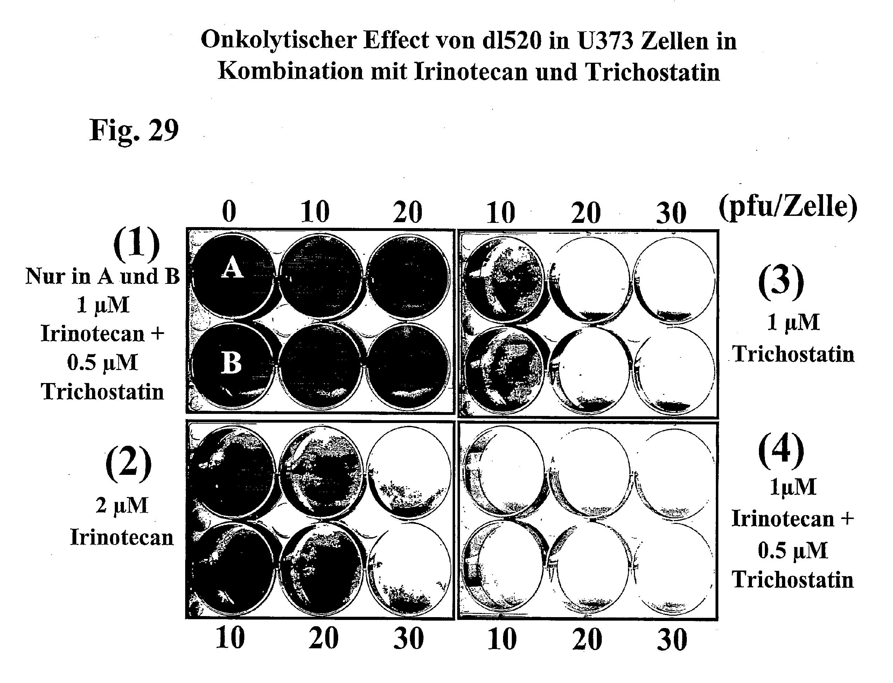

Das Ergebnis ist in Fig. 29 dargestellt. Die sechs in Panel 1 dargestellten Platten zeigen einen vollständigen Zellrasen, der durch die Inkubation mit einer Kombination von Irinotecan und Trichostatin A nicht beeinträchtigt wird, wie durch die Kristallviolettfärbung dargestellt. Die beiden nächsten Doppel-Löcher von Panel 1 zeigen den Zellrasen nach Infektion mit 10 bzw. 20 pfu/Zelle dl520. Auch bei diesen Bedingungen kommt es nicht zu einer Lyse der Zellen, was auf das Ausbleiben einer Replikation von dl520 zurückzuführen ist. Damit wird gezeigt, dass weder dl520 bei 10 und 20 pfu/Zelle noch 1 µM Irinotecan + 0.5 µM Trichostatin A alleine eine Zelllyse induzieren können.

Die weiteren in Fig. 29 dargestellten 6-Loch-Platten 2, 3 und 4, hierin auch als Panele 2, 3 und 4 bezeichnet, wurden grundsätzlich nach folgenden Schema behandelt. Die einzelnen Löcher wurden wie eingangs beschrieben mit U 373 Zellen beimpft und die Zellen darin kultiviert. Die Näpfe wurden, jeweils doppelt mit 10, 20 oder 30 pfu/Zelle dl 520 beimpft, wobei die Unterschiede der insgesamt drei 6-Loch-Platten in der Art der verwendeten Cytostatika bestand. Bei Panel 2 wurden 2µM Irinotecan, bei Panel 3 1 µM Trichostatin A und bei Panel 4 1 µM Irinotecan und 0,5 µM Trichostatin A zu den einzelnen Löchern zugesetzt.

In der 6-Loch Platte 2 (Panel 2) mit 2 µM Irinotecan werden die Zellen mit 30 pfu/Zelle dl520 lysiert. In der 6-Loch Platte 3 (Panel 3) mit 1 µM Trichostatin A werden die Zellen bei 20 und 30 pfu/Zelle dl520 lysiert. In der 6-Loch Platte 4 (Panel 4) mit 1 µM Irenotecan + 0.5 µM Trichostatin A werden die Zellen dagegen bereits bei 10 pfu/Zelle dl520 lysiert.

Die in den Figs. 26 bis 29 in ihrem Ergebnis dargestellten Versuche zeigen, dass die Kombination bestehend aus Irinotecan + Trichostatin A + dl520 eine effektivere Zellyse der Tumorzellen induziert als jede Substanz für sich alleine. Dies wird dadurch erreicht, dass Trichostatin A einerseits die CAR-Expression erhöht und somit die Infizierbarkeit der Zellen merklich verbessert. Andererseits wird durch Irinotecan YB-1 in den Zellkern transloziert und induziert somit eine verbesserte adenovirale Replikation. Zudem dient das zelluläre YB-1 nach Infektion mit dl520 der adenoviralen Replikation und steht für DNA-Reparaturvorgänge nicht mehr zur Verfügung. Je nach Betrachtungsweise führt dies zur verbesserten Wirksamkeit einerseits von dl520 und einer verbesserten Wirksamkeit der Cytostatika andererseits.

## Patentansprüche

1. Adenovirus, der ein E1B55kD-Protein und/oder ein E4orf6-Protein zeitlich vor einem E1A-Protein exprimiert, wobei der Adenovirus in Zellen repliziert, die YB-1 im Kern aufweisen, aber nicht in Zellen, die YB-1 nicht im Kern aufweisen.

2. Adenovirus nach Anspruch 1, **dadurch gekennzeichnet, dass** der Adenovirus eine Kombination aus E1B55kD-Protein und E4orf6-Protein zeitlich vor einem E1A-Protein exprimiert.

3. Adenovirus nach einem der Ansprüche 1 bis 2, **dadurch gekennzeichnet, dass** das E1A-Protein ein E1A12S-Protein ist.

4. Adenovirus nach einem der Ansprüche 1 bis 3, **dadurch gekennzeichnet, dass** der Adenovirus mindestens eine Nukleinsäure umfasst, die für ein Protein codiert, das aus der Gruppe ausgewählt ist, die aus E1B55kD-Protein, E4orf6-Protein und E1A-Proteinen besteht, wobei das mindestens eine Protein unter der Kontrolle eines Promotors steht, der verschieden ist von dem Promotor, der die Expression des Proteins in einem Wildtyp-Adenovirus steuert.

5. Adenovirus nach Anspruch 4, **dadurch gekennzeichnet, dass** das mindestens eine Protein ein E1B55kD-Protein ist.

6. Adenovirus nach einem der Ansprüche 4 bis 5, **dadurch gekennzeichnet, dass** das mindestens eine Protein ein E1A-Protein, bevorzugterweise ein E1A12S-Protein ist.

7. Adenovirus nach einem der Ansprüche 4 bis 6, **dadurch gekennzeichnet, dass** die Expression des E1B55kD-Proteins von einem Promotor gesteuert wird, wobei der Promotor ausgewählt ist aus der Gruppe, die Tumor-spezifische Promotoren, Organ-spezifische Promotoren, Gewebe-spezifische Promotoren, heterologe Promotoren und adenovirale Promotoren umfasst, wobei der adenovirale Promotor verschieden ist von dem E1B-Promotor.

8. Adenovirus nach einem der Ansprüche 4 bis 7, **dadurch gekennzeichnet, dass** die Expression des E4orf6-Proteins von einem Promotor gesteuert wird, wobei der Promotor ausgewählt ist aus der Gruppe, die Tumor-spezifische Promotoren, Organ-spezifische Promotoren, Gewebe-spezifische Promotoren, heterologe Promotoren und adenovirale Promotoren umfasst, wobei der adenovirale Promotor verschieden ist von dem E4-Promotor.

9. Adenovirus nach einem der Ansprüche 7 und 8, wobei der adenovirale Promotor der E1A-Promotor ist.

10. Adenovirus nach einem der Ansprüche 4 bis 9, **dadurch gekennzeichnet, dass** die Expression des E1A-Proteins von einem Promotor gesteuert wird, wobei der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei der adenovirale Promotor verschieden ist von dem E1A-Promotor.

11. Adenovirus nach einem der Ansprüche 7 bis 10, **dadurch gekennzeichnet, dass** der die Expression des E1A-Proteins steuernde Promotor YB-1-gesteuert oder YB-1-regulierbar ist.

12. Adenovirus nach einem der Ansprüche 7 bis 11, **dadurch gekennzeichnet, dass** der die Expression des E1A-Proteins steuernde Promotor der adenovirale E2-late-Promotor ist.

13. Adenovirus nach einem der Ansprüche 1 bis 12, **dadurch gekennzeichnet, dass** das E4orf6-Protein und das E1B55kD-Protein unter der Kontrolle eines gleichen oder eines gemeinsamen Promotors stehen.

14. Adenovirus nach einem der Ansprüche 1 bis 13, **dadurch gekennzeichnet, dass** der Adenovirus das Protein YB-1 durch mindestens ein adenovirales Protein im Zellkern bereitstellt oder die Bereistellung von YB-1 im Zellkern durch mindestens ein adenovirales Protein vermittelt, wobei bevorzugterweise das adenovirale Protein verschieden ist von E1A, wobei das adenovirale Protein ein Komplex aus E4orf6 und E1B55kD ist.

15. Adenovirus nach einem der Ansprüche 1 bis 14, **dadurch gekennzeichnet, dass** der Adenovirus das Protein YB-1 durch mindestens ein adenovirales Protein für die adenovirale Replikation bereitstellt oder die Bereitstellung von YB-1 für die adenovirale Replikation durch mindestens ein adenovirales Protein vermittelt, wobei das adenovirale Protein ein Komplex aus E4orf6 und E1B55kD ist.

16. Adenovirus nach einem der Ansprüche 1 bis 15, **dadurch gekennzeichnet, dass** die Nukleinsäure des Adenovirus zumindest eine funktional inaktive adenovirale Region aufweist, wobei die Region ausgewählt ist aus der Gruppe, die aus der E1-Region, der E3-Region, der E4-Region und Kombinationen davon besteht.

17. Adenovirus nach Anspruch 16, **dadurch gekennzeichnet, dass** die Region die E1-Region ist.

18. Adenovirus nach Anspruch 16 oder 17, **dadurch gekennzeichnet, dass** die Region die E3-Region ist.

19. Adenovirus nach einem der Ansprüche 16 bis 18, **dadurch gekennzeichnet, dass** die Region die E4-Region ist.

20. Adenovirus nach einem der Ansprüche 16 bis 19, **dadurch gekennzeichnet, dass** die Region aus der E1-Region, der E3-Region und der E4-Region besteht.

21. Adenovirus nach einem der Ansprüche 1 bis 20, **dadurch gekennzeichnet, dass** der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E1B55kD-Protein ist.

22. Adenovirus nach Anspruch 21, **dadurch gekennzeichnet, dass** der Promotor verschieden ist von dem E1B-Promotor.

23. Adenovirus nach Anspruch 22, **dadurch gekennzeichnet, dass** der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei der Promotor verschieden ist vom E1B-Promotor.

24. Adenovirus nach einem der Ansprüche 1 bis 23, **dadurch gekennzeichnet, dass** der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E4orf6-Protein ist.

25. Adenovirus nach Anspruch 24, **dadurch gekennzeichnet, dass** der Promotor verschieden ist von dem E4-Promotor.

26. Adenovirus nach Anspruch 25, **dadurch gekennzeichnet, dass** der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei die adenoviralen Promotoren verschieden sind von dem E4-Promotor.

27. Adenovirus nach einem der Ansprüche 21 bis 26, **dadurch gekennzeichnet, dass** der Promotor der E1A-Promotor ist.

28. Adenovirus nach einem der Ansprüche 1 bis 27, **dadurch gekennzeichnet, dass** der Adenovirus mindestens eine Expressionskassette umfasst, wobei die Expressionskassette mindestens einen Promotor und eine für ein adenovirales Protein codierende Nukleinsäure umfasst, wobei das adenovirale Protein ein E1A-Protein, bevorzugterweise ein E1A12S-Protein ist.

29. Adenovirus nach Anspruch 28, **dadurch gekennzeichnet, dass** der Promotor verschieden ist von dem E1A-Promotor.

30. Adenovirus nach Anspruch 29, **dadurch gekennzeichnet, dass** der Promotor ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht.

31. Adenovirus nach einem der Ansprüche 1 bis 30, **dadurch gekennzeichnet, dass** der Adenovirus eine Nukleinsäure umfasst, wobei die Nukleinsäure für YB-1 codiert.

32. Adenovirus nach Anspruch 31, **dadurch gekennzeichnet, dass** die für YB-1 codierende Nukleinsäure unter der Kontrolle eines Promotors steht, wobei der Promotor bevorzugterweise der E2-late-Promotor ist.

33. Adenovirus nach Anspruch 31 oder 32, **dadurch gekennzeichnet, dass** die für YB-1 codierende Nukleinsäure unter der Kontrolle eines Promotors steht, wobei der Promotor YB-1 abhängig ist bzw. YB-1- reguliert ist.

34. Adenovirus nach einem der Ansprüche 28 bis 33, **dadurch gekennzeichnet, dass** die für YB-1 codierende Nukleinsäure ein Teil der Expressionskassette umfassend eine für ein E1A-Protein codierende Nukleinsäure ist, bevorzugterweise eine für ein E1A 12S-Protein codierende Nukleinsäure ist.

35. Adenoviurs nach Anspruch 34, **dadurch gekennzeichnet, dass** die für das E1A-Protein codierende Nukleinsäure von der für das YB-1 codierende Nukleinsäure durch eine IRES-Sequenz getrennt ist.

36. Adenovirus nach einem der Ansprüche 21 bis 35, **dadurch gekennzeichnet, dass** die für das E4orf6-Protein codierende Nukleinsäure und die für das das E1B55kD-Protein codierende Nukleinsäure in einer Expressionskassette enthalten sind, und die beiden codierenden Sequenzen durch eine IRES-Sequenz voneinander getrennt sind.

37. Adenovirus nach Anspruch 36, **dadurch gekennzeichnet, dass** der Promotor der Expressionskassette ausgewählt ist aus der Gruppe, die aus Tumor-spezifischen Promotoren, Organ-spezifischen Promotoren, Gewebe-spezifischen Promotoren, heterologen Promotoren und adenoviralen Promotoren besteht, wobei die adenoviralen Promotoren verschieden sind von dem E4-Promotor und verschieden sind von dem E1B-Promotor, bevorzugterweise verschieden sind von dem Wildtyp-E4-Promotor und verschieden sind von dem Wildtyp-E1B-Promotor.

38. Adenovirus nach einem der Ansprüche 1 bis 37, **dadurch gekennzeichnet, dass** der Adenovirus eine Expressionskassette umfassend einen Promotor und eine Nukleinsäuresequenz umfasst, wobei die Nukleinsäuresequenz ausgewählt ist aus der Gruppe, die aus Aptameren, Ribozymen, Aptazymen, Antisense-Molekülen und siRNAs besteht.

39. Adenovirus nach einem der Ansprüche 1 bis 37, **dadurch gekennzeichnet, dass** der Adenovirus eine Expressionskassette umfassend einen Promotor und eine Nukleinsäuresequenz umfasst, wobei die Nukleinsäuresequenz eine codierende Nukleinsäure ist, wobei die Nukleinsäure für ein Molekül codiert, das ausgewählt ist aus der Gruppe, die aus Peptiden, Polypeptiden, Proteinen, Antikalinen, Antikörpern und Antikörperfragmenten besteht.

40. Adenovirus nach einem der Ansprüche 1 bis 37, **dadurch gekennzeichnet, dass** der Adenovirus eine Expressionskassette umfassend einen Promotor und eine Nukleinsäuresequenz umfasst, wobei die Nukleinsäuresequenz ausgewählt ist aus der Gruppe, die aus apoptoseinduzierende Genen, Prodrug-Genen, Protease-Inhibitoren, Tumorsuppressorgenen, Zytokinen und Angiogenese-Inhbitoren besteht.

41. Adenovirus nach einem der Ansprüche 1 bis 40, **dadurch gekennzeichnet, dass** der Adenovirus replikationsfähig ist in Zellen, die dereguliertes YB-1 aufweisen, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

42. Adenovirus nach einem der Ansprüche 1 bis 41, **dadurch gekennzeichnet, dass** die Zellen YB-1 Zellzyklus-unabhängig YB-1 im Kern aufweisen.

43. Nukleinsäure codierend für einen Adenovirus nach einem der Ansprüche 1 bis 42.

44. Replikationssystem umfassend eine Nukleinsäure nach Anspruch 43 und eine Nukleinsäure eines Helfervirus, wobei die Nukleinsäure des Helfervirus eine oder mehrere der Expressionskassetten des Adenovirus nach einem der Ansprüche 1 bis 42 umfasst.

45. Replikationssystem nach Anspruch 44, **dadurch gekennzeichnet, dass** dem Adenovirus oder der diesen codierenden Nukleinsäure die vom Helfervirus umfasste Expressionskassette fehlt.

46. Vektor umfassend eine Nukleinsäure nach Anspruch 43 und/oder ein Replikationssystem nach einem der Ansprüche 44 bis 45.

47. Vektor nach Anspruch 46, **dadurch gekennzeichnet, dass** der Vektor ein Expressionsvektor ist

48. Zelle umfassend einen Adenovirus nach einem der Ansprüche 1 bis 42 und/oder eine Nukleinsäure nach Anspruch 43 und/oder ein Replikationssystem nach Anspruch 44 oder 45 und/oder einen Vektor nach Anspruch 46 oder 47.

49. Zelle nach Anspruch 48, **dadurch gekennzeichnet, dass** die Zelle eine eukaryontische Zelle, bevorzugterweise eine tierische Zelle, bevorzugtererweise eine Säugtierzelle ist.

50. Zelle nach Anspruch 49, **dadurch gekennzeichnet, dass** die Säugetierzelle eine Zelle ist, die ausgewählt ist aus der Gruppe, die aus Zellen von Mäusen, Ratten, Meerschweinchen, Schweinen, Schafen, Ziegen, Rindern, Pferden, Hunden, Katzen und Menschen besteht.

51. Nicht-menschlicher Organismus, bevorzugterweise ein Säugetierorganismus umfassend einen Adenovirus nach einem der Ansprüche 1 bis 42, eine Nukleinsäure nach Anspruch 43, ein Replikationssystem nach Anspruch 44 oder 45, einen Vektor nach einem der Ansprüche 46 oder 47 oder eine Zelle nach einem der Ansprüche 48 bis 50, wobei bevorzugterweise der Organismus ausgewählt ist aus der Gruppe, die aus Mäusen, Ratten, Meerschweinchen, Schweinen, Schafen, Ziegen, Rindern, Pferden, Hunden und Katzen besteht.

52. Verwendung eines Adenovirus nach einem der Ansprüche 1 bis 42, einer Nukleinsäure nach Anspruch 43, eines Replikationssystem nach Anspruch 44 oder 45, eines Vektor nach einem der Ansprüche 46 oder 47 oder einer Zelle nach einem der Ansprüche 48 bis 50 zur in vitro Replikation von Adenovirus.

53. Verwendung eines Adenovirus nach einem der Ansprüche 1 bis 42, einer Nukleinsäure nach Anspruch 43, eines Replikationssystem nach Anspruch 44 oder 45, eines Vektor nach einem der Ansprüche 46 oder 47 oder einer Zelle nach einem der Ansprüche 48 bis 50 zur in vitro Herstellung von Adenovirus.

54. Verwendung eines Adenovirus nach einem der Ansprüche 1 bis 42, einer Nukleinsäure nach Anspruch 43, eines Replikationssystem nach Anspruch 44 oder 45, eines Vektor nach einem der Ansprüche 46 oder 47 oder einer Zelle nach einem der Ansprüche 48 bis 50 zur Herstellung eines Medikamentes.

55. Verwendung nach einem der Ansprüche 53 bis 54, **dadurch gekennzeichnet, dass** die Zelle, in der der Adenovirus repliziert, YB-1 im Kern aufweist, bevorzugterweise YB-1 Zellzyklus-unabhängig im Kern aufweist.

56. Verwendung nach einem der Ansprüche 53 bis 54, **dadurch gekennzeichnet, dass** die Zelle, in der der Adenovirus repliziert, dereguliertes YB-1 aufweist, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

57. Verwendung nach Anspruch 54, **dadurch gekennzeichnet, dass** das Medikament für die Behandlung von Tumorerkrankungen ist.

58. Verwendung nach Anspruch 57, **dadurch gekennzeichnet, dass** die Tumorerkrankung ausgewählt ist aus der Gruppe, die aus malignen Erkrankungen, Krebs, Krebserkrankungen und Tumoren besteht.

59. Verwendung nach Anspruch 58, **dadurch gekennzeichnet, dass** die Tumoren ausgewählt sind aus der Gruppe, die aus soliden, nichtsoliden, malignen und benignen Tumoren besteht.

60. Verwendung nach Anspruch einem der Ansprüche 57 bis 59, **dadurch gekennzeichnet, dass** zumindest ein Teil der den Tumor ausbildenden Zellen YB-1 im Kern aufweist, bevorzugterweise YB-1 Zellzyklus-unabhängig im Kern aufweist.

61. Verwendung nach Anspruch einem der Ansprüche 57 bis 60, **dadurch gekennzeichnet, dass** zumindest ein Teil der den Tumor ausbildenden Zellen dereguliertes YB-1 aufweist, wobei dereguliertes YB-1 ein solches ist, das im Cytoplasma von Tumorzellen in einer Form vorhanden ist, die quantitativ und/oder qualitativ verschieden ist von der in Nicht-Tumorzellen.

62. Verwendung nach einem der Ansprüche 57 bis 61, **dadurch gekennzeichnet, dass** zumindest ein Teil der den Tumor ausbildenden Zellen Rb-positiv oder Rb-negativ ist.

63. Verwendung nach einem der Ansprüche 57 bis 61, **dadurch gekennzeichnet, dass** zumindest ein Teil der den Tumor ausbildenden Zellen eine Resistenz gegen Antitumormittel eine Mehrfachresistenz gegen pharmakologische Wirkstoffe und/oder eine durch Bestrahlung bedingte Resistenz aufweisen.

64. Verwendung nach Anspruch 63, **dadurch gekennzeichnet, dass** die Resistenz gegen gegen Zytostatika gerichtet ist.

65. Verwendung nach einem der Ansprüche 57 bis 64, **dadurch gekennzeichnet, dass** der Patient für den das Medikament bestimmt ist, eine Mehrzahl von Zellen aufweist, wobei die Zellen solche Zellen sind, wie in einem der Ansprüche 60 bis 64 beschrieben.

66. Verwendung nach einem der Ansprüche 57 bis 65, **dadurch gekennzeichnet, dass** das Medikament mindestens ein weiteres pharmazeutisch aktiven Mittels umfasst.

67. Verwendung nach einem der Ansprüche 57 bis 65, **dadurch gekennzeichnet, dass** das Medikament zusammen mit einem weiteren pharmazeutisch aktiven Mittel verabreicht wird oder werden soll.

68. Verwendung nach Anspruch 66 oder 67, **dadurch gekennzeichnet, dass** das weitere pharmazeutische Mittel ausgewählt ist aus der Gruppe, die aus Zytokinen, Metalloproteinase-Inhibitoren, Angiogenese-Inhibitoren, Zytostatika, Tyrosinkinase-Inhibitoren, Zellzyklus-Inhibitoren, Proteosomen-Inhibitoren, Inhibitoren der Signaltransduktionskaskade, Proteinkinasen und rekombinanten Antikörpern besteht.

69. Verwendung nach einem der Ansprüche 57 bis 65, **dadurch gekennzeichnet, dass** das Medikament vor, während oder nach einer Bestrahlung verabreicht wird.

70. Verwendung nach Anspruch 69, **dadurch gekennzeichnet, dass** die Bestrahlung zum Zwecke der Behandlung eines Tumors verabreicht wird.

71. Verwendung nach einem der Ansprüche 57 bis 70, **dadurch gekennzeichnet, dass** die Zelle oder der zu behandelnde Organismus einer Maßnahme unterzogen wird, wobei die Maßnahme aus der Gruppe ausgewählt ist, die aus Bestrahlung, Gabe von Zytostatika und Hyperthermie besteht.

72. Verwendung nach einem der Ansprüche 57 bis 71, **dadurch gekennzeichnet, dass** die Maßnahme lokal oder systemisch angewandt wird.

73. Verwendung nach einem der Ansprüche 69 bis 72, **dadurch gekennzeichnet, dass** die Bestrahlung mit energiereicher Strahlung erfolgt, bevorzugterweise mit einer Strahlung, wie sie bei der Behandlung von Tumorerkrankungen verwendet wird.

74. Verwendung nach Anspruch 57, **dadurch gekennzeichnet, dass** die Tumorerkrankung ausgewählt ist aus der Gruppe, die aus Brusttumoren, Knochentumoren, Magentumoren, Darmtumoren, Gallenblasentumoren, Bauspeicheldrüsentumoren, Lebertumoren, Nierentumoren, Gehirntumoren, Eierstocktumoren, Haut- und Hautanhangsorgantumoren, Kopf-/Nackentumoren, Gebärmuttertumoren, Synovialtumoren, Kehlkopftumoren, Speiseröhrentumoren, Zungentumoren und Prostatatumoren, bevorzugterweise eine der vorstehenden Tumorerkrankungen, die wie in einem der vorangehenden Ansprüche beschrieben ausgeprägt ist.

75. Verwendung nach Anspruch 57, **dadurch gekennzeichnet, dass** der tumor-spezifische Promotor ein solcher Promotor ist, der spezifisch ist für den Tumor, für den das Medikament verwendet wird.

76. Pharmazeutische Zusammensetzung umfassend einen Adenovirus nach einem der Ansprüche 1 bis 42, eine Nukleinsäure nach Anspruch 43, ein Replikationssystem nach Anspruch 44 oder 45, einen Vektor nach einem der Ansprüche 46 oder 47 oder eine Zelle nach einem der Ansprüche 48 bis 50, und optional ein pharmazeutisch geeignetes Trägermittel.

77. Verwendung nach einem der Ansprüche 54 bis 75, **dadurch gekennzeichnet, dass** das Medikament eine Kombination von mindestens zwei Mittel umfasst, wobei ein jedes Mittel einzeln und unabhängig aus der Gruppe ausgewählt ist, die aus Cytostatika besteht.

78. Verwendung nach Anspruch 77, **dadurch gekennzeichnet, dass** mindestens zwei der Mittel an unterschiedlichen Zielmolekülen angreifen.

79. Verwendung nach Anspruch 78, **dadurch gekennzeichnet, dass** mindestens zwei der Mittel über einen unterschiedlichen Wirkmechanismus aktiv sind.

80. Verwendung nach einem der Ansprüche 77 bis 79, **dadurch gekennzeichnet, dass** mindestens ein Mittel die Infizierbarkeit einer Zelle erhöht, in der das Virus repliziert.

81. Verwendung nach einem der Ansprüche 77 bis 80, **dadurch gekennzeichnet, dass** mindestens ein Mittel die Verfügbarkeit einer Komponente der Zelle beeinflusst, wobei die Komponente die Aufnahme des Virus vermittelt.

82. Verwendung nach einem der Ansprüche 77 bis 81, **dadurch gekennzeichnet, dass** mindestens ein Mittel den Transport von YB-1 in den Zellkern vermittelt, bevorzugterweise erhöht.

83. Verwendung nach einem der Ansprüche 77 bis 82, **dadurch gekennzeichnet, dass** mindestens ein Mittel ein Histon-Deacylase-Inhibitor ist.

84. Verwendung nach Anspruch 83, **dadurch gekennzeichnet, dass** der Histon-Deacylase-Inhibitor aus der Gruppe ausgewählt ist, die aus Trichostatin A, FR 901228, MS-27-275, NVP-LAQ824, PXD101 Apicidin und Scriptaid besteht.

85. Verwendung nach einem der Ansprüche 77 bis 83, **dadurch gekennzeichnet, dass** mindestens ein Mittel aus der Gruppe ausgewählt ist, die aus Trichostatin A, FR 901228, MS-27-275, NVP-LAQ824, PXD101, Apicidin und Scriptaid besteht.

86. Verwendung nach einem der Ansprüche 77 bis 85, **dadurch gekennzeichnet, dass** mindestens ein Mittel ein Topoisomerase-Inhibitor ist.

87. Verwendung nach Anspruch 86, **dadurch gekennzeichnet, dass** der Topoisomerase-Inhibitor aus der Gruppe ausgewählt ist, die aus Camptothecin, SN-38, Topotecan, DX-895If, Irinotecan 9-aminocamptothecin, 9-nitrocamptothecin, Etoposid und Daunorubicin besteht.

88. Verwendung nach einem der Ansprüche 84 bis 87, **dadurch gekennzeichnet, dass** das Mittel aus Trichostatin A und Irinotecan besteht.

89. Verwendung nach Anspruch 87, **dadurch gekennzeichnet, dass** mindestens eine Einheitsdose des Virus von mindestens einer Einheitsdose von einem oder den mindestens zwei Mitteln getrennt ist.

90. Kit umfassend einen Virus nach einem der Ansprüche 1 bis 42, und mindestens zwei Mittel, wobei ein jedes Mittel einzeln und unabhängig aus der Gruppe ausgewählt ist, die aus Cytostatika besteht.

## Claims

1. An adenovirus expressing an E1B55kD protein and/or an E4orf6 protein prior to an E1A-protein, wherein the adenovirus is replicating in cells which have YB-1 in the nucleus, but no in cells which do not have YB-1 in the nucleus.

2. The adenovirus according to claim 1, **characterised in that** the adenovirus is expressing a combination of an E1B55kD protein and an E4orf6 protein prior to an E1A protein.

3. The adenovirus according to any of claims 1 to 2, **characterised in that** the E1A protein is an E1A12S protein.

4. The adenovirus according to any of claims 1 to 3, **characterised in that** the adenovirus comprises at least one nucleic acid coding for a protein which is selected from the group consisting of E1B55kD protein, E4orf6 protein and E1A proteins, wherein the at least one protein is under the control of a promoter which is different from the promoter controlling the expression of the protein in a wild type adenovirus.

5. The adenovirus according to claim 4, **characterised in that** the at least one protein is an E1B55kD protein.

6. The adenovirus according to any of claims 4 to 5, **characterised in that** the at least one protein is an E1A protein, preferably an E1A12S protein.

7. The adenovirus according to any of claims 4 to 6, **characterised in that** the expression of the E1B55kD protein is controlled by a promoter, wherein the promoter is selected from the group comprising tumor-specific promoters, organ-specific promoters, tissue-specific promoters, heterologous promoters and adenoviral promoters, wherein the adenoviral promoter is different from the E1B promoter.

8. The adenovirus according to any of claims 4 to 7, **characterised in that** the expression of the E4orf6 protein is controlled by a promoter, wherein the promoter is selected from the group comprising tumor-specific promoters, organ-specific promoters, tissue-specific promoters, heterologous promoters and adenoviral promoters, wherein the adenoviral promoter is different from the E4 promoter.

9. The adenovirus according to claims 7 and 8, wherein the adenoviral promoter is the E1A promoter.

10. The adenovirus according to any of claims 4 to 9, **characterised in that** the expression of the E1A protein is controlled by a promoter, wherein the promoter is selected from the group consisting of tumor-specific promoters, organ-specific promoters, tissue-specific promoters, heterologous promoters and adenoviral promoters, wherein the adenoviral promoter is different from the E1A promoter.

11. The adenovirus according to any of claims 7 to 10, **characterised in that** the promoter controlling the expression of the E1A protein is YB-1 controlled or can be regulated by YB-1.

12. The adenovirus according to any of claims 7 to 11, **characterised in that** the promoter controlling the expression of the E1A protein is the adenoviral E2 late promoter.

13. The adenovirus according to any of claims 1 to 12, **characterized in that** the E4orf6 protein and the E1B55kD protein under the control of the same or a common promoter.

14. The adenovirus according to any of claims 1 to 13, **characterized in that** the adenovirus provides the protein YB-1 in the nucleus through at least one adenoviral protein or mediates the provision of YB-1 in the nucleus through at least one adenoviral protein, wherein preferably the adenoviral protein is different from E1A, wherein the adenoviral protein is a complex of E4orf6 and E1B55kD.

15. The adenovirus according to any of claims 1 to 14, **characterized in that** the adenovirus provides the protein YB-1 for adenoviral replication through at least one adenoviral protein or mediates the provision of YB-1 for adenoviral replication through at least one adenoviral protein, wherein the adenoviral protein is a complex of E4orf6 and E1B55kD.

16. The adenovirus according to any of claims 1 to 15, **characterised in that** the nucleic acid of the adenovirus comprises at least one functionally inactive adenoviral region, wherein the region is selected from the group consisting of the E1 region, the E3 region, the E4 region and combinations thereof.

17. The adenovirus according to claim 16, **characterised in that** the region is the E1 region.

18. The adenovirus according to claim 16 or 17, **characterised in that** the region is the E3 region.

19. The adenovirus according to any of claims 16 to 18, **characterised in that** the region is the E4 region.

20. The adenovirus according to any of claims 16 to 19, **characterised in that** the region consists of the E1 region, the E3 region and the E4 region.

21. The adenovirus according to any of claims 1 to 20, **characterised in that** the adenovirus comprises at least one expression cassette, wherein the expression cassette comprises at least one promoter and a nucleic acid coding for an adenoviral protein, wherein the adenoviral protein is an E1B55kD protein.

22. The adenovirus according to claim 21, **characterised in that** the promoter is different from the E1B promoter.

23. The adenovirus according to claim 22, **characterised in that** the promoter is selected from the group consisting of tumor-specific promoters, organ-specific promoters, tissue-specific promoters, heterologous promoters and adenoviral promoters, wherein the promoter is different from the E1B promoter.

24. The adenovirus according to any of claims 1 to 23, **characterised in that** the adenovirus comprises at least one expression cassette, wherein the expression cassette comprises at least one promoter and a nucleic acid coding for an adenoviral protein, wherein the adenoviral protein is an E4orf6 protein.

25. The adenovirus according to claim 24, **characterised in that** the promoter is different from the E4 promoter.

26. The adenovirus according to claim 25, **characterised in that** the promoter is selected from the group consisting of tumor-specific promoters, organ-specific promoters, tissue-specific promoters, heterologous promoters and adenoviral promoters, wherein the adenoviral promoters are different from the E4 promoter.

27. The adenovirus according to any of claims 21 to 26, **characterised in that** the promoter is the E1A promoter.

28. The adenovirus according to any of claims 1 to 27, **characterised in that** the adenovirus comprises at least one expression cassette, wherein the expression cassette comprises at least one promoter and a nucleic acid coding for an adenoviral protein, wherein the adenoviral protein is an E1A protein, preferably an E1A12S protein.

29. The adenovirus according to claim 28, **characterised in that** the promoter is different from the E1A promoter.

30. The adenovirus according to claim 29, **characterised in that** the promoter is selected from the group consisting of tumor-specific promoters, organ-specific promoters, tissue-specific promoters, heterologous promoters and adenoviral promoters.

31. The adenovirus according to any of claims 1 to 30, **characterised in that** the adenovirus comprises a nucleic acid, wherein the nucleic acid codes for YB-1.

32. The adenovirus according to claim 31, **characterised in that** the nucleic acid coding for YB-1 is under the control of a promoter, wherein the promoter is preferably the E2 late promoter.

33. The adenovirus according to any of claims 31 to 32, **characterised in that** the nucleic acid coding for YB-1 is under the control of a promoter, wherein the promoter is YB-1 dependent and YB-1 controlled, respectively.

34. The adenovirus according to any of claims 28 to 33, **characterised in that** the nucleic acid coding for YB-1 is part of the expression cassette comprising a nucleic acid coding for an E1A protein, preferably a nucleic acid coding for an E1A12S protein.

35. The adenovirus according to claim 34, **characterised in that** the nucleic acid coding for the E1A protein is separated from the nucleic acid coding for YB-1 through an IRES sequence.

36. The adenovirus according to any of claims 21 to 35, **characterised in that** the nucleic acid coding for the E4orf6 protein, and the nucleic acid coding for the E1B55kD protein, are contained in an expression cassette, wherein the two coding sequences are separated through an IRES sequence.

37. The adenovirus according to claim 36, **characterised in that** the promoter of the expression cassette is selected from the group consisting of tumor-specific promoters, organ-specific promoters, tissue-specific promoters, heterologous promoters and adenoviral promoters, wherein the adenoviral promoters are different from the E4 promoter and different from the E1B promoter, preferably different from the wild type E4 promoter and different from the wild type E1B promoter.

38. The adenovirus according to any of claims 1 to 37, **characterised in that** the adenovirus comprises an expression cassette comprising a promoter and a nucleic acid sequence, wherein the nucleic acid sequence is selected from the group consisting of aptamers, ribozymes, aptazymes, antisense molecules and siRNA.

39. The adenovirus according to any of claims 1 to 37, **characterised in that** the adenovirus comprises an expression cassette comprising a promoter and a nucleic acid sequence, wherein the nucleic acid sequence is a coding nucleic acid, wherein the nucleic acid codes for a molecule which is selected from the group consisting of peptides, polypeptides, proteins, anticalines, antibodies and antibody fragments.

40. The adenovirus according to any of claims 1 to 37, **characterised in that** the adenovirus comprises an expression cassette, wherein the expression cassette comprises a promoter and a nucleic acid sequence, wherein the nucleic acid sequence is selected from the group consisting of apoptosis inducing genes, prodrug genes, protease inhibitors, tumor suppressor genes, cytokines and angiogenesis inhibitors.

41. The adenovirus according to any of claims 1 to 40, **characterised in that** the adenovirus is capable of replicating in cells comprising deregulated YB-1, wherein deregulated YB-1 is one which is present in the cytoplasma of tumor cells in a form which is quantitatively or qualitatively different from the one in a non-tumor cell.

42. The adenovirus according to any of claims 1 to 41, **characterised in that** the cells contain YB-1 in the nucleus independent of the cell cycle.

43. A nucleic acid coding for an adenovirus according to any of claims 1 to 42.

44. A replication system comprising a nucleic acid according to claim 43 and a nucleic acid of a helper virus, wherein the nucleic acid of the helper virus comprises one or more of the expression cassettes of the adenovirus according to any of claims 1 to 42.

45. The replication system according to claim 44, **characterised in that** the adenovirus or the nucleic acid coding therefor is lacking the expression cassette comprised by the helper virus.

46. A vector comprising a nucleic acid according to claim 43 and/or a replication system according to any of claims 44 to 45.

47. The vector according to claim 46, **characterised in that** the vector is an expression vector.

48. A cell comprising an adenovirus according to any of claims 1 to 42 and/or a nucleic acid according to claim 43 and/or a replication system according to claim 44 or 45 and/or a vector according to claim 46 or 47.

49. The cell according to claim 48, **characterised in that** the cell is a eukaryotic cell, preferably an animal cell, more preferably a mammalian cell.

50. The cell according to claim 49, **characterised in that** the mammalian cell is a cell selected from the group consisting of cells of mice, rats, guinea pigs, pigs, sheep, goats, cattle, horses, dogs, cats and human beings.

51. A non-human organism, preferably a mammal organism, comprising an adenovirus according to any of claims 1 to 42, a nucleic acid according to claim 43, a replication system according to claim 44 or 45, a vector according to any of claims 46 or 47 or a cell according to any of claims 48 to 50, wherein the organism is preferably selected from the group consisting of mice, rats, guinea pigs, pigs, sheep, goats, cattle, horses, dogs and cats.

52. Use of an adenovirus according to any of claims 1 to 42, a nucleic acid according to claim 43, a replication system according to claim 44 or 45, a vector according to any of claims 46 or 47, or a cell according to any of claims 48 to 50, for replication of an adenovirus, preferably for in vitro replication of an adenovirus.

53. Use of an adenovirus according to any of claims 1 to 42, a nucleic acid according to claim 43, a replication system according to claim 44 or 45, a vector according to any of claims 46 or 47, or a cell according to any of claims 48 to 50 for in vitro manufacture of an adenovirus.

54. Use of an adenovirus according to any of claims 1 to 42, a nucleic acid according to claim 43, a replication system according to claim 44 or 45, a vector according to any of claims 46 or 47, or a cell according to any of claims 48 to 50 for the manufacture of a medicament.

55. Use according to any of claims 53 to 54, **characterised in that** the cell in which the adenovirus replicates, has YB-1 in its nucleus, preferably has YB-1 in its nucleus independent of the cell cycle.

56. Use according to any of claims 53 to 54, **characterised in that** the cell in which the adenovirus replicates, comprises deregulated YB-1, wherein deregulated YB-1 is one which is present in the cytoplasma of tumor cells in a form which is quantitatively or qualitatively different from the one in a non-tumor cell.

57. Use according to claim 54, **characterised in that** the medicament is for the treatment of tumor diseases.

58. Use according to claim 57, **characterised in that** the tumor disease is selected from the group consisting of malignant diseases, cancer, cancer diseases and tumors.

59. Use according to claim 58, **characterised in that** the tumors are selected from the group comprising solid, non-solid, malignant and benign tumors.

60. Use according to any of claims 57 to 59, **characterised in that** at least one part of the tumor forming cells have YB-1 in the nucleus, preferably have YB-1 in the nucleus independent of the cell cycle.

61. Use according to any of claims 57 to 60, **characterised in that** at least a part of the cells forming the tumor comprises deregulated YB-1, wherein deregulated YB-1 is one which is present in the cytoplasma of tumor cells in a form which is quantitatively or qualitatively different from the one in a non-tumor cell.

62. Use according to any of claims 57 to 61, **characterised in that** at least a part of the cells forming the tumor are Rb positive or Rb negative.

63. Use according to any of claims 57 to 61, **characterised in that** at least a part of the cells forming the tumor have a resistance against anti-tumor agents, a multiple resistance against pharmaceutically active agents and/or a resistance caused by irradiation.

64. Use according to claim 63, **characterised in that** the resistance is against cytostatics.

65. Use according to any of claims 57 to 64, **characterised in that** the patient for whom the medicament is intended, comprises a plurality of cells, wherein the cells are cells as described in any of claims 60 to 64.

66. Use according to any of claims 57 to 65, **characterised in that** the medicament comprises at least one further pharmaceutically active agent.

67. Use according to any of claims 57 to 65, **characterised in that** the medicament is administered together with a further pharmaceutically active agent or is intended therefor.

68. Use according to any of claims 66 or 67, **characterised in that** the further pharmaceutically active agent is selected from the group consisting of cytokines, metalloproteinase inhibitors, angiogenesis inhibitors, cytostatics, tyrosine kinase inhibitors, cell cycle inhibitors, proteosome inhibitors, inhibitors of the signal transduction cascade, protein kinases and recombinant antibodies.

69. Use according to any of claims 57 to 65, **characterised in that** the medicament is administered prior, during or after irradiation.

70. Use according to claim 69, **characterised in that** the radiation is administered for the purpose of treating a tumor.

71. Use according to any of claims 57 to 70, **characterised in that** the cell or the organism to be treated is subject to a measure, wherein the measure is selected from the group consisting of irradiation, administration of cytostatics and hyperthermia.

72. Use according to any of claims 57 to 71, **characterised in that** the measure is applied locally or systemically.

73. Use according to any of claims 69 to 72, **characterised in that** the irradiation uses high-energy radiation, preferably uses any irradiation as used in the treatment of tumor diseases.

74. Use according to claim 57, **characterised in that** the tumor disease is selected from the group consisting of breast tumors, bone tumors, gastric tumors, intestinal tumors, gall-bladder tumors, pancreas tumors, liver tumors, kidney tumors, brain tumors, ovarian tumors, skin tumors, tumors of cutaneous appendages, head and neck cancer, uterine tumors, synovial tumors, laryngeal tumors, oesophageal tumors, lingual tumors and prostate tumors, preferably one of the preceding tumor diseases having the characteristics as described in any of the preceding claims.

75. Use according to claim 57, **characterized in that** the tumor-specific promoter is a promoter which is specific for the tumor for which the medicament is used.

76. A pharmaceutical composition comprising an adenovirus according to any of claims 1 to 42, a nucleic acid according to claim 43, a replication system according to claim 44 or 45, a vector according to any of claims 46 or 47, or a cell according to any of claims 48 to 50 and optionally a pharmaceutically acceptable carrier.

77. Use according to any of claims 54 to 75, **characterised in that** the medicament comprises a combination of at least two agents, wherein each agent is individually and independently selected from the group comprising cytostatics.

78. Use according to claim 77, **characterized in that** at least two of the agents address different target molecules.

79. Use according to claim 79, **characterized in that** at least two of the agents are effective by different modes of action.

80. Use according to any of claim 77 to 79, **characterized in that** at least one agent increases the abilitiy of a cell to be infected, wherein the virus replicates in said cell.

81. Use according to any of claims 77 to 80, **characterized in that** at least one agent influences the availability of a component of the cell, wherein the component mediates the uptake of the virus.

82. Use according to any of claims 77 to 81, **characterized in that** at least one agent mediates the transport of YB-1 into the nucleus, preferably increases said transport.

83. Use according to any of claims 77 to 82, **characterized in that** at least one agent is a histone deacylase inhibitor.

84. Use according to claim 83, **characterized in that** the histone deacylase inhibitor is selected from the group consisting og Trichostatin A, FR 901228, MS-27-275, NVP-LAQ824, PXD101 Apicidin and Scriptaid.

85. Use according to any of claims 77 to 83, **characterized in that** at least one agent is selected from the group consisting of Trichostatin A, FR 901228, MS-27-275, NVP-LAQ824, PXD101, Apicidin and Scriptaid.

86. Use according to any of claims 77 to 85, **characterized in that** at least one agent is a topoisomerase inhibitor.

87. Use according to claim 86, **characterized in that** the topoisomerase inhibitor is selected from the group consisting of Camptothecin, SN-38, Topotecan, DX-895If, Irinotecan, 9-aminocamptothecin, 9-nitrocamptothecin, Etoposid and Daunorubicin.

88. Use according to any of claims 84 to 87, **characterized in that** the agent comprises Trichostatin A and Irinotecan.

89. Use according to claim 87, **characterized in that** at least one unit dose of the virus is separated from at least one unit dose of one of or the at least two agents.

90. A kit comprising a virus according to any of claims 1 to 42, and at least two agents, wherein any agent is individually and independently selected from the group consisting of cytostatics.

## Revendications

1. Adénovirus qui exprime en ordre chronologique une protéine E1B55kD et/ou une protéine E4orf6, avant une protéine E1A, l'adénovirus se répliquant dans des cellules qui présentent YB-1 dans le noyau, mais non pas dans les cellules ne présentant pas de YB-1 dans le noyau.

2. Adénovirus selon la revendication 1, **caractérisé en ce que** l'adénovirus exprime, en ordre chronologique, une combinaison d'une protéine E1B55kD et d'une protéine E4orf6 dans le temps avant une protéine E1A.

3. Adénovirus selon l'une des revendications 1 à 2, **caractérisé en ce que** la protéine E1A est une protéine E1A12S.

4. Adénovirus selon l'une des revendications 1 à 3, **caractérisé en ce que** l'adénovirus comprend au moins un acide nucléique qui code pour une protéine qui est choisie dans le groupe consistant en une protéine E1B55kD, une protéine E4orf6 et des protéines E1A, la ou les protéines se trouvant sous le contrôle d'un promoteur qui est différent du promoteur qui dirige l'expression de la protéine dans un adénovirus de type sauvage.

5. Adénovirus selon la revendication 4, **caractérisé en ce que** ladite au moins une protéine est une protéine E1B55kD.

6. Adénovirus selon l'une des revendications 4 à 5, **caractérisé en ce que** ladite au moins une protéine est une protéine E1A, de préférence une protéine E1A12S.

7. Adénovirus selon l'une des revendications 4 à 6, **caractérisé en ce que** l'expression de la protéine E1B55kD est dirigée par un promoteur, le promoteur étant choisi dans le groupe qui comprend les promoteurs spécifiques de tumeur, les promoteurs spécifiques d'organe, les promoteurs spécifiques de tissu, les promoteurs hétérologues et les promoteurs adénoviraux, le promoteur adénoviral étant différent du promoteur E1B.

8. Adénovirus selon l'une des revendications 4 à 7, **caractérisé en ce que** l'expression de la protéine E4orf6 est dirigée par un promoteur, le promoteur étant choisi dans le groupe comprenant les promoteurs spécifiques de tumeur, les promoteurs spécifiques d'organe, les promoteurs spécifiques de tissu, les promoteurs hétérologues et les promoteurs adénoviraux, le promoteur adénoviral étant différent du promoteur E4.

9. Adénovirus selon l'une des revendications 7 et 8, le promoteur adénoviral étant le promoteur E1A.

10. Adénovirus selon l'une des revendications 4 à 9, **caractérisé en ce que** l'expression de la protéine E1A est dirigée par un promoteur, le promoteur étant choisi dans le groupe consistant en les promoteurs spécifiques de tumeur, les promoteurs spécifiques d'organe, les promoteurs spécifiques de tissu, les promoteurs hétérologues et les promoteurs adénoviraux, le promoteur adénoviral étant différent du promoteur E1A.

11. Adénovirus selon l'une des revendications 7 à 10, **caractérisé en ce que** le promoteur qui dirige l'expression de la protéine E1A est dirigé par YB-1 ou peut être régulé par YB-1.

12. Adénovirus selon l'une des revendications 7 à 11, **caractérisé en ce que** le promoteur qui dirige l'expression de la protéine E1A est le promoteur adénoviral tardif E2.

13. Adénovirus selon l'une des revendications 1 à 12, **caractérisé en ce que** la protéine E4orf6 et la protéine E1B55kD sont sous le contrôle d'un même promoteur ou d'un promoteur commun.

14. Adénovirus selon l'une des revendications 1 à 13, **caractérisé en ce que** l'adénovirus fournit la protéine YB-1 à travers au moins une protéine adénovirale dans le noyau cellulaire, ou que la fourniture de YB-1 dans le noyau cellulaire est réalisée à travers au moins une protéine adénovirale, la protéine adénovirale étant de préférence différente de E1A, la protéine adénovirale étant un complexe d'E4orf6 et d'E1B55kD.

15. Adénovirus selon l'une des revendications 1 à 14, **caractérisé en ce que** l'adénovirus fournit la protéine YB-1 à travers au moins une protéine adénovirale pour la réplication adénovirale, ou réalise la fourniture de YB-1 pour la réplication adénovirale à travers au moins une protéine adénovirale, la protéine adénovirale étant un complexe d'E4orf6 et d'E1B55kD.

16. Adénovirus selon l'une des revendications 1 à 15, **caractérisé en ce que** l'acide nucléique de l'adénovirus présente au moins une région adénovirale fonctionnellement inactive, la région étant choisie dans le groupe qui consiste en la région E1, la région E3, la région E4 et des combinaisons de celles-ci.

17. Adénovirus selon la revendication 16, **caractérisé en ce que** la région est la région E1.

18. Adénovirus selon la revendication 16 ou 17, **caractérisé en ce que** la région est la région E3.

19. Adénovirus selon l'une des revendications 16 à 18, **caractérisé en ce que** la région est la région E4.

20. Adénovirus selon l'une des revendications 16 à 19, **caractérisé en ce que** la région consiste en la région E1, la région E3 et la région E4.

21. Adénovirus selon l'une des revendications 1 à 20, **caractérisé en ce que** l'adénovirus comprend au moins une cassette d'expression, la cassette d'expression comprenant au moins un promoteur et un acide nucléique codant pour une protéine adénovirale, la protéine adénovirale étant une protéine E1B55kD.

22. Adénovirus selon la revendication 21, **caractérisé en ce que** le promoteur est différent du promoteur E1B.

23. Adénovirus selon la revendication 22, **caractérisé en ce que** le promoteur est choisi dans le groupe consistant en les promoteurs spécifiques de tumeur, les promoteurs spécifiques d'organe, les promoteurs spécifiques de tissu, les promoteurs hétérologues et les promoteurs adénoviraux, le promoteur étant différent du promoteur E1B.

24. Adénovirus selon l'une des revendications 1 à 23, **caractérisé en ce que** l'adénovirus comprend au moins une cassette d'expression, la cassette d'expression comprenant au moins un promoteur et un acide nucléique codant pour une protéine adénovirale, la protéine adénovirale étant une protéine E4orf6.

25. Adénovirus selon la revendication 24, **caractérisé en ce que** le promoteur est différent du promoteur E4.

26. Adénovirus selon la revendication 25, **caractérisé en ce que** le promoteur est choisi dans le groupe consistant en les promoteurs spécifiques de tumeur, les promoteurs spécifiques d'organe, les promoteurs spécifiques de tissu, les promoteurs hétérologues et les promoteurs adénoviraux, les promoteurs adénoviraux étant différents du promoteur E4.

27. Adénovirus selon l'une des revendications 21 à 26, **caractérisé en ce que** le promoteur est un promoteur E1A.

28. Adénovirus selon l'une des revendications 1 à 27, **caractérisé en ce que** l'adénovirus comprend au moins une cassette d'expression, la cassette d'expression comprenant au moins un promoteur et un acide nucléique codant pour une protéine adénovirale, la protéine adénovirale étant une protéine E1A, de préférence une protéine E1A12S.

29. Adénovirus selon la revendication 28, **caractérisé en ce que** le promoteur est différent du promoteur E1A.

30. Adénovirus selon la revendication 29, **caractérisé en ce que** le promoteur est choisi dans le groupe consistant en les promoteurs spécifiques de tumeur, les promoteurs spécifiques d'organe, les promoteurs spécifiques de tissu, les promoteurs hétérologues et les promoteurs adénoviraux.

31. Adénovirus selon l'une des revendications 1 à 30, **caractérisé en ce que** l'adénovirus comprend un acide nucléique, l'acide nucléique codant pour YB-1.

32. Adénovirus selon la revendication 31, **caractérisé en ce que** l'acide nucléique codant pour YB-1 est sous le contrôle d'un promoteur, le promoteur étant de préférence le promoteur tardif E2.

33. Adénovirus selon la revendication 31 ou 32, **caractérisé en ce que** l'acide nucléique codant pour YB-1 est sous le contrôle d'un promoteur, le promoteur étant dépendant de YB-1 ou étant régulé par YB-1.

34. Adénovirus selon l'une des revendications 28 à 33, **caractérisé en ce que** l'acide nucléique codant pour YB-1 est une partie de la cassette d'expression comprenant un acide nucléique codant pour une protéine E1A, et de préférence est un acide nucléique codant pour une protéine E1A 12S.

35. Adénovirus selon la revendication 34, **caractérisé en ce que** l'acide nucléique codant pour la protéine E1A est séparé par une séquence IRES de l'acide nucléique codant pour YB-1.

36. Adénovirus selon l'une des revendications 21 à 35, **caractérisé en ce que** l'acide nucléique codant pour la protéine E4orf6 et l'acide nucléique codant pour la protéine E1B55kD sont contenus dans une cassette d'expression, et les deux séquences codantes sont séparées l'une de l'autre par une séquence IRES.

37. Adénovirus selon la revendication 36, **caractérisé en ce que** le promoteur de la cassette d'expression est choisi dans le groupe consistant en les promoteurs spécifiques de tumeur, les promoteurs spécifiques d'organe, les promoteurs spécifiques de tissu, les promoteurs hétérologues et les promoteurs adénoviraux, les promoteurs adénoviraux étant différents du promoteur E4 et étant différents du promoteur E1B, de préférence différents du promoteur E4 de type sauvage et différents du promoteur E1B de type sauvage.

38. Adénovirus selon l'une des revendications 1 à 37, **caractérisé en ce que** l'adénovirus comprend une cassette d'expression comprenant un promoteur et une séquence d'acide nucléique, la séquence d'acide nucléique étant choisie dans le groupe consistant en les aptamères, les ribozymes, les aptazymes, les molécules antisens et les pARNi.

39. Adénovirus selon l'une des revendications 1 à 37, **caractérisé en ce que** l'adénovirus comprend une cassette d'expression comprenant un promoteur et une séquence d'acide nucléique, la séquence d'acide nucléique étant un acide nucléique codant, l'acide nucléique codant pour une molécule qui est choisie dans le groupe consistant en les peptides, les polypeptides, les protéines, les anticalines, les anticorps et les fragments d'anticorps.

40. Adénovirus selon l'une des revendications 1 à 37, **caractérisé en ce que** l'adénovirus comprend une cassette d'expression comprenant un promoteur et une séquence d'acide nucléique, la séquence d'acide nucléique étant choisie dans le groupe consistant en les gènes inducteurs d'apoptose, les gènes-prodrogues, les inhibiteurs de protéase, les gènes suppresseurs de tumeur, les cytokines et les inhibiteurs de l'angiogenèse.

41. Adénovirus selon l'une des revendications 1 à 40, **caractérisé en ce que** l'adénovirus peut se répliquer dans des cellules qui présentent une YB-1 dérégulée, l'YB-1 dérégulée étant telle que celle présente dans le cytoplasme de cellules tumorales sous une forme qui quantitativement et/ou qualitativement est différente de celle dans les cellules non tumorales.

42. Adénovirus selon l'une des revendications 1 à 41, **caractérisé en ce que** les cellules présentent dans le noyau une YB-1 indépendante du cycle cellulaire de l'YB-1.

43. Acide nucléique codant pour un adénovirus selon l'une des revendications 1 à 42.

44. Système de réplication comprenant un acide nucléique selon la revendication 43 et un acide nucléique d'un virus assistant, l'acide nucléique du virus assistant comprenant une ou plusieurs des cassettes d'expression de l'adénovirus selon l'une des revendications 1 à 42.

45. Système de réplication selon la revendication 44, **caractérisé en ce que** l'adénovirus ou l'acide nucléique codant pour ce dernier ne contient pas la cassette d'expression présente dans le virus assistant.

46. Vecteur comprenant un acide nucléique selon la revendication 43 et/ou un système de réplication selon l'une des revendications 44 à 45.

47. Vecteur selon la revendication 46, **caractérisé en ce que** le vecteur est un vecteur d'expression.

48. Cellule comprenant un adénovirus selon l'une des revendications 1 à 42 et/ou un acide nucléique selon la revendication 43 et/ou un système de réplication selon la revendication 44 ou 45 et/ou un vecteur selon la revendication 46 ou 47.

49. Cellule selon la revendication 48, **caractérisée en ce que** la cellule est une cellule eucaryote, de préférence une cellule animale, de préférence une cellule de mammifère.

50. Cellule selon la revendication 49, **caractérisée en ce que** la cellule de mammifère est une cellule choisie dans le groupe consistant en les cellules de souris, de rats, de cobayes, de porcs, de moutons, de chèvres, de bovins, de chevaux, de chiens, de chats et d'humains.

51. Organisme non humain, de préférence organisme mammifère comprenant un adénovirus selon l'une des revendications 1 à 42, un acide nucléique selon la revendication 43, un système de réplication selon la revendication 44 ou 45, un vecteur selon l'une des revendications 46 ou 47 ou une cellule selon l'une des revendications 48 à 50, l'organisme étant de préférence choisi dans le groupe consistant en les souris, les rats, les cobayes, les porcs, les moutons, les chèvres, les bovins, les chevaux, les chiens et les chats.

52. Utilisation d'un adénovirus selon l'une des revendications 1 à 42, d'un acide nucléique selon la revendication 43, d'un système de réplication selon la revendication 44 ou 45, d'un vecteur selon l'une des revendications 46 ou 47 ou d'une cellule selon l'une des revendications 48 à 50 pour la réplication in vitro d'un adénovirus.

53. Utilisation d'un adénovirus selon l'une des revendications 1 à 42, d'un acide nucléique selon la revendication 43, d'un système de réplication selon la revendication 44 ou 45, d'un vecteur selon l'une des revendications 46 ou 47 ou d'une cellule selon l'une des revendications 48 à 50 pour la production in vitro d'un adénovirus.

54. Utilisation d'un adénovirus selon l'une des revendications 1 à 42, d'un acide nucléique selon la revendication 43, d'un système de réplication selon la revendication 44 ou 45, d'un vecteur selon l'une des revendications 46 ou 47 ou d'une cellule selon l'une des revendications 48 à 50 pour la fabrication d'un médicament.

55. Utilisation selon l'une des revendications 53 à 54, **caractérisée en ce que** la cellule, dans laquelle se réplique l'adénovirus, comprend une YB-1 dans le noyau, de préférence la contient dans le noyau indépendamment du cycle cellulaire de l'YB-1.

56. Utilisation selon l'une des revendications 53 à 54, **caractérisée en ce que** la cellule, dans laquelle se réplique l'adénovirus, comprend une YB-1 dérégulée, l'YB-1 dérégulée étant telle qu'elle est présente dans le cytoplasme de cellules tumorales sous une forme qui quantitativement et/ou qualitativement est différente de celle dans les cellules non tumorales.

57. Utilisation selon la revendication 54, **caractérisée en ce que** le médicament est destiné au traitement de maladies tumorales.

58. Utilisation selon la revendication 57, **caractérisée en ce que** la maladie tumorale est choisie dans le groupe consistant en les maladies malignes, le cancer, les maladies cancéreuses et les tumeurs.

59. Utilisation selon la revendication 58, **caractérisée en ce que** les tumeurs sont choisies dans le groupe consistant en les tumeurs solides, non solides, malignes et bénignes.

60. Utilisation selon l'une des revendications 57 à 59, **caractérisée en ce qu'**au moins une partie des cellules constituant la tumeur comprend une YB-1 dans le noyau, de préférence la comprend dans le noyau indépendamment du cycle cellulaire de l'YB-1.

61. Utilisation selon l'une des revendications 57 à 60, **caractérisée en ce qu'**au moins une partie des cellules constituant la tumeur comprend une YB-1 dérégulée, l'YB-1 dérégulée étant telle que celle présente dans le cytoplasme de cellules tumorales sous une forme qui quantitativement et/ou qualitativement est différente de celle dans les cellules non tumorales.

62. Utilisation selon l'une des revendications 57 à 61, **caractérisée en ce qu'**au moins une partie des cellules constituant la tumeur est Rb-positive ou Rb-négative.

63. Utilisation selon l'une des revendications 57 à 61, **caractérisée en ce qu'**au moins une partie des cellules constituant la tumeur présente une résistance aux agents anti-tumoraux, une multi-résistance aux principes actifs pharmacologiques et/ou une résistance occasionnée par une irradiation.

64. Utilisation selon la revendication 63, **caractérisée en ce que** la résistance est dirigée contre les cytostatiques.

65. Utilisation selon l'une des revendications 57 à 64, **caractérisée en ce que** le patient auquel est destiné le médicament présente une majorité de cellules, les cellules étant les cellules telles que décrites dans l'une des revendications 60 à 64.

66. Utilisation selon l'une des revendications 57 à 65, **caractérisée en ce que** le médicament comprend au moins un agent pharmaceutiquement actif supplémentaire.

67. Utilisation selon l'une des revendications 57 à 65, **caractérisée en ce que** le médicament est administré ou doit être administré en même temps qu'un agent pharmaceutiquement actif supplémentaire.

68. Utilisation selon la revendication 66 ou 67, **caractérisée en ce que** l'agent pharmaceutique supplémentaire est choisi dans le groupe consistant en les cytokines, les inhibiteurs de métalloprotéinases, les inhibiteurs de l'angiogenèse, les cytostatiques, les inhibiteurs de la tyrosine-kinase, les inhibiteurs du cycle cellulaire, les inhibiteurs des protéosomes, les inhibiteurs de la cascade de transduction du signal, les protéine-kinases et les anticorps recombinants.

69. Utilisation selon l'une des revendications 57 à 65, **caractérisée en ce que** le médicament est administré avant, pendant ou après une irradiation.

70. Utilisation selon la revendication 69, **caractérisée en ce que** l'irradiation est effectuée dans le but de traiter une tumeur.

71. Utilisation selon l'une des revendications 57 à 70, **caractérisée en ce que** la cellule ou l'organisme à traiter est soumis à une mesure, la mesure étant choisie dans le groupe consistant en une irradiation, l'administration de cytostatiques et une hyperthermie.

72. Utilisation selon l'une des revendications 57 à 71, **caractérisée en ce que** la mesure est utilisée au niveau local ou systémique.

73. Utilisation selon l'une des revendications 69 à 72, **caractérisée en ce que** l'irradiation est réalisée avec un rayonnement de haute énergie, de préférence avec un rayonnement tel celui qui est utilisé lors du traitement des maladies tumorales.

74. Utilisation selon la revendication 57, **caractérisée en ce que** la maladie tumorale est choisie dans le groupe consistant en les tumeurs du sein, les tumeurs des os, les tumeurs de l'estomac, les tumeurs de l'intestin, les tumeurs de la vésicule biliaire, les tumeurs du pancréas, les tumeurs hépatiques, les tumeurs des reins, les tumeurs du cerveau, les tumeurs des ovaires, les tumeurs de la peau et des appendices de la peau, les tumeurs de la tête et du cou, les tumeurs de l'utérus, les tumeurs synoviales, les tumeurs du larynx, les tumeurs de l'oesophage, les tumeurs de la langue et les tumeurs de la prostate, de préférence l'une des maladies tumorales ci-dessus qui sont exprimées comme décrit dans l'une des revendications précédentes.

75. Utilisation selon la revendication 57, **caractérisée en ce que** le promoteur spécifique de tumeur est un promoteur qui est spécifique de la tumeur pour laquelle le médicament est utilisé.

76. Composition pharmaceutique comprenant un adénovirus selon l'une des revendications 1 à 42, un acide nucléique selon la revendication 43, un système de réplication selon la revendication 44 ou 45, un vecteur selon l'une des revendications 46 ou 47 ou une cellule selon l'une des revendications 48 à 50, et en option un support pharmaceutiquement approprié.

77. Utilisation selon l'une des revendications 54 à 75, **caractérisée en ce que** le médicament comprend une combinaison d'au moins deux agents, chaque agent étant, à titre individuel et d'une manière indépendante, choisi dans le groupe consistant en les cytostatiques.

78. Utilisation selon la revendication 77, **caractérisée en ce qu'**au moins deux des agents attaquent différentes molécules cibles.

79. Utilisation selon la revendication 78, **caractérisée en ce qu'**au moins deux des agents sont actifs par des mécanismes d'action différents.

80. Utilisation selon l'une des revendications 77 à 79, **caractérisée en ce qu'**au moins un agent augmente l'infectiosité d'une cellule dans laquelle se réplique le virus.

81. Utilisation selon l'une des revendications 77 à 80, **caractérisée en ce qu'**au moins un agent influe sur la disponibilité d'un composant de la cellule, le composant assurant l'absorption du virus.

82. Utilisation selon l'une des revendications 77 à 81, **caractérisée en ce qu'**au moins un agent assure, de préférence augmente le transport de l'YB-1 dans le noyau cellulaire.

83. Utilisation selon l'une des revendications 77 à 82, **caractérisée en ce qu'**au moins un agent est un inhibiteur de l'histone-désacylase.

84. Utilisation selon la revendication 83, **caractérisée en ce que** l'inhibiteur de l'histone-désacylase est choisi dans le groupe consistant en la trikostatine A, le FR 901228, le MS-27-275, le NVP-LAQ824, le PXD101, l'apicidine et le scriptaid.

85. Utilisation selon l'une des revendications 77 à 83, **caractérisée en ce qu'**au moins un agent est choisi dans le groupe consistant en la trikostatine A, le FR 901228, le MS-27-275, le NVP-LAQ824, le PXD101, l'apicidine et le scriptaid.

86. Utilisation selon l'une des revendications 77 à 85, **caractérisée en ce qu'**au moins un agent est un inhibiteur de la topoisomérase.

87. Utilisation selon la revendication 86, **caractérisée en ce que** l'inhibiteur de la topoisomérase est choisi dans le groupe consistant en la camptothécine, le Sn-38, le topotécan, le DX-895If, l'irinotécan, la 9-aminocomptothécine, la 9-nitrocamptothécine, l'étoposide et la daunorubicine.

88. Utilisation selon l'une des revendications 84 à 87, **caractérisée en ce que** l'agent consiste en la tricostatine A et l'irinotécan.

89. Utilisation selon la revendication 87, **caractérisée en ce qu'**au moins une dose unitaire du virus est séparée d'au moins une dose unitaire d'un ou des au moins deux agents.

90. Trousse comprenant un virus selon l'une des revendications 1 à 42, et au moins deux agents, chaque agent étant individuellement et d'une manière indépendante choisi dans le groupe consistant en les cytostatiques.
